(19) Europäisches Patentamt
European Patent Office
Office européen des brevets

(11) EP 1 820 799 A1

(12) **EUROPEAN PATENT APPLICATION**
published in accordance with Art. 158(3) EPC

(43) Date of publication:
**22.08.2007 Bulletin 2007/34**

(21) Application number: **05806052.6**

(22) Date of filing: **10.11.2005**

(51) Int Cl.:
*C07D 243/12* (2006.01) *A61K 31/55* (2006.01)
*A61P 3/10* (2006.01) *A61P 13/12* (2006.01)
*C07D 401/14* (2006.01) *C07D 403/10* (2006.01)
*C07D 403/14* (2006.01) *C07D 413/14* (2006.01)
*C07D 491/113* (2006.01)

(86) International application number:
**PCT/JP2005/020599**

(87) International publication number:
**WO 2006/051851 (18.05.2006 Gazette 2006/20)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.11.2004 JP 2004325791**
**30.03.2005 JP 2005099388**

(71) Applicant: **Wakamoto Pharmaceutical Co., Ltd.**
**Chuo-ku,**
**Tokyo 103-8330 (JP)**

(72) Inventors:
- **OHTAKE, Y.,**
**c/o WAKAMOTO PHARMACEUTICAL CO., LTD.**
**Tokyo 103-8330 (JP)**
- **FUKAYA, Y.,**
**c/o WAKAMOTO PHARMACEUTICAL CO., LTD.**
**Tokyo 103-8330 (JP)**
- **MIHARA, Y.,**
**c/o WAKAMOTO PHARMACEUTICAL CO., LTD.**
**Tokyo 103-8330 (JP)**
- **OISHI, T.,**
**c/o WAKAMOTO PHARMACEUTICAL CO., LTD.**
**Tokyo 103-8330 (JP)**
- **TAKASHIMA, Y.,**
**c/o WAKAMOTO PHARMACEUTICAL CO. LTD**
**Tokyo 103-8330 (JP)**
- **SHOJI, T.,**
**c/o WAKAMOTO PHARMACEUTICAL CO., LTD.**
**Tokyo 103-8330 (JP)**
- **MORIZONO, D.,**
**c/o WAKAMOTO PHARMACEUTICAL CO., LTD**
**Tokyo 103-8330 (JP)**

(74) Representative: **Hart Davis, Jason et al**
**Cabinet Beau de Lomenie**
**158 rue de l'Université**
**F-75340 Paris Cedex 07 (FR)**

(54) **2,3,4,5-TETRAHYDRO-1H-1,5-BENZODIAZEPINE DERIVATIVE AND MEDICINAL COMPOSITION**

(57) The present invention has its object to provide a 2,3,4,5-tetrahydro-1H-1,5-benzodiazepine derivative represented with the Formula (1)

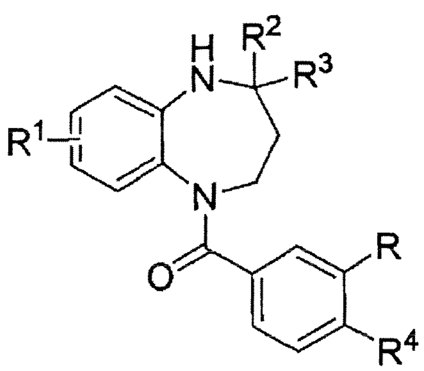

(1)

EP 1 820 799 A1

, or the pharmaceutically acceptable salt, which is effective as a therapeutic and prophylactic agent for diabetes, diabetic nephropathy, or glomerulosclerosis.

## Description

### TECHNICAL FIELD

The present invention relates to a new 2,3,4,5-tetrahydro-1H-1,5-benzodiazepine derivative having vasopressin receptor antagonism that is expected of as a therapeutic and prophylactic agent for diabetes, diabetic nephropathy, or glomerulosclerosis, and its pharmaceutically acceptable salt, and pharmaceutical composition.

### BACKGROUND ART

Arginine vasopressin (AVP) is a peptide hormone consisting of 9 amino acids produced and secreted in a hypothalamus-pituitary system. An AVP receptor is categorized into three sub-types of V1a, V1b, and V2. The V1a receptor is broadly distributed in a vascular smooth muscle, etc., and the AVP is known to show vasoconstrictor action, etc. mediated by the V1a receptor. The V2 receptor is distributed in distal renal tubules, collecting tubules, etc. of a kidney, and the AVP is known to show antidiuretic action mediated by the V2 receptor. Further, the V1b receptor distributed in the central anterior pituitary is known to be involved in hormone secretion.

The excessive secretion of the AVP is known to be involved in the induction and the progress of various morbid states.

For example, selective antagonists for V2 OPC-31260 and VP-343 improved the condition in which hyponatremia is a main morbid state in a rat model of the syndrome of inappropriate secretion of ADH (SIADH) (for example, refer to Non-Patent Documents 1 and 2). An inhibitor of both V1a and V2 receptors YM087 improved the condition in rat and dog models of cardiac failure (for example, refer to Non-Patent Documents 3 and 4) .

An increase of the vasopressin amount in blood is observed in a diabetic patient and a rat model of diabetes, and it has been reported that, in type II diabetic mouse (db/db mouse), a selective antagonist for vasopressin V1a FR218944 suppresses the increase of neutral fat in blood (for example, refer to Non-Patent Document 5).

Bardoux, et al. reported that when diabetes is induced by administrating streptozotocin (STZ) to a rat with vasopressin deficiency (Brattleboro rat), diabetic nephropathy decreases compared to a normal rat with diabetes induced by STZ (refer to Non-Patent Document 6).

Tahara, et al. have disclosed that a selective antagonist for V1a improves nephropathy in a rat model of STZ induced diabetic nephropathy (refer to Patent Document 1).

It has been reported that OPC-21268 decreases albumin discharge into urine of a patient with noninsulin dependent diabetic nephropathy (refer to Non-Patent Document 7).

It has been reported that the administration of a vasopressin V2 agonist dDAVP increases albumin discharge into urine of a healthy person and rat (refer to Non-Patent Document 8).

It has been reported that a selective antagonist for V2 SR121463 improves nephropathy in a model of STZ induced diabetic nephropathy (refer to Non-Patent Document 9).

OPC-21268 improves blood pressure, serum neutral fat, creatinine, urea nitrogen, and creatinine clearance, and histopathologically suppresses hardening of glomerulus in glomerulosclerosis in a rat model in which one of the kidneys is extracted and with high cholesterol (refer to Non-Patent Document 10).

The possibility of a vasopressin receptor antagonist being an effective therapeutic and prophylactic agent for diabetes, diabetic nephropathy, and glomerulosclerosis disease has been reported as described above. A compound having strong vasopressin receptor antagonism, long pharmacodynamic action persistence, and high safety is expected to the therapeutic and prophylactic agent for these diseases.

On the other hand, a nitrogen-containing aromatic five-membered ring-condensed benzazepine represented with Formula (A) (refer to Patent Document 2) and an N-sulfonylindoline derivative represented with Formula (B) (refer to Patent Document 3) have been disclosed as a non-peptidic compound having affinity for the vasopressin receptor.

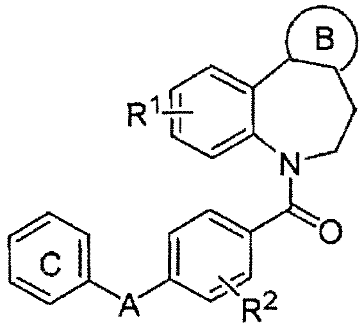

(A)

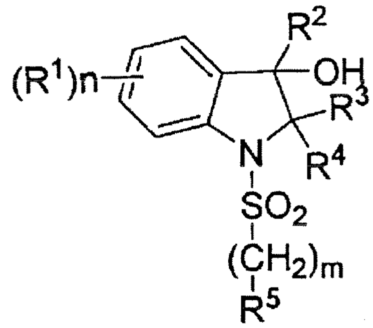

(B)

(The definition of the symbols in the Formulas refers to those in the document.)

Benzoheterocyclic derivatives represented with Formulas (C) (refer to Patent Document 4), (D) (refer to Patent Document 5), and (E) (refer to Patent Document 6) have been disclosed as compounds highly related with Formula (1) in the present invention.

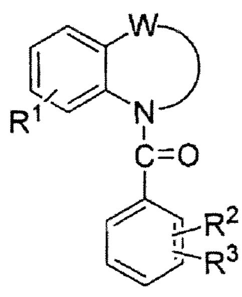

(C)

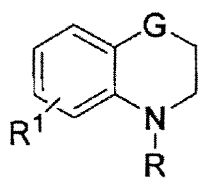

(D)

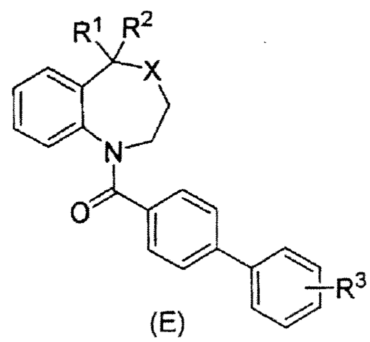

(E)

(The definition of the symbols in the Formulas refers to those in the document.)

Various compounds having various vasopressin receptor antagonisms have been discovered as described above. However, these compounds do not show strong vasopressin receptor antagonism, long pharmacodynamic action persistence, and high safety.

Patent Document 1: International Publication 02/02553
Patent Document 2: International Publication 95/03305
Patent Document 3: Japanese Kokai Publication Hei-4-234361
Patent Document 4: Japanese Kokai Publication Hei-4-154765
Patent Document 5: Japanese Kokai Publication Hei-8-301848
Patent Document 6: Japanese Kokai Publication Hei-6-16643
Non-Patent Document 1: Kidney International, 1993, vol. 44(1), 19-23.
Non-Patent Document 2: Biological and Pharmaceutical Bulletin, 2000, vol. 23(11), 1323-1327.
Non-Patent Document 3: European Journal of Pharmacology, 2002, vol. 450, 169-177.
Non-Patent Document 4: European Journal of Pharmacology, 1999, vol. 376, 239-246.
Non-Patent Document 5: Drug Development Research, 2003, vol. 60, 241-251.
Non-Patent Document 6: Proceeding of the National Academy of Sciences of the United State of America, 1999, vol. 96, 10397-10402.
Non-Patent Document 7: Journal of Diabetes and Its Complications, 1995, vol. 9, 326-329.
Non-Patent Document 8: Nephrology Dialysis Transplantation, 2003, vol. 18, 497-506.
Non-Patent Document 9: Nephrology Dialysis Transplantation, 2003, vol. 18, 1755-1763.
Non-Patent Document 10: Nephron, 1996, vol. 73, 629-636

SUMMARY OF THE INVENTION

The present invention provides a new 2,3,4,5-tetrahydro-1H-1,5-benzodiazepine derivative having strong vasopressin receptor antagonism, long pharmacodynamic action persistence, and high safety that is expected to a therapeutic and prophylactic agent for diabetes, diabetic nephropathy, and glomerulosclerosis, and pharmaceutical composition.

The inventors of the present invention found as the result of devoted study that the new 2,3,4,5-tetrahydro-1H-1,5-benzodiazepine derivative having the structural characteristics indicated in 1 to 3 below has strong vasopressin receptor antagonism, long pharmacodynamic action persistence, and high safety, and came to complete the present invention.

1. a nitrogen in position 5 of a 1, 5-benzodiazepine ring is unsubstituted,
2. the derivative has a substituent in position 4 of the 1,5-benzodiazepine ring, and
3. a substituent in position 3 of a benzoyl that is in position 1 of the 1,5-benzodiazepine ring and a benzene ring or an indole ring in position 4 of the benzoyl.

That is, the present invention is
a 2,3,4,5-tetrahydro-1H-1,5-benzodiazepine derivative represented with

(1) the following Formula (1)

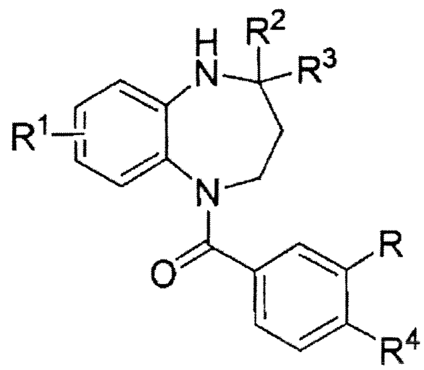

(1)

[In the Formula, $R^1$ represents hydrogen, lower alkyl, amino, or hydroxy. $R^2$ and $R^3$ are the same or different from each other representing hydrogen, lower alkyl, di-lower alkylamino lower alkyl, or hydroxy lower alkyl. However, the case that both of $R^2$ and $R^3$ are hydrogen is excluded. When $R^2$ and $R^3$ are different, they represent an optically-active substance or a racemic body. $R^4$ is a group represented with

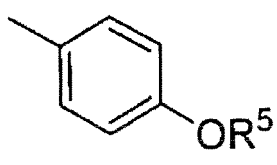

($R^5$ represents hydrogen, lower alkyl, or halogen-substituted lower alkyl.), a group represented with

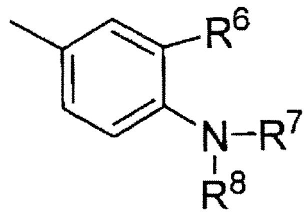

($R^6$ represents hydrogen, lower alkyl, lower alkoxy, halogen, or di-lower alkylamino. $R^7$ and $R^8$ are the same or different from each other representing hydrogen, lower alkyl, or lower alkanoyl.), or 1H-indol-5-yl. R represents hydrogen, hydroxy, morpholino, lower alkylpiperazinyl, lower alkoxy, amino lower alkyl, hydroxy lower alkyl, morpholino lower alkyl, di-lower alkylamino lower alkylcarbonyl, morpholinocarbonyl, lower alkylpiperazinylcarbonyl, piperazinyl lower alkoxy, a group represented with

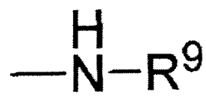

($R^9$ represents hydrogen, lower alkyl, amino lower alkyl, lower alkylpiperazinylcarbonyl lower alkyl, or lower alkylsulfonyl.), a group represented with

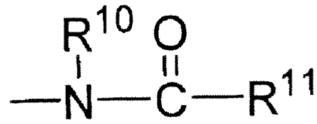

($R^{10}$ represents hydrogen, or lower alkyl. $R^{11}$ represents hydrogen, lower alkyl, lower alkylpiperazinyl lower alkyl, lower alkoxy, amino lower alkyl, amino, di-lower alkylamino, hydroxy lower alkyl, lower alkoxy lower alkyl, or morpholino.), a group represented with

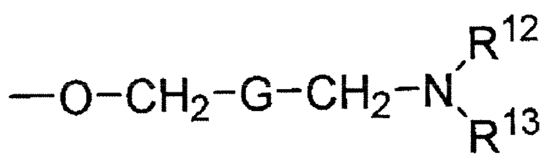

($R^{12}$ and $R^{13}$ are the same or different from each other representing hydrogen or lower alkyl. G represents -CH=CH- or -C≡C-.), a group represented with

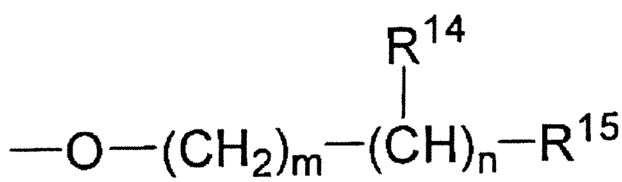

($R^{14}$ represents lower alkyl. $R^{15}$ represents hydroxy, lower alkoxy, imidazolyl, 2,5-dioxoimidazolidin-1-yl, 3-methyl-2,4-dioxoimidazolidin-1-yl, 2,4-dioxothiazolidin-3-yl, 4-oxopiperizin-1-yl,2-oxopyrrolidin-1-yl, 2-oxooxazolidin-3-yl, 3-oxopiperazin-1-yl, or (1,4-dioxa-8-azaspiro[4,5]dec-8-yl,

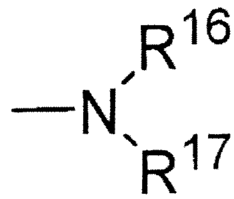

($R^{16}$ and $R^{17}$ are the same or different from each other representing hydrogen, lower alkyl, hydroxy lower alkyl, lower alkoxy lower alkyl, lower alkanoyl, amino lower alkanoyl, lower alkoxy lower alkylcarbonyl, or di-lower alkylamino lower alkyl. Further, $R^{16}$ and $R^{17}$ may form a 6- to 7-membered saturated heterocycle by bonding each other through a nitrogen atom or an oxygen atom and accompanying a nitrogen atom to which $R^{16}$ and $R^{17}$ are bonding. It may have lower alkyl or lower alkanoyl on the heterocycle as a substituent.), or a group represented with

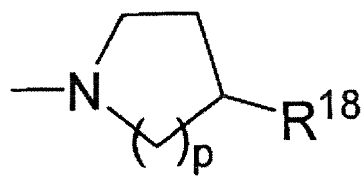

(p represents 1 or 2. $R^{18}$ represents hydrogen, di-lower alkylamino, amino, aminocarbonyl, di-lower alkylaminocarbonyl, formamido, hydroxy, or morpholino.). m represents an integer of 2 to 6, and n represents an integer of 0 to 3.}, or a group represented with

$$-CO_2-(CH_2)_m-R^{19}$$

($R^{19}$ represents di-lower alkylamino, morpholino, lower alkylpiperazinyl, lower alkylpiperizino, pyridyl lower alkyl, or lower alkylpyrrolidinyl. m is the same as described above.)] , and the pharmaceutically acceptable salt,
(2) the compound described above (1) in which $R^4$ is represented with

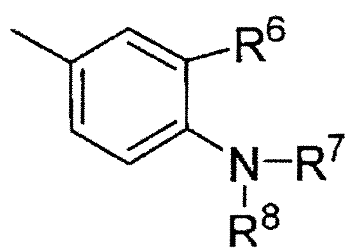

($R^6$, $R^7$, and $R^8$ are the same as described above.), and the pharmaceutically acceptable salt,
(3) the compound described above (1) in which $R^4$ is represented with 1H-indol-5-yl, and the pharmaceutically acceptable salt,
(4) the compound described above (2) in which R is represented with

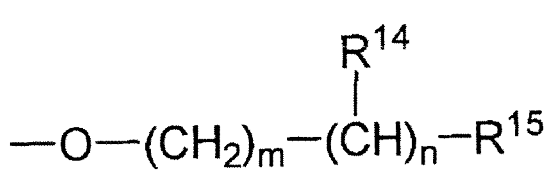

($R^{14}$, $R^{15}$, m, and n are the same as described above.), and the pharmaceutically acceptable salt,
(5) the compound described above (3) in which R is represented with

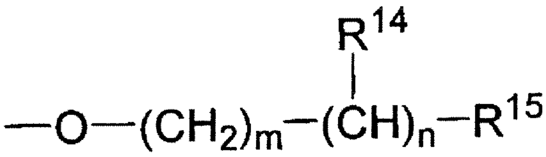

($R^{14}$, $R^{15}$, m, and n are the same as described above.), and the pharmaceutically acceptable salt,
(6) the compound described above (1) selected from the group consisting of N-[3-[2-(1H-indol-5-yl)-5-[[(4S)-4-methyl-2,3,4,5-tetrahydro-2H-2,5-benzodiazepin-1-yl]carbonyl]phenoxy] propyl]-N-methyl-N-propylamine; (4S)-1-[4-(1H-indol-5-yl)-3-[3-(4-isopropyl-1-piperazinyl)p ropoxy]benzoyl]-4-methyl-2,3, 4,5-tetrahydro-1H-1,5-benzodiazepine; (4S)-1-[4-(1H-indol-5-yl)-3-[3-(4-propyl-1-piperazinyl) propoxy]benzoyl]-4-methyl-2,3,4,5-tetrahydro-1H-1,5-benzodiazepine; N-methyl-2'-[3-[methyl(propyl)amino]propoxy]-4'-[[(4S)-4-methyl-2,3,4,5-tetrahydro-1H-1,5-benzodiazepin-1-yl]carbonyl][1,1'-biphenyl]-4-amine; (4S)-1-[4-(1H-indol-5-yl)-3-[2-(4-methyl-1-piperazinyl) ethoxy]benzoyl]-4-methyl-2,3,4,5-tetrahydro-1H-1,5-benzodiazepine; N-[4-[2-(1H-indol-5-yl)-5-[[(4S)-4-methyl-2,3,4,5-tetrahydro-1H-1,5-benzodiazepin-1-yl]carbonyl]phenoxy] butyl]-N-methyl-N-propylamine; (4S)-2-[3-[3-(4-butyl-1-piperazinyl)propoxy)-4-(1H-indol-5-yl)benzoyl]-4-methyl-2,3,4,5-tetrahydro-1H-1,5-benzodiazepine; and N-ethyl-2'-[3-[methyl(propyl)amino]propoxy]-4'-[[(4S)-4-methyl-2,3,4,5-tetrahydro-1H-1,5-benzodiazepin-1-yl]carbonyl][1,1'-biphenyl]-4-amine, and the pharmaceutically acceptable salt,
(7) the compound described above (1) which is N-[4-[2-(1H-indol-5-yl)-5-[[(4S)-4-methyl-2,3,4,5-tetrahydro-1H-1,5-benzodiazepin-1-yl]carbonyl]phenoxy] butyl]-N-methyl-N-propylamine·1/2 fumarate,
(8) the compound described above (1) which is (4S)-1-[4-(1H-indol-5-yl)-3-[2-(4-methyl-1-piperazinyl) ethoxy]benzoyl]-4-methyl-2,3,4,5-tetrahydro-1H-1,5-benzodiazepine-1/2 fumarate,
(9) the compound described above (1) which is (4S)-1-[3-[3-(4-butyl-1-piperazinyl)propoxy]-4-(1H-indol-5-yl)benzoyl]-4-methyl-2,3,4,5-tetrahydro-1H-1,5-benzodiazepine.1/2 fumarate,
(10) a pharmaceutical composition containing the compound in any of (1) to (9) or the pharmaceutically acceptable salt,
(11) a therapeutic and prophylactic agent for diabetes, diabetic nephropathy, and glomerulosclerosis having the compound described above described above in any of (1) to (9), or the pharmaceutically acceptable salt as an effective component.

DETAILED DESCRIPTION OF THE INVENTION

Each group shown in the above-described Formula (1) is more specifically as follows.

Examples of "lower alkyl" may be straight-chain or branched alkyls having 1 to 6 carbon atoms, such as methyl, ethyl, propyl, isopropyl, butyl, isobutyl, tert-butyl, pentyl, and hexyl, for instance.

Examples of "lower alkylpiperazinyl" may be piperazinyls in which a straight-chain or branched alkyl having 1 to 6 carbon atoms is substituted, such as 4-methylpiperazinyl, 4-ethylpiperazinyl, 4-propylpiperazinyl, 4-isopropylpiperazinyl, 4-butylpiperazinyl, 4-isobutylpiperazinyl, 4-tert-butylpiperazinyl, 4-pentylpiperazinyl, and 4-hexylpiperazinyl, for instance.

Examples of "lower alkoxy" may be straight-chain or branched alkoxys having 1 to 6 carbon atoms, such as methoxy, ethoxy, propoxy, isopropoxy, butoxy, isobutoxy, tert-butoxy, pentyloxy, isopentyloxy, tert-pentyloxy, neopentyloxy, 2-methylbutoxy, 1,2-dimethylpropoxy, 1-ethylpropoxy, and hexyloxy, for instance.

Examples of "amino lower alkyl" may be straight-chain or branched alkyls having 1 to 6 carbon atoms and amino-substituted, such as aminomethyl, 2-aminoethyl, 3-aminopropyl, 2-aminopropyl, 3-amino-2-methypropyl, 4-aminobutyl, 3-aminoisobutyl, 5-aminopentyl, and 6-aminohexyl, for instance.

Examples of "hydroxy lower alkyl" may be straight-chain or branched alkyls having 1 to 6 carbon atoms and hydroxy-substituted, such as hydroxymethyl, 2-hydroxyethyl, 3-hydroxypropyl, 2-hydroxypropyl, 3-hydroxy-2-methylpropyl, 4-hydroxybutyl, 5-hydroxypentyl, and 6-hydroxyhexyl, for instance.

Examples of "morpholino lower alkyl" may be straight-chain or branched alkyls having 1 to 6 carbon atoms and morpholino-substituted, such as morpholinomethyl, 2-morpholinoethyl, 3-morpholinopropyl, 2-morpholinopropyl, 3-morpholino-2-methylpropyl 4-morpholinobutyl, 3-morpholinobutyl, 5-morpholinopentyl, and 6-morpholinohexyl, for instance.

Examples of "di-lower alkylamino lower alkyl" may be straight-chain or branched alkyls having 1 to 6 carbon

atoms and having, as a substituent, an amino having two of straight-chain or branched alkyls having 1 to 6 carbon atoms, such as dimethylaminomethyl, diethylaminomethyl, dipropylaminomethyl, 2-(dimethylamino)ethyl, 2-(diethylamino)ethyl, 2-(dipropylamino)ethyl, 3-(dimethylamino)propyl, 3-(diethylamino)propyl, 3-(dipropylamino)propyl, ethylmethylaminomethyl, methylpropylaminomethyl, 2-(ethylmethylamino)ethyl, 2-(methylpropylamino)ethyl, 3-(ethylmethylamino)propyl, 3-(methylpropylamino)propyl, and 2-(methylpropylamino)propyl, for instance.

Examples of "di-lower alkylamino lower alkylcarbonyl" may be straight-chain or branched alkyl carbonyls having 1 to 6 carbon atoms and having, as a substituent, an amino having two of straight-chain or branched alkyls having 1 to 6 carbon atoms, such as dimethylaminomethylcarbonyl, diethylaminomethylcarbonyl, dipropylaminomethylcarbonyl, 2-(dimethylamino) ethylcarbonyl, 2- (diethylamino) ethylcarbonyl, 2- (dipropylamino) ethylcarbonyl, 3- (dimethylamino) propylcarbonyl, 3- (diethylamino)propylcarbonyl, 3-(dipropylamino)propylcarbonyl, ethylmethylaminomethylcarbonyl, methylpropylaminomethylcarbonyl, 2- (ethylmethylamino) ethylcarbonyl, 2-(methylpropylamino)ethylcarbonyl, 3-(ethylmethylamino)propylcarbonyl, 3-(methylpropylamino)propylcarbonyl, and 2-(methylpropylamino)propylcarbonyl, for instance.

Examples of "lower alkylpiperazinylcarbonyl" may be piperazinylcarbonyls in which a straight-chain or branched alkyl having 1 to 6 carbon atoms is substituted, such as (4-methyl-1-piperazinyl) carbonyl, (4-ethyl-1-piperazinyl)carbonyl, (4-propyl-1-piperazinyl)carbonyl, (4-isopropyl-1-piperazinyl) carbonyl, (4-butyl-1-piperazinyl) carbonyl, (4-isobutyl-1-piperazinyl) carbonyl, (4-tert-butyl-1-piperazinyl) carbonyl, (4-pentyl-1-piperazinyl)carbonyl, and (4-hexyl-1-piperazinyl) carbonyl, for instance.

Examples of "piperazinyl lower alkoxy" may be straight-chain or branched alkyls having 1 to 6 carbon atoms and piperazinyl-substituted, such as 1-piperazinylmethoxy, 2-(1-piperazinyl)ethoxy, 3-(1-piperazinyl)propoxy, 4-(1-piperazinyl)butoxy, 5-(1-piperazinyl)pentyloxy, 6-(1-piperazinyl)hexyloxy, and 3-(1-piperazinyl)butoxy, for instance.

Examples of "lower alkylpiperazinylcarbonyl lower alkyl" may be straight-chain or branched alkyls having 1 to 6 carbon atoms and having a piperazinylcarbonyl in which a straight-chain or branched alkyl having 1 to 6 carbon atoms is substituted, such as (4-methyl-1-piperazinyl)carbonylmethyl, (4-ethyl-1-piperazinyl)carbonylmethyl, (4-prapyl-1-piperazinyl)carbonylmethyl, (4-isopropyl-1-piperazinyl)carbonylmethyl, (4-butyl-1-piperazinyl)carbonylmethyl, (4-isobutyl-1-piperazinyl)carbonylmethyl, (4-tert-butyl-1-piperazinyl)carbonylmethyl, (4-pentyl-1-piperazinyl)carbonylmethyl, (4-hexyl-1-piperazinyl)carbonylmethyl, 2-[(4-methyl-1-piperazinyl)carbonyl]ethyl, 2-[(4-ethyl-1-piperazinyl)carbonyl]ethyl, 3- [(4-methyl-1-piperazinyl)carbonyl]propyl, 3-[(4-ethyl-1-piperazinyl)carbonyl]propyl, and 4-[(4-isopropyl-1-piperazinyl) carbonyl]butyl,for instance.

Examples of "lower alkylsulfonyl" may be straight-chain or branched alkylsulfonyls having 1 to 6 carbon atoms, such as methylsulfonyl, ethylsulfonyl, propylsulfonyl, isopropylsulfonyl, butylsulfonyl, tert-butylsulfonyl, pentylsulfonyl, and hexylsulfonyl, for instance.

Examples of "lower alkylpiperazinyl lower alkyl" may be straight-chain or branched alkyls having 1 to 6 carbon atoms and having piperazinyls in which a straight-chain or branched alkyl having 1 to 6 carbon atoms is substituted, such as (4-methyl-1-piperazinyl)methyl, (4-ethyl-1-piperazinyl)methyl, (4-propyl-1-piperazinyl)methyl, (4-isopropyl-2-piperazinyl)methyl, (4-butyl-1-piperazinyl)methyl, (4-isobutyl-1-piperazinyl)methyl, (4-tert-butyl-1-piperazinyl)methyl, (4-pentyl-1-piperazinyl)methyl, (4-hexyl-l-piperazinyl)methyl, 2-(4-methyl-1-piperazinyl)ethyl, 2-(4-ethyl-1-piperazinyl) ethyl, 3-(4-methyl-1-piperazinyl)propyl, 3-(4-ethyl-1-piperazinyl)propyl, and 4-(4-isopropyl-1-piperazinyl)-3-methylbutyl, for instance.

Examples of "di-lower alkylamino" may be aminos having two of straight-chain or branched alkyls having 1 to 6 carbon atoms, such as dimethylamino, diethylamino, dipropylamino, ethylmethylamino, and methylpropylamino, for instance.

Examples of "lower alkoxy lower alkyl" may be straight-chain or branched alkyls having 1 to 6 carbon atoms and having a straight-chain or branched alkoxy having 1 to 6 carbon atoms, such as methoxymethyl, 2-methoxyethyl, 3-methoxypropyl, 4-ethoxybutyl, 6-propoxyhexyl, 5-isopropoxypentyl, and 1,1-dimethyl-2-butoxyethyl, for instance.

Examples of "lower alkoxy lower alkylcarbonyl" may be straight-chain or branched alkylcarbonyls having 1 to 6 carbon atoms and having a straight-chain or branched alkoxy having 1 to 6 carbon atoms, such as methoxymethylcarbonyl, 2-methoxyethylcarbonyl, 3-methoxypropylcarbonyl, 4-ethoxybutylcarbonyl, 6-propoxyhexylcarbonyl, 5-isopropoxypentylcarbonyl, and 1,1-dimethyl-2-butoxyethylcarbonyl, for instance.

Examples of "lower alkanoyl" may be straight-chain or branched alkanoyls having 1 to 6 carbon atoms, such as formyl, acetyl, propionyl, butyryl, isobutyryl, pantanoyl, tert-butylcarbonyl, and hexanoyl, for instance.

Examples of "amino lower alkanoyl" may be straight-chain or branched alkanoyls having 1 to 6 carbon atoms and having an amino, such as 2-aminoacetyl, 3-aminopropionyl, 2-aminopropionyl, 4-aminobutyryl, 5-aminopentanoyl, and 6-aminohexanoyl, for instance.

Examples of "lower alkoxycarbonyl lower alkylcarbonyl" may be straight-chain or branched alkylcarbonyls having 1 to 6 carbon atoms and having a straight-chain or branched alkoxycarbonyl having 1 to 6 carbon atoms, such as methoxycarbonylmethylcarbonyl, ethoxycarbonylmethylcarbonyl, propoxycarbonylmethylcarbonyl, isopropoxycarbonylmethylcarbonyl, butoxycarbonylmethylcarbonyl, isobutoxycarbonylmethylcarbonyl, sec-butoxycarbonylmethylcarbonyl, tert-butoxycarbonylmethylcarbonyl, pentyloxycarbonylmethylcarbonyl, isopentyoxycarbonylmethylcarbonyl, neopentyloxycarbonylmethylcarbonyl, tert-yloxycarbonylmethylcarbonyl, hexyloxycarbonylmethycarbonyl, 2-(methoxycarbonyl)ethylcarbonyl, 2-(ethoxycarbonyl)ethycarbonyl, 3-(methoxycarbonyl)propylcarbonyl, 3-(ethoxycarbonyl)propylcarbonyl, and 4-(methoxycarbonyl)butylcarbonyl, for instance.

Examples of "lower alkylpiperizino" may be piperizinos in which a straight-chain or branched alkyl having 1 to 6 carbon atoms is substituted, such as 4-methylpiperizino, 4-ethylpiperizino, 4-propylpiperizino, 4-isopropylpiperizino, 4-butylpiperizino, 4-isobutylpiperizino, 4-tert-butylpiperizino, 4-pentylpiperlzino, and 4-hexylpiperizino, for instance.

Examples of "lower alkylpyrrolidinyl" may be pyrrolidinyls in which a straight or branched alkyl having 1 to 6 carbon atoms is substituted, such as 1-methylpyrrolidinyl, 2-methylpyrrolidiny, 3-methylpyrrolidinyl, 1-ethylpyrrolidinyl, 2-ethylpyrrolidinyl, 1-propylpyrrolidinyl, 1-isopropylpyrrolidinyl, 1-butylpyrrolidinyl, 1-isobutylpyrrolodinyl, 1-tert-butylpyrrolidinyl, 1-pentylpyrrolodinyl, and 1-hexylpyrrolidinyl, for instance.

Examples of "di-lower alkylaminocarbonyl" may be aminocarbonyls having two of straight or branched alkyls having 1 to 6 carbon atoms, such as dimethylaminocarbonyl, diethylaminocarbonyl, dipropylaminocarbonyl, ethylmethylaminocarbonyl, and methylpropylaminocarbonyl, for instance.

Examples of "pyridyl lower alkyl" may be pyridylalkyls in which an alkyl part is a straight or branched alkyl having 1 to 6 carbon atoms, such as (4-pyridyl)methyl, 1- (3-pyridyl) ethyl, 2- (2-pyridyl) ethyl, 3- (2-pyridyl) propyl, 4-(3-pyridyl) butyl, 5- (4-pyridyl) pentyl, 6-(2-pyridyl)hexyl, 1, 1-dimethyl-2-(3-pyridyl) ethyl, and 2-methyl-3-(4-pyridyl) propyl, for instance.

Examples of "halogen-substituted lower alkyl" may be straight-chain or branched alkyls having 1 to 6 carbon atoms and having to 3 halogen atoms, such as trifluoromethyl, trichloromethyl, chloromethyl, bromomethyl, fluoromethyl, iodomethyl, difluoromethyl, dibromomethyl, 2-chloroethyl, 2,2,2-trifluoroethyl, 2,2,2-trichloroethyl, 3-chloropropyl, 2,3-dichloropropyl, 4,4,4-trichlorobutyl, 5-chlcropentyl, and 6-bromohexyl, for instance.

Examples of "a 6- to 7-membered saturated heterocycle formed by $R^{16}$ and $R^{17}$ which are bonding each other through a nitrogen atom, an oxygen atom or a sulfur atom and accompanying a nitrogen atom to which $R^{16}$ and $R^{17}$ are bonding" include piperazinyl, morpholino, thiomorpholino, and perhydro-1,4-diazepine, for instance.

Examples of "the above-described heterocycle in which a group selected from lower alkyl or lower alkanoyl is substituted" may be the above-described heterocycle in which 1 to 3 groups selected from the group consisting of straight or branched alkyls having 1 to 6 carbon atoms and straight or branched alkanoyls having 1 to 6 carbon atoms are substituted, such as 4-methylpiperazinyl, 4-propylpiperazine, 4-isopropylpiperazinyl, 4-butyl-3,5-dimethylpiperazinyl, 3,5-dimethylpiperazinyl, 3,5-dimethylmorpholino, 4-isobutylpiperazinyl, 4-acetylpiperazinyl, and 4-butylylpiperazinyl, for instance.

"Halogen" means a monovalent group of a halogen atom, and specific examples include fluorine, chlorine, bromine, and iodine, for instance.

There is a case that the compound in the present invention represented in Formula (1) contains an asymmetric carbon depending on the type of the substituent, and an optical isomer could exist based on this. The present invention includes all mixtures and separated substances of these optical isomers. Further, there is a case that a tautomer exists in the compound in the present invention. However, the present invention includes substances separated from and mixtures of these isomers.

There is a case that the compound in the present invention forms a salt, and the salt is included in the present invention as long as it is a pharmaceutically acceptable salt. Specifically, it includes acid adduct salts with inorganic acid such as hydrochloric acid, hydrobromic acid, hydroiodic acid, sulfuric acid, nitric acid, and phosphoric acid, and with organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid, salts with an inorganic base containing metal such as sodium, potassium, calcium, and magnesium, and with an organic base such as methylamine, ethylamine, ethanolamine, lysine, and ornithine, and ammonium salt, for instance. Furthermore, the present invention includes various hydrates, solvates, and compounds having crystal polymorphism of the compound in the present invention and its pharmaceutically acceptable salts.

Manufacturing Method

The compound in the present invention and its pharmaceutically acceptable salts can be manufactured by applying various known synthesizing methods using characteristics based on its basic skeleton and the type of the substituent. A typical manufacturing method is exemplified below. Moreover, there is a case that it is effective in terms of manufacturing to replace the functional group to an appropriate protective group, that is a group capable of being easily converted into the functional group, at the stage from a raw material to an intermediate depending on the functional group. After that, the protective group is removed depending on necessity, and a desired compound can be obtained. Examples of such functional groups include a hydroxyl group, a carboxyl group, and an amino group, examples of the protective groups for these are the protective groups described in "Protective Group in Organic Synthesis-third edition"

by Greene and Wuts, and these may be used appropriately depending on the reaction condition.

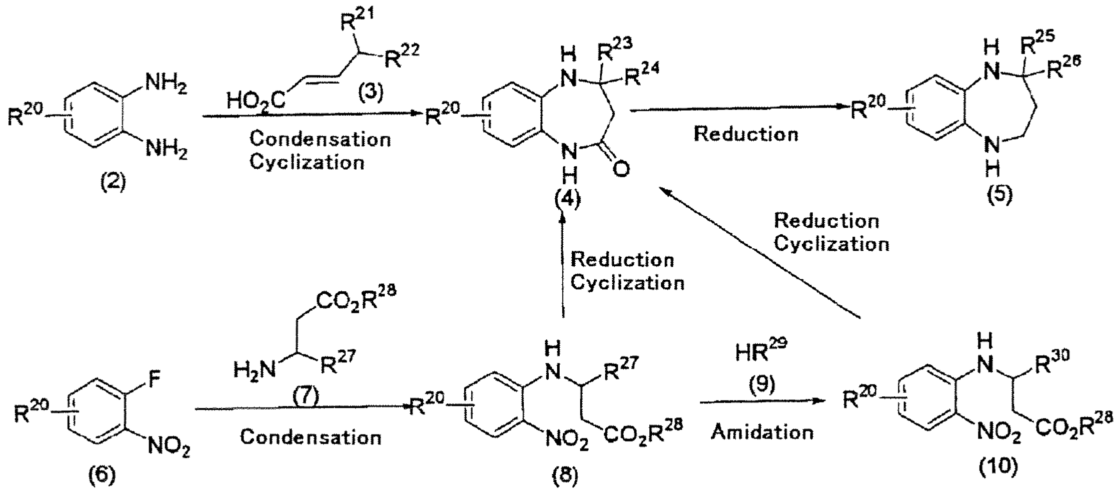


(In the Formula, $R^{20}$ represents hydrogen, lower alkyl, amino in which a protective group is introduced, or hydroxy in which a protective group is introduced. The protective group of amino is preferably amide, and further preferably p-toluenesulfonic acid amide . The protective group of hydroxy is preferably ether, and further preferably benzyl ether. $R^{21}$ and R $^{22}$ represent hydrogen, lower alkyl, or hydroxy lower alkyl in which the protective group is introduced. However, the case that both $R^{21}$ and $R^{22}$ are hydrogen is excluded. $R^{23}$ and $R^{24}$ represent hydrogen, lower alkyl, di-lower alkylaminocarbonyl, or hydroxy lower alkyl in which the protective group is introduced. However, the case that both $R^{23}$ and $R^{24}$ are hydrogen is excluded. $R^{25}$ and $R^{26}$ represent hydrogen, lower alkyl, di-lower alkylamino lower alkyl, or hydroxy lower alkyl in which the protective group is introduced. However, the case that both $R^{25}$ and $R^{26}$ are hydrogen is excluded. $R^{27}$ represents lower alkyl or carboxy. $R^{28}$ represents hydrogen, lower alkyl or benzyl. $R^{29}$ represents di-lower alkylamino. $R^{30}$ represents di-lower alkylaminocarbonyl.)

Compound (4) can be synthesized by referring to Heterocycles, 1991, vol. 32, p. 1131 for a reaction between Compound (2) and Compound (3) . Compound (8) can be synthesized by performing the condensation reaction in the presence of a base for a reaction between Compound (6) and Compound (7).

The base used in the condensation reaction includes an organic base such as N-methylmorpholine, trimethylamine, triethylamine, N.N'-dimethylamine, pyridine, 1,5-diazabicyclo[4,3,0]none-5-ene (DBN), and 1, 8-diazabicyclo [2,2,2]octane (DABCO), and an inorganic base such as potassium carbonate, sodium carbonate, potassium hydrogen carbonate, and sodium hydrogen carbonate, for instance.

The solvent used in the reaction is a single solvent, for example halogenated hydrocarbons such as methylene chloride, dichloroethane, and chloroform, aromatic hydrocarbons such as benzene, toluene, and xylene, ethers such as diethyl ether, tetrahydrofuran, 1,4-dioxane, and dimethoxyethane, esters such as ethyl acetate, and an aprotic polar solvent such as N,N-dimethylformamide, dimethylsulfoxide, and hexamethylphosphorous triamide, or a mixed solvent of these or a mixed solvent thereof with water. Normally, the reaction is performed at about the reaction temperature of 0˚C to about the boiling point of the solvent used, preferably at about room temperature to about the boiling point of the solvent used, and the reaction time is 5 minutes to 3 days, and preferably 10 hours to 3 days.

Compound (4) can be introduced by performing reduction of Compound (8) from a nitro group to an amino group and continuing the cyclization reaction.

The reduction method include chemical reduction and a catalytic reduction, and it can be performed with a normal method. The preferred reducing agents to be used in the chemical reduction are a metal such as tin, zinc, and iron, and a metal compound such as nickel chloride, chromium chloride, and chromium acetate, and the reduction can be performed under the acid, neutral, or basic condition. The acids to be used are organic acid such as formic acid, acetic acid, trifluoroacetic acid, and p-toluenesulfonic acid, or inorganic acid such as hydrochloric acid and hydrobromic acid. On the other hand, in the case of using base, the bases to be used include ammonia, ammonium chloride, and sodium hydroxide. Furthermore, an aluminum hydride compound such as aluminum hydride, lithium aluminum hydride, and sodium aluminum hydride, and a brohydride compound such as sodium brohydride, lithium brohydride, sodium cyano brohydride, Super Hydride (R), borane, and diborane give a good result as the reducing agent. The preferred catalysts to be used in the catalytic reduction include a palladium catalyst such as palladium carbon, palladium oxide, spongy palladium, and palladium colloid, a nickel catalyst such as raney nickel, nickel oxide, and reduced nickel, and a platinum catalyst such as a platinum plate, platinum oxide, and spongy platinumn, for instance. The reduction reaction

is normally performed in a solvent, and the solvent is a single solvent, for example alcohols such as methanol, ethanol and propanol, ethers such as diethyl ether, 1,4-dioxane, tetrahydrofuran and dimethoxyethane, or water, or a mixed solvent thereof. The reaction temperature is not especially limited, and the reaction can be performed under cooling or heating.

A cyclization reaction using a condensing agent and a cyclization reaction by heat can be used for the cyclization reaction. In the cyclization reaction using a condensing agent, a condensing agent is used such as dicyclohexylcarbodiimide (DCC), 1,1'-carbonylbis-1H-imidazol- (CDI), diphenylphosphoryl azide (DPPA), diethylphosphoryl cyanide, and 2-ethyl-3-(3-dimethylaminopropyl)carbodiimide hydrochloride (EDCI·HCl), for instance. Ethers such as diethyl ether, 1,4-dioxane, tetrahydrofuran, and dimethoxyethane are used as the solvent. The reaction temperature is not especially limited, and the reaction is preferably performed under cooling or at room temperature. The cyclization reaction by heat is generally performed under reflux by heating in an inert solvent. Examples of the solvent used are ethers such as diethyl ether, 1,4-dioxane, tetrahydrofuran, and dimethoxyethane, and aromatic hydrocarbons such as benzene, toluene, and xylene. The reaction is preferably performed at a reaction temperature of the boiling point of the solvent used.

Compound (5) can be synthesized by reducing the amido body (4). A reduction method in which a hydride reducing agent is used can be preferably used. Examples of the hydride reducing agent to be used include lithium aluminum hydride, lithium brohydride, sodium brohydride, and diborane, for instance. The use amount of the reducing agent is at least an equal molar amount to a raw material compound, and preferably in the range of an equal to 15 times the molar amount. In this reduction reaction, an appropriate solvent, for example, a single solvent including water, alcohols such as methanol, ethanol, and propanol, and ethers such as diethyl ether, 1,4-dioxane, tetrahydrofuran, and dimethoxyethane, or a mixed solvent thereof, is normally used. Normally, the reaction temperature is about -60˚C to 150˚C, preferably -30˚C to 100˚C, the reaction time is about 10 minutes to about 24 hours, and preferably about 30 minutes to about 15 hours. Moreover, in the case of using lithium aluminum hydride or diboran as a reducing agent, an anhydride solvent of ethers such as diethyl ether, 1, 4-dioxane, tetrahydrofuran, and dimethoxyethane is preferably used.

Among the compounds of Compound (8), the compound in which $R^{27}$ is carboxy can be led to Compound (4) by performing amidation reaction with Compound (9) and then performing reduction reaction and cyclization reaction. Because of being a free acid, a condensation agent such as dicyclohexylcarbodiimide (DCC), 1,1'-carbonylbis-1H-imidazol (CDI), diphenylphosphoryl azide (DPPA), diethylphosphoryl cyanide, and 1-ethyl-3- (3-dimethylaminopropyl) carbodiimide hydrochloride (EDCI·HCl) is used in the amidation reaction. Normally, the appropriate solvent is a single solvent, for example halogenated hydrocarbons such as methylene chloride, dichloroethane, and chloroform, aromatic hydrocarbons such as benzene, toluene, and xylene, ethers such as diethyl ether, tetrahydrofuran, 1,4-dioxane, and dimethoxyethane, alcohols such as methanol, ethanol, and propanol, and an aprotic polar solvent such as N,N-dimethylformamide, dimethylsulfoxide, and hexamethylphosphorous triamide, or a mixed solvent thereof. The reaction temperature is not especially limited, the reaction is preferably performed under cooling or at room temperature. The reduction reaction and the cyclization reaction after that can be performed with the same method as described before.

Further, protection and deprotection of the amino group may be performed in Compound (5) after that depending on necessity.

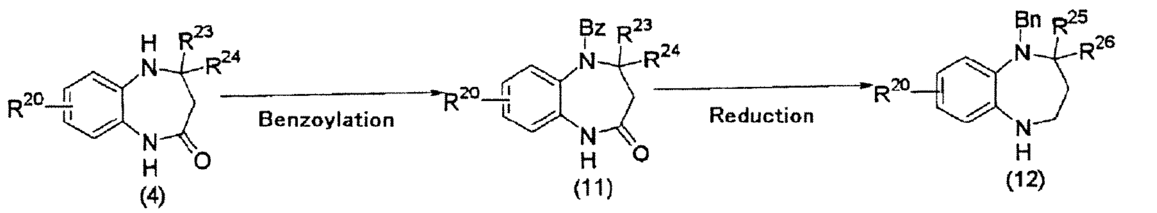


(4) (11) (12)

(In the Formula, $R^{20}$ , $R^{23}$,$R^{24}$, $R^{25}$, and $R^{26}$ are the same as described before.)

Compound (11) can be synthesized by benzoylating Compound (4). The benzoylation can be performed in an appropriate solvent in the presence of a base in the same manner as a known acylation reaction. The solvent used in the acylation reaction is a single solvent, for example halogenated hydrocarbons such as methylene chloride, dichloroethane, and chloroform, aromatic hydrocarbons such as benzene, toluene, and xylene, ethers such as diethyl ether, tetrahydrofuran, 1, 4-dioxane, and dimethoxyethane, alcohols such as methanol, ethanol, and propanol, and an aprotic polar solvent such as N,N-dimethylformamide, dimethylsulfoxide, and hexamethylphosphorous triamide, or a mixed solvent thereof. Further, the base used in the reaction includes alkali metals such as lithium, sodium, and potassium, alkali earth metals such as magnesium and calcium, these hydrides, hydroxides, an inorganic base such as hydrocarbonate and bicarbonate, and an organic base such as N-methylmorpholine, trimethylamine, triethylamine, N,N-dimethylamine, pyridine, 1,5-diazabicyclo[4,3,0]none-5-ene (DBN), and 1,8-diazabicyclo[2,2,2]octane (DABACO), for example. Normally, the reaction is performed at the reaction temperature of about -40˚C to about the boiling point of the solvent

used, preferably about 10˚C to about the boiling point of the solvent used, the reaction time is about 5 minutes to about 20 hours, and preferably about 5 minutes to about 10 hours.

The deprotection may be performed in Compound (12) after that depending on necessity.

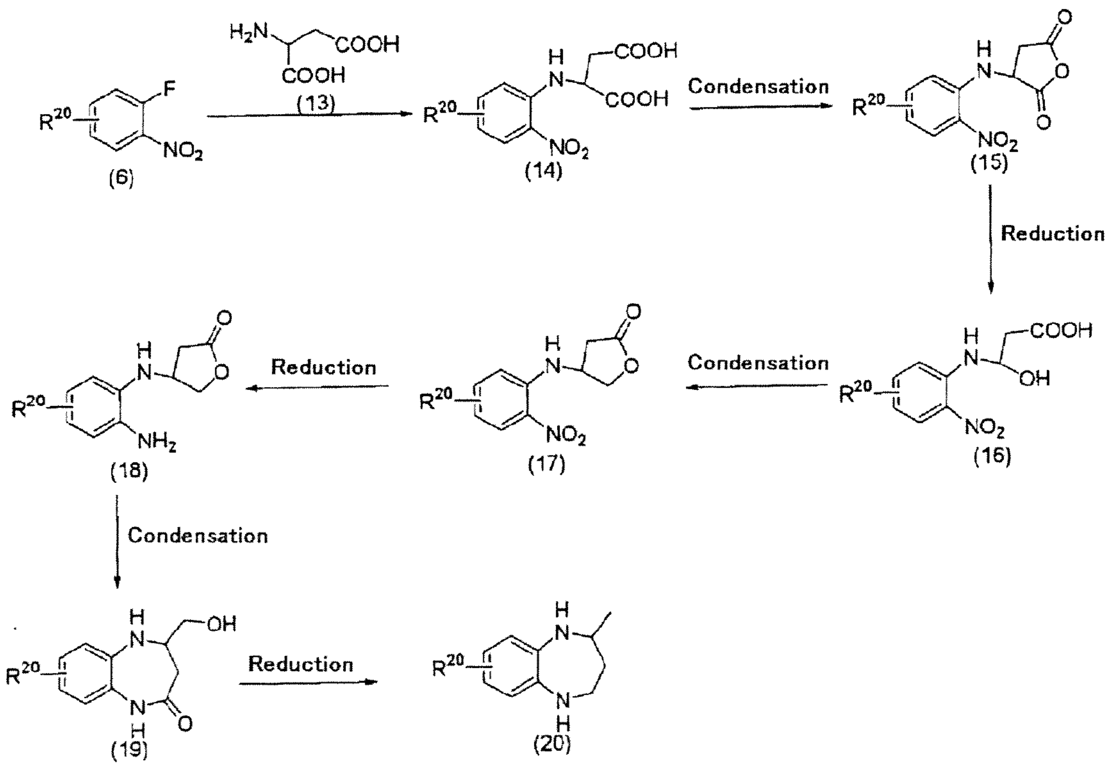


(In the Formula, $R^{20}$ is the same as described before.)

Compound (14) can be obtained by performing amination of Compound (6) using an optically active body or a racemic body of aspartic acid. This amination reaction can be performed in the condition the same as the synthesis of Compound (8). The condensation reaction from Compound (14) to Compound (15) is performed by an acid anhydride for example.

The appropriate acid anhydride includes acetic anhydride, trifluoroacetic anhydride, methanesulfonic anhydride, benzenesulfonic anhydride, and trifluoromethanesulfonic anhydride, for instance. The appropriate solvent is not especially limited. However, the examples include an aliphatic hydrocarbon-based solvent such as pentane, hexane, and cyclohexane, an aromatic hydrocarbon-based solvent such as benzene, toluene, and xylene, halogenated hydrocarbons such as methylene chloride, dichloroethane, chloroform, and carbon tetrachloride, ethers such as tetrahydrofuran, 1,4-dioxane, ethylene glycol dimethyl ether, siloxane, diethyl ether, and diphenylther, an aprotic polar solvent such as acetonitrile, N,N-dimethylformamide, and dimethylsulfoxide, esters such as ethyl acetate, pyridine, acetic acid, propionic acid, trufluoroacetic acid, dichloroacetic acid, and methanesulfonic acid, and these can be used alone or as a mixture of these. Normally, the reaction is performed at the reaction temperature of 0˚C to 100˚C, preferably room temperature to 50˚C, the reaction time is 1 to 24 hours, and preferably about 5 to about 15 hours.

The reduction reaction from Compound (15) to Compound (16) can be performed in the same condition as the reduction of Compound (4). Sodium brohydride can be preferably used as the reducing agent.

A dehydration condensation reaction from Compound (16) to Compound (17) is performed for example by refluxing using a solvent that does not mix with water. The appropriate solvent is not especially limited. However, the examples include an aliphatic hydrocarbon-based solvent such as pentane, hexane, and cyclohexane, an aromatic hydrocarbon-based solvent such as benzene, toluene, and xylene, halogenated hydrocarbons such as methylene chloride, dichloroethane, chloroform, and carbon tetrachloride, esters such as ethyl acetate, and these can be used alone or as a mixture of these.

The reaction is performed at room temperature or under heating, and preferably at the boiling point of the solvent used. The reaction time is about 30 minutes to about 6 days, and preferably about 6 hours to about 4 days.

The reduction reaction from Compound (17) to Compound (18) can be performed by referring to the reduction of Compound (4) . Palladium carbon can be preferably used as the reducing agent. The condensation reaction from Compound (18) to Compound (19) can be performed by referring to the synthesis example of Compound (17).

In the reduction reaction from Compound (19) to Compound (20), the synthesis of Compound (4) can be used as a reference. There is a method of using lithium aluminum hydride after treating with sodium brohydride or Super Hydride (R) as the reducing agent.

Further, an additive may be added in the reaction, examples of the additive include trimethylsilyl chloride, acid halides such as methanesulfonyl chloride, sulfonic acid esters such as methanesulfonic acid methyl ester and trifluoromethanesulfonic acid methyl ester, methyl iodide, dimethyl sulfate, and methanesulfonic acid, for instance.

Ethers such as tetrahydrofuran, 1,4-dioxane, ethylene glycol dimethyl ether, diethylene glycol dimethyl ether, and triethylene glycol dimethyl ether and others such as dimethylsulfoxide, for instance, are used as the solvent.

Further, this reduction reaction can be performed after introducing a leaving group to a hydroxy group depending on the case.

The leaving group includes halogen such as bromine and iodine, and methanesulfonate, for instance. It can be synthesized by performing a reaction of Compound (19) with an acid halide such as methanesulfonyl chloride in the presence of an appropriate base. The base to be used includes triethylamine and pyridine, for instance.

Further, in the case of converting into halogen, a metal halide such as lithium and sodium, halogen, phosphinic trihalide, thionyl halide, etc. can be used as a halogenating agent.

The halogen is preferably bromine, and can be brominated using phosphorous tribromide. Further, it can be converted using bromine in the presence of triphenylphosphine.

The solvent to be used is not especially limited as long as it does not hinder the reaction, examples include aliphatic hydrocarbons such as hexane and heptane, aromatic hydrocarbons such as benzene, toluene, and xylene, halogenated hydrocarbons such as methylene chloride, chloroform, carbon tetrachloride, dichloroethane, chlorobenzene, and dichlorobenzene, ethers such as diethyl ether, diisopropyl ether, tetrahydrofuran, 1,4-dioxane, dimethoxyethane, and diethylene glycol dimethy ether, and others such as acetnitrile, N,N-dimethylformamide, N,N-dimethylacetamide, N-methyl-2-pyrrolidone, N-methylpyrrolidinone, and dimethylsulfoxide, and these can be used alone or as a mixture of these. The above-described "ethers" can be preferably used, and further preferably tetrahydrofuran can be used.

The reaction temperature is 0˚C to the boiling point of the solvent used and preferably about room temperature to about 80˚C.

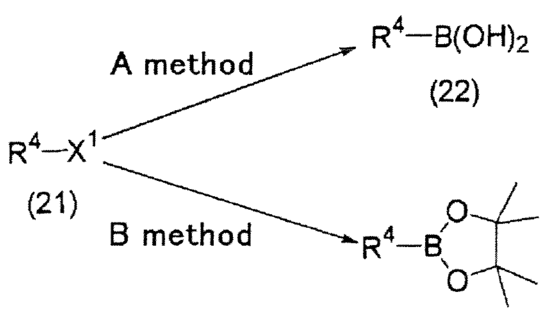


(21) (22) (23)

(In the Formula, $R^4$ is the same as described above. $X^1$ represents halogen or trifluoromethanesulfonyloxy.)

Compound (22) can be synthesized from Compound (21) using an A method. That is, the A method is performed by adding an organic lithium compound to Compound (21) in the appropriate solvent, conducting treatment of these normally at -80˚C to 50˚C, preferably -80˚C to -30˚C for a reaction time of about 30 minutes to about 5 hours, and then reacting the resultant with boronic esters such as trimethyl borate and triisopropyl borate. The appropriate solvent includes ethers such as tetrahydrofuran, 1,4-dioxane, and diethylene glycol dimethyl ether. Further, the organic lithium compound includes alkyl- and aryllithiums, and lithiumamides such as methyllithium, n-butyllithium, phenyllithium, and lithium diisopropylamide, for instance.

For all others, Compound (23) was synthesized according to a B method, that is according to a report in Journal of Organic Chemistry, 2000, vol. 65, p.164.

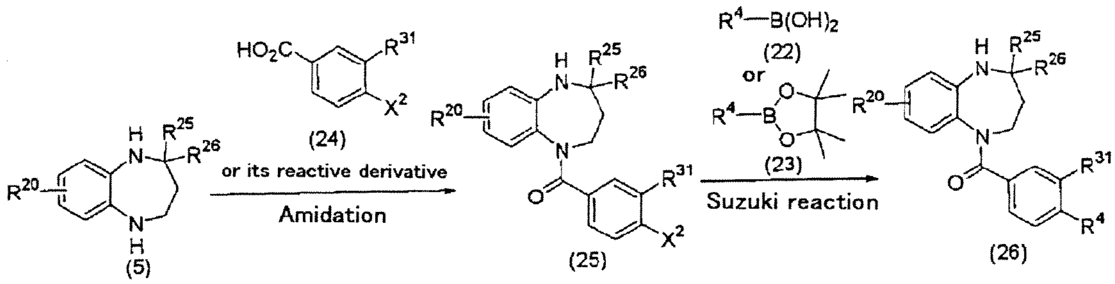

(5) (24) (25) (26)

[In the Formula, $R^4$, $R^{20}$, $R^{25}$, $R^{26}$ are the same as described above . $R^{31}$ represents hydrogen, lower alkoxy, nitro, cyano, hydroxy in which a protective group is introduced, halogen, lower alkoxycarbonyl, halogen-substituted lower alkyl, or

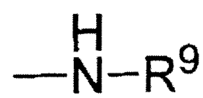

($R^9$ is the same as described above). $X^2$ represents halogen, trifluoromethanesulfonic acid ester, or nitro.]

Compound (25) can be synthesized by acylating Compound (5) with Compound (24) or its reactive derivative.

The reactive derivative of Compound (24) is normal ester thereof such as methyl ester, ethyl ester, tert-butyl ester; acid halide thereof such as acid chloride and acid bromide; acid azide thereof; active ester thereof with N-hydroxybenzotriazol, p-nitrophenol, N-hydroxysuccinimide, etc.; symmetric acid anhydride thereof; mixed acid anhydride thereof with halocarboxylic acid alkylester such as alkylcarbonate halide, pivaloyl halide, and p-toluenesulfonyl chloride; and mixed acid anhydride thereof such as phosphoric mixed acid anhydride obtained by reacting diphenylphosphoryl and N-methylmorpholine.

In the case of reacting Compound (24) with a free acid or reacting it without separating an active ester, a condensing agent such as dicyclohexylcarbodiimide (DCC), 1,1'-carbonylbis-1H-imidazol (CDI), diphenylphosphoryl azide (DPPA), diethylphosphoryl cyanide, and 1-ethyl-3-(3-dimethylaminopropyl)carbodiimide hydrochloride (EDCI-HCl), for instance, is preferably used.

Especially in the present invention, an acid chloride method, a method of performing the reaction in the coexistence of an active esterifying agent and a condensing agent, a method of treating normal ester with amine, etc. are useful.

The reaction solvent is various depending on the reactive derivative and the condensing agent to be used. However, the reaction is performed in an organic solvent that is inert in a reaction of halogenated hydrocarbons, aromatic hydrocarbons, ethers, esters, an aprotic polar solvent, etc. at a reaction temperature of -5˚C to 100˚C, and preferably room temperature to 50˚C. The reaction time is 10 minutes to 24 hours, and preferably 30 minutes to 3 hours.

Moreover, there is an advanced case of proceeding the reaction smoothly by using an excessive amount of Compound (5), or using a base such as N-methylmorpholine, trimethylamine, triethylamine, diisopropylethylamine, N, N-dimethylaniline, pyridine , 4-(N,N-dimethylamino)pyridine, picoline, and lutidine, for instance. Further, a salt consisting of weak base and strong acid such as pyridine hydrochloride, pyridine p-toluenesulfonate, and N,N-dimethylaniline hydrochloride may be used. Pyridine can be used also as a solvent. Especially, the reaction is preferably performed in a solvent such as methylene chloride and tetrahydrofuran and in the presence of a base such as pyridine and triethylamine.

Compound (26) can be synthesized with the Suzuki reaction using Compound (25) and Compound (22) or Compound (23). In this method, the synthesis can be performed by referring to a report in Heterocycles, 1992, vol. 34, p. 1395, Merck Index 13th Edition ONR-102, and the references in the documents.

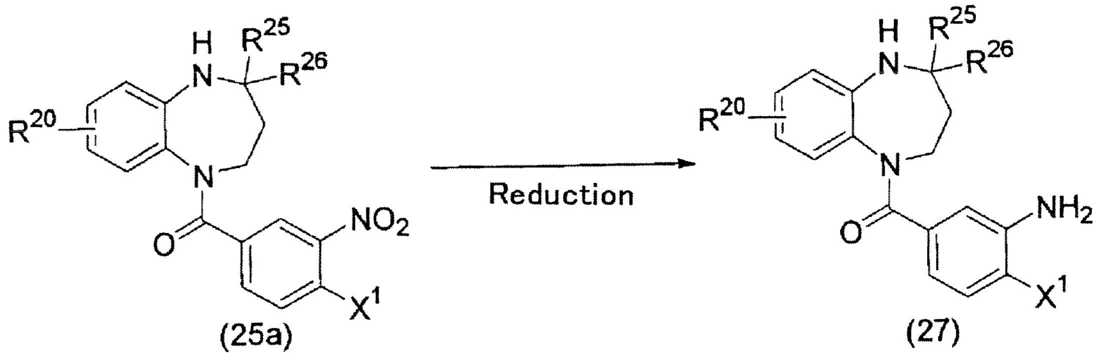


(25a) (27)

(In the Formula, $R^{20}$, $R^{25}$, $R^{26}$, and $X^1$ are the same as described above.)

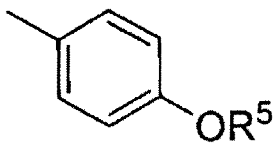

(In the Formula, $R^5$ is the same as described above.)

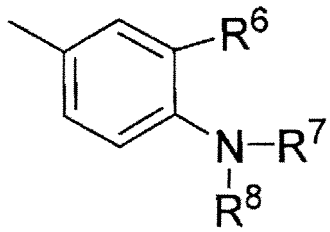

(In the Formula, $R^6$, $R^7$, and $R^8$ represent the same as described above, or 1H-indol-5-yl.)

Compound (27) can be synthesized by the reduction reaction from Compound (25a) with the same operation as the previous reduction method of the synthetic method of Compound (4).

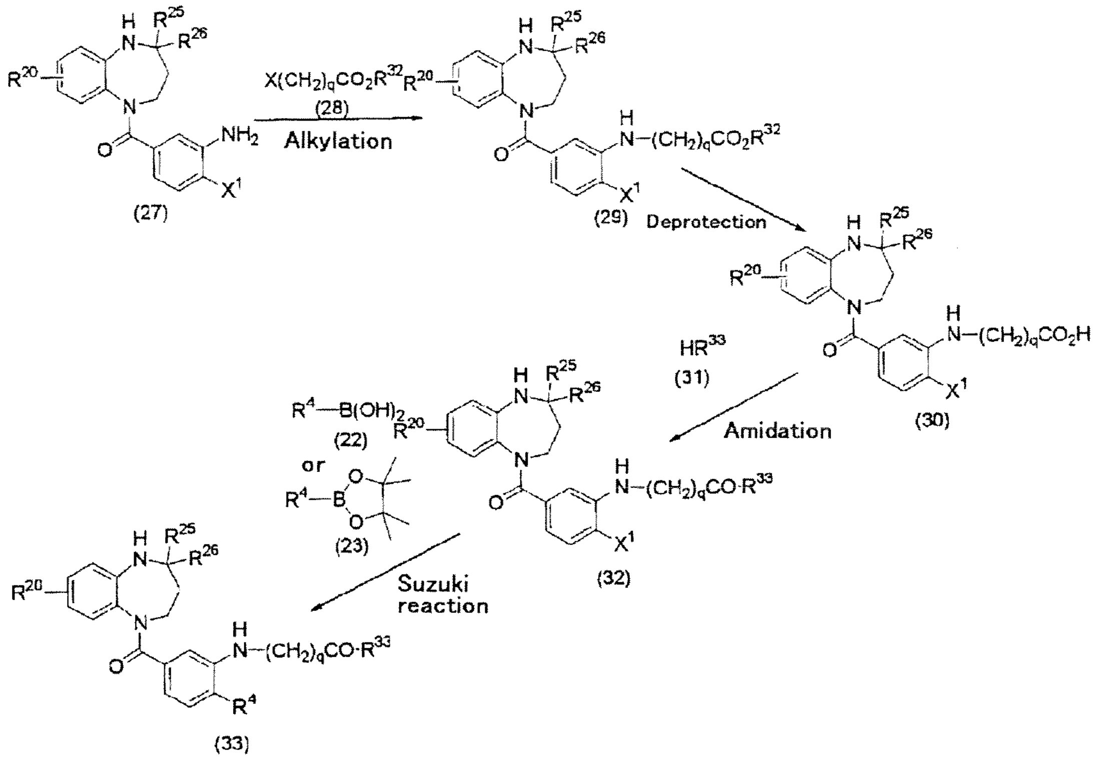


(In the Formula, $R^4$, $R^{20}$, $R^{25}$, $R^{26}$, and $X^1$ are the same as described above. $R^{32}$ represents a protective group of carboxylic acid. $R^{33}$ represents lower alkylpiperazinyl. q represents an integer of 1 to 6.)

Compound (29) can be synthesized by performing an alkylation of Compound (27) using Compound (28). The alkylation reaction can be performed generally in an appropriate solvent and in the presence of a basic compound. Examples of the solvent to be used include a single solvent including aromatic hydrocarbons such as benzene, toluene, and xylene, ethers such as tetrahydrofuran, 1,4-dioxane, and ethylene glycol dimethyl ether, halogenated hydrocarbons such as methylene chloride, chloroform, and carbon tetrachloride, acetone, acetonitrile, dimethylsulfoxide, and N,N-dimethylformamide, or a mixed solvent thereof, for instance. Further, examples of the basic compound include carbonate such as sodium carbonate, potassium carbonate, sodium hydrocarbonate, and potassium hydrocarbonate, metal hydroxide such as sodium hydroxide and potassium hydroxide, metal alcholate such as sodium hydride, potassium, sodium, sodium amide, sodium methylate, and sodium ethylate, and organic base such as pyridine, trimethylamine, triethylamine, N,N-dimethylamine, pyridine, 1,5-diazabicyclo[4,3,0]none-5-ene (DBN), and 1,8-diazabicyclo[2,2,2]octane (DABACO), for instance. An alkali metal halide such as sodium iodide and potassium iodide may be added in the reaction system. The reaction temperature is about room temperature to about 100˚C, preferably 50˚C to 80˚C or the boiling point of the solvent used.

The reaction time is 10 minutes to 48 hours, and preferably 30 minutes to 15 hours.

Compound (30) can be synthesized by deprotecting Compound (29).

Compound (32) can be synthesized by amidation of Compound (30) and Compound (31). The amidation reaction can be performed with the same operation as the previous amidation reaction in the synthesis of Compound (10).

Compound (33) can be synthesized by treating Compound (32) using Compound (22) or Compound (23) with the same operation as the previous Suzuki reaction on the synthesis of Compound (26).

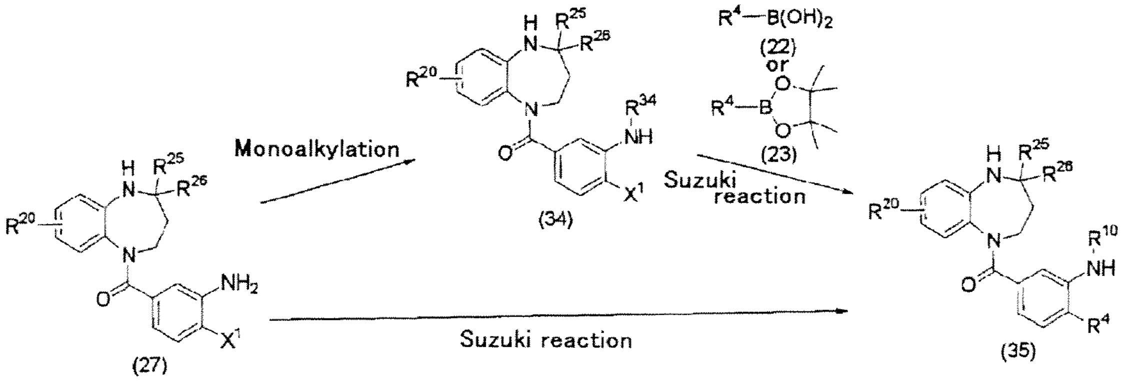


(22) (23) (34) (27) (35)

(In the Formula, $R^4$, $R^{10}$, $R^{20}$, $R^{25}$, $R^{26}$, and $X^1$ are the same as described above. $R^{34}$ represents lower alkyl.)

Compound (34) can be synthesized by monoalkylating Compound (27). In the monoalkylation, Compound (34) can be synthesized with a method of condensing an aliphatic aldehyde compound such as acetaldehyde and propionaldehyde with Compound (27) and treating the produced imine with a reducing agent. The reducing agent previously used in the reduction described in the synthesis of Compound (4) can be used as the reduction agent. Further, a monoalkyl body (34) can be synthesized by cleaving a formyl group by hydrolysis after the synthesis of N-alkylformanilide by lower alkyl orthoformate and aromatic primary amine described in "Seimitsu Yuki Gosei [Jikken Manual] ("Exact Organic Synthesis [Laboratory Manual]) (revised 2nd edition)", 2-6 amine [F-9a-b] and [F-9b], p.91.

Compound (35) can be synthesized by treating Compound (34) or Compound (27) using Compound (22) or Compound (23) with the same operation as the previous Suzuki reaction of the synthesis of Compound (26).

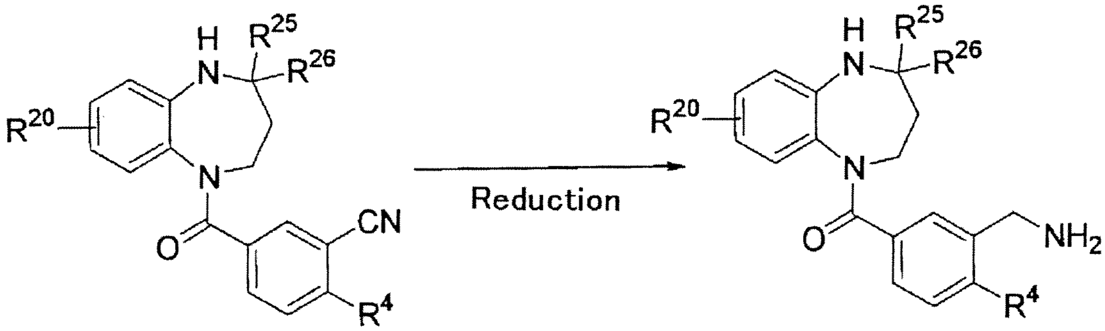


(26a) (36)

(In the Formula, $R^4$, $R^{20}$ , $R^{25}$ , and $R^{26}$ are the same as described above.)

Compound (36) can be synthesized by reducing Compound (26a). The reducing agent previously used in the reduction described in the synthesis of Compound (4) can be used as the reduction agent.

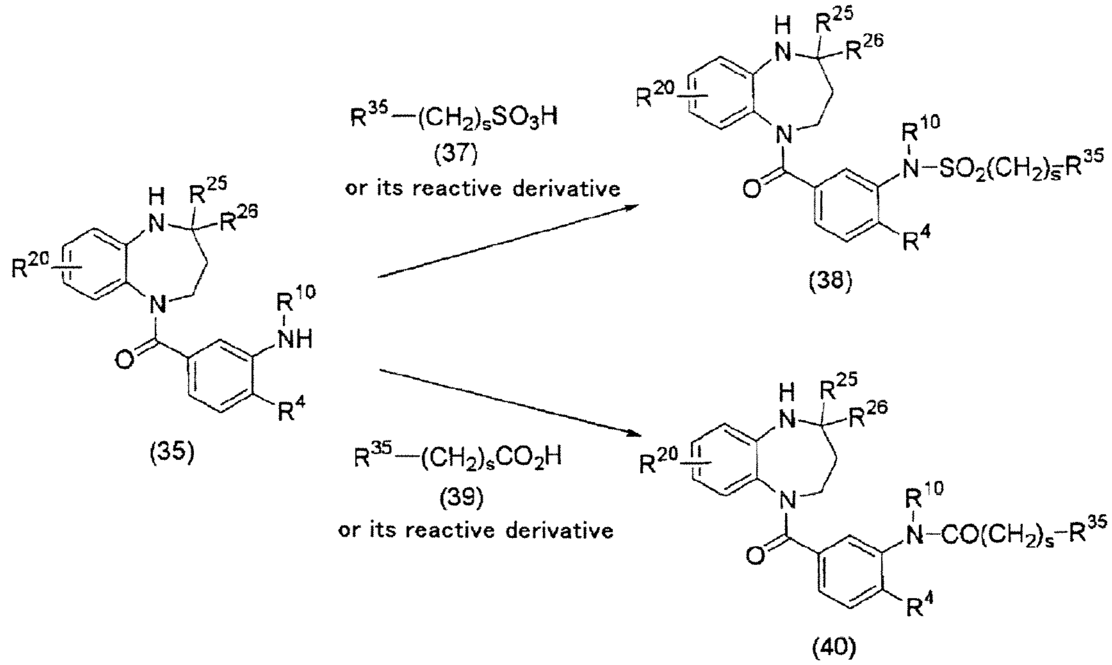


(In the Formula, $R^4$, $R^{10}$, $R^{20}$, $R^{25}$, and $R^{26}$ are the same as described above. $R^{35}$ represents hydrogen, alkoxy, morphorino, lower alkylpiperazinyl, amino, or, di-lower alkyl amino. s represents an integer of 0 to 2.)

The condensation reaction from Compound (35) to Compound (38) and Compound (40) can be performed with the same operation as the synthesis of Compound (8). Further, the reactive derivative of Compound (37) and Compound (39) canbe synthesized by referring to the reactive derivative of Compound (24).

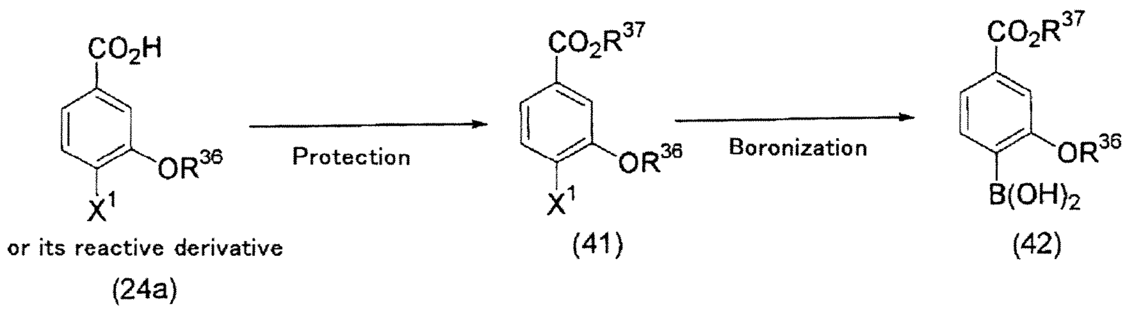


(In Formula, $R^{31}$ and $X^1$ are the same as described above. $R^{36}$ represents a protective group of a hydroxide group. $R^{37}$ represents a protective group of carboxylic acid.)

Compound (41) can be synthesized by protecting a carboxyl group of Compound (24a). An example of the protective group of the carboxyl group used is an ester type protective group. The operation of esterification is performed according to a normal method in which groups other than the carboxyl group are not changed. Further specifically, the reaction is performed in an organic solvent such as amide (for example, N,N-dimethylformamide, and N-methyl-2-pyrrolidinone), pyridine, halogenated hydrocarbons (for example, methylene chloride, dichloroethane, and chloroform), and ether (for example, tetrahydrofuran, 1,4-dioxane, and dimethoxyethane). The reaction temperature is -40°C to 80°C, and it is performed by the reaction of acid or an acid reactive derivative (for example, acid chloride, reactive ester, or anhydride) in the presence of a condensing agent such as carbodiimide (for example, dicyclohexylcarbodiimide), and a catalyst such as tertiary amine (trialkylamine such as triethylamine and diisopropylethylamine, pyridine, or a derivative) and 4-N-dimethylaminopyridine depending on the case.

The synthesis of Compound (42) is synthesized with the same method as the synthetic method of Compounds (22) and (23) .

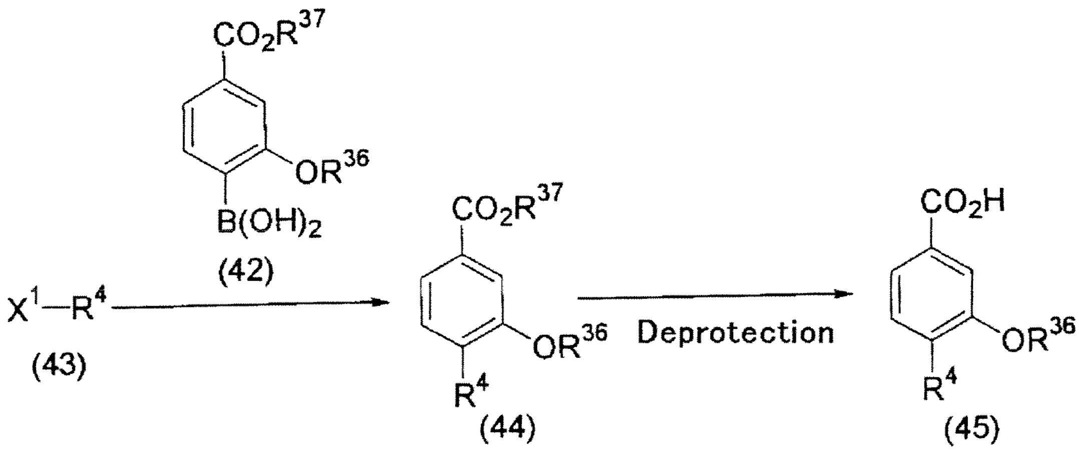


(In the Formula, $R^4$ , $R^{36}$, $R^{37}$, and $X^1$ are the same as described above.)

After Compound (43) is made into Compound (44) with the Suzuki reaction, Compound (45) can be obtained by removing a protective group. The Suzuki reaction can be performed in a condition referring to the synthesis of Compound (26).

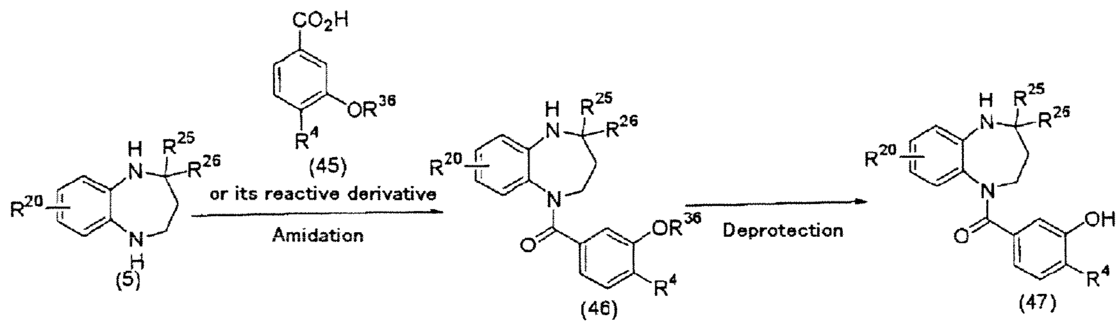


(In the Formula, $R^4$, $R^{20}$, $R^{25}$, $R^{26}$, and $R^{36}$ are the same as described above.)

Compound (46) can be obtained by performing amidation of Compound (5) using Compound (45) or its reactive derivative. Then, a protective group of phenolic hydroxide group is removed and Compound (47) is produced. The reactive derivative of Compound (45) can refer to a reactive derivative of Compound (24).

The deprotection can be performed, for example, by hydrolysis of an acetyl group using an appropriate base such as a potassium hydroxide solution. The reaction temperature is room temperature to 100˚C, and preferably 50˚C to 80˚C.

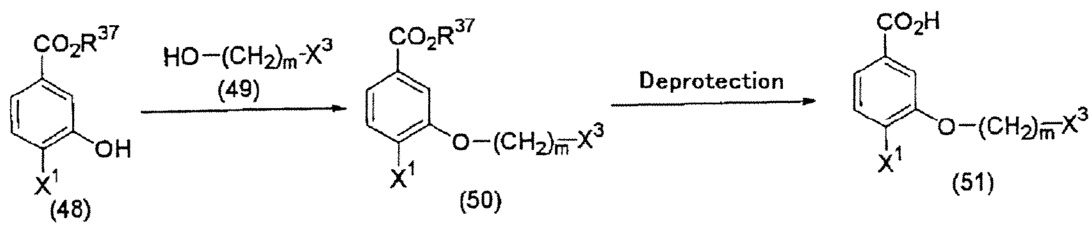


(In the Formula, $R^{37}$, $X^1$ and m are the same as described above. $X^3$ represents halogen, trifluoromethanesulfonic acid ester, or methanesulfonic acid ester.)

A conversion from Compound (48) to Compound (50) can be performed in a basic condition or in a condition of the Mitsunobu reaction. In the basic condition, the conversion can be performed using a base such as potassium carbonate in a solution such as N,N-dimethylformaide and methylethylketone. In the Mitsunobu reaction, the conversion can be performed by referring to Merck Index 13th Edition ONR-70 and the references in the document. Then, deprotection can be performed by referring to the operation of deprotection of Compound (44).

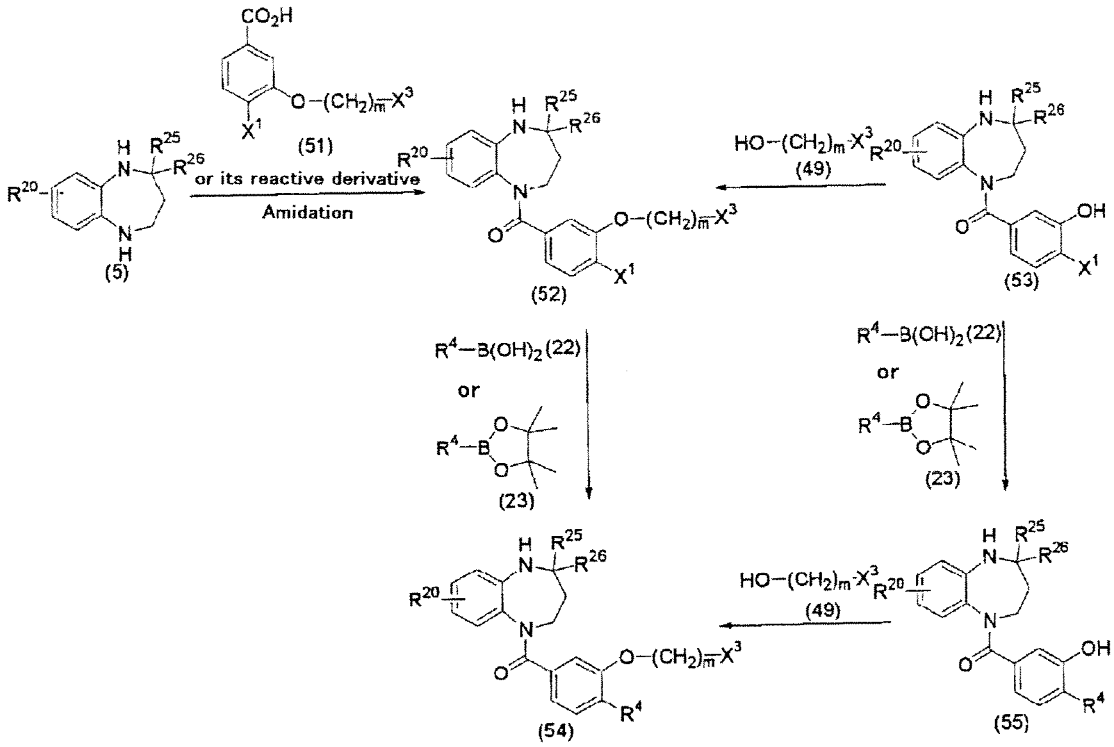


(In the Formula, $R^4$, $R^{20}$, $R^{25}$, $R^{26}$ , $X^1$, $X^3$, and m are the same as described above.)

Compound (52) can be obtained by amidating Compound (5) . This amidation reaction can be performed by referring to a method of synthesizing Compound (25). A reactive derivative of Compound (51) can be obtained by referring to the case of the reactive derivative of Compound (24).

Compound (52) and Compound (54) can be obtained respectively by performing the Mitsunobu reaction on Compound (53) and Compound (55) using Compound (49). The Mitsunobu reaction can be performed by referring to a method of synthesizing Compound (50) .

Compound (54) and Compound (55) can be obtained respectively by performing the Suzuki reaction on Compound (52) and Compound (53) using Compound (22) or Compound (23). The Suzuki reaction can be performed by referring to a method of synthesizing Compound (26)

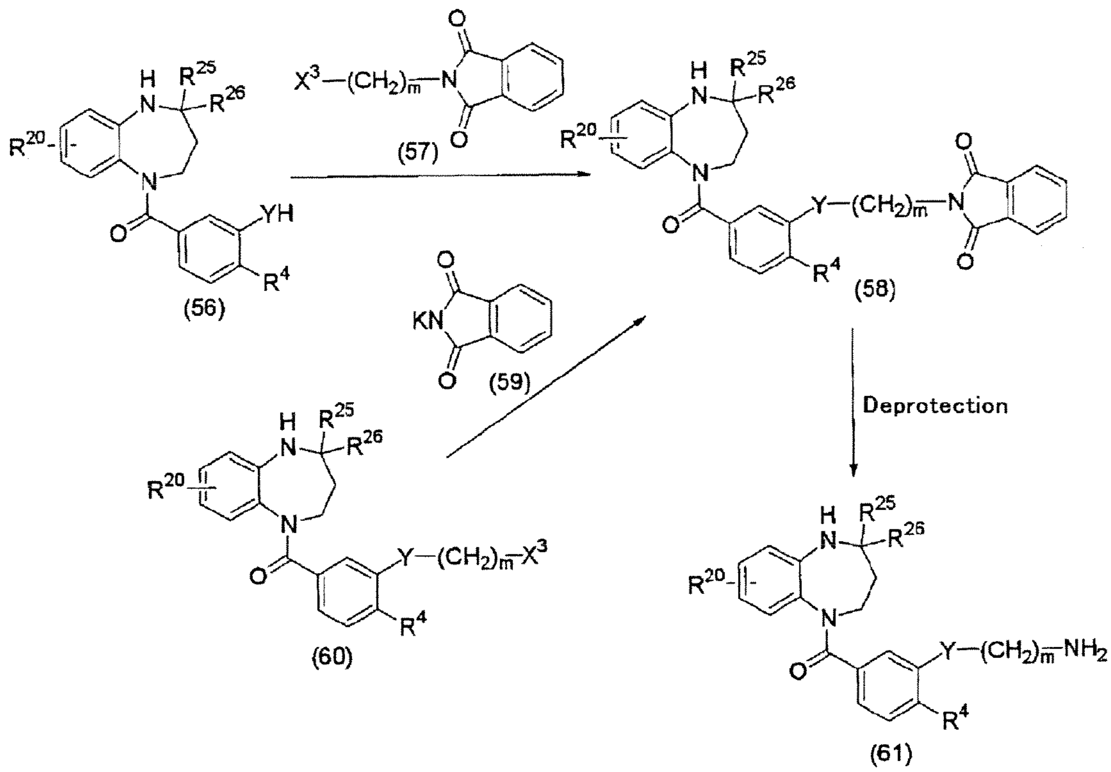


(In the Formula, $R^4$, $R^{20}$, $R^{25}$, $R^{26}$, $X^3$, and m are the same as described above. -Y- represents -O- or -NH-.)

Compound (58) can be synthesized with an alkylation of Compound (56). The alkylation reaction can be performed by referring to the synthetic method of Compound (29). Further, Compound (58) can be obtained also by performing a phthalimidation of Compound (60). In this case, potassium phthalimide etc. is used and the detail of other reaction conditions is the same as the alkylation reaction used in the synthesis of Compound (58).

Compound (61) can be obtained by reacting Compound (58), which is a phthalimide derivative, with hydrazine or a hydrazine derivative, for example hydrazine monohydride, at 0.5 to 5.0 times, preferably in the range of 1.0 to 2.0 times the molar amount of the Compound (58). Alcohols such as methanol and ethanol are used alone as a solvent, or alcohols and an appropriate inert solvent, preferably a mixed solvent with aliphatic hydrocarbons such as hexane and heptane can be used. The reaction is performed at a reaction temperature of 0˚C to 100˚C, preferably 20˚C to 80˚C, a reaction time of 10 minutes to 15 hours, and preferably 30 minutes to 3 hours.

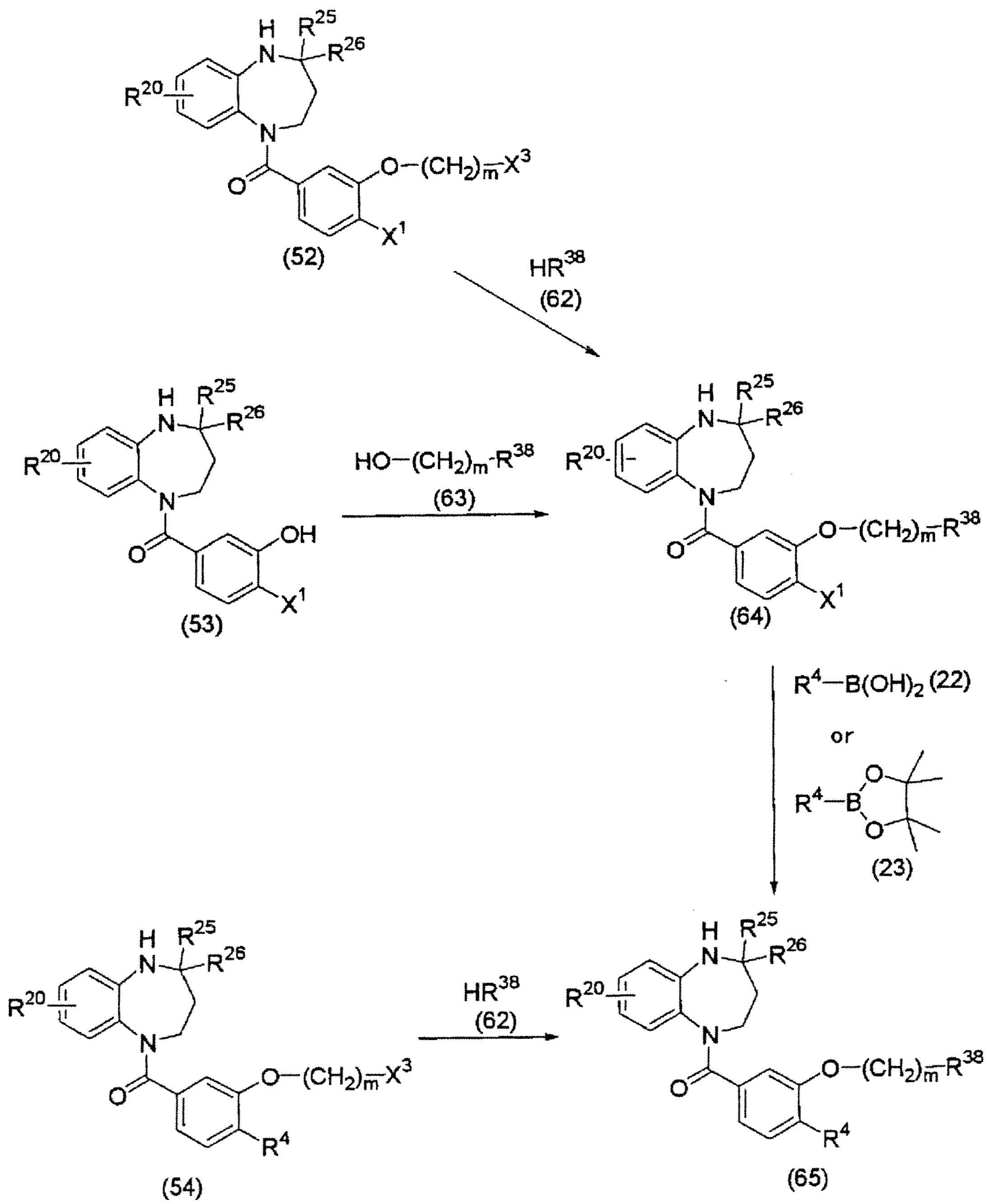


(52) (53) (64) (54) (65)

(In the Formula, $R^4$, $R^{20}$, $R^{25}$, $R^{26}$, $X^1$, $X^3$, and m are the same as described above. $R^{38}$ represents di-lower alkylamino or lower alkylpiperazinyl.)

Compound (64) and Compound (65) can be synthesized respectively by performing amination of Compound (52) and Compound (54). The operation of the amination reaction is performed with the same operation as the method of synthesizing Compound (8).

Compound (64) can be synthesized by performing the Mitsunobu reaction on Compound (53) using Compound (63). The operation of the Mitsunobu reaction is performed with the same operation of the method of synthesizing Compound (50).

The Suzuki reaction is used in the conversion from Compound (64) to Compound (65). The operation of the Suzuki reaction is performed with the same operation as the method of synthesizing Compound (26).

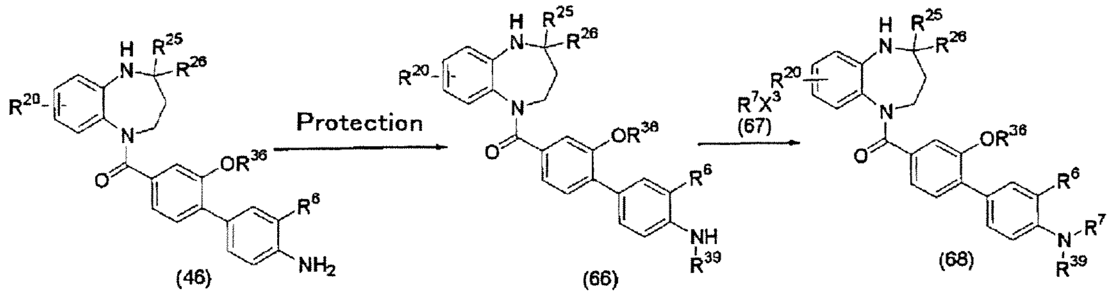


(In the Formula, $R^6$, $R^7$, $R^{20}$, $R^{25}$, $R^{26}$, $R^{36}$, and $X^3$ are the same as described above. $R^{39}$ represents a protective group of an amino group.)

Compound (66) can be obtained by introducing a protective group to Compound (46). A trifluoroacetyl group can preferably be listed as the protective group.

Furthermore, Compound (68) can be synthesized by performing an alkylation reaction using Compound (67). The operation of the alkylation reaction can be performed by referring to the method of synthesizing Compound (29).

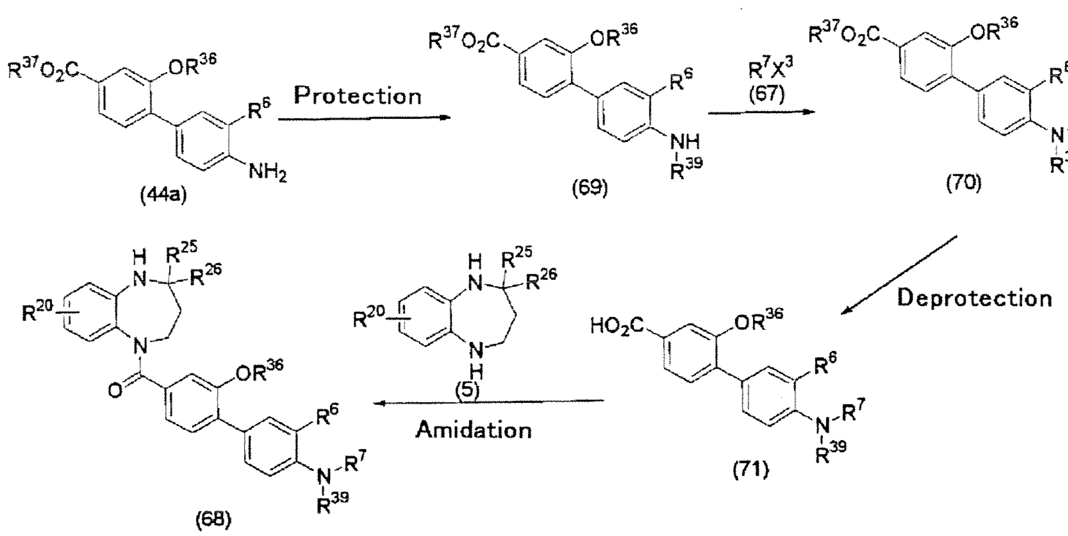


(In the Formula, $R^6$, $R^7$, $R^{20}$, $R^{25}$, $R^{26}$, $R^{36}$, $R^{37}$, $R^{39}$, and $X^3$ are the same as described above.)

Compound (70) can be obtained by performing a protection of an aniline part and an alkylation of Compound (44a) with the same procedure as the synthesis of Compound (68) . Compound (71) can be obtained by deprotecting a carboxyl group of Compound (70) with the same operation as Compound (45). Compound (68) can be synthesized by amidation of Compound (71) and its derivative using Compound (5) with the same operation as Compound (25). The reactive derivative of Compound (24) can be obtained by referring to the case of the reactive derivative of Compound (71).

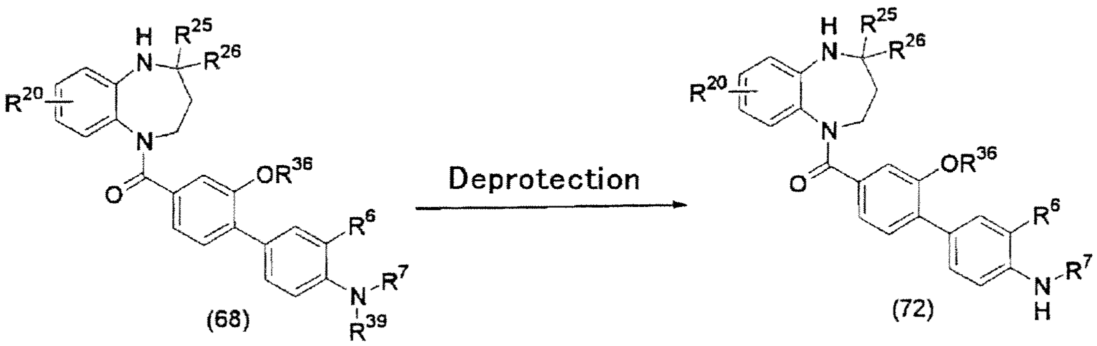

(In the Formula, $R^6$, $R^7$, $R^{20}$, $R^{25}$, $R^{26}$, $R^{36}$, and $R^{39}$ are the same as described above.)

Compound (68) is converted into Compound (72) by performing deprotection. For example, in the case of a trifluoroacetyl group, the reaction can be performed in an appropriate base, for example a sodium hydroxide solution.

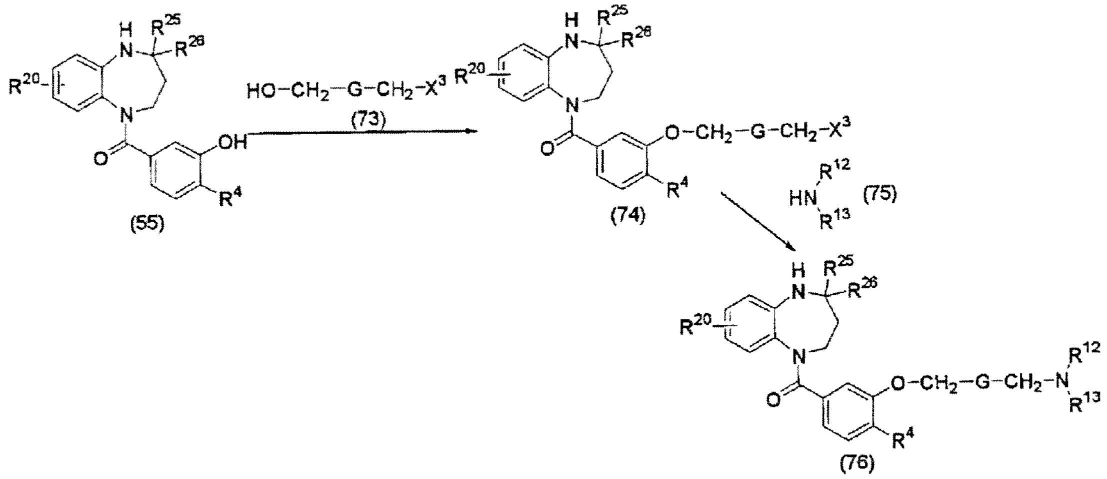


(In the Formula, $R^4$, $R^{12}$ $R^{13}$, $R^{20}$, $R^{25}$, $R^{21}$, $X^3$, and G are the same as described above.)

Compound (74) can be obtained by performing the Mitsunobu reaction on Compound (55) using Compound (73). The Mitsunobu reaction can be performed in the same condition as the synthesis of Compound (50).

The conversion to Compound (76) can be performed in the same condition as the synthesis of Compound (8).

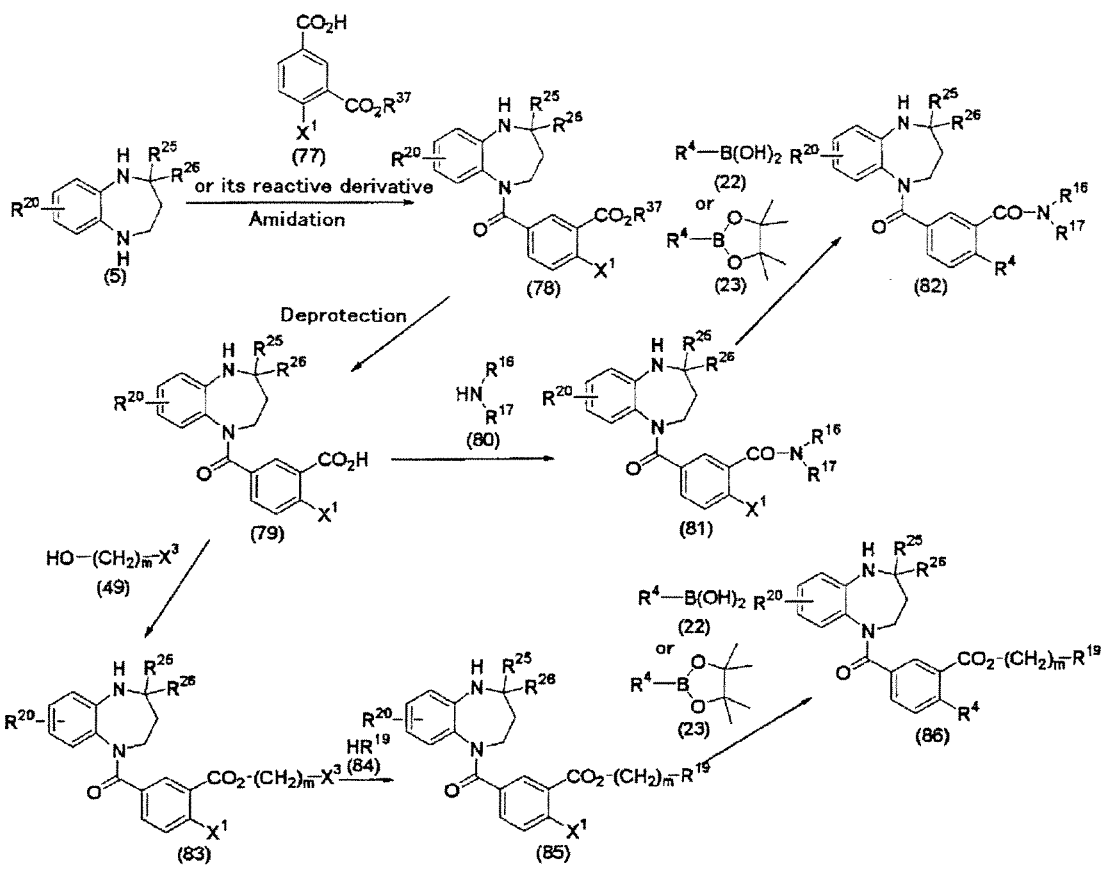


(In the Formula, $R^4$, $R^{16}$, $R^{17}$, $R^{19}$, $R^{20}$, $R^{25}$, $R^{26}$, $R^{37}$, $X^1$, $X^3$, and m are the same as described above.)

Compound (78) can be obtained by performing amidation of Compound (5) using Compound (77) and its derivative. The amidation reaction can be performed in the same condition as the method of synthesizing Compound (25). The reactive derivative of Compound (24) can be referred to as the reactive derivative of Compound (77).

Compound (79) can be obtained by removing a protective group of a carboxyl group of Compound (78). The operation of the deprotection can be performed in the same condition as the method of synthesizing Compound (30).

Compound (81) can be obtained by condensing Compound (79). The condensation reaction can be performed with the same operation as the synthesis of Compound (10) .

Compound (83) can be obtained by esterifying Compound (79), The esterification reaction can be performed with the same operation as the condensation reaction performed in the synthesis of Compound (10).

Compound (85) can be obtained by aminating Compound (83) using Compound (84). The operation of the amination reaction can be performed in the same condition as those of the method of synthesizing Compound (8).

Compound (82) and Compound (86) can be obtained respectively by performing the Suzuki reaction on Compound (81) and Compound (85) using Compound (22) or Compound (23). The Suzuki reaction can be performed in the same condition as those of the method of synthesizing Compound (26).

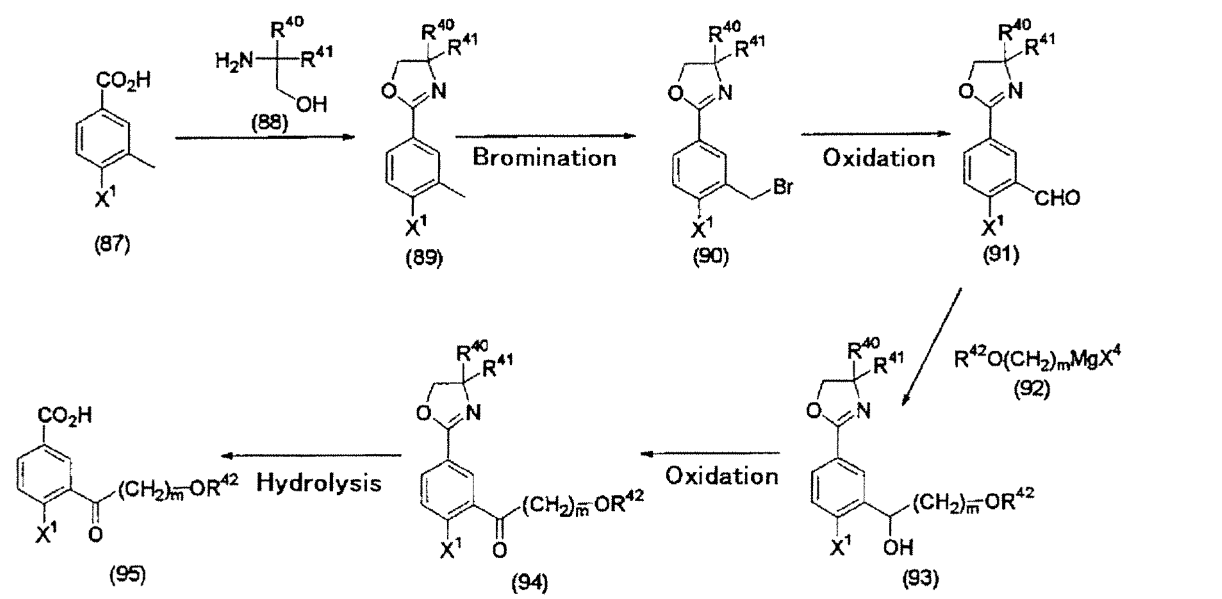


(In the Formula, $X^1$, and m are the same as described above. $R^{40}$ and $R^{41}$ represent lower alkyl. $R^{42}$ represents a protective group of a hydroxide group. $X^4$ represents halogen.)

Compound (89) can be obtained by condensing Compound (87), preferably using aminomethylpropanol. The condensation reaction of this case can be performed according to an example of synthesizing Compound (25). However, thionyl chloride can be preferably used as a condensing agent.

Bromination of Compound (89) to Compound (90) is performed using a brominating agent such as N-bromosuccinimide (NBS) and peroxides. The peroxide includes benzoyl peroxide, m-toluyl peroxide, 2,4-dichlorobenzoyl peroxide, tert-butyl hydroperoxide, cumene hydroperoxide, p-cumene hydroperoxide, diisopropyl peroxydicarbonate, di-n-propyl peroxydicarbonate, tert-butyl peroxyacetate, tert-butyl peroxyisobutyrate, and tert-butyl peroxypivalate, for instance, and preferably benzoyl peroxide.

The reaction can be performed in the presence of a solvent. The reaction solvent used here is not especially limited. However, it includes an aliphatic hydrocarbon-based solvent and aromatic hydrocarbon-based solvent, for instance. Normally, these solvents are used alone or mixed together. The reaction temperature is preferably 10˚C to 50˚C. The reaction time is 30 minutes to 24 hours, and preferably 1 hour to 13 hours.

Compound (91) can be obtained by performing an oxidation reaction on Compound (90) .

Examples of an oxidizing agent includes acetic anhydride-dimethylsulfoxide, phosphorous pentaoxide-dimethylsulfoxide, sulfur trioxide pyridine complex salt-dimethylsulfoxide, dicyclohexylcarbodiimide-dimethylsulfoxide, oxalyl chloride-dimethylsulfoxide, chromic acid, chromic acid complex such as chromic acid-pyridine complex, and manganese dioxide, for instance.

Further, a reduction reaction can be performed using 2-nitropropane and/or sodium as another method. In this case, alcohols such as propanol, ethanol, and methanol are preferably used as a reaction solvent, and further preferably ethanol can be used. The reaction temperature is 0˚C to 100˚C, and preferably room temperature to 50˚C. The reaction

can be performed with a reaction time of 10 minutes to 24 hours, and preferably 30 minutes to 15 hours.

Compound (93) can be obtained by acting a Grignard reaction agent (92) to Compound (91).

The solvent includes ethers such as diethyl ether, diisopropyl ether, diethylene glycol dimethyl ether, 1,4-dioxane, and tetrahydrofuran, aromatic hydrocarbons such as benzene, toluene, and xylene, and aliphatic hydrocarbons such as hexane and heptane, for instance. These solvents can be used alone or by mixing two or more kinds of these. The reaction temperature is normally -40°C to the boiling point of each solvent, preferably 0°C to room temperature, the reaction time is normally about a few minutes to about 24 hours, and preferably about 30 minutes to about 3 hours.

The oxidation reaction from Compound (93) to Compound (94) can be performed with the same operation as the synthesis of Compound (91).

The conversion from Compound (94) to Compound (95) can be achieved by removing a protective group with a hydrolysis reaction.

The base to be used includes an alkali metal oxide such as lithium hydroxide, sodium hydroxide, and potassium hydroxide, and preferred is sodium hydroxide. The inert solvent to be used is preferably a mixed solvent of an ether-based organic solvent such as tetrahydrofuran, diethyl ether, 1,2-dimethoxyethane, and 1,4-dioxane and water, and further preferably a mixed solvent of 1,4-dioxane and water (1:1). The reaction temperature is 0°C to 100°C, and preferably room temperature to 80°C. The reaction time is 10 minutes to 12 hours, and preferably 30 minutes to 6 hours.

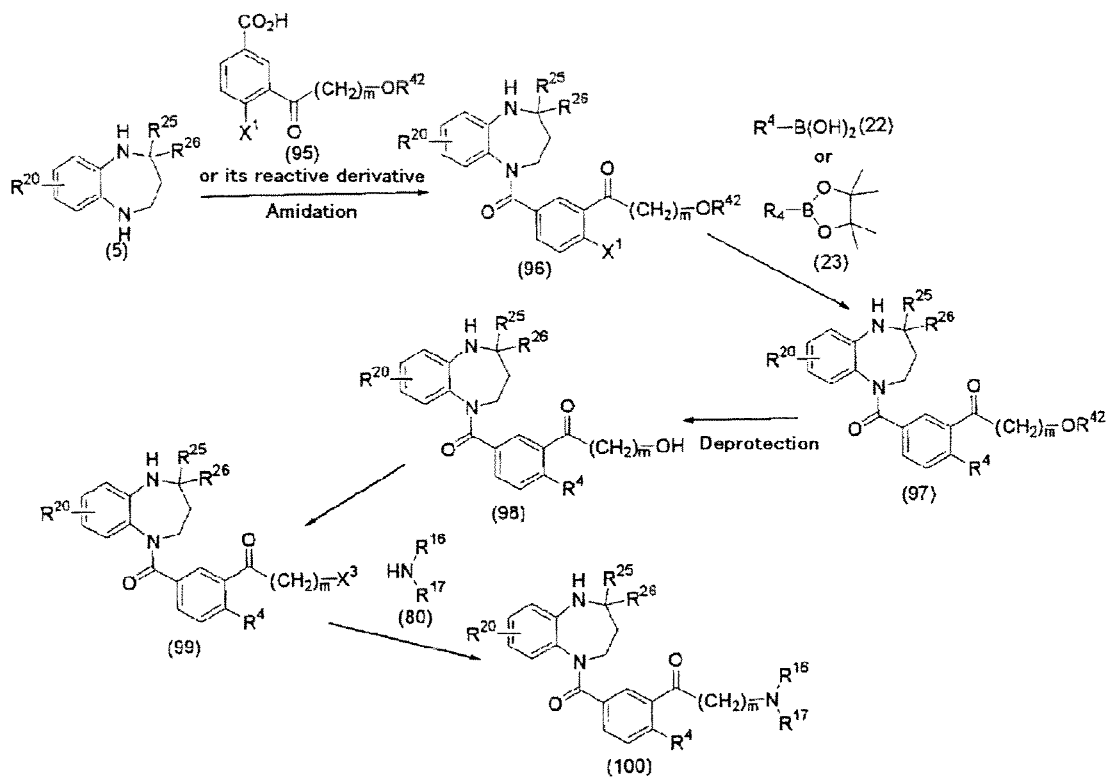


(In the Formula, $R^4$, $R^{16}$, $R^{17}$, $R^{20}$, $R^{25}$, $R^{26}$, $R^{42}$, $X^1$, $X^3$, and m are the same as described above.)

Compound (96) can be obtained by amidating Compound (5) using Compound (95) . The reactive derivative of Compound (95) can be obtained by referring to the case of the reactive derivative of Compound (24).

The amidation reaction can be performed in the same condition as the synthesis condition of Compound (10).

Compound (97) can be obtained by performing the Suzuki reaction on Compound (96) using Compound (22) or Compound (23) . The Suzuki reaction can be performed in the same conditions as those of the synthesis of Compound (26).

The conversion from Compound (97) to Compound (98) can be performed by deprotection of a hydroxide group.

The conversion from Compound (98) to Compound (99) is achieved by introducing a leaving group.

There is a method such as methanesulfonylation and halogenation as the introduction of a leaving group. However, it can be performed by referring to the method of introducing a leaving group in the synthesis of Compound (20).

Compound (100) can be obtained by aminating Compound (99) using Compound (80). The amination reaction can be performed in the same condition as those of the synthesis of Compound (10) .

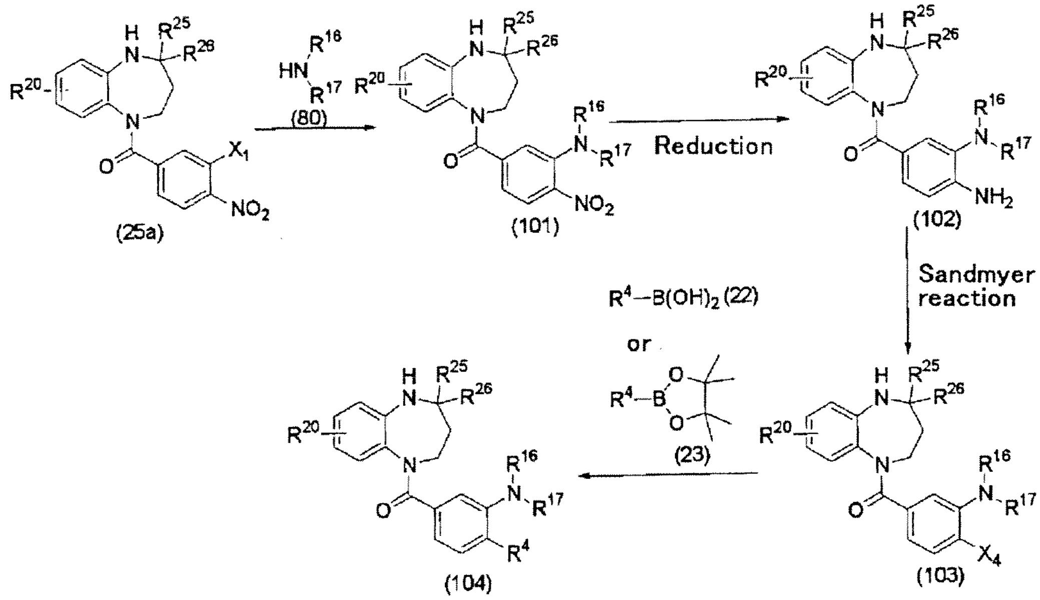

(25a) (101) (102) (103) (104)

(In the Formula, $R^4$, $R^{16}$, $R^{17}$, $R^{20}$, $R^{25}$, $R^{26}$, $X^1$, and $X^4$ are the same as described above.)

The amination reaction from Compound (25a) to Compound (101) can be achieved with the same method as the synthesis of Compound (8). The reduction reaction from Compound (101) to Compound (102) can be achieved with the same method as those of the synthesis of Compound (4). The conversion from Compound (102) to Compound (103) can be performed for example in the condition of the Sandmyer reaction (referred to in Merck Index 13th Edition ONR-93 and the references in the document). The Suzuki reaction from Compound (103) to Compound (104) can be achieved with the same method as those of the synthesis of Compound (26).

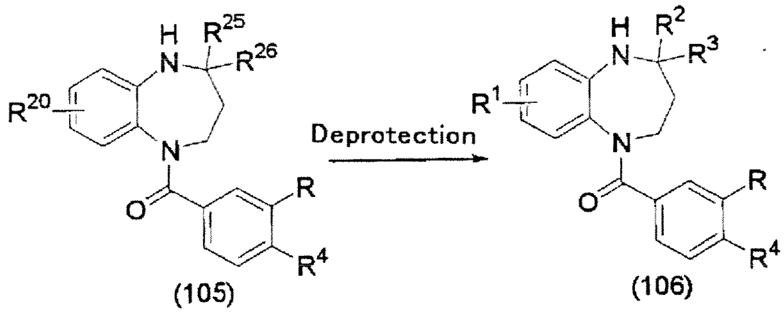

(105) (106)

(In the Formula, $R^1$, $R^2$, $R^3$, $R^4$, $R^{20}$, $R^{25}$, $R^{26}$, and R are the same as described above.)

In the case that Compound (105) has a protective group, Compound (106) can be synthesized by appropriately performing the operation of deprotection.

The medicine in the present invention can be prepared with the method that is normally used using one or more kinds of the compounds in the present invention shown in Formula (1) and a substance for medicine, an excipient and other additives normally used in pharmaceutical preparation. Administration may be any forms of an oral administration such as a tablet, a pill, a capsule, granule, powder, and liquid, and a non-oral administration, for example injection such as intravenous injection and intramuscular injection, suppository administration, nasal administration, transmucosal administration, and transdermal administration.

A tablet, powder, granule, etc. are used as components of the solid composition for oral administration in the present invention. In such solid composition, one or more kinds of active substances are mixed with at least one kind of inert diluents, for example lactose, mannitol, glucose, hydroxypropyl cellulose, microcrystalline cellulose, starch, polyvinylpyrrolidone, and magnesium metasilicate aluminate, for example. The composition may include additives other than the inert diluents, for example a lubricant such as magnesium stearate, a disintegrating agent such as fibrin calcium gluconate, a stabilizer such as lactose, and a solubilizing agent such as glutamic acid and aspartic acid, etc. A tablet or

a pill may be coated with a sugar-coating of sucrose, gelatin, hydroxypropyl cellulose, hydroxypropyl cellulose phthalate, etc. or a stomach soluble or an enteric film.

A liquid composition for oral administration contains an emulsifier, a solvent agent, a suspending agent, syrup, an elixir agent, etc. which are medicinally acceptable, and contains inert diluents used generally, for example purified water and ethanol. This composition may contain, in addition to the inert diluents, an adjuvant such as a wetting agent and a suspending agent, a sweetening gent, a flavoring agent, a fragrance, and a preservative.

The injection for non-oral administration contains an aseptic aqueous or non-aqueous solvent agent, suspending agent, and emulsifier. Examples of the aqueous solvent agent and suspending agent are distilled water for injection and a saline solution. Examples of the non-aqueous solvent agent and suspending agent include propylene glycol, polyethylene glycol, and vegetable oils such as olive oil, alcohols such as ethanol, and polysorbate-80, for instance. Furthermore, such a composition may contain a preservative, a wetting agent, an emulsifier, a dispersing agent, a stabilizer such as lactose, and a solubilizing agent such as glutamic acid and aspartic acid. These are sterilized by filtration through a bacteria-reserving filter, compounding of a sterilizing agent, or irradiation. Alternatively, an aseptic solid composition can be made at first, and then the composition can be dissolved into aseptic water or aseptic solvent for injection before use.

In the case of oral administration, the administration amount per day is normally about 0.0001 to 50 mg/kg, preferably appropriately about 0.001 to 10 mg/kg, further preferably appropriately 0.01 to 1 mg/kg, relative to the body weight, and it is administrated in one portion or by dividing into two to four portions. In the case of the intravenous injection, the administration amount per day is about 0.0001 to 1 mg/kg, preferably appropriately about 0.0001 to 0.1 mg/kg, relative to the body weight, and it is administrated in one portion or by dividing into a plurality of portions per day. The administration amount is determined appropriately by considering symptom, age, and sex and depending on individual cases. However, the administration amount is different among various conditions and there is a case that a smaller amount than the above-described amount is sufficient.

(Effect of the invention)

The compound and its salts in the present invention have a superior affinity to arginine vasopressin V1a receptor and V2 receptor, especially to an arginine vasopressin V1a receptor. That is, the compound and its salts in the present invention contain both antagonist for arginine vasopressin V1a receptor and V2 receptor, and arginine vasopressin V1a receptor-selective antagonist. The compound in the present invention for example has vasodilating action, hypotensive action, hepatic glucose release inhibiting action, mesangial cells proliferation inhibiting action, water diuretic action, platelet aggregation inhibiting action, vomit inhibiting action, urea excretion promoting reaction, the VIII factor secretion inhibiting reaction, cardiac function promoting reaction, mesangial cells contraction inhibiting reaction, hepatic gluconeogenesis inhibiting action, aldosterone secretion inhibiting action, endothelin production inhibiting action, renin secretion adjusting action, memory adjusting action, body temperature adjusting action, prostaglandin production adjusting action, etc., and therefore are useful as a vasodilating agent, a hypotensor, a water diuretic agent, a platelet aggregation inhibitor, a urea excretion promoter, an anti-cardiac failure agent, an anti-renal failure agent, etc., and is useful in prevention and treatment of hypertension, edema, brain edema, ascites, cardiac insufficiency, renal dysfunction, abnormal vasopressin secretion syndorome (SIADH), hepatocirrhosis, hyponatremia, hypokalemia, diabetes, circulatory failure, oscillation disease, water metabolism disorder, renal disease (nephritic syndrome, nephritis, diabetic nephropathy, chronic or acute renal failure), gastric ulcer, nausea, vomiting, various isochemic diseases such as faint, glomerulosclerosis, etc.

BEST MODE FOR CARRYING OUT THE INVENTION

Reference Example 1

o-Phenylenediamine (5 g, 43.9 mmol) and crotonic acid (3.8 g, 44.1 mmol) was stirred at 150˚C for 7 hours. Distilled water (50 mL) and acetone (20 mL) were added to the reaction solution after cooling, and the precipitated solid was collected by filtration and dried under reduced pressure, to obtain 4-methyl-1,3,4,5-tetrahydro-2H-1,5-benzodiazepin-2-one (3.42 g, 44.2%) as a white powder.
$^1$H-NMR($CDCl_3$)δ:1.33(3H,d,J=6.6Hz),2.44(1H,dd,J=13.9,7.3Hz), 2.65(1H,dd,J=13.9,4.4Hz),3.15-3.90(1H,br), 3.97-4.09(1H,m), 6.78(1H,d,J=8.1Hz),6.86-6.91(2H,m),6.97-7.04(1H,m),7.76 (1H,brs)ppm
FABMS:177(M+1)

Reference Example 2

A methylene chloride solution (10 mL) containing benzoyl chloride (1.13 g, 8.01 mmol) was added dropwise to a methylene chloride solution (40 mL) containing the compound in Reference Example 1 (1.0 g, 6.16 mmol) and

pyridine (0.73 g, 9.25 mmol) on an ice bath. The reaction solution was stirred at room temperature for 12 hours. After the reaction was completed, the reaction solution was concentrated under reduced pressure, and the residue obtained was poured into a sodium hydrogen carbonate solution and extracted with ethyl acetate. The organic layer was washed with saturated aqueous sodium chloride solution, dried over anhydrous magnesium sulfate, and concentrated under reduced pressure. The residue obtained was purified by silica gel column chromatography (chloroform/methanol, 248: 2), to obtain 5-benzoyl-4-methyl-1,3,4,5-tetrahydro-2H-1,5-benzodiazepin-2-one (1.52 g, 87.7%) as a brown oil. $^1$H-NMR($CDCl_3$)δ:1.34(3H,d,J=6.6Hz),2.41-2.62(2H,m),5.34 (1H,br),6.71-6.74(1H,m),6.88-6.93(1H,m),7.08-7.22(7H,m), 7.93(1H,brs)ppm
FABMS:281 (M+1)

Reference Example 3

The compound in Reference Example 2 (1.51 g, 5.39 mmol) was added to a tetrahydrofuran solution (40 mL) containing lithium aluminum hydride (1.61 g, 42.4 mmol), and the mixture was heated under reflux for 2.0 hours. Water was added to the reaction solution while the mixture was cooled with water. The mixture was stirred at room temperature for 15 hours and then filtered through Celite. The filtrate obtained was diluted with ethyl acetate. The ethyl acetate layer was washed with water, dried over anhydrous sodium sulfate, and concentrated under reduced pressure. The residue obtained was purified by silica gel column chromatography (chloroform/ethyl acetate, 240:10), to obtain 1-benzyl-2-methyl-2-methyl-2,3,4,5-tetrahydro-1H-1,5-benzodiazepine (0.86 g, 63.2%) as a brown oil.
$^1$H-NMR ($CDCl_3$) δ:0.96 (3H, d J=6.6Hz),1.54-1.66(1H,m),1.85-1.97(1H,m),3.02-3.11(1H,m), 3.25-3.35(1H,m), 3.41-3.450(1H,m),3.74(1H,br),4.19(2H,m), 6.62(1H,dd,J=7.3,2.0Hz),6.72-6.85(2H,m),6.91 (1H,dd,J=7.3,1.5Hz),7.20-7.35(3H,m),7.44-7.44(2H,m)ppm
FABMS:253(M+1)

Reference Example 4

The compound in Reference Example 1 (2 g, 11.4 mmol) was added to a tetrahydrofuran solution (50 mL) containing lithium aluminum hydride (1 g, 26.35 mmol), and the mixture was heated under reflux for 16 hours. Water was added to the reaction solution while the mixture was cooled with water. The mixture was stirred at room temperature for 15 hours and then filtered through Celite. The filtrate obtained was diluted with ethyl acetate. The ethyl acetate layer was washed with water, dried over anhydrous sodium sulfate, and concentrated under reduced pressure. The residue obtained was purified by silica gel column chromatography (chloroform/ethyl acetate, 198:2), to obtain 2-methyl-2,3,4,5-tetrahydro-1H-1,5-benzodiazepine (1.61 g, 87.4%) as a brown oil.
$^1$H-NMR(CDCl3)δ:12.9(3H,d,J=6.6Hz),1.50-1.68(1H,m),1.76-1.88 (1H,m),2.70-2.82(1H,m),2.91-3.05(1H,m),3.15-4.05 (3H,m), 6.77-6.80(4H,m)ppm
FABMS : 163 (M + 1)

Reference Example 5

(+) -α-Bromo-D-camphor-8-sulfonic acid (24.65 g, 74 mmol) was added to an ethanol solution (120 mL) containing the compound in Reference Example 1 (13.18 g, 74 mmol), and the mixture was heated under reflux until it became a homogeneous solution. The mixture was then cooled gradually to room temperature, and the resulting crude crystals were collected by filtration. The crude crystals were recrystallized (ethanol) twice, to obtain a salt. Methylene chloride was added to an aqueous ammonia suspension (50 mL) of the salt, until the solvent is mixed uniformly. The organic layer was concentrated under reduced pressure, to obtain
(4S)-4-methyl-1,3,4,5-tetrahydroxy-2H-1,5-benzodiazepin-2-one (3 g, 45%) as a colorless solid.
(2S)-2-methyl-2,3,4,5-tetrahydro-1H-1,5-benzodiazepine was obtained by the same procedure as in Reference Example 4 by using the compound thereof. (yield 88.8%)
$^1$H-NMR($CDCl_3$)δ:1.30(3H,d,J=6.35Hz),1.52-1.66 1H,m), 1.77-1.86(1H,m),2.70-2.80(1H,m),2.91-3.02(1H,m),3.32-3.41 (1H,m),3.63(1H,br),6.69-6.78(4H,m)ppm
FABMS:163(M+1)

Reference Example 6

(2R)-2-Methyl-2,3,4,5-tetrahydro-1H-1,5-benzodiazepine was obtained by the same procedure as in Reference Example 5 using the compounds in Reference Example 1 and (-)-α-bromo-L-camphor-8-sulfonic acid. (yield 85.4%)
$^1$H-NMR($CDCl_3$)δ:1.30(3H,d,J=6.4Hz),1.52-1.66(1H,ml),1.77-1.86 (1H,m),2.70-2.80(1H,m),2.91-3.02(1H,m),3.32-3.41

(1H,m),3.63 (1H,br),6.69-6.78(4H,m)ppm
FABMS:163(M+1)

Reference Example 7

A mixture of o-phenylenediamine (6 g, 55.4 mmol) and 3-methylcrotonic acid (7 g, 55.4 mmol) was heated at 180°C for 4 hours. Chloroform was added while the mixture was hot. The precipitated crystals were collected by filtration and dissolved in methanol; the methanol solution was subjected to activated carbon treatment; and the filtrate was concentrated. The crystals obtained were washed with a small amount of methanol, to obtain 4,4-dimethyl-1,3,4,5-tetrahydro-2H-1,5-benzodiazepin-2-one (2.17 g, 41.2%) as a pale yellow oil.
$^1$H-NMR(DMSO-$d_6$)δ:1.23(6H,s),2.19(2H,s),4.80(1H,s), 6.76-6.95(4H,m),9.43(1H,brs)ppm
FABMS:190(M+1)

Reference Example 8

The compound in Reference Example 7 (2 g, 10.52 mmol) was added to a tetrahydrofuran solution (50 mL) containing lithium aluminum hydride (1 g, 26.35 mmol), and the mixture was heated under reflux for 3 hours. Water was added to the reaction solution while the mixture was cooled with water. The mixture was stirred at room temperature for 15 hours and then filtered through Celite. The filtrate obtained was diluted with ethyl acetate. . Ethyl acetate layer was washed with water, dried over anhydrous sodium sulfate, and concentrated under reduced pressure. The residue obtained was purified by silica gel column chromatography (hexane/ethyl acetate, 7:3), to obtain 2,2-dimethyl-2,3,4,5-tetrahydro-1H-1,5-benzodiazepine (1.43 g, 77.2%) as brown crystals.
$^1$H-NMR($CDCl_3$) δ:1.22(6H,s),1.77(2H,d,J=2.9Hz),3.09 (2H,d,J=2.9Hz),6.63-6.82(4H,m)ppm
FABMS:176(M+1)

Reference Example 9

4-Ethyl-1,3,4,5-tetrahydro-2H-1,5-benzodiazepin-2-one (4.48 g, 46.7%) was prepared as pale brown crystals in the same procedure to Reference Example 1 by using o-phenylenediamine (5.73 g, 50.4 mmol) and trans-2-pentenoic acid (6.55 g, 65.5 mmol).
$^1$H-NMR($CDCl_3$)δ:1.01(3H, t, J=7.3Hz), 1.65 (2H, quint., J=7.3Hz), 2.41-2.70(2H,m),3.40-4.15(2H,m),6.75-6.84(1H,m), 6.85-6.94 (2H,m),6.97-7.05(1H,m),7.99(1H,brs)ppm
FABMS:190(M+1)

Reference Example 10

2-Ethyl-2,3,4,5-tetrahydro-1H-1,5-benzodiazepine was prepared as pale brown crystals in the same procedure to Reference Example 3 using the compounds in Reference Example 9. (yield 80.7%)
$^1$H-NMR($CDCl_3$)δ:1.03(3H,t,J=7.3Hz),4.54-1.67(2H,m),1.82-1.93 (1H,m),2.71-2.84(2H,m),3.25-3.95(2H,m),6.66-6.79 (4H,m)ppm
FABMS:176(M+1)

Reference Example 11

A mixture of o-phenylenediamine (4.67 g, 42.0 mmol) and (2E)-4-methyl-2-pentenoic acid (6.09 g, 53.3 mmol) was heated under reflux at 120°C for 8 hours. The reaction solution was cooled and purified by silica gel column chromatography (chloroform), to obtain 4-isopropyl-1,3,4,5-tetrahydro-2H-1,5-benzodiazepine (4.08 g, 48.7%) as pale brown crystals.
$^1$H-NMR($CDCl_3$) δ:1.03(6H,d,J=6.8Hz),1.57-1.92(2H,m),2.62-2.82 (2H,m),3.67-3.86(3H,m),6.63-6.79(4H,m)ppm
FABMS:190(M+1)

Reference Example 12

2-Isopropyl-2,3,4,5-tetrahydro-1H-1,5-benzodiazepine was prepared as a pale brown oil in the same procedure to Reference Example 3 using the compounds in Reference Example 11. (yield 90.4%)
$^1$H-NMR($CDCl_3$)δ:2.40 (3H,s), 2.84-2.90 (2H,m), 3.11-3.16 (2H,m), 3.21-3.98 (1H,br), 3.70 (2H,s), 6.73 (1H, d, J=7.3Hz), 6.83 (1H, dt, J=1.5,7.3Hz), 7.04-7.14 (2H,m), 7.29-7.55 (7H,m), 7.89 (1H, d, J =8.9Hz), 7.94 (1H,s)ppm
FABMS:163(M+1)

Reference Example 13

1.1 M borane-tetrahydrofuran solution (60 mL, 66 mmol) was added dropwise gradually to a tetrahydrofuran solution (40 mL) containing monoethyl malonate (8.5 g, 56 mmol) on an ice bath. The mixture was stirred at room temperature for 15 hours, 50% aqueous acetic acid solution (1.6 mL) was added gradually thereto, for decomposition of excess borane, and stirred at room temperature additionally for 30 minutes. The reaction solution was concentrated under reduced pressure; the residue obtained was dissolved in ethyl acetate; and the solution was washed with saturated sodium hydrogen carbonate solution and then with saline solution. The organic layer was dried over anhydrous sodium sulfate and concentrated under reduced pressure, and the residue obtained was purified by column chromatography (hexane/ethyl acetate, 4:1 to 3:1), to obtain ethyl (2E)-4-hydroxy-2-butenylate (3.79 g, 52.0%) as a colorless oil.
$^1$H-NMR($CDCl_3$)δ:1.30(3H,t,J=6.8Hz),1.60(1H,s),4.21 (2H,q,J=6.8Hz),4.31-4.40(2H,m),6.11(1H,dt,J=15.7,2.0Hz), 7.13 (1H,d,J=15.7,3.9Hz) ppm
FABMS: 131(M+1)

Reference Example 14

Diphenyl-tert-butylsilyl chloride (8.7g, 31.7 mmol) was added gradually to an N,N-dimethylformamide solution (15 mL) containing the compound in Reference Example 13 (3.79 g, 29.1 mmol) and imidazole (4.36 g, 64.0 mmol) while the solution was water-cooled and the mixture was stirred at room temperature for 3 hours. The reaction solution was added with ice and extracted with ethyl acetate. The organic layer was washed with saturated aqueous citric acid solution and then with water . The organic layer was dried over anhydrous sodium sulfate and concentrated under reduced pressure, and the residue obtained was purified by column chromatography (hexane/ethyl acetate, 495:5 to 490:10), to obtain ethyl
(2E)-4-[[tert-butyl(diphenyl)silyl)oxy]-2-butenate (9.4 g, 87.7%) as a colorless oil.
$^1$H-NMR($CDCl_3$) δ:1.08(9H,s),1.32(3H,t,J=6.8Hz),4.22 (2H,q,J=6.8Hz),4.34(2H,t,J=2.9Hz),6.22-6.33(1H,m), 6.91-7.04 (1H,m),7.34-7.48(6H,m),7.61-7.72(4H,m)ppm
FABMS:369(M+1)

Reference Example 15

Aqueous 0.5N potassium hydroxide solution (46 mL, 23 mmol) was added to a methanol solution (100 mL) containing the compound in Reference Example 14 (7.74 g, 21.0 mmol), and the solution was stirred at room temperature for 20 hours. The reaction solution was concentrated under reduced pressure; the residue obtained was diluted with ethyl acetate; and the solution was washed with saturated aqueous citric acid solution and then with water. The organic layer was dried over anhydrous sodium sulfate and concentrated under reduced pressure, to obtain (2E)-4-[[tert-butyl (diphenyl)silyl)oxy]-2-butenoic acid (6.61 g, 92.5%) as a colorless oil. $^1$H-NMR($CDCl_3$)δ:1.09(9H,s),4.38-4.40(2H,m), 6.49 (1H,d,J=14.7Hz),7.13(1H,dt,J=15.6,2.9Hz),7.33-7.55(1H,m), 7.58-7.72(1H,m),8.07-8.18(1H,m),8.73-12.95(1H,br) ppm

Reference Example 16

4-[[[tert-Butyl(diphenyl)silyl]oxy]methyl] 1,3,4,5-tetrahydro-2H-1,5-benzodiazepin-2-one was prepared as a pale yellow oil in the same procedure to Reference Example 1 by using o-phenylenediamine and the compound in Reference Example 15. (yield 38.7%)
$^1$H-NMR($CDCl_3$) δ:1.10(9H,s) ,2.37-2.46(2H,m), 3.53-3.68(2H,m), 3.93-4.07(1H,m),6.77(1H,d,J=7.8Hz),6.91-(2H,d, J=3.9Hz), 6.98-7.07(1H,m),7.34-7.49(6H,m),7.61-7.71(1H,m),8.00 (1H,brs),8.11(1H,d,J=6.8Hz)ppm
FABMS:430(M+1)

Reference Example 17

2-[[[tert-Butyl(diphenyl)silyl]oxy]methyl]-2,3,4,5-tetrahydro-1H-1,5-benzodiazepine was prepared as a pale brown oil in the same procedure to Reference Example 3 using the compounds in Reference Example 16. (yield 51.0%)
*$^1$H-NMR($CDCl_3$) δ:1.11(9H,s), 1.52-1.71(2H,m), 2.63-2.77 (1H,m) , 2.86-2.99(1H,m),3.30-3.40(1H,m),3.49-3.79(2H, m),4.05-4.96 (1H,br),6.71-6.86(4H,m),7.33-7.47(6H,m),7.64-7.73(4H,m)ppm
FABMS:417(M+1)

Reference Example 18

A mixture of 80% sulfuric acid (50.5 g) , D, L-aspartic acid (50 g) , and benzylalcohol (125 g) was stirred under heat at 70˚C for 6 hours. The mixture cooled to room temperature was added gradually to a mixed solution of 28% sodium hydrogen carbonate solution (250 mL) and ether (150 mL) while the mixture was stirred mechanically. After stirring for one hour, the precipitated crystals were collected by filtration and dried in air. The crude crystals were recrystallized from water (700 mL), to obtain o-benzyl-4-oxohomoserine (17 g, 20%) as colorless crystals. $^1$H-NMR (DMSO-$d_6$) δ : 2.61 (1H, dd, J=16.6, 7.8Hz), 2.91 (1H, dd, J=16.6, 5.0Hz), 3.49 (1H, dd, J=7.8, 5.0Hz), 5.10 (2H,s), 7.3-7.4(5H,m)ppm

Reference Example 19

A mixture of the compound in Reference Example 18 (10 g, 44.8 mmol), 2-fluoronitrobenzene (6.31 g, 44.8 mol), triethylamine (9.95 g, 98.6 mmol) and acetonitrile (50 mL) was stirred while heated under reflux for 24 hours. After removal of the solvent under reduced pressure, the residue was acidified with hydrochloric acid and extracted with ethyl acetate. The ethyl acetate layer was dried over anhydrous magnesium sulfate and concentrated under reduced pressure. The residue obtained was purified by silica gel column chromatography (hexane/ethyl acetate, 1:1), to obtain 4-(benzyloxy)-2-(2-nitroanilino)-4-oxobutanoic acid (11.1g, 69%) as a yellow solid.
$^1$H-NMR($CDCl_3$)δ:3.06(2H,d, J=5.3Hz),4.70-4.74(1H,m),5.17 (2H,ABq,J=12.2Hz),6.76(1H,t,J=8.3Hz),6.84(1H,d, J=8.3Hz), 7.2-7.4(5H,m),7.45(1H,t,J=8.3Hz),8.20(1H,d,J=8.3Hz),8.49 (1H,d,J=8.3Hz)ppm

Reference Example 20

Dimethylamine (15 mL, 2 M tetrahydrofuran solution), N,N-dicyclohexylcarbodiimide (6.18 g, 30 mmol), and HOBt (4.59 g, 30 mmol) were added in series to a tetrahydrofuran solution (100 mL) containing the compound in Reference Example 19 (10.4 g, 30 mmol), and the mixture was stirred at room temperature for 2 hours. The precipitate was collected by filtration and washed with ethyl acetate, and the filtrate and the washing solution were combined and concentrated under reduced pressure. The residue obtained was dissolved in ethyl acetate, and the solution was washed with aqueous sodium bicarbonate, with dilute hydrochloric acid, and with water successively, and then dried. After concentration under reduced pressure, the residue obtained was purified by silica gel column chromatography (hexane/ethyl acetate, 1:1 to 1:2), to obtain benzyl 4-(dimethylamino)-3-(2-nitroanilino)-4-oxobutanoate (10 g, 90%) as a yellow solid. $^1$H-NMR($CDCl_3$)δ:2.79(dd,1H,J=16.0,5.6Hz),2.98(s,3H),3.06 (dd,1H,J=16.0,7.3Hz),3.07(s,3H),4.95-5.05(m, 1H),5.13 (ABq,2H,J=12.2),6.71(t,1H,J=8.3Hz),6.90(d,1H,J=8.3Hz), 7.3-7.46(m,6H),8.19(d,1H,J=8.3Hz),8.39(d,1H, J=8.8)ppm

Reference Example 21

The compound in Reference Example 20 in methanol (110 mL) was reduced catalytically in the presence of 5% palladium carbon (4 g) at room temperature under atmospheric pressure. After removal of the catalyst by filtration, , methanol was removed under reduced pressure, and tetrahydrofuran (100 mL) was added to the residue . N,N-Dicyclohexylcarbodiimide (5.54 g) and HOBt (4.11 g) were added in series to the solution obtained on an ice bath, and the mixture was stirred at room temperature for 15 hours. The precipitate was collected by filtration and washed with ethyl acetate. The washing solution and the filtrate were combined and concentrated under reduced pressure. The residue obtained was purified by silica gel column chromatography (chloroform/methanol, 100:3), to obtain N,N-dimethyl-4-oxo-2,3,4,5-tetrahydro-1H-1,5-benzodiazepine-2-carboxamide (2.7 g, 43%).
$^1$H-NMR(DMSO-$d_6$)δ:2.33-2.44(1H,m),2.58(1H,dd,J=13.6,10.3Hz), 2.83(3Hs),3.05 (3H,s),4.63 (1H,dt,J=10.3,3.4Hz), 5.42(1H,s), 6.68-6.93(4H,m),9.55(1H,s)ppm

Reference Example 22

N,N-dimethyl(2,3,4,5-tetrahydro-1H-1,5-benzodiazepin-2-yl) methanamine was obtained as a pale brown oil in the same procedure to Reference Example 3 using the compounds in Reference Example 21. (yield 80%)
$^1$H-NMR ($CDCl_3$) δ:1.41-1.55 (1H, m), 1.64-1.72 (1H, m) ,2.14 (1H, dd, J=12.2, 3.4 Hz), 2.26 (6H, s), 2.51 (1H, t, J=12.2 Hz), 2.67-2.78(1H,m),3.37-3.44(1H,m),6.70-6.85(4H,m)ppm

Reference Example 23

An N,N-dimethylformamide solution (10 mL) containing benzyl bromide (4.54 mL, 38.2 mmol) was added

dropwise to an N,N-dimethylformamide solution (50 mL) containing 3-fluoro-4-nitrophenol (5 g, 31.8 mol) and potassium carbonate (6.6 g, 47.7 mmol) under a nitrogen stream at room temperature. After stirring for 3 hours at room temperature, the reaction mixture was poured into saturated aqueous sodium chloride solution and extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 4:1), to obtain 4-benzyloxy-2-fluoro-1-nitrobenzene (7.43 g, 94.5%) as a pale yellow solid.
$^1$H-NMR($CDCl_3$)δ:5.14(2H,s),6.78-6.86(2H,m),7.37-7.42(5H,m), 8.06-8.13(1H,m)ppm
FABMS:248(M+1)

Reference Example 24

An aqueous solution (30 mL) containing the compound in Reference Example 23 (6.71g, 27.2 mmol), DL-3-amino-n-butyric acid (4.21 g, 40.8 mmol), and sodium bicarbonate (4.57 g, 54.4 mmol) was stirred under reflux for 2 days. Then, the reaction mixture was poured into ethyl acetate and extracted with saturated sodium hydrogen carbonate solution. 1N hydrochloric acid aqueous solution was added to the aqueous phase until the aqueous phase became acidic, and the aqueous phase was extracted with ethyl acetate. The organic layer was washed with saturated aqueous sodium chloride solution, dried over anhydrous magnesium sulfate, and concentrated under reduced pressure, to obtain 3-(5-benzyloxy-2-nitroanilino)butanoic acid (8.07 g, 89.8%).
$^1$H-NMR($CDCl_3$)δ:1.39(3H,d,J=6.4Hz),2.52(1H,dd,J=15.6,7.3Hz), 2.73(1H,dd,J=15.6,5.1Hz),4.01-4.11(1H,m),5.13 (2H,s), 6.29-6.35(2H,m),7.31-7.43(5H,m),8.15(1H,d,J=9.3Hz),8.32 (1H,d J=7.8Hz)ppm
FABMS:331(M+1)

Reference Example 25

A conc. hydrochloric acid solution (2 mL) containing stannous chloride (948 mg, 5.0 mmol) was added to a tetrahydrofuran solution (10 mL) containing the compound in Reference Example 24 (330 mg, 1.0 mmol) on an ice bath. Then, the mixture was stirred under reflux for a day. The reaction mixture was concentrated under reduced pressure, and the residue obtained was dissolved with ethyl acetate and poured into saturated aqueous sodium chloride solution. The organic layer was washed with saturated aqueous sodium bicarbonate and then with saturated aqueous sodium chloride solution. The organic layer was dried over anhydrous magnesium sulfate and then concentrated under reduced pressure. The residue obtained was purified by silica gel column chromatography (hexane/ethyl acetate, 1:4), to obtain 7-benzyloxy-4-methyl-1,3,4,5-tetrahydro-2H-1,5-benzodiazepin-2-one (178 mg, 63%) as a colorless solid. $^1$H-NMR (DMSO-$d_6$) δ:1.27 (3H,d,J=6.4Hz), 2.28 (1H,dd,J=13.2,7.3Hz),2.51(1H,dd,J=13.2,3.9Hz),3.88-3.92 (1H,m),5.08(2H,s), 5.42(1H,d,J=2.4Hz),6.48 (1H,dd,J=8.8,2.9Hz),6.61(1H,d,J=2.9Hz),6.85(1H,d,J=8.3Hz), 7.39-7.54(5H,m),9.37(1H,s) ppm
FABMS:283 (M+1)

Reference Example 26

Benzyl 4-methyl-2,3,4,5-tetrahydro-1H-1,5-benzodiazepin-7-yl ether was obtained by the same procedure as in Reference Example 3 using the compounds in Reference Example 25. (yield 84.7%)
$^1$H-NMR ($CDCl_3$) δ:1.28 (3H, d, J=6 . 4Hz) , 1.52-1.66 (1H,m) , 1.75-1.84 (1H,m) , 2.64-2.74 (1H,m) ,2.92-2.99 (1H,m), 3.26-3.34 (1H,m),3.47 (2H,br), 4.97 (2H,s), 6.38-6.43 (2H,m), 6.61-6.65 (1H,m), 7.26-7.43(5H,m)ppm
FABMS:269(M+1)

Reference Example 27

3-(5-Methyl-2-nitroanilino)butanoic acid was obtained by the same procedure as in Reference Example 24 by using 3-fluoro-4-nitrotoluene and DL-3-amino-n-butyric acid.
(yield 80.8%)
$^1$H-NMR(DMSO-$d_6$)δ:1.29(3H,d,J=6.4Hz)2.32(3H,s)2.60-2.62 (2H,m),4.12-4.22(1H,m),6.S2(1H,dd,J=8.8,1.SHz),6.92 (1.H,s), 7.96(1H,d,J=8.8Hz),8.22(1H,d,J=8.8Hz)ppm
FABMS:239(M+1)

Reference Example 28

4,7-Dimethyl-1,3,4,5-tetrahydro-2H-1,5-benzodiazepin-2-one was obtained by the same procedure as in Reference Example 25 using the compounds in Reference Example 27. (yield 32.7%)

$^1$H-NMR($CDCl_3$)δ:1.32(3H,d,J=6.4Hz),2.26(3H,s),2.38-2.46 ($CDCl_3$)δ:1.32 (3H ,d,J=6.4Hz),2.26(3H ,s), 2.38-2.46 ($^1$H, m), 2.58-2.65 (1H,m), 3.96-4.05 (1H,m:), 6.60-6.80 (3H, m), 7.89(1H,br)ppm
FABMS:191(M+1)

Reference Example 29

2,8-Dimethyl-2,3,4,5-tetrahydro-1H-1,5-benzodiazepine was obtained by the same procedure as in Reference Example 3 using the compounds in Reference Example 28. (yield 81.5%)
$^1$H-NMR($CDCl_3$) δ : 1.28 (3H, d, J = 6.4Hz) 1.52-1.66 (1H,m), 1.75-1.84 (1H,m), 2.66-2.75 (1H,m), 2.87-2.99 (1H,m), 3.29-3.37(1H,m), 3.56 (2H,br),6.56-6.63(3H,m)ppm
FABMS:177(M+1)

Reference Example 30

A tetrahydrofuran solution (20 mL) containing tosyl chloride (6.29 g, 33.0 mmol) was added dropwise to a tetrahydrofuran solution (50 mL) containing 4-fluoro-3-nitroaniline (4. 68 g, 30.0 mmol) and pyridine (3.64 mL, 45.0 mmol) on an ice bath. The reaction solution was stirred at room temperature for 18 hours and concentrated under reduced pressure. The residue obtained was poured into saturated aqueous sodium chloride solution and extracted with ethyl acetate. The organic layer was washed with saturated aqueous sodium chloride solution, dried over anhydrous magnesium sulfate, and concentrated under reduced pressure. The residue obtained was purified by silica gel chromatography (hexane/ethyl acetate, 3:2), to obtain N-(4-fluoro-3-nitrophenyl)-4-methylbenzenesulfonamide (7.74 g, 83.1%) as a colorless solid.
$^1$H-NMR($CDCl_3$)δ:2.41(3H,s),7.04(1H,br),7.17-7.24(1H,m),7.29 (2H,d,J=7.8Hz),7.40-7.46(1H,m),7.68(2H,d,J=8.3Hz), 7.73 (1H,dd,J=6.4,2.9Hz)ppm
FABMS:311(M+1)

Reference Example 31

3-[4-[[(4-Methylphenyl)sulfonyl]amino]-2-nitroanilino] butanoic acid was obtained by the same procedure as in Reference Example 24 using the compounds in Reference Example 30 and DL-3-amino-n-butyric acid.

Reference Example 32

The compound in Reference Example 31 (8.83 g, 21.3 mmol) in methanol (150 mL) was reduced catalytically in the presence of 10% palladium carbon (4.0 g) under a hydrogen stream at room temperature for 15 hours. After removal of the catalyst by filtration, the solution was concentrated under reduced pressure. The residue obtained was used without purification in the next reaction. A toluene solution (200 mL) containing the crude product was stirred as heated under reflux for 8 hours and then concentrated under reduced pressure. The residue obtained was poured into saturated aqueous sodium chloride solution and extracted with ethyl acetate. The organic layer was washed with saturated aqueous sodium chloride solution, dried over anhydrous magnesium sulfate, and concentrated under reduced pressure. The residue obtained was purified by recrystallization (ethyl acetate and chloroform), to obtain 4-methyl-N-(2-methyl-4-oxo-2,3,4,5-tetrahydro-1H-1,5-benzodiazepzn-7-yl)benzenesulfonamide (1.4 g, 37.8%) as colorless granular crystals.
$^1$H-NMR(DMSO-$d_6$,120˚C)δ:1.15(3H,d,J=5.9Hz), 2.16 (1H, dd, J=13 . 5, 7.1Hz), 2.34(3H, s), 2.40 (1H, dd, J=13.5,4.2Hz), 3.74(1H,br),4.73(1H,s),6.57(1H,dd,J=8.3,2.4Hz),6.66 (1H, d, J=8.3Hz),6.71(1H, d, J=2.4Hz), 7.28(2H, d, J=8.1Hz), 7.60 (2H,d,J=8.1Hz)ppm
FABMS:346(M+1)

Reference Example 33

4-Methyl-N-(2-methyl-2,3,4,5-tetrahydro-1H-1,5-benzodiazepin-7-yl)benzenesulfonamide was obtained by the same procedure as in Reference Example 3 using the compounds in Reference Example 32. (yield 44.1%)
$^1$H-NMR(DMSO-$d_6$,120˚C)δ:1.13 (3H, d, J=6.4Hz),1.27-1.40(1H,m), 1.68-1.78(1H,m),2.34(3H,s), 2.60-2.67(1H,m), 2.90-2.99(1H,m), 3.22(3H,br),4.05(1H,br),4.72(1H,br),6.22 (1H,dd,J=8.3,2.4Hz),6.42(1H,d,J=2.4=4Hz),6.52(1H,d, J=8.3Hz), 7.27(1H,d,J=8.3Hz),7.58(1H,d,J=8.3Hz),8.99(1H,br)ppm
FABMS:332(M+1)

Reference Example 34

4-Bromo-3-nitrotoluene (25.3 g, 0.117 mol) was added to a mixed solution of pyridine and water (1:1, 300 mL), and potassium permanganate (36.9 g, 0.234 mol) was added thereto over 5 hours while the solution was stirred under heat at 50˚C. Then, excess potassium permanganate was quenched with methanol. The reaction mixture was concentrated under reduced pressure; the residue obtained was washed with ether and dissolved in water; and aqueous 4 N hydrochloric acid solution was added thereto until the solution becomes acidic, allowing precipitation of solid. The crude product was recrystallized from methanol and water, to obtain 4-bromo-3-nitrobenzoic acid (14.0 g, 48.6%) as pale yellow needle crystals.
$^1$H-NMR($CDCl_3$) δ :7.89(1H,d,J=8.3Hz),B.12(1H,dd,J=8.3,2.0Hz), 8.53(1H,d,J=1.95Hz)ppm
FABMS:247(M+1)

Reference Example 35

The compound in Reference Example 34 (4.94 g, 20.1 mmol) and nickel chloride-hexahydrate (9.56 g, 40.2 mmol) were mixed in methanol (100 mL) and the solution was stirred. Then, sodium borohydride (3.8 g, 0.1 mol) was added gradually, while the solution was cooled on an ice bath to keep the reaction temperature low. Then, the mixture was stirred at room temperature for 30 minutes and concentrated under reduced pressure; the residue was dissolved in ethyl acetate; the solution was filtered through Celite; and the filtrate was washed with saturated aqueous citric acid solution and then saturated aqueous sodium chloride solution. The organic layer was dried over anhydrous magnesium sulfate and concentrated under reduced pressure, and the precipitated crystals were washed with hexane, to obtain 3-amino-4-bromobenzoic acid (2.95 g, 68%) as a colorless solid.
$^1$H-NMR(DYSO-$d_6$),δ:7.01(1H,dd,J=8.3,2.5Hz),7.39 (1H, d, J = 2.5Hz), 7.45 (1H, d, J = 8.3Hz) ppm
FABMS:215(M+1)

Reference Example 36

A conc. hydrochloric acid solution (45 mL) containing stannous chloride (21.6 g, 114 mmol) was added to an ethanol solution (60 mL) containing 4-bromo-3-nitrotoluene (5 g, 23.1 mmol) while the mixture was cooled on an ice bath. The reaction solution was stirred at 60˚C for 0.5 hour. After the reaction was completed, ethanol was evaporated under reduced pressure, and 12 N aqueous sodium hydroxide solution was added to the aqueous solution until pH 12 or more. The aqueous solution was extracted with ether; the organic layer was washed with saturated aqueous sodium chloride solution, dried over anhydrous magnesium sulfate, and concentrated under reduced pressure, to obtain 2-bromo-5-methylaniline (4.21 g, 97.5%) as a pale yellow solid.
$^1$H-NMR ($CDCl_3$) δ: 2.22 (3H, s), 2.73-4.79 (2H,br), 6.44 ($^1$H, dd, J=8.06, 2.2Hz), 6.59 (1H, d, J=1.5Hz), 7.23-7.29(1H, m) ppm
FABMS:187(M+1)

Reference Example 37

Acetic anhydride (16.1 mL, 0.171 mol) was added to a benzene solution (200 mL) containing the compound in Reference Example 36 (21.17 g, 0.114 mol), and the mixture was stirred under reflux for 14 hours. After the reaction was completed, the reaction solution was concentrated under reduced pressure. The residue obtained was recrystallized (from ethyl acetate), to obtain N-(2-bromo-5-methylphenyl)acetamide (20 g, 76.9%) as a colorless solid.
$^1$H-NMR($CDCl_3$) δ:2.23(3H, s), 2.32(3H, s), 6.80(1H,d, J=7.8Hz), 7.39(1H,d,J=8.8Hz), 7.43-7.65 (1H,br),8.17(1H,brs) ppm
FABMS:228(M+)

Reference Example 38

Potassium permanganate (4.74 g, 30.0 mmol) was added to a mixed tert-butyl alcohol and water solution (69/46 mL) containing the compound in Reference Example 37 (2.28 g, 10.0 mmol) while the solution was heated at 60˚C, and the mixture was stirred for 5 hours. After the reaction was completed, most of the reaction solvent was evaporated under reduced pressure, and the residue was filtered through Celite, removing manganese residue. 4 N aqueous hydrochloric acid solution was added to the filtrate until the solution becomes acidic; the precipitated solid was collected by filtration and washed thoroughly with distilled water, to obtain 3-acetylamino-4-bromobenzoic acid (1.27 g, 49.3%) as a colorless solid.
$^1$H-NMR (CDCl3)δ:2.11 (3H, s), 7.63 (1H, dd, J=8 . 8,2.0Hz) , 7.78 (1H, d, J=7.8Hz), 8.16(1H, d, J=2.0Hz), 9.59(1H, s),

12.56-13.85 (1H,br)ppm

Reference Example 39

An aqueous solution (50 mL) containing 3-fluoro-4-nitrotoluene (2.0 g, 12.9 mmol) and potassium permanganate (4.07 g, 25.8 mmol) was heated under reflux for 15 hours. The reaction solution was cooled and then aqueous phase was washed with ethyl acetate. The aqueous phase was acidified with 4 N aqueous hydrochloric acid solution, and the precipitated crystals were collected by filtration. The filtrate was extracted with ethyl acetate five times. The crystals collected by filtration were dissolved in ethyl acetate, and combined with the ethyl acetate extraction solution. The ethyl acetate layer combined was washed with water and then with saturated aqueous sodium chloride solution, dried over anhydrous magnesium sulfate, and concentrated under reduced pressure, to obtain 3-fluoro-4-nitrobenzoic acid (1.07 g, 82.9%) as pale yellow crystals.
$^1$H-NMR($CDCl_3$)δ:8.01-8.06(1H,m) ,8.12-8.18 (1H,m) ppm
FABMS:186(M+1)

Reference Example 40

The compound in Reference Example 37 (0.7 g, 3.07 mmol) was added to a dimethylformamide solution (3 mL) containing 60% sodium hydride (0.16mg, 4.0 mmol), and the mixture was stirred at 50˚C for 1 hour. The reaction solution was cooled to room temperature; methyl iodide (0.5mL) was added thereto; and the mixture was stirred at room temperature for 15 hours. The reaction solution was poured into ice water and extracted with ethyl acetate. The organic layer was washed with salt water and then concentrated under reduced pressure. The residue obtained was purified by silica gel column chromatography (hexane/ethyl acetate, 4:1 to 3:2), to obtain N-(2-bromo-5-methylphenyl)-N-methylacetamide (0.73 g, 96.2%) as a colorless solid.
$^1$H-NMR($CDCl_3$) δ:1.81 (3H, s), 2.35(3H, s), 3.17(3H, s), 7.02-7.12 (2H, m), 7.54(1H, d, J=8.8Hz) ppm
FABMS:242(M+1)

Reference Example 41

3-[Acetyl(methyl)amino]-4-bromobenzoic acid was prepared in the same procedure to Reference Example 38 using the compounds in Reference Example 40. (yield 78.0%)
$^1$H-NMR (CDC13, 40□) δ: 1.84 (3H, s) , 3.22 (3H, s) , 3.7-4. 9 (2H,br), 7.82(1H,d,J=7.8Hz),7.94-8.03(2H,m)ppm
FABMS:272(M+1)

Reference Example 42

An aqueous sodium nitrite solution (9 mL) was added dropwise into a conc. hydrochloric acid solution (50 mL) containing the compound in Reference Example 35 (1.08 g, 5.0 mmol) while the solution was cooled on an ice bath. After stirring for 15 minutes, the solution was made neutral by addition of 4 N aqueous sodium hydroxide solution. Toluene (20 mL) was added to the copper cyanide solution thus prepared, and the reaction solution above was added thereto, while the solution was stirred and cooled on an ice bath. After stirring for 30 minutes, the reaction solution was cooled to room temperature, stirred for 3 hours, and then heated to 50˚C. After stirring for 3 hours, the reaction solution was made neutral by addition of 4 N aqueous hydrochloric acid solution and extracted with ethyl acetate. The organic layer was washed with saturated aqueous sodium chloride solution, dried over anhydrous magnesium sulfate, and concentrated under reduced pressure. The residue obtained was purified by silica gel column chromatography (chloroform/methanol, 95:5), to obtain 4-bromo-3-cyanobenzoic acid (366 mg, 31.7%) as a colorless solid.
$^1$H-NMR($CDCl_3$)δ:2.8S(3H,s),7.60(1H,dd,J=8.3,2.0Hz), 7.66(1H,d,J=8.3Hz),7.79(1H,d,J=2.0Hz)ppm
FABMS:244(M+1)

Reference Example 43

An acetic acid solution (20 mL) of bromine (20 mL, 0.38 mol) was added dropwise to an acetic acid solution (500 mL) containing 3-hydroxybenzoic acid (53 g, 0.384 mol) at room temperature, and the mixture was stirred for 5 days. The reaction mixture was concentrated under reduced pressure, and the residue obtained was extracted with ethyl acetate. The organic layer was washed with saturated aqueous sodium chloride solution, dried over anhydrous magnesium sulfate, and concentrated under reduced pressure. The residue obtained was recrystallized from water, to obtain 4-bromo-3-hydroxybenzoic acid (23.33 g, 28%) as colorless granular crystals.
$^1$H-NMR(DMSO-$d_6$) δ:7.27(1H,dd,J=7.8,2.0Hz)7.52 (1H,d,J=2.0Hz),7.61(1H,d,J=7.8Hz),10.65(1H,brs),13.04 (1H,brs)ppm

Reference Example 44

Conc. sulfuric acid (4.43 g, 45.2 mmol) was added to a methanol solution (74 mL) containing the compound in Reference Example 43 (4. 91 g, 22. 6 mmol), and the mixture was heated under reflux for 6 hours. The reaction solution was concentrated under reduced pressure, added with water, and extracted with ether. The organic layer was washed with water and then with saturated sodium hydrogen carbonate solution, dried over anhydrous magnesium sulfate, and concentrated under reduced pressure. The residue obtained was left at room temperature. The precipitated crystals were washed with hexane, to obtain methyl 4-bromo-3-hydroxybenzoate (4.38 g, 84.0%) as colorless crystals.
$^1$H-NMR($CDCl_3$) δ:3.92(3H,s),5.89(1H,brs),7.44-7.58(2H,m), 7.65-7.73(1H,m)ppm
FABMS:231(M+1)

Reference Example 45

Triphenylphosphine (1.32 g, 4.94 mmol) and 98% 2-bromoethanol (0.62 g, 4.94 mmol) were added to a tetrahydrofuran solution (20 mL) containing the compound in Reference Example 44 (0.51 g, 2.22 mmol), and then, a tetrahydrofuran solution (10 mL) of diethyl azodicarboxylate (0.86 g, 4.94 mmol) was added dropwise thereto, while the mixture was cooled in an ice/salt bath. After stirring at room temperature for 15 hours, the solution was concentrated under reduced pressure, added with water and extracted with ethyl acetate. The organic layer was washed with water, dried over sodium sulfate, and concentrated under reduced pressure. The residue obtained was purified by silica gel column chromatography (hexane/ethyl acetate, 5:1), to obtain methyl 4-bromo-3-(2-bromoethoxy)benzoate (0.67 g, 89.4%) as yellow crystals.
$^1$H-NMR ($CDCl_3$) δ:3.71 (2H, t, J=6.8Hz), 3.92(3H,s), 4.41 (2H,t,J=6.8Hz),7.55(2H,dd,J=2.0,6.8Hz),7.63 (1H,dd, J=2.0,6.8Hz),ppm
FABMS:339(M+1)

Reference Example 46

Aqueous 0. 5 N potassium hydroxide solution (0.76 mL, 0.38 mmol) was added to a methanol solution (5 mL) containing the compound in Reference Example 45 (0.12 g, 0.35 mmol), and the mixture was heated under reflux for 1 hour. The reaction solution was concentrated under reduced pressure, added with water, and extracted with ethyl acetate. The aqueous phase was acidified with 4 N aqueous hydrochloric acid solution and extracted with ethyl aceta te . The organic layer was washed with saline solution, dried over sodium sulfate, and concentrated under reduced pressure, to obtain 4-bromo-3-(2-bromoethoxy)benzole acid (0.11 g, 96.3%) as a colorless solid.
$^1$H-NMR(DMSO-$d_6$)δ:3.84(2H,t,J=5.1Hz),4.48(2H,t,J=5.1Hz), 7.48(1H,dd,J=1.5,8.1Hz),7.55(1H,d,J=1.5Hz),7.74 (1H, d,J=8.1Hz)ppm
FABMS:327(M+1)

Reference Example 47

Benzyl bromide (17.8 mL, 0.15 mol) was added to a dimethylformamide solution (100 mL) containing the compound in Reference Example 43 (10.9 g, 50 mmol) and potassium carbonate (20.7 g, 0.15 mol) at room temperature, and the mixture was stirred for one day at room temperature. The reaction mixture was poured into saturated aqueous sodium chloride solution, and the organic layer was extracted with ethyl acetate. The organic layer was washed with saturated aqueous sodium chloride solution, dried over anhydrous magnesium sulfate, and concentrated under reduced pressure. The crude product obtained was used without purification in the next reaction. Aqueous 4 N sodium hydroxide solution (20 mL) was added to a methanol solution (200 mL) of the crude product obtained, and the mixture was stirred under reflux for 6 hours. After the reaction was completed, the reaction solution was concentrated under reduced pressure, and 4 N aqueous hydrochloric acid solution was added to the residue until acidic, allowing precipitation of colorless solid. The precipitated solid was collected by filtration and washed thoroughly with water, to obtain 3-benzyloxy-4-bromobenzoic acid (14.5 g, 94.2%) as a colorless solid.
$^1$H-NMR ($CDCl_3$) δ: 5.23 (2H, s) 7.34-7.45 (2H, m), 7.48-7. 54 (2H, m), 7.58-7.71(3H,m)ppm

Reference Example 48

4-Bromo-3-methoxybenzoic acid was prepared in the same procedure to Reference Example 47 using the compounds in Reference Example 43. (yield 88.9%)

$^1$H-NMR ($CDCl_3$) δ:3.98 (3H, s), 7.58-7.68 (3H, m) ppm

Reference Example 49

A mixed solution of pyridine (20 mL) and acetic anhydride (20 mL) containing the compound in Reference Example 43 (10 g, 46.1 mmol) was heated under reflux for 6 hours. The reaction solution was poured into ice water and extracted with ethyl acetate. The organic layer was washed with 1 N aqueous hydrochloric acid solution and then with saturated aqueous sodium chloride solution, dried over anhydrous sodium sulfate, and concentrated under reduced pressure. The crystals obtained were washed with hexane, to obtain 3-acetyloxy-4-bromobenzoic acid (11.3 g, 94.3%) as a colorless solid.
$^1$H-NMR($CDCl_3$)($CDCl_3$)δ:2.39(3H,s),4.10-5.20(1H,br),7.71-7.76(1H,m), 7.82-7.88(2H,m)ppm
FABMS:259(M+1)

Reference Example 50

39% Nitric acid (300 mL) suspension containing 4-bromo-m-xylene (50 g, 0.26 mol) was heated under reflux for 24 hours. The reaction solution was cooled on an ice bath and then left in a refrigerator (5˚C) for 3 days. The precipitated crystals were collected by filtration, washed with distilled water and then with hexane, and dried under reduced pressure, to obtain 4-bromo-3-methylbenzoic acid (33.54 g, 60.0%) as colorless crystals.
$^1$H-NMR(DMSO-$d_6$)δ:2.40(3H,s),7.63-7.75(2H,m)7.90 (1H,dd,J=3.9,5.9Hz),12.75-13.66(1H,br)ppm

Reference Example 51

Methyl iodide (9.3 mL, 0.15 mol) was added to an N,N-dimethylformamide solution (70 mL) containing the compound in Reference Example 50 (24.61 g, 0.114 mol) and potassium carbonate (25 g, 0.18 mol), and the mixture was stirred at 50˚C for 2.5 hours. The reaction solution was concentrated to half in volume under reduced pressure, added with 1 N aqueous hydrochloric acid solution, and extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 240: 10), to obtain methyl 4-bromo-3-methylbenzoate (21.7 g, 83.1%) as a pale yellow oil.
$^1$H-NMR ($CDCl_3$) δ:2.44 (3H, s), 3.91 (3H, s), 7.60 (1H, d, J=8.1Hz), 7.7 0 (1H, dd, J=2.2, 8.8Hz), 7.89-7.92 (1H, m) ppm

Reference Example 52

Benzoyl peroxide (0.51g, 2.1 mmol) was added to a carbon tetrachloride solution (250 mL) containing the compound in Reference Example 51 (22.7 g, 94. 7 mmol) and N-bromosuccinimide (17.2 g, 94.7 mmol), and the mixture was heated under reflux for 6 hours. The reaction solution was cooled to room temperature; insoluble matter is removed by filtration; and the filtrate was concentrated under reduced pressure. The residue was dissolve in hexane (100 mL) and left at 5˚C for 1.5 hours. The precipitated solid was collected by filtration and dried, to obtain methyl 4-bromo-3-bromomethylbenzoate (13.4 g, 46.1%) as a pale yellow solid.
$^1$H-NMR($CDCl_3$)δ:4.64(2H,s),7.71(1H,d,J=8.1Hz),7.89 (1H,dd,J=2.2,8.1Hz),8.19(1H,d,J=2.2Hz)ppm
FABMS:309(M+1)

Reference Example 53

Triethylamine (0.84 mL, 5.99 mmol) was added to an acetonitrile solution (28 mL) containing the compound in Reference Example 52 (1.42 g, 4.61 mmol) and morpholine (1.21 mL, 13.8 mmol) under a nitrogen atmosphere, and the mixture was heated under reflux for 13 hours. The reaction solution was concentrated under reduced pressure, and the residue obtained was purified by silica gel chromatography (hexane/ethyl acetate, 6:1), to obtain methyl 4-bromo-3-(4-morpholinyl methyl)benzoate (1.38 g, 96%) as a pale yellow oil.
$^1$H-NMR (DMSO-$d_6$) δ :2.43 (4H,t,J=4.4Hz) 3.36 (2H,s) 3.57-3.60 (6H,m), 7.70(2H,s), 8.01(1H,s)ppm
FABMS:301(M+1)

Reference Example 54

Sodium hydroxide (180 mg, 4.6mmol) was added to a methanol solution (20 mL) containing the compound in Reference Example 53 (0.727 g, 2.33 mmol), and the mixture was stirred at room temperature 23 hours. After the reaction was completed, 4 N aqueous hydrochloric acid solution was added to the reaction solution and the organic

matter was extracted with ethyl acetate. The organic layer was washed with saturated aqueous sodium chloride solution, dried over anhydrous magnesium sulfate, and concentrated under reduced pressure. The residue obtained was recrystallized from ethyl acetate, to obtain 4-bromo-3- (4-morpholinylmethyl) benzoic acid (0.337 g, 43%) as a white solid.
$^1$H-NMR(DMSO-$d_6$) δ:2.57(4H,t,J=2.2Hz),3.64(6H,m),3.92(3H,s), 7.80(2H,s),8.09(1H,s)ppm
FABMS:313(M+1)

Reference Example 55

4-Bromo-3-bromomethylbenzoic acid was prepared in the same procedure to Reference Example 52 using the compound in Reference Example 50.
$^1$H-NMR($CDCl_3$) δ:4.64(2H,s),7.71(1H,d,J=8.1Hz),7.89 (1H,dd,J=2.2,8.1Hz),8.19(1H,d,J=2.2Hz)ppm

Reference Example 56

10% aqueous sodium hydroxide solution (1.24 g, 31.1 mmol) was added to a methanol solution (200 mL) containing dimethyl 4-bromoisophthalate (8.0 g, 31.1 mmol), and the mixture was heated under reflux for 24 hours. After the reaction was completed, methanol was evaporated under reduced pressure, and the aqueous phase was washed with ethyl acetate. The aqueous phase was acidified with 4 N aqueous hydrochloric acid solution and extracted with ethyl acetate. The organic layer was washed with saturated aqueous sodium chloride solution, dried over anhydrous magnesium sulfate, and concentrated under reduced pressure. The residue obtained was washed with ethyl acetate, to obtain 4-bromo-3-(methoxycarbonyl)benzoic acid (2.0 g, 26.5%) as a colorless solid.
$^1$H-NMR($CDCl_3$) δ: 3.97(3H,s),7.80(1H,d,J=8.3Hz),8.02 (1H,dd,J=8.3,2.4Hz),8.50(1H,d,J=2.4Hz)ppm

Reference Example 57

Thionyl chloride (5.0 mL, 68.0 mmol) was added to a tetrahydrofuran solution (40 mL) containing the compound in Reference Example 48 (3.92 g, 0.17 mmol) and N,N-dimethylformamide (2 drops) at room temperature, and the mixture was stirred at room temperature for 2 hours. After the reaction was completed, the mixture was concentrated sufficiently under reduced pressure (azeotropic distillation with toluene), to obtain the acid chloride. On the other hand, a hexane solution (9.6 mL, 25.5 mmol) of 2.66 M butyllithium was added to a tetrahydrofuran solution (30 mL) of 3-ethyl-3-pentanol (2.96 g, 25.5 mmol) while the mixture was cooled on an ice bath under nitrogen stream. The mixture was stirred for 2 hours on an ice bath, and a tetrahydrofuran solution (20 mL) of the acid chloride prepared above was added thereto dropwise on an ice bath. After dropwise addition, the mixture was stirred at room temperature for 2 hours. After the reaction was completed, the reaction mixture was concentrated under reduced pressure; the residue was poured into saturated aqueous sodium chloride solution; and the organic matter was extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 20:1), to obtain 1,1-diethylpropyl 4-bromo-3-methoxy benzoate (4.86 g, 86.8%) as a pale yellow oil.
$^1$H-NMR(CDCl3)δ:0.88(9H,t,J=7.3Hz),1.98(6H,q,J=7.3Hz),3.95 (3H,s),7.46(1H,dd,J=8.3,2.0Hz),7.53(1H,d,J=2.0Hz), 7.58 (1H,d,J=8.3Hz)ppm
FABMS:330(M+1)

Reference Example 58

A hexane solution (5.6 mL, 14.8 mmol) of 2.64 M butyl lithium was added to a tetrahydrofuran solution (50 mL) containing the compound in Reference Example 57 (4.86 g, 14.8 mmol) under a nitrogen stream at dry-ice temperature (-76 to -70˚C). The mixture was stirred at the dry ice temperature additionally for 2 hours; triisopropyl borate (3.44 mL, 14.8 mmol) was added thereto; and the mixture was stirred as the same temperature for 1 hour. After the reaction was completed, 4 N aqueous hydrochloric acid solution (14.8 mL) was added thereto. The reaction mixture was concentrated under reduced pressure; the residue was poured into saturated aqueous sodium chloride solution; and the organic matter was extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 4:1), to obtain
4-[(1,1-diethylpropoxy)carbonyl]-2-methoxyphenylboronic acid (1.74 g, 40.0%) as a pale yellow oil.
$^1$H-NMR ($CDCl_3$) δ: 0.89 (9H, t, J=7.3Hz), 1.99 (6H, q, J =7.3Hz) 3. 97 (3H,s ) , 5.83 (2H, s) , 7.55 (1H,d,J=1 . 5Hz) , 7.63 (1H, dd, J=7.8,1.5Hz) , 7.88 (1H, d, *J*=7.8Hz) ppm

Reference Example 59

2,1-Diethylpropyl 3-benzyloxy-4-bromobenzoate was prepared in the same procedure to Reference Example 47 using the compounds in Reference Example 47. (yield 51.7%)
$^1$H-NMR($CDCl_3$)δ:0.86(9H,t,J=7.3Hz), 1.91-2.00(6H,m),5.20 (2H,s),7.31-7.51(6H,m),7.55-7.62(2H,m)ppm

Reference Example 60

4-[(1,1-Diethylpropoxy)carbonyl]-2-benzyloxyphenylbor onic acid was obtained by the same procedure as in Reference Example 58 using the compounds in Reference Example 59. (yield 51.7%)
$^1$H-NMR ($CDCl_3$)δ:0.88 (9H, t, J=7.3Hz),1.94-2.02 (6H,m), 5.19 (2H,s),7.35-7.47(5H,m),7.61-7.66(2H,m),7.91(1H,d, J=7.3Hz) ppm

Reference Example 61

4-Bromo-3-(methoxymethoxy)benzoic acid was prepared in the same procedure to Reference Example 47 using the compounds in Reference Example 43. (yield 91.3%)
$^1$H-NMR($CDCl_3$)δ:3.54(3H,s), 5.33(2H,s),7.64-7.65(2H,m), 7.84(1H,d,J=1.5Hz)ppm

Reference Example 62

1,1-Diethylpropyl 4-bromo-3-(methoxymethoxy)benzoate was prepared in the same procedure to Reference Example 57 using the compounds in Reference Example 61. (yield 74.8%)
$^1$H-NMR($CDCl_3$) δ:0.88 (9H, t, J=7.3Hz), 1.97(6H, q, J=7.3Hz), 3.53 (3H,s), 5.29 (2H,s), 7.50 (1H, dd, J=8.3,2.0Hz), 7.59 (1H, d, J=8.3Hz), 7.76(1H, d, J=2.0Hz)ppm
FABMS:360(M+1)

Reference Example 63

4- [(1, 1-Diethylpropoxy) carbonyl] -2- (methoxymethoxy) phenylboronic acid was prepared in the same procedure to Reference Example 58 using the compounds in Reference Example 62. (yield 74.8%)
$^1$H-NMR($CDCl_3$) δ: 0.89 (9H,t,J=7.3Hz),1.98(6H,q,J=7.3Hz),3.52 (3H,s),5.34(2H,s),5.88(2H,s),7.67(1H,dd, J=7.8,1.0Hz), 7.74 (1H,d,J=1.0Hz), 7.90(1H,d,J=7.8Hz),

Reference Example 64

Thionyl chloride (25.5 mL, 349 mmol) was added to a tetrahydrofuran solution (120 mL) containing the compound in Reference Example 50 (15.0 g, 69.8 mmol) at room temperature under a nitrogen atomosphere, and the mixture was stirred for 16 hours. Then, the reaction solution was concentrated under reduced pressure; the residue was dissolved in methylene chloride (1,800 mL); 2-amino-2-methyl-1-propanol (10.6 g, 119 mmol) was added thereto; and the mixture was stirred at room temperature for 16 hours. The reaction solution was then concentrated under reduced pressure; thionyl chloride (45 mL, 617 mmol) was added thereto; and the mixture was stirred at room temperature for 30 minutes. The reaction solution was then concentrated under reduced pressure and poured into 1 N aqueous hydrochloric acid solution, and the organic matter was extracted with ether. The aqueous phase was made basic by addition of 4 N aqueous sodium hydroxide solution and extracted with ether, and the ether layer was washed with saturated aqueous sodium chloride solution. The ether layer was dried over anhydrous magnesium sulfate, and concentrated, to obtain 2-(4-bromo-3-methylphenyl)-4,4-dimethyl-4,5-dihydro-1,3-oxa zole (11.6 g, 62.1%) as a colorless solid.
$^1$H-NMR($CDCl_3$) δ:1.38(9H,s),2.42(3H,s),4.38(2H,s),7.54-7.61 (2H,m),7.83-7.84(1H,m)ppm
FABMS:216(M+1)

Reference Example 65

2-[4-Bromo-3-(bromomethyl)phenyl]-4,4-dimethyl-4,5-dihydro-1,3-oxazole was prepared in the same procedure to Reference Example 52 using the compounds in Reference Example 64. It was used without purification in the reaction of Reference Example 66.

Reference Example 66

2-Nitropropane (4.27 mL, 47.6 mmol) and the compound in Reference Example 65 (43.3 mmol) were added to an ethanol (160 mL) solution containing sodium (1.08 g, 47.0 mmol) at room temperature under a nitrogen atmosphere, and the mixture was stirred for 18 hours. The reaction solution was concentrated and poured into 1 N aqueous sodium hydroxide solution; the organic matter was extracted with ethyl acetate; and the extract was washed with saturated aqueous sodium chloride solution. The extract was dried over anhydrous magnesium sulfate and concentrated under reduced pressure, and the residue obtained was purified by column chromatography (hexane/ ethyl acetate, 10:1), to obtain
2-bromo-5-(4,4-dimethyl-4,5-dihydro-1,3-oxazol-2-yl) benzaldehyde (6.12 g, 50,2%) as a yellow solid. $^1$H-MMR($CDCl_3$) δ:1.41(6H,s),4.14(2H,s),7.70(1H,d,J=8.3Hz), 8.02(1H,dd,J=2.4 and 8.3Hz),8.42(1H,d,J=2.4Hz),10.4(1H,s)ppm
FABMS: 283 (M+1)

Reference Example 67

Anhydrous tetrahydrofuran (50 mL) was added to a container containing magnesium (0.346 g, 14 mmol) and iodine (trace amount) at room temperature under a nitrogen atmosphere, and benzyl 4-bromobutyl ether (2.73 mL, 14.0 mmol) was added thereto additionally. After stirring at 50˚C for 2.5 hours, the reaction solution was cooled on an ice bath, and added to a tetrahydrofuran solution (100 mL) containing the compound in Reference Example 66 (3.29 g, 11.7 mmol) gradually with a cannula. The mixture was stirred at room temperature for 3 hours. The reaction solution was treated with saturated aqueous ammonium chloride solution and extracted with ethyl acetate; the extract was washed with saturated sodium bicarbonate aqueous solution and then with saturated aqueous sodium chloride solution, dried over anhydrous magnesium sulfate, and concentrated under reduced pressure, to obtain 5-(benzyloxy)-1-[2-bromo-5-(4,4-dimethyl-4,5-dihydro-1,3-oxazol-2-yl)phenyl]-1-pentanol. It was used without purification in the reaction of Reference Example 68. Reference Example 68

Under a nitrogen atomospher, Celite (6g) and pyridinium chlorochromate (3.02 g, 14.0 mmol) were added to a methylene chloride solution (100 mL) containing the compound in Reference Example 67 (14 mmol). After stirring for 16 hours, 2-propanol (1.3 mL, 16.8 mmol) was added thereto, and the mixture was stirred for 30 minutes. The mixture was diluted with ether and filtered through Celite, and the filtrate was concentrated under reduced pressure. The residue obtained was purified by column chromatography (hexane/ethyl acetate, 5:1), to obtain 5-benzyloxy-1-[2-bromo-5-(4,4-dimethyl-4,5-dihydro-1,3-oxazol-2-yl)phenyl]-1-pentanone (1.19 g, 22.9%) as a yellow solid.
$^1$H-NMR($CDCl_3$) δ:1.38 (6H,s) 1.65-1.85(4H,m) 2.95(2H, t, J=7.3Hz ), 3.51 (2H, t, J=6.3Hz), 4.12 (2H,s), 4.50(2H,s), 7.26-7.34 (5H,m), 7.64 (1H,d,*J*=8.3Hz), 7.82(1H,dd,J=2.4 and 8.3Hz), 7.91 (1H,d,J=2.4Hz) ppm
FABMS:445(M+1)

Reference Example 69

Aqueous 4 N hydrochloric acid solution (15 mL) and ethanol (9 mL) were added to the compound in Reference Example 68 (3 g, 6.75 mmol), and the mixture was stirred for 2 hours. The reaction solution was concentrated under reduced pressure and extracted with ethyl acetate; and the extract was washed with saturated aqueous sodium chloride solution, dried over anhydrous magnesium sulfate, and concentrated under reduced pressure. Ethanol (60 mL) and 4 N aqueous sodium hydroxide solution (18 mL) were added to the residue, and the mixture was stirred at 60˚C for 3 hours. After concentration, ethyl acetate was added to the residue; the aqueous phase was acidified with 4 N aqueous hydrochloric acid solution, and the organic matter was extracted with ethyl acetate. The extract was washed with saturated aqueous sodium chloride solution, dried over anhydrous magnesium sulfate, and concentrated under reduced pressure, to obtain 3-[5-(benzyloxy)pentanoyl]-4-bromobenzoic acid (2.02 g, 76.5%) as a colorless solid.
$^1$H-NMR($CDCl_3$)δ:1.69-1.88(4H,m),2.96(2H,t,J=7.3Hz),3.52 (2H,t,J=5.9Hz),4.51(2H,s),7.26-7.37(5H,m),7.72 (1H,d, J=8.8Hz),7.97(1H,dd,J=2.2 and .8Hz), 8.05(1H,d,J=2.2Hz)ppm
FABMS: 392 (M+1)

Reference Example 70

A tetrahydrofuran solution (10 mL) of 5-bromoindole (1.0 g, 5.1 mmol) was added to a tetrahydrofuran suspension (20 mL) containing potassium hydride (682 mg, 5.1 mmol) at dry ice temperature under a nitrogen stream. After stirring for 15 minutes, 1.6 M tert-butyl lithium n-pentane solution (6.4 mL, 10.2 mmol) was added thereto. After stirring for 10 minutes, a tetrahydrofuran solution (3 mL) of triisopropyl borate (2.35 mL, 10.2 mmol) was added additionally, and the mixture was stirred at room temperature for 30 minutes. After the reaction was completed, the reaction solution was poured into 1 M aqueous phosphoric acid solution, and the organic matter was extracted with ether . The organic

layer was washed with saturated aqueous sodium chloride solution, dried over anhydrous magnesium sulfate, and concentrated under reduced pressure. The residue obtained was purified by silica gel column chromatography (hexane/ethyl acetate, 2:3), to obtain 1H-indol-5-ylboronic acid (352 mg, 42.9%) as a colorless solid.
$^1$H-NMR (DMSO-$d_6$) δ:6.42 (1H,s), 7.29-7.31(1H,m), 7.32 (1H,d,J=8.3 Hz), 7.52-7.55(1H,m), 7.74(1H,s),8.05(1H,s), 11.1(2H,br)ppm

Reference Example 71

Pinacolborane (4.35 mL, 6.0 mmol) was added to a dioxane solution (16 mL) containing 1-iodo-4-nitrobenzene (5.0 g, 20.0 mmol), $PdCl_2$ (dppf) (490 mg, 0.6mmol), and triethylamine (8.45 mL, 60.0 mmol) at room temperature under a nitrogen stream. The mixture was stirred while heated at 80˚C for 16 hours; the reaction solution was concentrated under reduced pressure; the residue was poured into saturated aqueous sodium chloride solution; and the organic matter was extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 9:1), to obtain
4,4,5,5-tetramethyl-2-(4-nitrophenyl)-1,3,2-dioxaborolane (3.48 g, 70.0%) as a yellow solid.
$^1$H-NMR ($CDCl_3$) δ:1.37 (12H, s), 7.96(2H, d, J=8.8Hz), 8.20 (2H, d, J=8.8Hz) ppm

Reference Example 72

10% Palladium carbon (200 mg) was added to a methanol solution (10 mL) of the compound in Reference Example 71 (1.0 g, 4.02 mol) at room temperature under a hydrogen stream, allowing catalytic reduction. After stirring for 21 hours, the catalyst was removed by filtration; and the filtrate was concentrated under reduced pressure, to obtain 4-(4,4,5,5-tetramethyl-1,3,2-dioxaborolan-2-yl)phenylamine (752 mg, 85.4%) as a brown solid.
$^1$H-NMR($CDCl_3$) δ:1.32 (12H, s) 3.83 (2H,br), 6.64-6.68 (2H,m), 7.60-7.73(2H,m)ppm

Reference Example 73

Acetyl chloride (139 mg, 1.77 mmol) was added to a tetrahydrofuran solution (10 mL) containing the compound in Reference Example 72 (387 mg, 1.77 mmol) and pyridine (0.17mL, 2.12 mmol) at room temperature under nitrogen stream. After stirring for 4 hours, the reaction mixture was concentrated under reduced pressure. The residue was poured into aqueous ammonium chloride solution, and the organic matter was extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 9:1), to obtain 4-(4,4,5,5-tetramethyl-1,3,2-dioxaborolan-2-yl)phenylamine (425 mg, 92%) as a colorless solid.

Reference Example 74

2,2,2-Trifluoro-N-[4-(4,4,5,5-tetramethyl-1,3,2-dioxaborolan-2-yl)phenyl]acetamide was prepared in the same procedure to Reference Example 73 using the compounds in Reference Example 72. (yield 87.1%)
$^1$H-NMR($CDCl_3$)δ:1.35(12H,s),7.57-7.59(2H,m),7.60-7.82(2H,m), 7.96(1H,br)ppm
FABMS:316(M+1)

Reference Example 75

Methyl iodide (0.12 mL, 2.0 mmol) was added to an acetone solution (10 mL) containing the compound in Reference Example 74 (315 mg, 1.0 mmol) and potassium carbonate (276 mg, 2.0 mmol), and the mixture was stirred at 60˚C for 21 hours. The reaction solution was concentrated under reduced pressure; the concentrated residue obtained was dissolved in ethyl acetate; and the solution was washed with saturated aqueous sodium chloride solution, dried over anhydrous magnesium sulfate, and concentrated under reduced pressure. The residue obtained was purified by silica gel column chromatography (hexane/ethyl acetate, 9:1), to obtain 2,2,2-trifluoro-N-methyl-N-[4-(4,4,5,5-tetramethyl-1,3,2-dioxaborolan-2-yl)phenyl]acetamide (280 mg, 85.1%) as a pale yellow oil.

Reference Example 76

N-Ethyl-2,2,2-trifluoro-N-[4-(4,4,5,5-tetramethyl-1,3,2-dioxaborolan-2-yl)phenyl]acetamide was prepared in the same procedure to Reference Example 75 using the compounds in Reference Example 74 (yield 71.1%).

$^{1}$H-NMR($CDCl_3$) δ:1.67(3H, t, J=1.7Hz),1.36(12H,s) , 3.79 (2H ,q,J=7.1Hz),7.21(2H,d,J=8.3Hz),7.87(2H,d,J=8.3Hz) ppm
FABMS:345(M+1)

Reference Example 77

Tosyl chloride (1.0 g, 5.25 mmol) was added to a pyridine solution (10 mL) of the compound in Reference Example 72 (1.1 g, 5.0 mmol), and the mixture was stirred while heated to 50˚C under nitrogen stream for 21.5 hours. After the reaction was completed, the reaction mixture was poured into aqueous citric acid solution, and the organic matter was extracted with ethyl acetate. The organic layer was washed with saturated aqueous sodium chloride solution, dried over anhydrous magnesium sulfate, and concentrated under reduced pressure. The residue obtained was purified by silica gel column chromatography (hexane/ethyl acetate, 4:1), to obtain 4-methyl-N-[4-(4,4,5,5-tetramethyl-1,3,2-dioxaborolan-2-yl) phenyl]benzenesulfonamide (1.29 g, 69.0%) as a colorless solid.
$^{1}$H-NMR($CDCl_3$) δ:1.31 (12H, s), 2.36(3H, s), 6.84 (1H, s), 7.07 (2H, d, J=8.8Hz), 7.21(2H, d, J=8.3Hz), 7.65-7.69 (4H, m) ppm
FABMS:374(M+1)

Reference Example 78

Diethyl azodicarboxylate (40% toluene solution) (3.05 mL, 6.72 mmol) was added to a tetrahydrofuran solution (40 mL) containing the compound in Reference Example 77 (1.29 g, 3.36 mol), triphenylphosphine (1.76 g, 6.72 mmol) and 2-propanol (0.52 mL, 6.72 mmol) under nitrogen stream, while the solution was cooled on an ice bath. The reaction solution was stirred at room temperature for 13 hours, and the solvent was evaporated under reduced pressure. The residue obtained was purified by silica gel column chromatography (hexane/ethyl acetate, 9:1), to obtain N-isopropyl-4-methyl-N-[4-(4,4,5,5-tetramethyl-1,3,2-dioxaborolan-2-yl)phenyl]benzenesulfonamide (1.16 g, 83.1%) as a colorless solid.
$^{1}$H-NMR($CDCl_3$)δ:1.03(6H,d,J=6.8Hz),1.35(12H,s),2.42(3H,s), 4.47(1H,sep,J=6.8Hz),7.02-7.06(2H,m),7.23-7.26(2H, m), 7.60-7.63(2H,m),7.75-7.78(2H,m)ppm
FABMS:416(M+1)

Reference Example 79

n-Butyllithium (3.7 mL, 2.4 mmol hexane solution) was added dropwise to an ether solution (20 mL) of 4-bromo-(difluoromethoxy)benzene (2 g, 9 mmol) at -60˚C over 10 minutes, and the mixture was stirred at the same temperature for 1 hour. An ether solution (10 mL) of triisopropyl borate (1.88 g, 10 mmol) was added dropwise to the solution at -60˚C over 5 minutes, and the mixture was stirred at the same temperature for 1 hour and at room temperature for additional 1 hour. 10% aqueous phosphoric acid solution (10 mmol) was added thereto at 10˚C, and the mixture was stirred for 1 hour. The ether layer was separated and extracted with aqueous 1 N sodium hydroxide solution, and the extract was acidified and extracted with ether. The ether layer was dried and concentrated under reduced pressure. The residue obtained was purified by silica gel column chromatography (hexane/ethyl acetate, 7:3), to obtain difluoromethyl 4-(4,4,5,5-tetramethyl-1,3,2-dioxaborolan-2-yl)phenyl ether (0.06 g, 11%) as a colorless solid.
$^{1}$H-NMR($CDCl_3$) δ:6.56(1H,t,J=73.2Hz),7.14(2H,d,J=8.3Hz) 7.76(1H,d,J=8.3Hz)ppm

Reference Example 80

A methylene chloride solution (16 mL) of trifluoromethanesulfonic acid anhydride (8.0 mL, 47. 7 mmol) was added dropwise to a methylene chloride solution (50 mL) containing 3-fluoro-4-nitrophenol (5.0 g, 31.8 mmol) and pyridine (5.2 mL, 63.7 mmol) under nitrogen stream while cooled on an ice bath, and the mixture was stirred at room temperature for 3 days. After the reaction was completed, the reaction mixture was concentrated under reduced pressure, and the residue obtained was added with saturated aqueous sodium chloride solution and extracted with ethyl acetate. The organic layer obtained was washed with saturated aqueous sodium chloride solution, dried over anhydrous magnesium sulfate, and concentrated under reduced pressure. The residue obtained was purified by silica gel column chromatography (hexane/ethyl acetate, 9:1), to obtain
3-fluoro-4-nitrophenyl trifluoromethanesulfonate (9.48 g, quantitative yield) as a pale yellow oil.
1H-NMR($CDCl_3$)δ: 7.26-7.33(2H,m) , 8.20-8.26(2H,m)ppm
FABMS:290(M+1)

Reference Example 81

2,2,2-Trifluoro-N-(4-iodo-2-methylphenyl)acetamide was prepared in the same procedure to Reference Example 74 by using 4-iodo-2-methylaniline. (yield 98.4%)
$^1$H-NMR(DMSO-$d_6$)δ:1.57(2H,s),2.25(3H,s),7.60 (3H,m)ppm
FABMS:330(M+1)

Reference Example 82

N-Ethyl-2,2,2-trifluoro-N-(4-iodo-2-methyl-phenyl) acetamide was prepared in the same procedure to Reference Example 75 using the compounds in Reference Example 81. (yield 94.9%)
$^1$H-NMR(DMSO-$d_6$)δ:1.18(3H,t, J=7.3Hz),2.19(3H,s),3.12 (1H,q,J=6.6Hz),4.23(1H,q,J=6.6Hz),6.84(1H,d,J=8.0Hz), 7.57(2H,dd,J=1.58.0Hz),7.68(1H,s)ppm
FABMS:358(M+1)

Reference Example 83

N-Ethyl-2,2,2-trifluoro-N-[2-methyl-4-(4,4,5,5-tetramethyl 1, 3, 2-dioxaborolan-2-yl) phenyl]acetamide was prepared in the same procedure to Reference Example 71 using the compounds in Reference Example 82. (yield 78.6%)
$^1$H-NMR(D.MSO-$d_6$)δ:1.19(3H,t,J=7.3Hz),1.36(12H,s),1.57(2H,s), 2.25(3H,s),3.14(1H,q,J=7.3Hz),4.24(1H,q, J=7.3Hz),7.11 (1H,d,J=7.3Hz),7.67(1H,d,J=7.3Hz),7.76(1H,s)ppm

Reference Example 84

A toluene solution (20 mL) containing phthalic anhydride (10 g, 67.5 mmol) and 2-ethanolamine (4.12 g, 67.5 mmol) was heated under reflux for 18 hours, while a water separator was used. The crystals precipitated when cooled to room temperature were collected by filtration. The crystals obtained were recrystallized from ethanol, to obtain 2- (2-hydroxyethyl) -1H-isoindol-1, 3 (2H) -dione (8.62 g, 66.8%) as a colorless solid.
$^1$H-NMR(CD3OD)δ:3.72-3.84(4H,m),4.61(1H,brs),7.77-7.90(4H,m) ppm
FABMS: 192 (M+1)

Reference Example 85

An N,N-dimethylformamide solution (20 mL) containing anhydrous potassium phthalate (7.6 g, 41 mmol) and 1,6-dibromohexane (10 g, 41 mmol) was stirred at 100˚C for 6 hours. The reaction solution was cooled and then poured into saturated aqueous citric acid solution, and the organic matter was extracted with ethyl acetate. The organic layer was washed with water, dried over anhydrous sodium sulfate, and concentrated under reduced pressure. The residue obtained was purified by silica gel column chromatography (hexane/ethyl acetate, 450:50), to obtain 2-(6-bromohexyl)-1H-isoindol-1, 3 (2H) -dione (6.78 g, 53.3%) as pale yellow crystals.
$^1$H-N-MR($CDCl_3$)δ:1.31-1.36(4H,m)1.63-1.76(1H,m),1.79-1.93 (1H,m),3.36-3.46(1H,m),3.69(1H,t,J=7.3Hz),7.68-7.75 (2H,m), 7.81-7.89 (2H, m) ppm
FABMS:312 (M+1)

Reference Example 86

A mixed solution of 3-aminopropanol (10 g, 0.13 mol) and ethyl formate (30 g, 0.38 mol) was heated under reflux for 5 hours and concentrated under reduced pressure. The concentrated residue obtained was purified by distillation under reduced pressure (5 mmHg, 142-145˚C), to obtain 3-hydroxypropyl formamide (12 g, 89.5%) as a colorless oil.
$^1$H-NMR($CDCl_3$) δ:1.62-1.83(2H,m),2.89(1H,brs),3.42-3.55 (2H,m),3.63-3.77(2H,m),6.08(1H,brs),8.20(1H,s),7.81-7.89 (2H,m)ppm

Reference Example 87

A tetrahydrofuran solution (100 mL) of the compound in Reference Example 86 (10.67 g, 0.10 mol) was added gradually dropwise to a tetrahydrofuran suspension (100 mL) containing lithium aluminum hydride (6.6 g, 0.17 mol) at room temperature. After dropwise addition, the mixture was heated under reflux for 5 hours. Aqueous 7.5N potassium hydroxide solution (20 mL) was added gradually dropwise to the reaction solution previously ice-cooled. After dropwise addition, the mixture was heated under reflux for 30 minutes. The reaction solution was cooled, and the insoluble matter

was separated by filtration. The insoluble matter was washed with tetrahydrofuran, and the filtrate and the washing solution were combined and concentrated under reduced pressure . The concentrated residue obtained was purified by distillation under reduced pressure (25mmHg, 90-92˚C), to obtain 3-(methylamino)-1-propanol (7.95 g, 86.6%) as a colorless oil.
$^1$H-NMR ($CDCl_3$) δ:1.65-1.75 (2H,m) ,2.43 (3H, s), 2.72 (2H,t,J=5.9Hz),3.78-3.84(2H,m),6.08(1H,brs),8.20(1H,s)ppm

Reference Example 88

A dioxane solution (10 mL) of benzyl chloroformate (4.3 g, 25.9 mmol was added gradually dropwise to a mixed solution of dioxane (15 mL) and 1.4 N aqueous sodium hydroxide solution (20 mL) containing the compound in Reference Example 87 (2.11 g, 23.7 mmol). After dropwise addition, the mixture was stirred at 50˚C for 30 minutes. The reaction solution was concentrated under reduced pressure, and the residue obtained was acidified with saturated aqueous citric acid solution and extracted with ethyl acetate. The organic layer was washed with water, dried over anhydrous sodium sulfate, and concentrated under reduced pressure. The residue obtained was purified by silica gel column chromatography (hexane/ethyl acetate, 1:1 to 1:2), to obtain benzyl 3-hydroxypropyl(methyl)carbamate (4.37 g, 82.6%) as a colorless oil.
$^1$H-NMR($CDCl_3$)6:1.66-1.80(2H,m)2.92(3H,*s)*3.32-3.67(3H,m), 5.15(2H,s),7.28-7.40(5H,m)ppm

Reference Example 89

Conc. sulfuric acid (5mL) was added to a methanol solution (150 mL) of urocanic acid (10 g, 72.4 mmol), and the mixture was heated under reflux for 9 hours. The reaction solution was concentrated under reduced pressure, and the residue obtained was made basic by addition of saturated sodium hydrogen carbonate solution and extracted with ethyl acetate. The organic layer was washed with water, dried over anhydrous sodium sulfate, and concentrated under reduced pressure, to obtain (2E)-3-(1H-imidazol-4-yl)-2-propenoic acid methyl ester (5.83 g, 53.3%) as colorless crystals.
$^1$H-NMR($CDCl_3$)δ:3.79(3H,s),6.49(1H,d,J=5.4Hz),7.29(1H,s), 7.62(1H,d,J=6.1Hz),7.71(1H,s)ppm
FABMS : 153 (M+1)

Reference Example 90

10% Palladium carbon (1g) was added to a methanol solution (100 mL) of the compound in Reference Example 89 (5.88 g, 38.6 mmol), and the mixture was agitated vigorously at room temperature under hydrogen stream for 10.5 hours. The catalyst was collected by filtration and washed with methanol, the filtrate and the washing solution were combined and concentrated under reduced pressure, to obtain methyl 3-(1H-imidazol-4-yl)propanoate (5.39 g, 90.6%) as colorless crystals.
1H-NMR($CDCl_3$)δ:2.68(2H,t,J=6.8Hz),2.93(2H,t,J=6.8Hz),3.69 (3H,s),6.81(1H,s),7.55(1H,m),9.50-10.50(1H,br)ppm
FABMS:155(M+1)

Reference Example 91

A tetrahydrofuran solution (100 mL) of the compound in Reference Example 90 (5.39 g, 35.0 mmol) was added gradually dropwise to a tetrahydrofuran suspension (50 mL) of lithium aluminum hydride (2.65 g, 69.8mmol) at room temperature. After dropwise addition, the mixture was stirred at 50˚C for 5 hours. The reaction solution was cooled on an ice bath, and 2.5N aqueous potassium hydroxide solution (20 mL) was added thereto gradually. After dropwise addition, the mixture was stirred while heated under reflux for 30 minutes. The reaction solution was cooled, and the insoluble matter was separated by filtration. The insoluble matter was washed with tetrahydrofuran, and the filtrate and the washing solution were combined and concentrated under reduced pressure. The concentrated residue obtained was purified by distillation under reduced pressure (25 mm Hg, 90-92 ˚C), to obtain 3- (1H-imidazol-4-yl) -1-propanol (7.95 g, 86.6%) as a colorless oil.
$^1$H-NMR(DMSO-$d_6$,120˚C)δ:1.62-1.76(2H,m),2.42-2.60(2H,m), 3.35-3.48(2H,m),4.53(1H,brs),6.52-6.85(1H,m),7.48 (1H,s), 11.78(1H,brs)ppm
FABMS:127 (M+1)

Reference Example 92

Triethylamine (5 mL) and dimethylaminopyridine (0.32 g, 2.62 mmol) were added to a mixed solution of acetonitrile (20 mL) and a dioxane/water mixture (1:1, 5 mL) containing the compound in Reference Example 91 (3.26 g, 25.8 mmol). Di-tert-butyl carbonate (6.77 g, 31 mmol) was added additionally at room temperature, and the mixture was

stirred for 10 hours. The reaction solution was concentrated under reduced pressure. The residue obtained was purified by silica gel column chromatography (chloroform/methanol, 248:2), to obtain tert-butyl 4-(3-hydroxypropyl)-1H-imidazole-1-carboxylate (5.06 g, 86.5%) as colorless crystals.
$^1$H-LNMR ($CDCl_3$) δ:1.61(9H,s),1.89(2H,quint.,J=6.6Hz),2.69 (1H,t,J=6.6Hz),3.03(1H,t,J=5.9Hz),3.72(2H,q,J=5.9Hz), 7.11 (1H,s),8.00(1H,s) ppm
FABMS:227(M+1)

Reference Example 93

Benzyl 3-(hydroxymethyl)-1-piperidinecarboxylate was prepared in the same procedure to Reference Example 88 by using 3-(hydroxyrnethyl)-1-piperidine. (yield 91.9%) $^1$H-NMR($CDCl_3$)δ:1.23-1.82(8H,m),2.05-3.91(6H,m), 5.13-5.14 (2H,m),7.29-7.37(5H,m)ppm
FABMS:250(M+1)

Reference Example 94

An anhydrous ethanol solution (10 mL) of ethyl chloroacetate (2.1 g, 17.1 mmol) was added dropwise to an anhydrous ethanol solution (30 mL) of ethylenediamine (6 g, 99.8 mmol) at room temperature over 2.5 hours. The mixture was stirred at room temperature for 2 hours, and sodium ethoxide (sodium 0.4 g/ethanol 20 mL) solution was added thereto dropwise, gradually. The mixture was stirred at room temperature for 14 hours; the precipitated sodium chloride was separated by filtration; and the filtrate was concentrated under reduced pressure. The crude compound obtained was heated at 200 ˚C for 5 minutes, and the reaction product was purified by NH-silica gel column chromatography (ethyl acetate/chloroform, 10:240), to obtain a red solid, which was further purified by recrystallization (acetone-water), to obtain 2-piperazinone (0.59 g, 34.5%) as orange solid.
$^1$H-NMR($CDCl_3$)δ:1.50-2.05(1H,m),3.01-3.08(2H,m),3.34-3.42 (2H,m),3.53(2H,s),6.60(1H,brs)ppm
FABMS:101 (M+1)

Reference Example 95

Formic acid (5 mL) was added dropwise to ice-cooled acetic anhydride (10 mL) gradually. After dropwise addition, the mixture was stirred at 50˚C for 2 hours. After the solution was cooled to room temperature, 1-benzyl-piperidinyl-4-yl amine (2 g, 10.5 mmol) was added thereto gradually. After stirring for 15 hours, the reaction solution was concentrated under reduced pressure; and the residue obtained was diluted with ethyl acetate. The ethyl acetate layer was washed with saturated sodium hydrogen carbonate solution and then with saturated aqueous sodium chloride solution, dried over anhydrous sodium sulfate, and concentrated under reduced pressure, to obtain 1-benzyl-4-piperidinyl formamide (1.88 g, 82.1%) as a pale yellow oil.
$^1$H-NMR($CDCl_3$)δ:1.40-1.60(2H,m),1.82-2.00(2H,m),2.04-2.20 (2H,m),2.65-2.90(2H,m),1.63(2H,s),3.83-3.99(1H,m), 7.21-7.35 (5H,m),8.12(1H,s)ppm
FABMS:219(M+1)

Reference Example 96

Palladium hydroxide 20% on carbon (0.2 g) was added to a methanol solution (30 mL) of the compound in Reference Example 95 (0.73 g, 3.34 mmol), and the mixture was stirred at room temperature under hydrogen stream for 8 hours. The catalyst was separated by filtration, and the filtrate was concentrated under reduced pressure, to obtain 4-piperidinyl formamide (0.4 g, 93.4%) as a colorless solid.
$^1$H-NMR ($CDCl_3$) δ:1.26-1.56(2H, m),1.84-2.02(2H, m), 2.61-2.77 (2H,m),3.02-3.17(2H,m,),3.90-4.07(1H,m),5.30-5.85 (1H,br), 8.13(1H,s)ppm
FABMS:219(M+1)

Reference Example 97

1-[(Benzyloxy)carbonyl]-4-piperidinecarboxylic acid was prepared in the same procedure to Reference Example 88 by using 4-piperidinecarboxylic acid. (yield 96%)
$^1$H-NMR($CDCl_3$) δ:1.10-3.10(1H,br) 1.58-1.78(2H,m), 1.83-2.05 (2H,m),2.45-2.60(1H,m),2.85-3.05(2H,m),5.13(2H,s), 7.30-7.41 (5H,m)ppm
FABMS:264 (M+1)

Reference Example 98

Thionyl chloride (0.33 mL, 4.8 mmol) and a drop of N, N-dimethylformamide were added to a tetrahydrofuran solution (20 mL) of the compound in Reference Example 97 (0.5 g, 1.9 mmol), and the mixture was stirred at room temperature for 3 hours. The reaction solution was concentrated under reduced pressure, and distilled azeotropically with toluene twice. The concentrated residue obtained was dissolved in tetrahydrofuran (5 mL). The solution was added dropwise to 50% aqueous dimethylamine solution while the solution was cooled with ice and water. The mixture was stirred at room temperature for 3 hours, and the reaction solution was extracted with ethyl acetate. The organic layer was washed with saturated aqueous sodium chloride solution, dried over anhydrous sodium sulfate, and concentrated under reduced pressure, to obtain benzyl 4-[(dimethylamino)carbonyl]-1-piperidinecarboxylate (0.545 g, 98.8%) as a colorless solid.
$^1$H-NMR($CDC1_3$) δ:1. 65-1.85 (4H,m) 2.61-2.2.74 (1H, m) 2.75-2.99 (5H,m),3.06(3H,s),4.13-4.35(2H,m),7.27-7.41(5H, s)ppm
FABMS:263(M+1)

Reference Example 99

5% Palladium carbon (0.1 g) was added to a methanol solution (25 mL) of the compound in Reference Example 98 (0.54 g, 1.86 mmol) , and the mixture was stirred at room temperature under hydrogen stream for 15 hours. The catalyst was separated by filtration, and the filtrate was concentrated under reduced pressure, to obtain N,N-dimethyl-4-piperidinecarboxamide (0.28 g, 96.4%) as a colorless oil.
$^1$H-NMR ($CDCl_3$) δ:1.57-1.77 (3H, m), 1.78-1.90 (3H, m) 2.59-2.73 (2H,m), 2.95 (3H,brs), 3.05 (3H,brs), 3.11-3.22(1H, m) ppm
FABMS : 157(M+1)

Reference Example 100

Potassium bicarbonate (2.76 g, 20 mmol) was added to an ethanol solution (30 mL) containing benzyl piperazinecarboxylate (2.2 g, 10 mmol) and 2-propane iodide (3.4 g, 20 mmol), and the mixture was heated under reflux for 24 hours . The reaction solution was cooled to room temperature; and the insoluble matter was separated by filtration. The filtrate was concentrated under reduced pressure, and the residue was purified by silica gel column chromatography (chloroform/methanol, 248:2), to obtain benzyl 4-isopropyl-1-piperazinecarboxylate (2.19 g, 83.3%) as a colorless oil.
$^1$H-NMR($CDCl_3$) δ:1.04(6H,d,J=6.6Hz)2.43-2.54(4H,m)2.65-2.76 (1H,m),3.48-3.55(4H,m),5.40(2H,s),7.28-7.38(5H,m) ppm
FABMS:263 (M+1)

Reference Example 101

1-Isopropylpiperazine was prepared in the same procedure to Reference Example 104 using the compounds in Reference Example 100. (yield 66.4%)
$^1$H-NMR ($CDCl_3$) δ:1.06 (6H,d,J=6.8Hz), 2.51-2.61 (4H,m), 2.62-2.74 (1H,m),2.80-2.99(6H,m)ppm
FABMS:129(M+1)

Reference Example 102

Benzyl 4-butyl-1-piperazinecarboxylate was prepared in the same procedure to Reference Example 100 by using benzyl piperazinecarboxylate and butyl iodide. (yield 97.8%) $^1$H-NMR($CDCl_3$) δ:0.91(3H,t, J=7.3Hz),1.23-1.53 (4H,m),2.29-2.45 (6H,m),3.52(4H,t,J=5.1Hz),5.13(2H,s),7.28-7.38(5H,m)ppm

Reference Example 103

1-Butyypiperazine was prepared in the same procedure to Reference Example 99 using the compounds in Reference Example 102. The compound obtained was dissolved in ethyl acetate (50 mL); 4N hydrochloric acid/ethyl acetate solution (15 mL) was added thereto; and the mixture was stirred at room temperature for 3 hours. The precipitated crystals were collected by filtration, washed with ethyl acetate, and dried, to obtain 1-butylpiperazine-2 hydrochloride salt (2.07 g, 90.0%) as a pale brown solid.
$^1$H-NMR(DMSO-$d_6$)δ:0.90(3H,t, J=7.3Hz),1.25-1.40(2H,m), 1.60-1.75(4H,m),3.02-3.90(10H,m),9.92(2H,brs),11.90 (1H,brs) ppm

Reference Example 104

Benzyl 4-isobutyl-1-piperazinecarboxylate was prepared in the same procedure to Reference Example 100 by using benzyl piperazinecarboxylate and 3-iodo-2-methylpropane. (yield 73.3%)
$^1$H-NMR($CDCl_3$) δ: 0.89 (6H,d, J=6. 6Hz) 1.68-1.85 (1H,m), 2.07 (2H,d,J=7.3Hz),2.26-2.44(4H,m),3.48(4H,t,J=5.1Hz), 5.13 (2H, s), 7.29-7.38 ( 5H, m) ppm

Reference Example 105

1-Isobutylpiperazine-2 hydrochloride salt was prepared in the same procedure to Reference Example 103 using the compounds in Reference Example 104. (yield 60.0%)
$^1$H-NMR(DMSO-$d_6$)δ:1.07 (3H,t,J=5.9Hz) ,2.10-2.30(1H,m), 2.80-4.30(10H,m),10.07(2H,brs),11.15-11.80(1H,br)ppm

Reference Example 106

1-Benzylpiperidin-4-one (3.79 g, 20.0 mmol) and dimethylamine hydrochloride salt (4.80 g, 58.9 mmol) were dissolved in ethanol (30 mL) under a nitrogen atmosphere. Molecular sieve 3A was added; hydrochloric acid/ethanol solution was added until the solution became pH 6; and sodium cyanoborohydride (2.51 g, 40.0 mmol) was added additionally. The mixture was stirred at room temperature for 7 days; the precipitate was separated by filtration; and the filtrate was concentrated under reduced pressure. Aqueous 1N sodium hydroxide solution was added to the residue, and the organic matter was extracted with ethyl acetate. The organic layer was washed with saturated aqueous sodium chloride solution and concentrated under reduced pressure. The residue obtained was purified by NH silica gel chromatography (hexane/ethyl acetate, 1:1), to obtain 1-benzyl-N,N-dimethyl-4-piperidine amine (0.52 g, 12%) as a pale yellow oil.

Reference Example 107

N,N-dimethyl-4-piperidine amine-2 hydrochloride salt was prepared in the same procedure to Reference Example 103 using the compounds in Reference Example 106. (yield 83%)
$^1$H-NMR(DMSO-$d_6$) δ: 1.95 (2H,t,J=11.7Hz), 2.20 (2H,d,J=12.4Hz), 2.69(6H,s),3.37(5H,m)ppm

Reference Example 108

Benzyl (3R, 5S)-3,5-dimethyl-1-piperazinecarboxylate was prepared in the same procedure to Reference Example 88 by using (3R,5S)-3,5-dimethyl-1-piperazine. (yield 77.7%)
$^1$H-NMR (DMSO-$d_6$) δ: 0.95 (6H, d, J=6.6Hz), 2.28 (2H,m), 2.64(2H,m), 3.85(2H,dd,J=2.2,10.9Hz),5.08(2H,s),7.32(H, m)ppm
FABMS:249(M+1)

Reference Example 109

Benzyl (3R,5S)-4-butyl-3,5-dimethyl-1-piperazinecarboxylate was prepared in the same procedure to Reference Example 100 using the compounds in Reference Example 108. (yield 44%) Reference Example 110
(2R, 6S)-2,6-Dimethylpiperazine (yield 73.3%) was prepared in the same procedure to Reference Example 99 using the compounds in Reference Example 109.
$^1$H-NMR(DMSO-$d_6$)δ:0.90 (9H,m),1.24(2H,m),1.38(2H,m),2.29 (2H,m) ,2.36(2H,m),2.56(2H,d,J=8.8Hz),2.68(2H,d, J=11.7Hz),
FABMS : 172 (M+1)

Reference Example 111

1-Benzyl-N-methyl-N-propyl-4-piperidinamine was prepared in the same procedure to Reference Example 106 by using 1-benzylpiperidin-4-one. (yield 34.2%)
$^1$H-NMR (DMSO-$d_6$)δ:0.82(3H,t,J=7.3Hz),1.32-1.48 (3H,m),1 .60 (2H,d,J=11.0Hz),1.87(2H,t,J=11.7Hz),2.12(3H,s), 2.31 (3H,t,J=7.3Hz),2.51(1H,d,J=1.5Hz),2.81(2H,d,J=11.7Hz), 3.41(4H,s),7.28(5H,m)ppm
FABMS:247(M+1)

Reference Example 112

N-Methyl-N-propyl-4-piperidynamine-2 hydrochloride salt was prepared in the same procedure to Reference Example 103 using the compounds in Reference Example 111. (yield 40.5%)
$^1$H-NMR(DMSO-$d_6$) δ: 0.88(3H,t,J=7.3HZ),1.61(2H, q),1.84(2H,m), 2.01(2H,m),2.72(2H,s),2.87(2H,dt,J=2.9,12.4Hz), 3.14(1H,br),3.30(2H,s),3.34(1H,s),9.33(1H,br)ppm

Reference Example 113

Pyridine (10.8 mL, 133 mmol) and thionyl chloride (10.8 mL, 133 mmol) were added to a chloroform solution (150 mL) of 2-butyne-1,4-diol (10.4 g, 121 mmol), and the mixture was stirred under nitrogen atomosphere for 6 hours. The reaction solution was added with water and extracted with chloroform. The organic layer was washed with aqueous sodium carbonate solution, and with water, dried over anhydrous magnesium sulfate, and concentrated under reduced pressure. The residue was distilled (75˚C, 1.5 mm Hg), to obtain 4-chloro-2-butyne-1-ol (4.01 g, 31.8%) as a colorless oil.
$^1$H-NMR (DMSO-$d_6$) δ: 2.43(1H,br), 4.20(2H,s), 4.33(2H,s) ppm

Reference Example 114

A tetrahydrofuran solution (40 mL) of 2-butyne-1,4-diol (10.0 g, 116 mL) was added dropwise to a tetrahydrofuran solution (100 mL) of lithium aluminum hydride (5.73g, 151 mmol), and the mixture was stirred at room temperature for 16 hours. The reaction solution was cooled to -10˚C, added with water (2.72mL, 151 mmol), 15% sodium hydroxide aqueous solution (2.72 mL, 151 mmol), and then water (8.16 mL, 453 mmol), one by one, and the mixture was stirred for 2 hours. The residue was separated by filtration, and the filtrate was dried, concentrated and distilled under reduced pressure (110˚C, 1.5 mm Hg), to obtain (2E)-2-butene-1,4-diol (8.11 g, 79.2%) as a colorless oil.
$^1$H-NMR(DMSO-$d_6$) δ:2.07(2H,s),4.17(4H,s),5.98(2H,s)ppm

Reference Example 115

Pyridine (5.05mL, 62.4 mmol) and thionyl chloride (62.4 mL, 4.55 mmol) were added to a chloroform solution (60 mL) of the compound in Reference Example 114 (5.00 g, 56.7 mmol), and the mixture was stirred under nitrogen atmosphere at room temperature for 2 hours. The reaction solution was concentrated under reduced pressure; toluene was added additionally; and the mixture was concentrated under reduced pressure. The product was used without purification in the next reaction.

Triethylamine (0.109 mL, 0.785 mol) was added to a mixed solution of acetonitrile (50 mL) and tetrahydrofuran (50 mL) containing the product obtained in the reaction above and N-methylpropylamine (17.4 mL, 170 mmol), and the mixture was stirred at 43˚C under nitrogen atmosphere for 6 hours. The reaction solution was cooled to room temperature and then concentrated under reduced pressure. Ethyl acetate was added to the residue, and the product was extracted with aqueous citric acid solution. The aqueous phase obtained was made basic by addition of sodium bicarbonate and the organic matter was extracted with ethyl acetate. The organic layer obtained was dried over anhydrous magnesium sulfate and concentrated under reduced pressure; the residue was distilled (120˚C, 1.5 mm Hg) and purified by NH-silica gel column chromatography (hexane/ethyl acetate, 1:1), to obtain
(2E)-4-[methyl (propyl) amino]-2-butene-1-ol (2.92 g, 35.9%) as a colorless oil.
$^1$H-NMR (DMSO-$d_6$) δ:0.89(3H, t, J=7.3Hz) ,1.50 (2H,q,J=7.3Hz) , 2.20(2H,s),2.30(2H,t,J=7.3Hz),2.99(3H,t,J=2.2Hz), 4.12 (2H,m),5.77(2H,s)ppm

Reference Example 116

Triethylamine (4.5 g, 44.5 mmol) was added to a tetrahydrofuran solution (60mL) of morpholine (3g, 32. 7 mmol), and a tetrahydrofuran solution (20 mL) of chloroacetyl chloride (3.9 g, 34.5 mmol) was added dropwise, gradually while the solution was ice-water-cooled. Ether was added to the residue; the precipitated triethylamine hydrochloride was removed by filtration; and the filtrate was concentrated under reduced pressure. The residue obtained was purified by distillation under reduced pressure (10 mm Hg, 110 to 112˚C), to obtain 4-(chloroacetyl)morpholine (4.1 g, 76.6%) as a pale yellow oil.
$^1$H-NMR($CDCl_3$)δ:3.50-3.79(8H,m),4.07(2H,s)ppm

Reference Example 117

Thionyl chloride (0.28 mL, 3,9 mmol) and N,N-dimethylformamide (2 drops) were added to a tetrahydrofuran

solution (10 mL) of the compound in Reference Example 48 (0.3 g, 1.30 mmol), and the mixture was stirred at room temperature for 3 hours. The solution was concentrated under reduced pressure, and dissolved in tetrahydrofuran (10 mL). The solution was added dropwise to a tetrahydrofuran solution (15 mL) containing the compound in Reference Example 4 (0.2 g, 1.23 mmol) and pyridine (0.145 g, 1.83 mmol), while the solution was ice-water-cooled. After stirring at room temperature for 2 hours, the reaction solution was washed with water, dried over anhydrous sodium sulfate, and concentrated under reduced pressure. The residue was purified by silica gel column chromatography (hexane/ethyl acetate, 20:5 to 19:6), to obtain 2-bromo-5-[(4-methyl-2,3,4,5-tetrahydro-1H-1,5-benzodiazepin-1-yl)carbonyl]phenyl methyl ether (0.34 g, 73.7%) as a colorless solid.
$^1$H-NMR($CDCl_3$)δ:2.34(3H,s),5.03-5.36(4H,br),6.01-6.12(2H,m), 6.66(1H,d,J=3.7Hz),6.70(1H,t,J=2.2Hz),6.82-6.93 (1H,m), 7.04-7.25(4H,m),7.29-7.49(6H,m),7.58(1H,d,J=3.7Hz),7.65 (1H,d,J=1.5Hz),7.76(1H,d,J=8.8Hz),8.00(1H,d, J=8.8Hz)ppm
FABMS:376(M+1)

Reference Example 118

2-Bromo-5-[(4,4-dimethyl-2,3,4,5-tetrahydro-1H-1,5-benzodiazepin-1-yl)carbonyl]phenyl methyl ether was prepared in the same procedure to Reference Example 117 by using the compounds in Reference Examples 8 and 48. (yield 84.5%)
$^1$H-NMR(DMSO-$d_6$,120°C) δ:1.21(6H,s),1.73(2H,t,J=5.9Hz),3.60 (3H,s),3.65-3.93(2H,m),3.78(1H,brs),6.49-6.62(2H, m),6.74 (1H,dd,J=7.8,2.0Hz),6.80(1H,d,J=2.0Hz),6.9S(2H,d,J=3.9Hz), 7.31(1H,d,J=8.8Hz)ppm
FABMS:390(M+1)

Reference Example 119

Triethylamine (0.46 mL, 3.25 mmol) was added to a methylene chloride solution (4 mL) of the compound in Reference Example 34 (400 mg, 1.63 mmol) under nitrogen stream while the mixture was cooled on an ice bath; then, 2, 4, 6-trichlorobenzoyl chloride (0.26 mL, 1.63 mmol) was added thereto; and the mixture was stirred at room temperature for 1.5 hours. The reaction mixture was added dropwise to a methylene chloride solution (50 mL) containing the compound in Reference Example 4 (264 mg, 1.63 mmol) and 4-dimethylamznopyridine (489 mg, 2.0 mmol). The mixture was stirred at room temperature for 4.5 hours, and then concentrated under reduced pressure. The residue obtained was poured into saturated aqueous citric acid solution, and the organic matter was extracted with ethyl acetate. The organic layer was washed with saturated sodium hydrogen carbonate solution and with saturated aqueous sodium chloride solution, dried over anhydrous magnesium sulfate, and concentrated under reduced pressure. The residue obtained was purified by column chromatography (ethyl acetate/chloroform, 1:4), to obtain 1-(4-bromo-3-nitrobenzoyl)-4-methyl-2,3,4,5-tetrahydro-1H-1,5-benzodiazepine (490 mg, 77.0%) as a yellow solid.
$^1$H-NMR($CDCl_3$)δ:1.38(3H,d,J=6.4Hz),1.75-1.90(1H,m),2.72-2.82 (1H,m),3.06-3.13(1H,m),3.58(1.H,br),5.00-5.06(1H, m), 6.61-6.69(2H,m),6.89(1H,d,J=7.8Hz),6.97-7.10(1H,m),7.25 (1H,dd,J=7.8,2.0HZ),7.48(1H,d,J=8.3Hz),7.67(1H,d, J=2.0Hz) ppm
FABMS: 391 (M+1)

Reference Example 120

A mixed solution of ethylene glycol dimethyl ether (20 mL) and water (10 mL) containing the compound in Reference Example 119 (490 mg, 1.26 mmol), the compound in Reference Example 70 (127 mg, 1.26 mol), tetrakis (triphenylphosphine)palladium (0) (44 mg, $3.78\times10^{-2}$ mmol) and sodium bicarbonate (318 mg, 3.78 mmol) was heated under reflux for 15.5 hours. The reaction solution was concentrated under reduced pressure, the residue obtained was poured into saturated aqueous sodium chloride solution; and the organic matter was extracted with ethyl acetate. The organic layer was washed with saturated aqueous sodium chloride solution, dried over anhydrous magnesium sulfate, and concentrated under reduced pressure. The residue was purified by silica gel chromatography (hexane/ethyl acetate, 3:2), to obtain
1-[4-(1H-indol-5-yl)-3-nitrobenzoyl]-4-methyl-2,3,4,5-tetrahydro-1H-1,5-benzodiazepine (435mg, 81%) as a colorless solid.
$^1$H-NMR(DMSO-$d_6$,120°C)δ:1.26(3H,d,J=6.3Hz),1.63-1.76(1H,m), 1.90-2.00(1H,m),3.37-3.50(1H,m),4.97(1H,s), 6.42-6.44(1H,m), 6.45-6.56(1H,m),6.69(1H,d,J=7.8Hz),6.91(1H,dd,J=8.3,1.5Hz), 6.96-7.00(2H,m),7.30-7.41(6H,m), 7.47(1H,dd,J=8.3,1.5Hz), 7.58(1H,d,J=2.5Hz)ppm
FABMS:427(M+1)

Reference Example 121

(4R)-1-(4-Bromobenzoyl)-4-methyl-2, 3,4, S-tetrahydro-1-H-1,5-benzodiazepine was prepared in the same procedure to Reference Example 117 using the compounds in Reference Example 6 and 4-bromobenzoyl chloride.

Reference Example 122

(4R)-1-(4-Bromobenzoyl)-4-methyl-2,3,4,5-tetrahydro-1H-1,5-benzodiazepine was prepared in the same procedure to Reference Example 117 using the compounds in Reference Example 5 and 4-bromobenzoyl chloride. (yield 57%)

Reference Example 123

1-(4-Bromo-3-nitrobenzoyl)-4 -isopropyl-2,3,4,5-tetrahydro-1H-1,5-benzodiazepine was prepared in the same procedure to Reference Example 117 by using the compounds in Reference Examples 12 and 34. (yield 80.3%)
$^1$H-NMR($CDCl_3$)δ:1.08 (6H,d,J=6.6Hz)1,71-2.12(2H,m)2.66-2.86 (2H,m),3.31-3.54(1H,m),4.97-5.10(1H,m),6.44-6.53 (1H,m), 6.55-6.74(2H,m),6.84-6.93(1H,m),6.96-7.12(1H,m),7.21-7.29(1 H,m),7.44-7.56(1H,m),7.63-7.71(1H,m)
FABMS:418(M+1)

Reference Example 124

1-(4-Bromo-3-nitrobenzoyl)-4-ethyl-2,3,4,5-tetrahydro-1H-1,5-benzodiazepine was prepared in the same procedure to Reference Example 117 by using the compounds in Reference Example 10 and 34. (yield 99.5%)
$^1$H-NMR ($CDCl_3$)δ:1.08 (3H, t, J=7.3Hz),1. 60-2.00 (3H,m), 2.71-2. 90 (2H,m), 3.50-3.86(1H,m), 4.70-5,10 (1H,m), 6.45-6.56(1H,m), 6.59-6.72(2H,m),6.90(1H,d,J=7.3Hz),6.96-7.12(1H,m), 7.45-7.55(1H,m),7.64-7.70(1H,m)ppm
FARMS:404(M+1)

Reference Example 125

The compound in Reference Example 119 (4.14 g, 10.6 mmol) and nickel chloride-hexahydrate (4.75 g, 20.0 mmol) were added to methanol (160 mL) , and the solution was stirred while cooled on an ice bath. Sodiumborohydride (1 .89 g, 50.0 mmol) was added gradually, so that the reaction temperature did not rise. Then, after stirring at room temperature for 30 minutes, the mixture was concentrated under reduced pressure, and the residue was mixed with ethyl acetate and filtered through Celite. The filtrate was concentrated under reduced pressure; and the residue was purified by silica gel column chromatography (hexane:ethyl acetate/3:2), to obtain 2-bromo-5-[(4-methyl-2,3,4,5-tetrahydro-1H-1,5-benzodiazepin-1-yl)carbonyl]phenylaniline (2.41 g, 63.2%) as a colorless solid.
$^1$H-NMR(DMSO-$d_6$,120˚C)δ:1.33(3H,d,J=6.4Hz),1.65-1.79(1H,m), 1.96-2.07(1H,m),3.51(2H,br),4.14(1H,br),4.88(1H, br),5.02 (2H,br),6.37(1H,dd,J=8.3,2.0Hz),6.54-6.60(1H,m),6.69 (1H,d,J=7.8Hz),6.92(1H,d,J=2.0Hz),6.97-7.04(2H,m), 7.17 (1H, d,J=8.3Hz)ppm
FABMS:361(M+1)

Reference Example 126

An N,N-dimethylformamide solution (4 mL) containing the compound in Reference Example 125 (360 mg, 1.0 mmol), sodium bicarbonate (126 mg, 1.69 mmol) and bromobutyric acid ethyl ester (344 mg, 1.5 mmol) was stirred while heated at 50˚C under nitrogen stream for one day. The reaction solution was poured into saturated aqueous sodium chloride solution, and the organic matter was extracted with ethyl acetate. The organic layer was washed with saturated aqueous sodium chloride solution, dried over anhydrous magnesium sulfate, and concentrated under reduced pressure. The residue was purified by silica gel column chromatography (hexane: ethyl acetate / 3:2) to obtain benzyl [2-bromo-5-[(4-methyl-2,3,4,5-tetrahydro-1H-1,5-benzodiazepin-1-yl)carbonyl]anilino]acetate (225 mg, 44.3%) as a colorless solid.
$^1$H-NMR (DMSO-$d_6$, 120˚C)δ:1.20 (3H, d, J=6.4Hz), 1.55-1. 68 (1H,m), 1.84-1.90(1H,m),3.37-3.51(2H,m),3.87(2H,d, J=5.9Hz), 4.05-4.15(1H,m),4,79(1H,br),5.13-5.22(1H,m),5.28(2H,s), 6.40-6.60(4H,m),6.84-6.90(2H,m),7.20(1H,d, J=8.3Hz), 7.26-7.39(5H,m)ppm
FABMS:509(M+1)

Reference Example 127

A mixed solution of methanol (20 mL) and water (1.2 mL) containing the compound in Reference Example 126 (358 mg, 0.704 mmol) and potassium carbonate (506 mg, 3.18 mmol) was stirred while heated under reflux for one day. The reaction mixture was poured into saturated aqueous citric acid solution; the precipitated solid was collected by filtration and washed with water, to obtain
[2-bromo-5-[(4-methyl-2,3,4,5-tetrahydro-1H-1,5-benzodiazepin-1-yl)carbonyl]anilino]acetic acid (162 mg, 55.0%) as a colorless solid.
$^1$H-NMR(DMSO-$d_6$, 120˚C) δ: 1.23 (3H,d,J=6.4Hz), 1.59-1.69(1H,m), 1.87-196(1H,m),3.38(2H,br),3.68(2H, s),4.03(1H, br),6.41-6.48 (3H,m),6.58(1H,d,J=7.3Hz),6.90-6.91(2H,m),7.19 (1H,d,J=7.8Hz)ppm
FABMS: 419 (M+1)

Reference Example 128

A methylene chloride solution (2 mL) of 1-ethyl-3-(3-dimethylaminopropyl)carbodiimide-hydrochloride salt (82 mg, 2.4 mmol) was added dropwise to an N,N-dimethylformamide solution (2 mL) containing the compound in Reference Example 127 (162 mg, 0.387 mmol), N-methylpiperazine (43 mg, 0.426 mmol), 3,4-dihydro-3-hydroxy-4-oxo-1,2,3-benzotriazine (70 mg, 0.426 mmol), and triethylamine (43 mg, 2.4 mmol) under nitrogen stream, while the mixture was cooled on an ice bath. After dropwise addition, the mixture was stirred at room temperature for 10 hours, and the reaction solution was then concentrated under reduced pressure. The residue was poured into saturated sodium bicarbonate, and the organic matter was extracted with ethyl acetate. The organic layer was washed with saturated aqueous sodium chloride solution, dried over anhydrous magnesium sulfate, and concentrated under reduced pressure. The residue was purified by NH-silica gel column chromatography (hexane:ethyl acetate/1:9), to obtain 2-bromo-N-[2-(4-methyl-1-piperazinyl)-2-oxoethyl]-5-[(4-methyl-2,3,4,5-tetrahydro-1H-1,5-benzodiazepin-1-yl) carbonyl]aniline (108 mg, 55.8%) as a colorless solid.
$^1$H-NMR(DMSO-$d_6$, 120˚C) δ :1.24 (3H,d, J=6.3Hz),1.57-1.71(1H,m) 1.89-1.99 (1H,m),2.24(3H,s), 2.36(4H,t,J=5.6Hz), 3.46-3.78 (8H,m),4.04(1H,br),4.85(1H,s),5.35(1H,s),6.41-6.60(4H,m), 6.91(1H,d,J=3.4Hz),7.19(1H,d,J=8.3Hz)ppm
FABMS:537(M+1)

Reference Example 129

Two drops of sulfuric acid was added to a methyl orthoformate solution (14 mL) of the compound in Reference Example 125 (720 mg, 2.0 mmol), and the mixture was stirred at an internal temperature of 70˚C for 5 hours. Then, the internal temperature was raised to 140˚C, until exsiccation. The residue was extracted with ethyl acetate and saturated aqueous bicarbonate solution. The organic layer was washed with saturated aqueous sodium chloride solution, dried over anhydrous magnesium sulfate, and concentrated under reduced pressure. Tetrahydrofuran (5 mL) and 1N aqueous hydrochloric acid solution (10 mL) were added to the crude product obtained, and the mixture was stirred while heated under reflux for 5 hours. After the reaction was completed, the mixture was made basic by addition of saturated aqueous bicarbonate solution. The aqueous phase was extracted with ethyl acetate; the organic layer was washed with saturated aqueous sodium chloride solution, dried over anhydrous magnesium sulfate, and concentrated under reduced pressure. The residue obtained was purified by silica gel column chromatography (hexane:ethyl acetate/2:3), to obtain 2-bromo-N-methyl-5-[(4-methyl-2,3,4,5-tetrahydro-1H-1,5-benzodiazepin-1-yl)carbonyl]aniline (154 mg, 20.6%) as a colorless solid.
$^1$H-NMR(DMSO-$d_6$,120˚C)δ:1.23(3H,d,J=6.4Hz),1.57-1.70(1H,m), 1.88-1.96(1H,m),2.60(3H,d,J=5.4Hz),3.39(2H,br), 4.06(1H,br), 4.86(2H,br),6.41-6.49(3H,m),6.57(1H,d,J=7.3Hz),6.86-6.92 (2H,m),7.17(1H,d,J=7.8Hz)ppm
FABMS:374(M+1)

Reference Example 130

2-Bromo-5-[(4-methyl-2,3,4,5-tetrahydro-1H-1,5-benzodiazepin-1-yl)carbonyl]benzonitrile was prepared in the same procedure to Reference Example 117 by using the compounds in Reference Examples 4 and 42. (yield 77.8%)
$^1$H-NMR(DMSO-$d_6$,120˚C)δ:1.24(3H,d,J=6.4Hz),1.60-1.73(1H,m), 1.89-1.96(1H,m),
3.38(2H,br),4.06(1H,br),4.99(1H,br),6.45-6.51(1H,m),6.61 (1H,d,J=7.8Hz),6.95-6.97(2H,m),7.38(1H,dd,J=8.3,2.0Hz), 7.53(1H,d,J=2.0Hz),7.64(1H,d,J=8.3Hz)ppm
FABMS:371(M+1)

Reference Example 131

2-(1H-indol-5-yl)-5-[(4-methyl-2,3,4,5-tetrahydro-1H-1,5-benzodiazepin-1-yl)carbonyl]benzonitrile was prepared in the same procedure to Reference Example 120 by using the compounds in Reference Examples 130 and 70. (yield 78.2%)
$^1$H-NMR(DMSO-$d_6$,120˚C)δ:1.26(3H,d,J=6.4Hz),1.62-1.76(1H,m), 1.92-1.96(1H,m),3.41(2H,br),4.09(1H,br),4.99(1H, s), 6.47-6.54(2H,m),6.66(1H,d,J=8.3Hz),6.97-6.98(2H,m), 7.13-7.19(1H,m),7.33-7.64(6H,m)ppm
FABMS:407 (M+1)

Reference Example 132

1-Benzyl-5-(4-bromo-3-nitrobenzoyl)-2-methyl-2,3,4,5-tetrahydro-1H-1,5-benzodiazepine was prepared in the same procedure to Reference Example 117 by using the compounds in Reference Examples 3 and 34. (yield 74.6%) $^1$H-NMP,(DMSO,120˚C)δ:1.03(3H,brs),1.42-1.65(1H,m),1.92-2.07 (1H,m),3.36-4.15(3H,m),4.41(2H,dd,J=5.9,20.5Hz), 6.62-6.76 (2H,m),7.02-7.34(6H,m),7.39(2H,d,J=6.8Hz),7.48 (1H,d,J=7.8Hz),7.56(1H,d,J=2.0Hz)ppm
FABMS:480(M+1)

Reference Example 133

1-Benzyl-5-[4-(1H-indol-5-yl)-3-nitrobenzoyl]-2-methyl-2,3,4,5-tetrahydro-1H-1,5-benzodiazepine was prepared in the same procedure to Reference Example 120 by using the compounds in Reference Examples 132 and 70. (yield 68.0%)
$^1$H-NMR(DMSO,120˚C)δ:1.05-(3H,brs),1.44-1.65(1H,m),1.93-2.09 (1H,m,)3.37-3.86(2H,m)3.89-4.14(1H,m)4.35-4.42 (2H,m), 6.40-6.45(1H,m),6.67-6.81 (2H,m), 6.88(1H, dd, J=2.0,8.8Hz), 7.11-7.32(8H,m),7.35-7.44(4H,m),7.53(1H,brs), 10.85(1H,brs) ppm
FABMS:517(M+1)

Reference Example 134

5-[(5-Benzyl-4-methyl-2,3,4,5-tetrahydro-1H-1,5-benzodiazepin-1-yl)carbonyl]-2-(1H-indol-5-yl)aniline was prepared in the same procedure to Reference Example 125 using the compounds in Reference Example 133. (yield 42.8%)
$^1$H-NHR(DMSO, 120˚C) δ:1.04(3H,d,J=6.8Hz),1.45-1.60(1H,m), 1.87-2.05(1H,m),3.33-3.69(3H,m),3.82-4.03(1H,m), 4.22 (1H,brs),4.43(2H,dd,J=5.9,20.5Hz),6.30(2H,dd,J=5.9,7.8Hz), 6.38-6.43(1H,m),6.62-6.76(4H,m),6.96-7.02(1H,m), 7.06-7.11 (2H,m),7.15-7.23 (1H, m) ,7.25-7.32 (3H,m) ,7.37.46(4H,m), 10.71(1H,brs)ppm
FABMS:487(M+1)

Reference Example 135

The compound in Reference Example 134 (0.20 g, 0.4 mmol) was added to a mixed solution of acetic anhydride (0.4 mL) and formic acid (0.8 mL), and the mixture was stirred at room temperature for 1 hour. The reaction solution was poured into ice water, and the precipitated crystals were collected by filtration. The crystals obtained were dissolved in ethyl acetate; the solution was washed with sodium bicarbonate aqueous solution and then with saline solution, dried over anhydrous sodium sulfate, and concentrated under reduced pressure. The residue obtained was purified by silica gel column chromatography (chloroform:methanol/ 248:2), to obtain 5-[(5-benzyl-4-methyl-2,3,4,5-tetrahydro-2H-1,5-benzodiazepin-1-yl)carbonyl]-2-(1H-indol-5-yl)phenyl formamide (0.17 g, 77.9%) as a yellow oil.
$^1$H-NMR (DM-SO,120˚C) δ :1.17(3H,d,J=6.8Hz),1.44-1.66(1H,m), 1.87-2.06(2H,m),3.32-3.68(2H,m),3.89-4.1.2(1H,m), 4.44 (2H, dd, J=6.8,21.4Hz), 6.41-6.41 (1H,m),6.62-6.84 (3H,m), 6.88-6.96(2H,m),7.05-7.35(5H,m),7.37-7.45(4H,m), 7.57-7.82 (1H,br), 7.96-8.03 (1H,m), 8.51-8.62 (1H,br), 10.78 (1H,brs) ppm
FABMS:515(M+1)

Reference Example 136

Potassium cyanate (53 mg, 0.62 mmol) was added to a mixed solution of acetic acid (1.5 mL) and water (9 mL) containing the compound in Reference Example 134 (0.28 g, 0.58 mmol) while cooled on an ice bath, and the mixture was stirred for 1 hour. The reaction solution was concentrated under reduced pressure; the concentrated residue obtained was added with water and made neutral by addition of aqueous sodium hydroxide solution. Then, the organic matter was extracted with ethyl acetate, and the organic layer was washed with water, dried over anhydrous sodium

sulfate, and concentrated under reduced pressure. The residue obtained was purified by recrystallization (ethyl acetate and hexane), to obtain N-[5-[(5-benzyl-4-methyl-2,3,4,5-tetrahydro-1H-1,5-benzodiazepin-1-yl)carbonyl]-2-(1H-indol-5-yl)phenyl]urea (0.28 g, 90.3%) as a colorless solid.
$^1$H-NMR(DMSO,120˚C)δ:1.03 ( 3H, d, J =5.9Hz),1.43-1.60(1H,m), 1.75(3H,s),1.88-2.03(1H,m),3.33-3.69(2H,m), 3.84-4.09(1H,m), 4.43 (2H, dd, J=8.8, 23.5Hz), 6.38-6. 45 (1H,m),6. 62-6.78 (3H,m) , 6.87-7.47(12H,m), 7.70(1H,brs), 10.75(1H,brs)ppm
FABMS:529(M+1)

Reference Example 137

(4R)-1-(4-Bromo-3-nitrobenzoyl)-4-methyl-2,3,4,5-tetrahydro-1H-1,5-benzadzazepine was prepared in the same procedure to Reference Example 117 by using the compounds in Reference Examples 6 and 34. (yield 59.0%) $^1$H-NMR($CDCl_3$)δ:1.38(3H,d,J=6.4Hz),1.75-1.90(1H,m),2.72-2.82 (1H,m),3.06-3.13(1H,m),3.58(1H,br),5.00-5.06(1H,m), 6.61-6.69(2H,m),6.89(1H,d,J=7.8Hz),6.97-7.10(1H,m),7.25 (1H,dd,J=7.8,2.0Hz),7.48(1H,d,J=8.3Hz),7.67(1H,d, J=2.0Hz) ppm
FABMS:391(M+1)

Reference Example 138

(4R)-1-[4-(1H-Indol-5-yl)-3-nitrobenzoyl]-4-methyl-2, 3, 4, 5-tetrahydro-1H-1, 5-benzodiazepine was prepared in the same procedure to Reference Example 120 by using the compounds in Reference Examples 137 and 70. (yield 98%)
$^1$H-NMR(DMSO-$d_6$,120˚C) δ:1.26 (3H, d, J=6.3Hz), 1.63-1.76(1H,m), 1.90-2.00(1H,m),3.37-3.50(1H,m),4.97(1H,s), 6.42-6.44(1H,m), 6.45-6.56(1H,m),6.69(1H,d,J=7.8Hz),6.91(1H,dd,J=8.3,1.5Hz), 6.96-7.00(2H,m),7.30-7.41(6H,m), 7.47(1H,dd,J=8.3,1.5Hz), 7.58(1H,d,J=1.5Hz)ppm
FABMS:427(M+1)

Reference Example 139

(4S)-1-(4-Bromo-3-nitrobenzoyl)-4-methyl-2,3,4,5-tetrahydro-1H-1,5-benzodiazepine was prepared in the same procedure to Reference Example 117 by using the compounds in Reference Examples 5 and 34. (yield 75.6%)
$^1$H-NMR(DMSO-$d_6$,120˚C)δ:1.24 (3H,d,J=5.9Hz),1.58-1.75 (1H,m), 1.82-1.99(1H,m),3.08-4.37(3H,m),4.97(1H,brs), 6.43-6.55 (1H,m),6.64(1H,d,J=7.8Hz),6.95(1H,d,J=3.9Hz),7.29-7.38 (1H,m),7.62-7.72(2H,m)ppm
FABMS:390(M+1)

Reference Example 140

(4S)-1-[4-(1H-Indol-5-yl)-3-nitrobenzoyl]-4-methyl-2,3,4,5-tetrahydro-1H-1,5-benzodiazepine was prepared in the same procedure to Reference Example 117 by using the compounds in Reference Examples 139 and 70. (yield 60.4%)
$^1$H-NMR(DMSO-$d_6$,120˚C) δ:1.26 (3H,d,J=6.8Hz),1.61-1.78 (1H,m), 1.87-2.03(1H,m),3.24-4.39(3H,m),4.97(1H,brs), 6.41-6.45 (1H,m),6.47-6.56(1H,m),6.69(1H,d,J=7.8Hz),6.86-7.01(3H,m), 7.28-7.43(4H,m),7.44-7.49(1H,m),7.54-7.61 (1H,m),10.85 (1H, brs) ppm
FABMS:426(M+1)

Reference Example 141

[5-(4-Bromo-3-nitrobenzoyl)-2,3,4,5-tetrahydro-1H-1,5-benzodiazepin-2-yl]methyl tert-butyl(diphenyl) ether was obtained by the same procedure as in Reference example 117 using the compounds in Reference Examples 17 and 34 (yield 71.9%).
FABMS:417(M+1)

Reference Example 142

4-[[[tert-Butyl(diphenyl)silyl]oxy]methyl]-1-[4-(1H-indol-5-yl)-3-nitrobenzoyl]-2,3,4,5-tetrahydro-1H-1,5-benzodiazepine was obtained by the same procedure as in Reference Example 120 using the compounds in Reference Examples 141 and 70 (yield 81.6%)
$^1$H-NMP(DMSO-$d_6$, 120˚C)δ:1.08(9H,s),1.77-2.07(2H,m),3.21-3.58 (1H,m),3.71-3.88(2H,m),3.94-4.46(1H,m),4.97(1H, s), 6.38-6.46(1H,m),6.53-6.62(1H,m),6.72(1H,d,J=7.8Hz), 6.84-6.93(2H,m),6.95-7.04(1H,m),7.28-7.47(11H,m),7.57

(1H,s),7.61-7.69(4H,m),10.85(1H,brs)ppm
FABMS:681(M+1)

Reference Example 143

10% Palladium-carbon (0.1 g) was added to the methanol solution (70 mL) of the compound (0.27g, 0.40mmol) in Reference Example 142 in a nitrogen stream by cooling with ice. After stirring the mixture at room temperature for 12 hours in a nitrogen stream, the reaction solution was filtered through a filter (0.22 μm: trade name FM-22, manufactured by Fuji Film Co.). Palladium-carbon was washed with methanol, and the filtrate and washing solution were combined and concentrated in vacuum. The residue obtained was purified by column chromatography (chloroform : methanol/ 249:1) to obtain 5-[[4-[[[tert-butyl-(diphenyl)silyl]oxy]methyl]-2,3,4,5-tetrahydro-1H-1,5-benzodiazepzn-1-yl]carbonyl]-2-(1H-indol-5-yl)aniline (0.21 g, 81.7%) as a colorless oil.
$^1$H-NMR(DMSO-$d_6$,120˚C)δ:1.19(9H,s),1.71-1.86(1H,m),1.96-2.11 (1H,m),3.42-3.78(2H,m),3.81-3.96(1H,m),4.44(2H, brs),4.92 (1H,brs),6.48-6.57(2H,m),6.62-6.72(1H,m),6.78-6.98(4H,m), 7.02-7.15(2H,m),7.36-7.42(1H,m),7.46-7.59 (8H,m),7.73-7.81 (4H,m),10.82(1H,brs)ppm
FABMS:651 (M+1)

Reference Example 144

A tetrahydrofuran solution (10 mL) of acetyl chloride (24 mg, 0.31 mmol) was gradually dropped into a tetrahydrofuran solution (50 mL) of the compound (0.2 g, 0.31 mmol) in Reference Examples 143 and pyridine (30 mg, 0.38 mmol). After stirring the mixture at room temperature for 2 hours, the reaction solution was poured into water, and the reaction product was extracted with ethyl acetate. After washing the organic layer with water, the organic layer was dried over anhydrous sodium sulfate and concentrated vacuum. The residue obtained was purified by silica gel column chromatography (chloroform : methanol / 249:1 to 247:3) to obtain N-[5-[[4-[[[tert-butyl(diphenyl)silyl]oxy]methyl]-2,3,4,5-tetrahydro-1H-1,5-benzodiazepin-1-yl]carbonyl]-2-(1H-indol-5-yl)phenyl]acetamide (0.21 g, 97.8%) as a pale yellow oil.
$^1$H-NMR(DMSO-$d_6$,120˚C)δ:1.08(9H,s) ,1.62-1.82(4H,m),1.86-2.03 (1H,m),3.33-3.65(2H,m),3.59-3.86(2H,m), 3.95-4.22(1H,m), 4.85(1H,brs),6.39-6.46(1H,m),6.49-6.58(1H,m),6.65-6.72 (1H,m),6.80-6.87(1H,m),6.89-7.06(4H,m), 7.26-7.31(1H,m), 7.36-7.48(8H,m),7.62-7.69(5H,m),8.36(1H,brs),10.75(1H,brs) ppm
FABMS:693(M+1)

Reference Example 145

N-Carbobenzyloxyglycine (0.21 g, 1.02 mmol), 1-ethyl-3-(3-dimethylaminopropyl)carbodiimide hydrochloride (0.19 g, 1.02 mmol) and 1-hydroxybenzotriazole hydrate (0.14 g, 1.02 mmol) were added to an N,N-dimethylformamide solution (3 mL) of the compound (0.49 g, 1.02 mmol) in Reference Example 134, and the solution was stirred at room temperature for 23 hours. After completing the reaction, water and ethyl acetate were added to the reaction mixture to extract the reaction product. The ethyl acetate layer was washed with saline solution, dried over anhydrous sodium sulfate and concentrated in vacuum. The residue obtained was purified by silica gel column chromatography (chloroform : methanol / 123:1) to obtain
benzyl 2-[5-[(5-benzyl-4-methyl-2,3,4,5-tetrahydro-1H-1,5-benzoazepin-1-yl)carbonyl]-2-(1H-indol-5-yl)anilino]-2-oxoethylcarbamate (0.60g, 87.6%) as an orange amorphous solid.
$^1$H-NMR (DMSO-$d_6$,120˚C) δ:1.03 (3H,d,J=5.9Hz),1.42-1. 65 (1H.m), 1.86-2.07(1H,m),3.32-4.74(4H,m),3.86-4.11(1H, m),4.44 (2H,q,J=4.7Hz),4.86(2H,s),6.41-6.46(1H,m),6.64-6.77(3H,m), 6.79-6.97(4H,m),7.03-7.14(2H,m),7.15-7.36 (2OH,m),7.37-7.47 (4H,m),8.02(1H,brs),8.47(1H,brs),10.76(1H,brs)ppm
FABMS : 678 (M+1)

Reference Example 146

N,N-Dimethylglycine (0.10 g, 0.72 mmol), 1-ethyl-3-(3-dimethylaminopropyl)carbodiimide hydrochloride (0.14 g, 0.72 mmol) and 1-hydroxybenzotriazale hydrate (0.12 g, 0.72 mmol) were added to an N,N-dimethylformamide solution (6 mL) of the compound (0.35 g, 0.72 mmol) in Reference Example 134, and the mixture was stirred at room temperature for 19 hours. After completing the reaction, the reaction product was diluted with water and extracted with ethyl acetate. The ethyl acetate layer was washed with saline solution, dried over anhydrous sodium sulfate and concentrated in vacuum. The residue obtained was purified by silica gel column chromatography (chloroform : methanol / 248:2) to obtain N-[5-(5-benzyl-4-methyl-2,3,4,5-tetrahydro-1H-1,5-benzodiaz
epin-1-yl)carbonyl]-2-[(1H-indol-5-yl)phenyl]-2-(dimethylamino)acetamide (0.30 g, 73.0%) as a colorless amorphous solid.

$^1$H-NMR(DMSO-$d_6$,120°C)δ:1.02(3H,d,J=6.8Hz),1.42-1.66(2H,m), 1.74-2.06(7H,m),3.28-3.75(3H,m),3.86-4.14(1H,m), 4.36-4.54 (2H,m),6.39-6.46(1H,m),6.64-6.78(3H,m),6.86-6.98(2H,m), 7.03-7.14(2H,m),7.15-7.36(4H,m),7.38-7.49(4H, m),8.21-8.31 (1H,m),9.26(1H,brs),10.79(1H,brs)ppm
FABMS: 572 (M+1)

Reference Example 147

After heating a thionyl chloride solution (4 mL) of 97% benzyloxyacetic acid (87 mg, 0.53 mmol) at 100°C for 2 hours, the solution was concentrated in vacuum, and the residue was azeotropically distilled twice with toluene. A tetrahydrofuran solution (5 mL) of the residue obtained was dropped into a tetrahydrofuran solution (5 mL) of the compound (0.21 g, 0.44 mmol) in Reference Example 134 and pyridine (45 mg, 0.57 mmol). After stirring for 2 hours at room temperature, the reaction product was diluted with water and extracted with ethyl acetate. The ethyl acetate layer was washed with an aqueous citric acid solution and saline solution, dried over anhydrous sodium sulfate and concentrated in vacuum. The residue obtained was purified by silica gel column chromatography (hexane: ethyl acetate / 1:1) to obtain N-[5-(5-benzyl-4-methyl-2,3,4,5-tetrahydro-1H-1,5-benzodiazepin-1-yl)carbonyl]-2-[(1H-indol-5-yl)phenyl]-2-(benzyloxy)acetamide (0.25 g, 89.2%) as a colorless amorphous solid.
$^1$H-NMR(DMSO-$d_6$,120°C)δ:1.02(3H,d,J=5.9Hz),1.42-1.63(1H,m), 1.85-2.08(1H,m),3.30-3.73(2H,m),3.79-4.14(3H,m), 4.29(2H,s), 4.51 (1H, dd, J=14.7, 32.2Hz) , 6.39-6.48 (1H,m), 6. 62-6.78 (3H,m) , 6.85-6.99(4H,m),7.02-7.23 (6H,m), 7.24-7.34 (3H, m), 7.34-7.50 (4H.m) ppm
FABMS:635(M+1)

Reference Example 148

N-[2-Bromo-5-[[4-(dimethylamino) methyl]-2,3,4,5-tetrahydro-1H-1,5-benzodiazepin-1-yl] carbonyl] phenyl] acetamide was obtained by the same procedure as in Reference Example 117 using the compounds in Reference Examples 22 and 38 (yield 43%).
$^1$H-NMR(DMSO-$d_6$)δ :1.4-1.60 (1H,m) 2.03 (3H, s) 2.05-2.15 (2H,m), 2.17(6H,s), 2.30(1H, dd, J=12.0,7.3Hz), 3.05-3.20 (1H,m), 3.40-3.60(1H,m),4.90-5.05(1H,m),5.89(1H,s),6.30 (1H,t,J=7.3Hz),6.44(1H,d,J=7.3Hz),6.64(1H,d,J=7.3Hz), 6.82-6.90(2H,m),7.36(1H,d,J=8.3Hz),7.59(1H,s),9.39(1H,s)ppm

Reference Example 149

1-[4-Bromo-3-(bromomethyl)benzoyl]-4-methyl-2,3,4,5-tetrahydro-1H-1,5-benzodiazepine was obtained by the same procedure as in Reference Example 117 using the compounds in Reference Examples 4 and 55 (yield 16.2%).
$^1$H-NMR(DMSO-$d_6$, 120°C) δ:1.24 (3H,d,J=6.8Hz), 1.58-1.75(1H,m), 1.80-2.00(1H,m),3.15-4.20(4H,m),4.40-4.67(2H, m),6.32-6.70 (2H,m),6.82-7.12(3H,m),7.24-7.54(2H,m),
FABMS:439(M+1)

Reference Example 150

A 1N aqueous sodium hydroxide solution (2 mL) was added to a 50% tetrahydrofuran-water solution (60 mL) of the compound (0.65 g, 1.48 mmol) in Reference Example 149, and the mixed solution was refluxed for 20 hours with heating. The reaction solution was concentrated in vacuum, and the reaction product was extracted with ethyl acetate. The organic layer was washed with water, dried over anhydrous sodium sulfate and concentrated in vacuum. The residue obtained was purified by silica gel column chromatography (chloroform : methanol / 499:1) to obtain [2-bromo-5-[(4-methyl-2,3,4,5-tetrahydro-1H-1,5-benzodiazepin-1-yl)carbonyl]phenyl]methanol (0.32 g, 57.6%) as a pale yellow oil.
$^1$H-NMR(DMSO-$d_6$, 120°C) δ: 1.23 (3H, d, J=5.9Hz), 1.53-1.74 (1H,m), 1.82-1.98(1H,m),3.12-3.78(2H,m),3.88-4.27(1H, m),4.41 (2H,d,J=4.9Hz),4.68-5.02(1H,m),6.38-6.49(1H,m),6.55 (1H,d,J=7.8Hz),6.82-6.97(3H,m),7.27(1H,d,J=7.8Hz), 7.46-7.53 (1H,m) ppm
FABMS : 375 (M+1)

Reference Example 151

1-Benzyl-5-[4-bromo-3-(2-bromoethoxy)benzoyl]-2-methyl-2, 3,4, 5-tetrahydro-1H-1,5-benzodiazepine was obtained by the same procedure as in Reference Example 117 using the compounds in Reference Examples 3 and 46 (yield 93.3%).
$^1$H-NMR(DMSO-$d_6$, 120°C) δ:0.82-1.11(3H,m), 1.39-1.67 (1H,m), 1.84-2.07(1H,m),2.81-3.26(2H,m),3.33-4.11(5H,m),4.47 (2H,dd,J=3.9,11.7Hz),6.48-6.71(3H,m),6.92-7.45(9H,m)ppm
FABMS:559(M+1)

Reference Example 152

5- [(5-Benzyl-4-methyl-2,3,4,5-)-tetrahydro-1H-1,5-benzodiazepin-1-yl)carbonyl]-2-(1H-indol-5-yl)phenyl 2-bromoethyl ether was obtained by the same procedure in Reference Example 120 using the compounds in Reference Examples 151 and 70 (yield 81.7%).
$^1$H-NMR(DMSO-$d_6$,120˚C)δ:1.04(3H,d,J=5.9Hz),1.44-1.66(1H,m), 1.90-2.10(1H,m),3.31-3.S0(3H,m),3.51-3.69(1H, m),3.72-3.86 (2H,m),3.88-4.14(1H,m),4.16-4.55(2H,m),6.38-6.42(1H,m), 6.66-6.77(3H,m),6.87(1H,d,J=7.8Hz), 7.01-7.39(9H,m),7.44 (1H,d,J=5.8Hz),7.57-7.64(1H,m),10.67(1H,brs)ppm
FABMS: S94 (M+1)

Reference Example 153

An N, N-dimethylformamide solution (6 mL) of the compound (0.69 g, 1.16 mmol) in Reference Example 152 and potassium phthalimide (0.24 g, 1.28 mmol) was stirred for 11 hours with heating at 60˚C. The reaction product was diluted with water and extracted with ethyl acetate. The ethyl acetate layer was washed with saline solution twice, dried over anhydrous sodium sulfate and concentrated in vacuum. The residue obtained was purified by silica gel column chromatography (chloroform) to obtain
2-[2-[5-[(5-benzyl-4-methyl-2,3,4,5-tetrahydro-1H-1,5-benzodiazepin-1-yl)carbonyl]-2-(1H-indol-5-yl)phenoxy] ethyl]-1H-isoindole-1,3(2H)-dione (0.68 g, 88.5%) as a pale yellow amorphous solid.
$^1$H-NMR (DMSO-$d_6$, 120˚C) δ:1.02 (3H, d, J=5.9 Hz), 1.43-1.65 (1H, m), 1.88-2.08(1H,m),3.33-3.83(6H,m),3.87-4.14 (1H,br),4.32-4.51 (2H,m),6.23-6.32(1H,m),6.65-6.73(2H,m),6.74-6.84(2H,m), 6.93-7.31(9H,m),7.35-7.44(3H,m), 7.65-7.77(4H,m),10.55 (1H,brs)ppm
FABMS:661(M+1)

Reference Example 154

2-[2-[2-(1H-indol-5-yl)-5-[(5-benzyl-4-methyl-2,3,4,5-tetrahydro-1H-1,5-benzodiazepin-1-yl)carbonyl]-phenoxy]ethyl]-1H-isoindole-1,3(2H)-dione by the same procedure in Reference Example 143 using the compound in Reference Example 153 (yield 24.5%).
$^1$H-NMR (DMSO-$d_6$, 120˚C) δ:1.25 (3H, d, J=5.9Hz) , 1.55-1. 73 (1H,m), 1.84-2.01(1H,m), 3.31-3.59 (2H,m), 3.73-3.86 (2H,m), 3.92-4.30 (3H,m), 4.87 (1H,brs), 6.22-6.28 (1H,m), 6.44-6.53 (1H,m), 6.58-6.66 (1H,m),6.81-7.03 (5H,m), 7.04-7.13 (2H,m), 7.15-7.20 (1H,m), 7.41 (1H,brs), 7.68-7.79 (4H,m), 10.54 (1H,brs)ppm
FABMS: 571 (M+1)

Reference Example 155

N-[5-{4-Bromo-3-nitrobenzoyl]-2-methyl-2,3,4,5-tetrahydro-1H-1,5-benzodiazepin-7-yl-4-methylbenzene sulfonamide was obtained by the same procedure in Reference Example 117 using the compounds in Reference Examples 33 and 34 (yield 83.0%).

Reference Example 156

Conc. sulfuric acid (5 mL) was added to the compound (500 mg, 0.894 mmol) in Reference Example 155, and the solution was stirred for 3 hours at 60˚C. The reaction mixture was poured into saturated saline solution, and the aqueous solution was washed with ethyl acetate. A saturated aqueous solution of sodium hydrogen carbonate was added to the aqueous layer until it became basic, and the aqueous layer was extracted with ethyl acetate. The organic layer was washed with saturated saline solution, dried over anhydrous magnesium sulfate and concentrated in vacuum to obtain
5-(4-bromo-3-nitrobenzoyl)-2-methyl-2,3,4,5-tetrahydro-1H-1, 5-benzodiazepin-7-yl amine (292 mg, 80.9%) as a pale yellow solid.
$^1$H-NMR (DMSO-$d_6$, 120˚C) δ:1.18 (3H,d,J=6.3Hz), 1.55-1.65 (1H,m), 1.79-1.83 (1H, m), 2.85-3.20 (3H, br), 4.21(3H, br), 6.01 (1H,d,J=2.0Hz),6.32(1H,dd,J=8.3,2.4Hz),6.75(1H,d,J=8.3Hz), 7.36(1H,dd,J=8.3,2.0Hz),7.67-7.71(2H,m)ppm
FABMS:406(M+1)

Reference Example 157

A tetrahydrofuran solution (3mL) of benzyl chloroformate (103 mg, 0.605 mmol) was dropped into a tetrahydrofuran solution (2 mL) of the compound (223 mg, 0.55 mmol) in Reference Example 156 and pyridine (57 mg, 0.714 mmol) in a nitrogen stream by cooling with ice. After stirring the reaction solution at room temperature for 1 hour, the solution was concentrated in vacuum. The residue obtained was poured into saturated aqueous ammonium chloride solution, and the reaction product was extracted with ethyl acetate. The organic layer was washed with saturated saline solution, dried over anhydrous magnesium sulfate and concentrated in vacuum. The residue obtained was purified by column chromatography (hexane : ethyl acetate / 3:2) to obtain benzyl 5-(4-bromo-3-nitrobenzoyl)-2-methyl-2,3,4,5-tetrahydro-1H-1,5-benzodiazepin-7-yl carbamate (1.4 g, 37.8%) as a pale yellow solid.
$^1$H-NMR(DMSO-$d_6$,120˚C) δ:1.22 (1H,d,J=6.3Hz),1.57-1.68(1H,m), 1.86-1.96(1H,m),3.26(1H,br),4.20(1H,br),4.75(1H,s),5.04 (1H,s),6.82(1H,d,J=2.4Hz),6.89(1H,d,J==8.8Hz), 7.03 (1H,dd,J=8.8,2.4Hz),7.25-7.38(5H,m),7.50-7.69(2H,m),8.83 (1H,br)ppm
FABMS:540(M+1)

Reference Example 158

Benzyl 5-[4-(1H-indol-5-yl)-3-nitrobenzoyl]-2-methyl-2,3,4,5-tetrahydro-1H-1,5-benzodiazepin-7-ylcarbamate was obtained by the same procedure as in Reference Example 120 using the compounds in Reference Examples 157 and 70 (yield 64.9%).
$^1$H-NMR(DMSO-$d_6$,120˚C) δ:1.23 (3H,d,J=6.4Hz),1.61-1.72 (1H,m), 1.89-1.96(1H,m),3.29 (2H,br),4.23 (1H,br),4.75 (1H,br),5.04 (2H,s),6.42(1H,s),6.47-6.92(3H,m),7.07(1H,dd,J=8.8,2.4Hz), 7.25-7.51(10H,m),7.59(1H,d,J=1.5Hz),8.85 (1H,br),10.9(1H,br) ppm
FABMS:576(M+1)

Reference Example 159

Benzyl 5-[3-amino-4-(1H-indol-5-yl)benzoyl]-2-methyl-2,3,4,5-tetrahydro-1H-1,5-benzodiazepin-7-ylcarbamate was obtained by the same procedure as in Reference Example 125 using the compound in Reference Example 158 (yield 88.0%).
$^1$H-NMR (DMSO-$d_6$,120˚C) δ:1.21 (3H,d, J=6.3Hz) , 1.53-1. 66 (1H,m), 1.85-1.90(1H,m),3.30(1H,br),3.43(1H,br),4.09 (1H,br),4.34 (2H,br),4.55(1H,br),5.06(2H,s),6.39-6.41(1H,m),6.48 (1H,dd,J=7.8,15Hz),6.77-6.89(4H,m),7.01(1H,dd,J=8.3,1.5Hz), 7.07(1H,dd,J=8.8,2.4Hz),7.23-7.45(7H,m),8.83(1H,br),10.7 (1H,br)ppm
FABMS:546(M+1)

Reference Example 160

A tetrahydrofuran solution (15mL) of acetyl chloride (43 mg, 0.55 mmol) was dropped into a tetrahydrofuran solution (20 mL) of the compound (300 mg, 0.55 mmol) in Reference Example 159 and pyridine (52 mg, 0.66 mmol) in a nitrogen stream by cooling with ice. After stirring for 1 hour, the reaction mixture was concentrated in vacuum. The residue obtained was purified by silica gel column chromatography (hexane : ethyl acetate / 1:4) to obtain benzyl 5-[3-(acetylamino)-4-(1H-indol-5-yl)benzoyl]-2-methyl-2,3,4,5-tetrahydro-1H-1,5-benzodiazepin-7-ylcarbamate (222 mg, 68.7%) as a colorless solid.
$^1$H-NMR(DMSO-$d_6$, 120˚C) δ: 1.22 (3H,d,J=6.4Hz), 1.55-1.69(1H,m), 1.76(3H,s), 1.85-1.93(1H,m), 3.30-3.45(2H,m), 4.14(1H,br), 4.61(1H,br), 5.05(2H,s),6.41-6.43(1H,m), 6.84-6.87(2H,m), 6.98-7.10(4H,m), 7.24-7.45(7H,m), 7.65-7.65 (1H,m),8.33 (1H,br), 8.79(1H,br), 10.8(1H,br)ppm
FABMS:588(M+1)

Reference Example 161

N-[2-Bromo-5-[(4,7-dimethyl-2,3,4,5-tetrahydro-1H-1,5-benzodiazepin-1-yl)carbonyl]phenyl]acetamide was obtained by the same procedure in Reference Example 117 using the compounds in Reference Examples 29 and 38 (yield 53.6%).
$^1$H-NMR(DMSO-$d_6$,120˚C)δ:1.33(3H,d,J=6.3Hz),1.66-1.80(1H,m), 1.96-2.00(1H,m),2.11(3H,s),2.22(3H,s),3.47(1H,br),4.15 (1H,br),4.85(1H,br),6.39(1H,d,J=8.3Hz),6.56(1H,d,J=7.8Hz), ppm
FABMS:417(M+1)

Reference Example 162

N-[5-[[7-(Benzyloxy)-4-methyl-2,3,4,5-tetrahydro-1H-1,5-benzodiazepin-1-yl]carbonyl]-2-bromophenyl]acetamide was obtained by the same procedure in Reference Example 117 using the compounds in Reference Examples 26 and 38 (yield 97.7%).
$^1$H-NMR(DMSO-$d_6$,120˚C) δ:1.22 (3H,d,J=6.3Hz), 1.45-1.70 (1H,m), 1.76-1.96(1H,m),2.00(3H,s),3.38(2H,br),4.86(1H, br),4.95 (2H,s),6.12(1H,d,J=8.8,2.9Hz),6.48(1H,d,J=8.8Hz),6.59 (1H,s),6.79-6.83(1H,m),7.17-7.37(6H,m),7.62-7.63 (1H,m), 8.82 (1H, br), FABMS:509 (M+1)

Reference Example 163

N-[5-[[7-(Benzyloxy)-4-methyl-2,3,4,5-tetrahydro-1H-1,5-benzodiazepin-1-yl]carbonyl]-2-(1H-indol-5-yl)phenyl]-acetamide was obtained by the same procedure in Reference Example 120 using the compounds in Reference Examples 162 and 70 (yield 97.7%).
$^1$H-NMR(DMSO-$d_6$,120˚C)δ:1.23(3H,d,J=6.4Hz) 1.27-1.72(1H,m), 1.78(3H,s),1.83-2.01(1H,m),3.44(2H,br),4.87(1H, br),4.96 (2H,s),6.16(1H,dd,J=8.3,2.9Hz),6.42-6.44(1H,m),6.55 (1H,d,J=8.3Hz),6.61(1H,d,J=2.4Hz),6.94-7.08(3H,m), 7.13-7.54(8H,m),7.65(1H,d,J=1.0Hz),8.36(1H,br),10.8 (1H,br)ppm
FABMS:545 (M+2)

Reference Example 164

2-Bromo-5-[[(4S)-4-methyl-2,3,4,5-tetrahydro-1H-1,5-benzodiazepin-1-yl]carbonyl]pherzyl acetate was obtained by the same procedure in Reference Example 117 using the compounds in Reference Examples 5 and 49 (yield 95%).
$^1$H-NMR(DMSO-$d_6$,120˚C)d:1.23(3H,d,J=6.6Hz),1.66(1H,m),1.87 (1H,m),2.23(3H,m),3.36(2H,br),4.08(1H,br),4.88 (1H,s),6.47 (1H,m),6.59(1H,d,J=8.0Hz),6.91-6.99(2H,m),7.07 (1H,d,J=2.2Hz),7.44(1H,d,J=8.0Hz)ppm
FABMS:424(M+1)

Reference Example 165

Sodium hydroxide (1.41g, 35.2 mmol) was added to a mixed solution (50/5 mL) of methanol and water of a compound obtained by the same procedure in Reference Example 164, and the solution was stirred at room temperature for 1.5 hours. After completing the reaction, the reaction solution was concentrated, and the reaction product was extracted with ethyl acetate by adding an aqueous citric acid solution until the reaction solution became acidic. The organic layer was washed with saturated saline solution, dried over anhydrous magnesium sulfate and concentrated in vacuum. The residue obtained was purified by silica gel column chromatography (hexane : ethyl acetate / 3:2) to obtain 2-bromo-5-[[(4S)-4-methyl-2,3,4,5-tetrahydro-1H-1,5-benzodiazepin-1-yl]carbonyl]phenol (4.11 g, 64.7%) as a colorless solid.
$^1$H-NMR(DMSO-$d_6$,120˚C)d:1.23 (3H,d,J=6.84Hz), 1.59-1.69(1H,d), 1.82-1.95(1H,m),3.30-4.22(3H,br),4.81(1H,br), 6.42-6.57 (3H,m),6.89-6.91(3H,m),7.17-7.20(1H,d,J=8.30Hz),9.62(1H,s) ppm
FABMS:362(M+1)

Reference Example 166

(4R)-1-[3-(Benzyloxy)-4-bromobenzoyl]-4-methyl-2,3,4,5-tetrahydro-1H-1,5-benzodiazepine was obtained by the same method in Reference Example 117 using the compounds in Reference Examples 6 and 47 (yield 89.2%).
$^1$H-NMR (DMSO-$d_6$, 120˚C) δ:1.24 (3H,d,J=5.9Hz), 1.60-1.70 (1H,m), 1.88(1H,br),3.37(2H,br) ,4.06(1H,br),4.87-4.94 (3H,m), 6.43-6.48(1H,m),6.S4(1H,d,J=7.8Hz),6.76-6.89(2H,m), 6.92-6.94(4H,m),7.30-7.47(6H,m)
FABMS:452(M+1)

Reference Example 167

2-Bromo-5-[[(4R)-4-methyl-2,3,4,5-tetrahydro-1H-1,5-benzodiazepin-1-yl]carbonyl]phenyl acetate was obtained by the same method in Reference Example 117 using the compounds in Reference Examples 6 and 49 (yield 55.9%).
$^1$H-NMR(DMSO-$d_6$, 120˚C) d:1.23(3H, d, J = 6.6Hz), 1.66 (1H,m), 1.87 (1H,m),2.23(3H,m),3.36(2H,br),4.08(1H,br),4.88 (1H,s),6.47 (1H,m),6.59(1H,d,J=8.0Hz),6.91-6.99(2H,m),7.07 (1H,d,J=2.2Hz),7.44(1H,d,J=8.0Hz)
FABMS:424(M+1)

Reference Example 168

2-Bromo-5-[[(4R)-4-methyl-2,3,4,5-tetrahydro-1H-1,5-benzodiazepin-1-yl]carbonyl]phenol was obtained by the same method in Reference Example 165 using the compound in Reference Example 167 (yield 51.0%).
$^1$H-NMR(DMSO-$d_6$,120˚C)d:1.23(3H,d,J=6.84Hz),1.59-1.69(1H,d), 1.82-1.95(1H,m), 3.30-4.12(3H,br),4.81(1H,br), 6.42-6.57 (3H,m),6.89-6.91(3H,m),7.17-7.20(1H,d,J=8.30Hz),9.62(1H,s) ppm
FABMS:362(M+1)

Reference Example 169

(4R)-1-[3-(Benzyloxy)-4-(1H-indol-5-yl)benzoyl]-4-methyl-2,3,4,5-tetrahydro-1H-1,5-benzodiazepine was obtained by the same method in Reference Example 120 using the compound in Reference Example 166 (yield 72.2%).
$^1$H-NMR (DMSO-$d_6$,120˚C) δ:1.25 (3H,d,J=6.4Hz),1.64-1.69 (1H,m) , 1.91(1H,br),3.41(2H,br),4.12(1H,br),4.74-4.84 (2H,m),4.93 (1H,s),6.38(1H,s),6.47-6.52(1H,m),6.61(1H,d,J=7.3Hz), 6.93-6.98(4H,m),7.14-7.35(9H,m),7.59(1H,s), 10.66(1H,br) ppm
FARMS:488 (M+1)

Reference Example 170

1-[3-(Benzyloxy)-4-bromobenzoyl]-4-methyl-2,3,4,5-tetrahydro-1H-benzodiazepine was obtained by the same method in Reference Example 117 using the compounds in Reference Examples 4 and 47 (yield 78.0%).
$^1$H-NMR(DMSO-$d_6$, 120˚C) δ:1.24 (3H, d, J=5. 9Hz), 1.60-1.70 (1H, m), 1.88 (1H, br), 3.37 (2H, br), 4.06 (1H, br), 4.87-4.94 (3H, m), 6.43-6.48(1H,m) 6.54(1H,d,J=7.8Hz),6.76-6.89(2H,m), 6.92-6.94(4H,m),7.30-7.47(6H,m)ppm
FABMS:452(M+1)

Reference Example 171

1-[3-(Benzyloxy)-4-(1H-indol-5-yl)benzoyl]-4-methyl-2, 3,4,5-tetrahydro-iH-1,5-benzodiazepine was obtained by the same method in Reference Example 120 using the compounds in Reference Examples 170 and 70 (yield 73.5%).
$^1$H-NMR(DMSO-$d_6$,120˚C) δ:1.25 (3H,d,J=6.4Hz), 1.64-1.69(1H,m), 1.91(1H,br),3.41(2H,br),4.12(1H,br),4.74-4.84(2H, m),4.93 (1H,s),6.3S(1H,s),6.47-6.52(1H,m),6.61(1H,d,J=7.3Hz), 6.93-6.98(4H,m),7,14-7.35(9H,m),7.59(1H,*s),*10.66 (1H,br) ppm
FABMS:488(M+1)

Reference Example 172

1-[3-(Benzyloxy)-4-bromobenzoyl]-4-ethyl-2,3,4,5-tetrahydro-1H-1,5-benzodiazepine was obtained by the same method in Reference Example 117 using the compounds in Reference Examples 10 and 47 (yield 78.4%).
$^1$H-NMR(DMSO-$d_6$, 120˚C) δ:0.98 (3H, t, J=6.8Hz), 1.45-1.75(3H,m), 1.87-2.01(1H,m),3.06-3.30(1H,br),3.35-4.40(2H, m),4.82-5.00 (3H,m),6.40-6.49(1H,m),6.53(1H,d,J=7.8Hz),6.78 (1H,dd,J=2.0,7.8Hz),6.87-6.98(3H,m),7.26-7.43(6H,m) ppm
FABMS:467(M+2)

Reference Example 173

1-[3-(Benzyloxy)-4-(1H-indol-5-yl)benzoyl]-4-ethyl-2, 3, 4, 5-tetrahydro-1H-1, 5-benzodiazepine was obtained by the same procedure in Reference Example 120 using the compounds in Reference Examples 172 and 70 (yield 53.4%).
$^1$H-NMR(DMSO-$d_6$,120˚C)δ:0.99(3H,t,J=7.8Hz),1.45-1.78(3H,m), 3.90-2.05(1H,m),3.22(1H,brs),3.45-4.30(2H,m),4.79 (2H,s), 4.90(1H,s),6.38(1H,brs),6.44-6.52(1H,m),6.61(1H,d,J=8.8Hz), 6.89-7.01(4H,m),7.13-7.37(8H,m),7.59(1H,s), 10.66(1H,brs)
FABMS : 503 (M+1)

Reference Example 174

Potassium carbonate (0.21 g, 1.52 mmol) was added to an N,N-dimethylformamide solution (5 mL) of the compound (0.4 g, 1.0 mmol) in Example 182 and N- (3-bromopropyl) phthalimide (0.33 g, 1.45 mmol) , and the solution was stirred for 15 hours at 80 ˚C. Water was poured into the reaction solution, and the reaction product was extracted with ethyl acetate. The organic layer was washed with water, dried over anhydrous sodium sulfate and concentrated in

vacuum to obtain
2-[3-[2-(1H-indol-5-yl)-5-[(4-methyl-2,3,4,5-tetrahydro-1H-1,5-benzodiazepin-1-yl)carbonyl]phenoxy]propyl]-1H-isoindol-1,3(2H)-dione (0.355 g) as a pale yellow oil. The product was used for the reaction in Example 30 without further purification.

Reference Example 175

2-[4-[2-(1H-Indol-5-yl)-5-[(4-methyl-2,3,4,5-tetrahydro-1H-1,5-benzodiazepin-1-yl)carbonyl]phenoxy] butyl]-1H-isoindole-1,3(2H)-dione was obtained by the same procedure in Reference Example 174 using the compound in Example 182 and N-(4-bromobutyl)phthalimide (yield 63.2%).
$^1$H-NMR(DMSO-$d_6$,120˚C)δ:1.25(3H,d,J=6.8Hz),1.50-1.65(5H,m), 1.87-2.00(1H,m),3.35-3.62(4H,m),3.65-3.3.77(2H, m), 3.90-4.35(1H,m),4.89(1H,s),6.34(1H,s),6.45-6.53(1H,m), 1.62(1H,d,J=6.8Hz),6.83-6.98(4H,m),7.00-7.16(2H,m), 7.19 (1H,t,J=2.9Hz),7.31(1H,d,J=7.8Hz),7.54(1H,m),7.76-7.85 (4H,m),10.62(1H,brs)ppm
FABMS: 599 (M+1)

Reference Example 176

2-[5-[2-(1H-Indol-5-yl)-5-[(4-methyl-2,3,4,5-tetrahydro-1H-1, 5-benzodiazepin-1-yl) carbonyl]phenoxy] pentyl]-1H-isoindole-1,3(2H)-dione was obtained by the same procedure in Reference Example 174 using the compound in Example 182 and N-(5-bromopentyl)phthalimide (yield 66.3%).
$^1$H-NMR(DMSO-$d_6$,120˚C)δ:1.20-1.38(5H,m),1.49-1.75(5H,m), 1.88-2.01(1H,m),3.33-3.73(6H,m),3.95-4.35(1H,m), 4.88(1H,s), 6.36-6.40(1H,m),6.44-6.52(1H,m),6.62(1H,d,J=7.8Hz), 6.82-6.97(4H,m),7.09-7.16(2H,m),7.21(1H,t, J=2.9Hz),7.31 (1H, d, J=8.8Hz), 7.54(1H,m), 7.76-7.85(4H,m), 10.62(1H,brs)ppm
FABMS:613(M+1)

Reference Example 177

2-[6-[2-(1H-Indol-5-yl)-5-[(4-methyl-2,3,4,5-tetrahydro-1H-1,5-benzodiazepin-1-yl) carbonyl]phenoxy] hexyl]-1H-isoindole-1,3(2H)-dione was obtained by the same procedure in Reference Example 174 using the compound in Example 182 and N-(6-bromohexyl)phthalimide (yield 70.2%).
$^1$H-NMR(DMSO-$d_6$,120˚C)δ:1.15-1.38(7H,m),1.48-1.75(5H,m), 1.88-2.00(1H,m), 3.32-3.73(6H,m),3.95-4.35(1H,br), 4.60-5.20 (1H,br),6.35-6.39(1H,m),6.44-6.52(1H,m),6.62(1H,d,*J*=6.8Hz), 6.82-6.98(4H,m), 7.10-7.16(2H,m),7.20(1H,t, *J*=1.9Hz),7.34 (1H,d,*J*=8.8Hz),7.55(1H,m),7.7S-7.8S(4H,m),10.62(1H,brs)ppm
FABMS:627(M+1)

Reference Example 178

Potassium carbonate (90 mg, 0.65 mmol) was added to an acetonitrile solution (15 mL) of the compound (0.2 g, 0.5 mmol) in Example 175 and N-(3-bromopropyl)phthalimide (0.135 g, 0.5 mmol), and the solution was refluxed with heating for 18 hours. Water was poured into the reaction solution, and the reaction product was extracted with ethyl acetate. The organic layer was washed with water, dried over anhydrous sodium sulfate and concentrated in vacuum. The residue obtained was purified by silica gel column chromatography (methylene chloride : ethyl acetate / 248:2) to obtain
2-[3-[2-(1H-indol-5-yl)-5-[4-methyl-2,3,4,5-tetrahydro-1H-1,5-benzodiazepin-1-yl]carbonyl]anilno]propyl]-1H-isoindole-1,3(2H)-dione (0.1 g, 34.3%) as a pale yellow oil.
$^1$H-NMR (DMSO-$d_6$, 120˚C) δ:1.24 (3H,d,J=6.8Hz), 1.55-1.77 (1H,m), 1.82-2.00(1H,m),2.13-2.26(2H,m),3.35-3.72(3H, m),3.92-4.15 (1H,br),4.29-4.30(2H,m),4.36(1H,brs),4.79(1H,brs),6.39-6.55 (3H,m), 6.63-6.69(1H,m),6.74-6.82(1H,m), 6.87-6.93(2H,m), 7.02-7.08(1H,m),7.35(1H,d,J=2.9Hz),7.40-7.48(2H,m), 7 . 75-7 . 83 (5H, m) ppm
FABMS:483 (M+1)

Reference Example 179

(4S)-1-(4-Bromo-3-methoxybenzoyl)-4-methyl-2,3,4,5-tetrahydro-1H-1,5-benzodiazepine was obtained by the same procedure in Reference example 117 using the compounds in Reference Examples 5 and 48 (yield 74%).
$^1$H-NMR(DMSO-$d_6$,120˚C)d:1.23(3H,d,J=6.6Hz),1.66(1H,m),1.89 (1H,m),3.38(2H,br),3.64(3H,s),4.11(1H,br),4.94(1H, br),6.47 (1H,m),6.58(1H,d,J=8.0Hz),6.77(1H,dd,J=2.2,8.0Hz),6.85 (1H,d,J=2.2Hz),6.93(2H,m),7.34(1H,d,J=8.0Hz) ppm
FABMS:376(M+1)

Reference Example 180

N-[2-bromo-5-[[(4S)-4-methyl-2,3,4,5-tetrehydro-1H-1,5-benzodiazepin-1-yl]carbonyl]phenyl]-N-methylacetamide was obtained by the same procedure in Reference Example 117 using the compounds in Reference Examples 5 and 41 (yield 70.0%).
$^1$H-NMR (DMSO-$d_6$, 120˚C) δ:1.23 (3H, d, J=6.6 Hz), 1.32-1.75 (4H, m), 1.84-1.99 (1H, m), 2.88 (3H, s), 3.15-4.65 (3H, m), 4.96(1H, brs), 6.40-6.52 (1H, m), 6.55-6.65(1H, m), 6.86-6.98 (2H, m), 7.06 (1H, s), 7.18-7.29 (1H, m),7.59 (1H, d, J=8.1 Hz) ppm
FABMS:416(M+1)

Reference Example 181

Trifluoroacetic acid (2 mL) was added at room temperature to a methylene chloride solution (10 mL) of the compound obtained by the same procedure in Reference Example 120 using the compound in Reference Example 58 and 4-bromonitrobenzene, and the solution was stirred for 2 hours. After completing the reaction, the reaction mixture was concentrated in vacuum. A saturated aqueous solution of sodium hydrogen carbonate was added to the residue obtained until solid is precipitated. The precipitated solid was filtered off, and thoroughly washed with water to obtain 2-methoxy-4'-nitro[1,1'-biphenyl]-4-carboxylic acid. The product was used for the reaction in Reference Example 182 without further purification.

Reference Example 182

(4S)-1-[(2-Methoxy-4'-nitro[1,2'-biphenyl]-4-yl) carbonyl]-4-methyl-2,3,4-5-tetrahydro-1H-1,5-benzodiazepine was obtained by the same procedure in Reference Example 117 using the compounds in Reference Examples 5 and 181 (yield 53.5%, total yield from Reference Example 181). $^1$H-NMR(DMSO-$d_6$,120˚C) δ:1.26 (3H,d,J=5.9Hz), 1.61-1.75 (1H,m), 1.89-1.96(1H,m),3.42(2H,br),3.58(3H,s),4.13(1H,br),4.94 (1H,s),6.46(1H,m),6.64(1H,d,J=6.8Hz),6.70-7.06 (4H,m),7.20 (1H,d,J=7.8Hz)7.62-7.67(2H,m),8.14-8.18(2H,m)ppm
FABMS:418(M+1)

Reference Example 183

Benzyl 3-[2-(1H-indol-5-yl)-5-[[(4S)-4-methyl-2,3,4,5-tetrahydro-1H-1,5-benzodiazepin-1-yl]carbonyl]phenoxy]propyl(methyl) carbamate was obtained by the same procedure in Reference Example 45 using the compounds in Example 180 and Reference Example 88. (yield 79.5%)
$^1$H-NMR(BMSO-$d_6$,120˚C)δ:1.25(3H,d,J=5.8Hz),1.59-1.80(3H,m), 1.87-2.02(1H,m),2.71(3H,s),3.18(2H,d,J=7.8Hz), 3.32-3.78 (3H,m),3.98-4.35(1H,br),4.88(1H,s),5.01(2H,s),6.38(1H,s), 6.43-6.52(1H,m),6.62(1H,d,J=6.8Hz),6.83-6.98 (4H,m), 7.11-7.17(2H,m),7.21-7.38(6H,m),7.56(1H,s),10.65(1H,brs)ppm
FABMS:604 (M+1)

Reference Example 184

1, 1-Diethylpropyl 2-(methoxymethoxy)-4'-nitro [1,1'-biphenyl]-4-carboxylate was obtained by the same procedure in Reference Example 120 using the compound in Reference Example 63 and 4-bromonitrobenzene (yield 80.6%).
$^1$H-NMR($CDCl_3$) δ:0.91 (9H, t, J=7.3 Hz), 2.00 (6H, q, J=7.3 Hz), 3.39 (3H,s),5.20(2H,s),7.38(1H,d,J=8.3Hz),7.68-7.76 (3H,m),7.87 (1H,s),8.28(2H,d,J=8.3Hz)ppm
FABMS:402(M+1)

Reference Example 185

Trifluoroacetic acid (8 mL) was added to a methylene chloride solution (80 mL) of the compound (1.61 g, 4.03 mmol) in Reference Example 184 at room temperature, and the solution was stirred for 1 hour. After completing the reaction, the reaction mixture was concentrated in vacuum. The residue obtained was poured into saturated saline solution, and the product was extracted with ethyl acetate. The organic layer was washed with saturated saline solution, dried over anhydrous magnesium sulfate and concentrated in vacuum. The residue obtained was thoroughly washed with hexane to obtain 2-(methoxymethoxy)-4'-nitro[1,1'-biphenyl]-4-carboxylic acid (1.11g, 90.8%) as a colorless solid.
$^1$H-NMR (DMSO-$d_6$) δ:3.42(3H,s),5.35(2H,s),7.62(1H,d,J=7.8Hz), 1.79(1H,dd,J=7.8,1.5Hz),7.88-7.94(3H,m),8.39(2H, d,J=8.8Hz) ppm
FABMS:304(M+1)

Reference Example 186

(4S)-1-[[2-(Methoxymethoxy)-4'-nitro[1,1'-biphenyl]-4-yl]carbonyl]-4-methyl-2,3,4,5-tetrahydro-1H-1,5-benzodiazepine was obtained by the same method in Reference Example 117 using the compounds in Reference Examples 5 and 185. The compound was used for the reaction in Reference Example 187 without further purification.

Reference Example 187

A tetrahydrofuran/isopropyl alcohol solution (10/2 mL) of the compound in Reference Example 186 and conc. hydrochloric acid (2 mL) was stirred and refluxed for 4 hours with heating. After completing the reaction, the reaction solution was concentrated in vacuum. The residue obtained was poured into saturated saline solution, and the reaction product was extracted with ethyl acetate. The organic layer was washed with saturated saline solution, dried over anhydrous magnesium sulfate and concentrated in vacuum to obtain 4- [[(4S)-4-methyl-2, 3, 4, 5-tetrahydro-1H-1,5-benzodiazepin-1-yl]carbonyl]-4'-nitro [1,1'-biphenyl]-2-ol. The compound was used for the reaction in Reference example 188 without further purification.

Reference Example 188

3-[[4-[[(4S)-4-Methyl-2,3,4,5-tetrahydro-1H-1,5-benzodiazepin-1-yl]carbonyl]-4-nitro[1,1'-biphenyl]-2-yl] oxy] propylamine was obtained by the same procedure in Example 24 from the compound obtained by the same procedure in Reference Example 174 using the compound in Reference example 187 and N-(3-bromopropyl)phthalimide (yield 31.1%).
$^1$H-NMR(DMSO-$d_6$,120˚C)δ:1.25(3H,d,J=5.9Hz), 1.58-1.74 (3H,m) , 1.91(1H,br),2.55-2.60(2H,m),3.42(2H,br), 3.81-3.96(2H,m), 4.12(1H,br),4.93(1H,s),6.46-6.50(1H,m),6.64 (1H,d,J=7.8Hz),6.93-7.05(4H,m),7.20(1H,d,J=7.8Hz), 7.6S-7.68(2H,m),8.14-8.18(2H,m)ppm
FABMS:461(M+1)

Reference Example 189

1-Bromo-2-chloroethane (1.62 g, 11.3 mmol) was added to an acetone solution (30 mL) of the compound (1.50 g, 3.77 mmol) in Example 180 and potassium carbonate (1.56g, 11.3 mmol ) , and the solution was refluxed with heating for 3 days in a nitrogen atmosphere. The reaction solution was cooled to room temperature and concentrated in vacuum, and the reaction product was extracted with ethyl acetate by adding water. The organic layer was concentrated in vacuum, and the residue obtained was purified by silica gel column chromatography (hexane : ethyl acetate = 2:1) to obtain
(4S)-1-[3-(2-chloroethoxy)-4-(1H-indol-5-yl)benzoyl]-4-ir.ethyl-2,3,4,5-tetrahydro-1H-1,5-benzodiazepine (0.738 g, 42.5%) as a pale yellow oil.
$^1$H-NMR (DMSO-$d_6$,120˚C) δ:1.26 (3H,d,J=5.9Hz),1.66-1.69 (1H,m), 1.96 (1H, m), 3.64 (2H, t, J=5. 9Hz), 3.95 (2H, t, J=1. 45Hz), 3.95 (2H,t,J=1.45Hz),4.90 (1H, s), 6.39 (1H,s), 6.50 (1H,m), 6.4 (1H, d, J=7. 3Hz), 6.88 (1H, d, J=1. 5Hz), 6.93-6.99 (3H, m), 7.14 (1H,s), 7.17 (1H, t, J=1.5Hz), 7.20 (1H, t, J=1. 5Hz), 7.24 (1H, t, J=2.9Hz), 7.35(1H,d,J=8.8Hz), 7.61(1H,s),10.67(1H,s)ppm
FABMS:461(M+1)

Reference Example 190

1-[3-(3-Bromopropyl)-4-(1H-indol-5-yl)benzoyl]-4-methyl-2,3,4,5-tetrahydro-1H-1,5-benzodiazepine was obtained by the same procedure in Reference Example 45 using the compound in Example 182 and 3-bromopropanol (yield 98%) .
$^1$H-NMR(DMSO-$d_6$,120˚C)d:126(3H,d,J=6.6Hz),1.69(1H,m), 1.92-2.08 (3H,m), 3.41 (4H,t,J=6.6Hz), 3.81 (2H,m), 4.15 (1H,br), 1.40 (1H,s), 6.50-6.54 (1H,m), 6.63 (1H,d, J=7.3Hz), 6.89-6.98 (4H,m), 7.11-7.15 (2H,m),7.24 (1H,t, J=2.9Hz), 7.33 (1H, d, J=8.8Hz), 7.55 (1H,s), 10.67 (1H,br)ppm
FABMS:519(M+1)

Reference Example 191

(4S)-1-[3-(3-Bromopropoxy)-4-(1H-indol-5-yl)benzoyl]-4-methyl-2,3,4,5-tetrahydro-1H-1,5-benzodiazepine was obtained by the same procedure in Reference Example 45 using the compound in Example 180 and 3-bromopropanol (yield 89.2%).

$^1$H-NMR(DMSO-$d_6$,120˚C)δ:1.26(3H,d,J=5.9Hz),1.60-2.13(4H,m), 3.50-4.30(8H,m),6.40(1H,brs),6.48-6.57(1H,m), 6.62-6.69 (1H,m),6.85-6.98(4H,m),7.10-7.17(2H,m),7.22-7.27(1H,m), 7.32-7.42(1H,m),7.53-7.58(1H,m)ppm
FABMS:518(M+1)

Reference Example 192

(4S)-1-[3-(4-Bromobutoxy)-4-(1H-indol-5-yl)benzoyl]-4-methyl-2,3,4,5-tetrahydro-1H-1,5-benzodiazepine was obtained by the same procedure in Reference Example 45 using the compound in Example 180 and 4-bromobutanol (yield 90.9%).
$^1$H-NMR(DMSO-$d_6$,120˚C)δ:1.26(3H,d,J=6.6Hz),1.55-2.05(6H,m), 3.35-4.30(8H,m),6.40(1H,brs),6.48-6.57(1H,m), 6.62-6.69 (1H,m),6.85-6.90(1H,m),6.91-7.02(3H,m),7.10-7.18(2H,m), 7.22-7.27(1H,m),7.32-7.42(1H,m),7.52-7.59(1H, m)ppm
FABMS:533(M+1)

Reference Example 193

(4S)-1-[3-[(5-Bromopentyl)oxy]-4-(1H-indol-5-yl) benzoyl]-4-methyl-2,3,4,5-tetrahydro-1H-1,5-benzodiazepine was obtained by the same procedure in Reference Example 45 using the compound in Example 180 and 4-bromopentanol (yield 80%).
$^1$H-NMR(DMSO-$d_6$,120˚C)δ: 1.14-1.27 (4H,m) , 1.34-1.46 (2H,m), 1.48-1.55(2H,m),1.65-1.1.78(2H,m),1.89-2.00(2H, m),3.37 (3H,t,J=6.6Hz),3.68(2H,m),4.00-4.19(1H,m),6.39(1H,s), 6.46-6.52(1H,m),6.62(1H,d,J=7.3Hz),6.85-6.93(4H, m), 7.12-7.15(2H,m),7.24(1H,t,J=2.9Hz),7.33(1H,d,J=8.8Hz), 7.55(1H,s),10.66(1H,br)ppm
FABMS:548 (M+1)

Reference Example 194

(4R)-1-[3-(3-Bromopropoxy)-4-(1H-indol-5-yl)benzoyl]-4-methyl-2,3,4,5-tetrahydro-1H-1,5-benzoazepine was obtained by the same procedure in Reference Example 45 using the compound in Example 181 and 3-bromopropanol (yield 72.7%). $^1$H-NMR(DMSO-d6,120˚C)δ:1.26(3H,d,J=5.9Hz),1.60-2.13(4H,m), 3.504.30(8H,m),6.40(1H,brs), 6.48-6.57(1H,m),6.62-6.69 (1H,m),6.85-6.98 (4H,m), 7.10-7.17 (2H,m), 7.22-7.27 (1H,m), 7.32-7.42 (1H,m),7.53-7.58 (1H,m) ppm
FABMS:518(M+1)

Reference Example 195

(4S)-4-Methyl-1-[[2-[2-(4-morphonyl)ethoxy]-4'-nitro[1,1'-biphenyl]-4-yl]carbonyl]-2,3,4,5-tetrahydro-1H-1,5-benzodiazepine was obtained by the same procedure in Reference Example 45 using the compound in Reference Example 187 and 4-(2-hydroxyethyl)morpholine (yield 54.2%).
$^1$H-NMR(DMSO-$d_6$,120˚C) δ:1.25 (3H,d,J=5.9Hz),1.64-1.74 (1H,m), 1.91(1H,br),2.31-2.35(4H,m),2.51-2.55(4H,m), 3.49-3.57 (4H,m),3.88-3.90(2H,m),4.01-4.13(1H,m),4.94(1H,br), 6.45-6.51(1H,m),6.64(1H,d,J=7.8Hz),6.89-7.01(4H, m),7.22 (1H,d,J=7.8Hz),7.70-7.74(2H,m),8.13-8.17(2H,m)
FABMS:517 (M+1)

Reference Example 196

(4S)-1-[4-Bromo-3-[2-(4-morphonyl)ethoxy]benzoy]-4 methyl-2,3,4,5-1H-1,5-benzodiazepine was obtained by the same procedure in Reference Example 45 using the compound in Reference Example 165 and N-hydroxyethylmorpholine (yield 84.4%).
$^1$H-NMR(DMSO-$d_6$,120˚C)δ:1.24(3H,d,J=6.8Hz),1.58-1.72(1H,m), 1.89-1.96(1H,m),2.45-2.49(4H,m),2.62-2.66(2H,m), 3.39-3.59 (2H,br),3.55-3.59(4H,m),3.90-3.96(2H,m),4.11(1H,br),4.91 (1H,br),6.43-6.50(1H,m),6.58(1H,d,J=6.6Hz), 6.77 (1H,dd,J=7.82,2.0Hz),6.85-6.92(3H,m),7.34(1H,d,J=7.8Hz)ppm
FABMS:475(M+1)

Reference Example 197

(4S)-1-[4-Bromo-3-(4-morphonylmethyl)benzoyl]-4-methyl-2,3,4,5-tetrahydro-1H-1,5-benzodiazepine was obtained by the same procedure in Reference Example 117 using the compounds in Reference Examples 5 and 54 (yield 62%).

$^1$H-NMR(DMSO-$d_6$,120°C)d:1.22(3H,d,J=7.3),1.66(1H,m),1.96 (1H,m),2.22(1H,t,J=5.1Hz),3.36(2H,s)3.53(4H,t, J=4.4Hz), 1.06(1H,br),4.90(1H,br),6.40(1H,m),6.52(1H,d,J=6.6Hz), 6.83-6.89(2H,m),7.10(1H,dd,J=2.2,8.0Hz),7.23 (1H,s), 7.41(1H,d,J=8.1Hz)ppm
FABMS:446(M+1)

Reference Example 198

Methyl 2-bromo-5-[[(45)-4-methyl-2,3,4,5-tetrahydro-1H-1,5-benzodiazepin-1-yl]carbonyl]benzoate was obtained by the same procedure in Reference Example 117 using the compounds in Reference Examples 5 and 56 (yield 90.1%).

Reference Example 199

An aqueous solution of 5% sodium hydroxide (211 mg, 5.28 mmol) was added to a methanol solution of the compound (710 mg, 1.76 mmol) in Reference Example 198, and the solution was stirred at 50°C for 3 hours. After removing methanol by evaporation in vacuum, the aqueous layer was washed with ethyl acetate. The aqueous layer was acidified by adding 5N aqueous hydrochloric acid, and the reaction product was extracted with methylene chloride. The organic layer was washed with saturated saline solution, dried over anhydrous magnesium sulfate and concentrated in vacuum to obtain
2-brcmo-5-[[(4S)-4-methyl-2,3,4,5-tetrahydro-1H-1,5-benzodiazepin-1-yl]carbonyl]benzoic acid (641 mg, 93.5%) as a colorless solid.

Reference Example 200

3-Bromopropyl 2-bromo-5-[[(4S)-4-methyl-2,3,4,5-tetrahydro-1H-1,5-benzodiazepin-1-yl]carbonyl]benzoate was obtained by the same procedure in Reference Example 45 using the compound in Reference Example 199 and 3-bromopropanol (yield 73%). $^1$H-NMR(DMSO-$d_6$:120°C)δ:1.34(3H,d,J=6.8Hz),1.70-1.8(1H,m), 1.90-2.05(1H,m),2.35 (2H,quintet,J=6.3Hz),3.40-3.65(2H,m), 3.68(2H,t,J=6.3Hz),4.1-4.4(1H,m),4.44(2H,t,J=6.3Hz) 6.55-6.75(2H,m), 7.00-7.10(2H,m),7.36(1H,d,J=7.8Hz), 7.60-7.70(2H,m)ppm
FABMS:511(M+1)

Reference Example 201

An acetone solution (20 mL) of the compound (2 g, 5.1 mmol) in Reference Example 199, 1-bromo-2-chloroethane (2.2 g, 15.4 mmol) and potassium carbonate (2.12 g, 15.4 mmol) was refluxed for 16 hours with heating. After cooling, the reaction solution was poured into ice-water, and the reaction product was extracted with ethyl acetate. The organic layer was washed with saturated saline solution, dried over anhydrous magnesium sulfate and concentrated in vacuum. 2-chloroethyl
2-(1H-indol-5-yl)-5-[[(4S)-4-methyl-2,3,4,5-tetrahydro-1H-1,5-benzodiazepin-1-yl]carbonyl]benzoate was obtained by the same procedure in Reference Example 120 using the residue obtained and the compound in Reference Example 70. (yield 91%)
$^1$H-NMR(DMSO-$d_6$: 120°C) δ: 1.25 (3H, d, J=6.3 Hz), 1.6-1.8 (1H, m), 1.9-2.05(1H,m),2.7-3.3(2H,m),3.3-3.6(1H,m),3.50 (2H, t, J=5.9 Hz), 4.1-4.25 (1H, m), 4.20 (2H, t, J=5.9 Hz), 6.40 (1H, brs), 6.46-6.52 (1H, m), 6.63 (1H, d, J=7.8 Hz), 6.91-6.95(3H,m),7.25(1H,d,J=8.3Hz),7.28(1H,t,J=3.1Hz), 7.33-7.38(3H,m),7.59(1H,d,*J*=2.0Hz)ppm
FABMS: 488 (M+1)

Reference Example 202

3-Bromopropyl 2-(1H-indol-5-yl)-5-[[(4S)-4-methyl-2,3,4,5-tetrahydro-1H-2,5-benzodiazepin-1-yl]carbonyl] benzoate was obtained by the same procedure in Reference Example 120 using the compounds in Reference Examples 200 and 70. (yield 67%)
$^1$H-NMR(DMSO-$d_6$:120°C)δ:1.25(3H,d,J=6.3Hz),1.58-1.80(1H,m), 1.83(2H,tquintet,J=6.3Hz),1.90-2.05(1H,m), 2.80-3.60(3H,m), 3.08(2H,t,J=6.3Hz),4.05(2H,t,J=6.3Hz),6.38-6.42(1H,m), 6.45-6.55(1H,m),6.64(1H,d,J=7.8Hz), 6.91-6.95(3H,m), 7.20-7.40(5H,m),7.51(1H,s)ppm
FABMS:547(M+1)

Reference Example 203

4-Chlorobutyl 2-(1H-indol-5-yl)-5-[[(4S)-4-methyl-2,3,4,5-tetrahydro-1H-1,5-banzodiazepin-1-yl]carbonyl]benzoate was obtained by the same procedure in Reference Example 201 using the compound in Reference Example 199 and 4-chlorobutanol. (yield 81%)
$^1$H-NMR (DMSO-$d_6$: 120˚C) δ:1.25(3H,d,J=6.3Hz),1.3-1.5(4H,m), 1.6-1.8(1H,m),1.9-2.05(1H,m),3.3-3.6(1H,m),3.31 (2H,t,J=5.9Hz),4.1-4.25(1H,m),3.94(2H,t,J=5.9Hz),4.8-5.0 (1H,m),6.40(1H,brs),6.46-6.52(1H,m),6.63(1H,d,J=7.8Hz), 6.91-6.95(3H,m),7.25(1H,d,J=8.3Hz),7.28(1H,t,J=3.1Hz), 7.33-7.38(3H,m),7.50(1H,d,J=2.0Hz)ppm
FABMS:517 (M+1)

Reference Example 204

Morpholine (0.282 mL, 3.24 mmol), diethyl cyanophosphonate (0.299mL, 1.98mmol) and triethylamine (0.275 mL, 1.98 mmol) were added to an N,N-dimethylformamide solution (8mL) of the compound (0.632g, 1.62 mmol) in Reference Example 199, and the mixture was stirred at 50˚C for 5 hours. The reaction solution was concentrated in vacuum, the residue was poured into saturated saline solution and the reaction product was extracted with ethyl acetate. The organic layer was washed with saturated saline solution, dried over anhydrous magnesium sulfate and concentrated in vacuum. The residue obtained was purified by NH-silica gel column chromatography (chloroform) to obtain (4S)-1-[4-bromo-3-(4-morpholinecarbonyl)benzoyl]-4-methyl-2,3,4,5-tetrahydro-1H-1,5-benzodiazepine (0.499 g, 67.1%) as a colorless oil.

Reference Example 205

(4S)-1-[4-Bromo-3-[(4-methyl-1-piperazinyl)carbonyl] benzoyl-4-methyl-2,3,4,5-tetrahydro-1H-2,5-benzodiazepine was obtained by the same procedure in Reference Example 204 using the compound in Reference Example 199 and N-methyl piperazine. (yield 60.3%)
$^1$H-NMR(DMSO-$d_6$: 120˚C)5:1.23(3H,d,J=6.6Hz),1.57-1.74(1H,m), 1.84-2.00(1H,m),2.13-2.40(7H,m),2.70-3.08(4H, m),3.20-4.50 (3H,m),4.91(1H,brs),6.40-6.52(1H,m),6.58(1H,d,J=7.3Hz), 6.88-7.02(3H,m),7.13-7.27(1H,m),7.46(1H,d, J=8.1Hz)ppm
FABMS:472(M+1)

Reference Example 206

2-Bromo-5-[(4,7-dimethyl-2,3,4,5-tetrahydro-1H-1,5-benzodiazepin-1-yl)carbonyl]phenol was obtained by the same procedure in Reference Example 165 after applying the same procedure in Reference Example 117 using the compounds in Reference Examples 29 and 49. (yield 33.3%)
$^1$H-NMR(DMSO-$d_6$: 120˚C) δ:1.21 (3H, d, J=7.8 Hz), 1.52-1.70 (1H, m), 1.82-2.00(1H,m),2.11(3H,s),3.25-4.40(3H,m), 4.68(1H,m), 6.24-6.35(1H,m),6.43(1H,d,J=7.8Hz),6.48-6.58(1H,m),6.71 (1H, s), 6.89 (1H, d, J=2.0 Hz), 7.20 (2H, d, J=7.8 Hz), 9.62 (1H, brs) ppm
FABMS:376(M+1)

Reference Example 207

1-[4-Bromo-3-[2-(4-morphonyl)ethoxy]benzoyl]-4,7-dimethyl-2,3,4,5-tetrahydro-1H-1,5-benzodiazepine was obtained by the same procedure in Reference example 45 using the compound in Reference Example 206 and 2-(4-morphonyl)ethanol. (yield 96.5%)
$^1$H-NMR(DMSO-$d_6$:120˚C)δ:1.22(3H,d,J=6.8Hz),1.57-1.73(1H,m), 1.82-1.98(1H,m),2.12(3H,s),3.25-3.62(5H,m), 3.78-4.20(2H,m), 4.79(1H,brs),6.30(1H,d,J=7.8Hz),6.45(1H,d,J=7.8Hz), 6.70-6.88(3H,m),6.34(1H,d,J=7.8Hz) ppm
FABMS:490(M+1)

Reference Example 208

(4S)-1-[4-Bromo-3-(3-bromopropoxy)benzoyl]-4-methyl-2,3,4,5-tetrahydro-1H-1,5-benzodiazepine was obtained by the same procedure in Reference Example 45 using the compound in Reference example 165 and 3-bromopropanol. (qualitative yield) Reference Example 209

(4R)-1-[4-Bromo-3-(3-bromopropoxy)benzoyl]-4-methyl-2,3,4,5-tetrahydro-1H-,5-benzodiazepine was obtained by the same procedure in Reference Example 45 using the compound in Reference example 168 and 3-bromopropanol. (qualitative yield)

$^1$H-NMR(DMSO-$d_6$, 120˚C)δ:1.24(3H,d,J=6.8Hz),1.50-2.00(2H,m), 3.29-4.32(6H,m),6.42-6.49(1H,m),6.52-6.70(3H,m),6.74-6.95 (4H,m),7.33-7.37(1H,m)
FABMS:483(M+1)

Reference Example 210

An acetonitrile solution (5 mL) of the compound (482 mg, 1.0 mmol) in Reference Example 208, methylpropyl amine (0.2 mL, 2.0 mmol) and triethylamine (0.28 mL, 2.0 mmol) was stirred and refluxed with heating for 21 hours in a nitrogen stream. After completing the reaction, the reaction solution was concentrated in vacuum. The residue obtained was purified by NH-silica gel column chromatography (hexane : ethyl acetate / 1:20) to obtain
3-[2-bromo-5-[[(4S)-4-methyl-2,3,4,5-tetrahydro-1H-1,5-benzodiazepin-1-yl] carbonyl]phenoxy] -N-methyl-N-propyl-1-propanamine (391 mg, 82.4%) as a colorless oil.
$^1$H-NMR(DMSO-$d_6$: 120˚C)δ: 0.84 (3H,d,J=7.8Hz), 1.25 (3H,d,J=6.8Hz),1.33-1.52(2H,m),1.59-1.83(3H,m),1.85-2.00 (1H,m),2.17(3H,s),2.40-2.50(2H,m),3.25-4.40(7H,m),4.88 (1H,brs),6.42-6.53(1H,m),6.57(1H,d,J=7.8Hz),6.75 (1H,d J=7.8Hz),6.83(1H,d,J=2.0Hz),6.90-6.98(2H,m),7.33 (1H,d,J=7.8Hz)ppm
FABMS:475 (M+1)

Reference Example 211

3-[2-Bromo-5-[[(4R)-4-methyl-2,3,4,5-tetrah.ydro-IH-1,5-benzodiazepin-1-yl]carbonyl]phenoxy]-N-methyl-N-propyl-1-propanamine was obtained by the same procedure in Reference Example 210 using the compound in Reference Example 209. (yield 68.6%)
$^1$H-NMR(DMSO-$d_6$, 120˚C)δ:0.84(3H, t, J=7.3Hz),1.24 (3H,d,J=6.8Hz),1.37-1.77(5H,m),2.16(3H,s),2.28 (2H,t, J=6.8Hz),2.43(2H,t,J=6.8Hz),3.31-4.25(5H,m),4.88 (2H,m), 6.42-6.48 (1H,m), 6.57 (1H, d, J=7.8Hz), 6.75 (1H, dd, J=2.0 and 6.8Hz), 6.82 (1H,s), 6.91-6.93 (2H,m), 7.33 (1H,d,J=7.8Hz)ppm
FABMS:475(M+1)

Reference Example 212

A dimethylformamide solution (3 mL) of hydantoin (200 mg, 2.0 mmol) was dropped into a suspension of sodium hydroxide (80 mg, 2.0 mmol) in dimethylformamide (10 mL) at room temperature in a nitrogen stream. After stirring the reaction solution at 60˚C for 1.5 hours, the reaction solution was cooled with ice, and a N, N-dimethylformamide solution (2 mL) of the compound (482 mg, 1.0 mmol) in Reference Example 208 was dropped into the solution. The reaction solution was stirred for 1.5 hours again at 60˚C. After completing the reaction, the reaction solution was poured into saturated saline solution, and the reaction product was extracted with ethyl acetate. The organic layer was washed with saturated saline solution, dried over anhydrous magnesium sulfate and concentrated in vacuum. The residue obtained was purified by silica gel column chromatography (hexane : ethyl acetate / 1:4) to obtain
3-[3-[2-bromo-5-[[(4S)-4-methyl-2,3,4,5-tetrahydro-1H-1,5-benzodiazepin-1-yl]carbonyl]phenoxy]propyl]-2,4-imidazolidinedione (282 mg, 56.2%) as a colorless solid.
$^1$H-NMR(DMSO-$d_6$: 120˚C) δ:1.24 (3H,d,J=5.9Hz), 1.58-1.78 (1H,m), 1.80-2.01(3H,m), 3.30-3.64(4H,m), 3.75-4.45(6H, m), 4.89 (1H,brs), 6.42-6.54(1H,m), 6.55-6.63(1H,m), 6.73-6.86(2H,m), 6.89-6.98(2H,m), 7.33(2H,d,J=7.8Hz), 7.54(1H, brs)ppm
FABMS:503(M+1)

Reference Example 213

(4S)-1-(3-Fluoro-4-nitrobenzoyl)-4-methyl-2,3,4,5-tetrahydro-1H-1,5-benzodiazepine was obtained by the same procedure in Reference Example 117 using the compounds in Reference Examples 5 and 39. (yield 45.2%) $^1$H-NMR ($CDCl_3$) δ: 1.38 (3H,d,J=6.3Hz) 1.80-1.90 (2H,m), 2.70-2.75 (1H,m),3.08-3.80(2H,m),4.95-5.05(1H,m),6.46-6.63 (3H,m), 6.85-7.16(3H,m),7.75-7.81(1H,m)ppm
FABMS:330(M+1)

Reference Example 214

An acetonitrile solution of the compound (400 mg, 1.21 mmol) in Reference Example 213, morpholine (127 mg, 1.45 mmol) and potassium carbonate (250 mg, 1.82 mmol) was refluxed with heating for 15 hours. After completing the reaction, the reaction solution was concentrated in vacuum. Water was added to the residue obtained, and the reaction product was extracted with ethyl acetate. The ethyl acetate layer was washed with saturated saline solution,

dried over anhydrous magnesium sulfate and concentrated in vacuum to obtain (4S)-4-methyl-[3-(4-morphonyl)-4-nitrobenzoyl-2,3,4,5-tetrahydro-1H-benzodiazepine (450 mg, 86.3%) as pale yellow crystals.
$^1$H-NMR($CDCl_3$) δ : 1.38 (3H, d, J=6. 3Hz), 1.80-1.98 (2H,m), 2.68-2.84 (5H,m), 3.09-3.56 (2H,m), 3.72-3.76 (4H,m), 5.01-5.06 (1H,m), 6.47-6.66(2H,m),6.84-7.10(4H,m),7.56-7.59(1H,m)ppm
FABMS: 397 (M+1)

Reference Example 215

4-[[(4S)-4-Methyl-2,3,4,5-tetrahydro-1H-1,5-benzodiazepin-1-yl]carbonyl]-2-(4-morphonyl)phenylamine was obtained by the same procedure in Reference Example 143 using the compound in Reference Example 214. (yield 93.2%).
$^1$H-NMR($CDCl_3$)δ:1.3(3H,brs), 1.80-1.90(2H,m) 2.68-2.80 (5H,m),3.0-3.60(2H,m),3.72-3.73(4H,m),4.00-4.10(2H,m), 5.10-5.20(1H,m),6.49-6.70(4H,m),6.87-7,15(3H,m)ppm
FABMS:367 (M+1)

Reference Example 216

Hydrobromic acid (37%, 3mL) was added to an acetone solution (30 mL) of the compound (450 mg, 1.23 mmol) in Reference Example 215, and the solution was stirred at 0˚C for 5 minutes. Then, an aqueous solution (3mL) of sodium nitrite (93 mg, 1.35 mmol) was dropped in such a way that the temperature of the reaction solution did not increase above 0˚C. After completing dropping, the reaction solution was stirred for 30 minutes while the temperature was kept at 0˚C. Copper bromide (194 mg, 1.35 mmol) was then added in small portions. After the addition, the reaction solution was stirred at room temperature for 1 hour. After completing the reaction, water was added to the reaction solution, and the reaction product was extracted with ethyl acetate. The ethyl acetate layer was washed with saturated saline solution, dried over anhydrous magnesium sulfate and concentrated in vacuum. The residue obtained was purified by silica gel column chromatography (hexane : ethyl acetate / 5:1) to obtain (4S)-1-[4-bromo-3-(4-morphonyl)benzoyl]-4-methyl-2,3,4,5-tetrahydro-1H-1,5-benzodiazepine (360 mg, 65.9%) as colorless crystals.
$^1$H-NMR($CDCl_3$)δ:1.36(3H,d,J=6.3Hz),1.80-1.90(2H,m),2.60-2.80 (5H,m),3.00-3.50(2H,m),3.76-3.77(4H,m),5.00-5.10 (1H,m), 6.60-6.90(3H,m),6.90-7.00(2H,m),7.30-7.50(2H,m)ppm
FABMS : 431 (M+1)

Reference Examples 217

1-Propyl-4-piperazinyl 2-bromo-5-[[(4S)-4-methyl-2, 3, 4, 5-tetrahydro-1H-1,5-benzodiazepin-1-yl] carbonyl] benzoate was obtained by the same procedure in Reference Example 45 using the compound in Reference Example 199 and 4-hydroxy-1-propylpiperidine.
(yield 29%)
$^1$H-NMR(DMSO-$d_6$,120˚C) δ:0.87(3H,t,J=7.3Hz),1.24 (3H,d,J=6.3Hz),1.46(2H,quintet,J=7.3Hz),1.60-1.80(3H,m), 1.85-2.00(3H,m),2.25-2.35(4H,m),2.60-2.70(2H,m),3.20-3.60 (2H,m),3.90-4.30(1H,m),4.85-4.95(2H,m),6.40-6.60(2H, m), 6.91-6.95(2H,m),7.22-7.27(1H,m),7.47-7.52(1H,m)ppm
FABMS:515(M+1)

Reference Examples 218

4-Pyridinylmethyl 2-bromo-5-[[(4S)-4-methyl-2,3,4,5-tetrahydro-1H-1,5-benzodiazepin-1-yl]carbonyl]benzoate was obtained by the same procedure in Reference Example 45 using the compound in Reference Example 199 and 4-pyridinylmethanol. (yield 72%)
$^1$H-NMR(DMSO-$d_6$,120˚C)δ:2.23 (3H,d, J=6.3Hz), 1.60-1.75 (1H,m), 1.85-2.00(1H,m),3.30-3.50(2H,m),4.85(1H,s),5.31 (2H,s), 6.42-6.62(2H,m),6.85-6.95(2H,m),7.25-7.35(3H,m),7.53 (1H,d,J=8.3Hz),7.61(1H,d,J=2.5Hz),8.59(2H,dd, J=4.4,2.5Hz) ppm
FABMS:481(M+1)

Reference Examples 219

N-Isopropyl-4-methyl-N-[2'-[3-[methyl(propyl)amino]-propoxy]-4'-[[(4S)-4-methyl-2,3,4,5-tetrahydro-1H-1,5-benzodiazepin-1-yl]carbonyl[1,1'-biphenyl]-4-yl]benzene sulfonamide was obtained by the same procedure in Reference

Example 120 using the compounds in Reference Examples 210 and 78. (yield 75.4%)
$^1$H-NMR(DMSO-d6,120˚C)δ:80.79(3H,t,J=7.3Hz),1.03 (6H,d,J=6.8Hz),1.25(3H,d,J=5.9Hz),1.30-1.41(2H,m),1.60-1.74 (3H,m),1.83(1H,br),2.08(3H,s),2.19(2H,t,J=6.8Hz),2.39 (3H,s),3.45(2H,br),6.44-6.50(1H,m),6.62(1H,d,J=7.8Hz), 6.88-6.95(4H,m),6.99-7.10(2H,m),7.14(1H,d,J=7.8Hz), 7.33-7.52(4H,m),7.59-7.62(2H,m)ppm
FABMS: 683 (M+1)

Reference Examples 220

1-Ethyl-3-pyrrolidinyl 2-bromo-5-[[(4S)-4-methyl-2,3,4,5-tetrahydro-1H-1,5-benzodiazepin-1-yl]carbonyl]benzoate was obtained by the same procedure in Reference Example 45 using the compound in Reference Example 199 and 1-ethyl-3-pyrrolidinol. (yield 88%)
$^1$H-NMR,(DMSO-$d_6$, 120˚C)δ:1.05(3H,t,J=6.8Hz) 1.2S (3H,d,J=6.3Hz),1.60-2.00(2H,m),2.15-2.30(1H,m),2.40-2.55 (5H,m),2.60-2.80(2H,m),3.30-3.60(2H,m),3.90-4.40(1H,m), 4.85(1H,s),5.20-5.30(1H,m),6.40-6.60(2H,m),6.90-6.95 (2H,m), 7.23(1H,dd,J=7.8,2.5Hz),7.50(1H,d,J=7.8Hz),7.50-7.55(1H,m) ppm
FABMS:487(M+1)

Reference Examples 221

(4S)-4-Methyl-1-[3-(4-methyl-1-piperazinyl)-4-nitrobenzoyl]-2,3,4,5-tetrahydro-1H-1,5-benzodiazepine was obtained by the same procedure in Reference Example 214 using the compound in Reference Example 213 and N-methyl piperazine. (yield 87.2%)
$^1$H-NMR($CDCl_3$)δ:1.38(3H,d,J=6.3Hz),1.80-1.90(2H,m),2.25 (3H,s),2.78-2.94(5H,m),3.09-3.56(2H,m),3.72-3.76(4H, m), 5.01-5.06(1H,m),6.47-6.66(2H,m),6.84-7.10(4H,m),7.56-7.59 (1H,m)ppm
FAEMS:410 (M+1)

Reference Examples 222

2-{4-Methyl-1-pzperazinyl}-4-[[(4S)-4-methyl-2,3,4,5-tetrahydro-1H-1,5-benzodiazepin-1-yl]carbonyl]phenylamine was obtained by the same procedure in Reference Example 143 using the compound in Reference Example 221. (yield 30.8%)
$^1$H-NMR($CDCl_3$)δ:1.35 (3H,brs), 1.80-1.90(2H,m), 2.25(3H,s), 2.78-2.90(5H,m),3.00-3.60(2H,m),3.72-3.73(4H,m), 4.00-4.10 (2H,m),5.10-5.20(1H,m),6.49-6.70(4H,m),6.87-7.15(3H,m)ppm
FABMS:380 (M+1)

Reference Examples 223

(4S)-1-[4-Bromo-3-(4-methyl-1-piperazinyl)benzoyl]-4-methyl-2, 3, 4, 5-tetahydro-1H-1, 5-benzodiazepine was obtained by the same procedure in Reference Example 216 using the compound in Reference Example 222. (yield 79.4%)
$^1$H-NMR($CDCl_3$)δ:1.36(3H,d,J=6.3Hz),1.80-1.90(2H,m),2.25 (3H,s),2.80-2.95(5H,m),3.00-3.50(2H,m),3.76-3.77(4H,m), 5.00-5.10(1H,m),6.60-6.90(1H,m),6.90-7.00(2H,m),7.30-7.50 (2H,m) ppm
FABMS:443 (M+1)

Reference Examples 224

(4S)-1-[3-[(4-Chloro-2-butynyl)oxy]-4-(1H-indol-5-yl) benzoyl]-4-methyl-2,3,4,5-tetrahydro-1H-1,5-benzodiazepine was obtained by the same procedure in Reference Example 45 using the compounds in Reference Examples 180 and 113. (yield 88.2%)
$^1$H-NMR(DMSO-$d_6$,120˚C) δ:1.26(3H,d,J=6.6Hz),1.68(1H,m),1.96 (1H,m),3.44(2H,br),4.03(1H,br),4.35(2H,s),4.52(2H, s),6.40 (1H,s),6.50(1H,m),6.64(1H,d,J=7.3Hz),6.90-7.00(4H,m),7.13 (1H,dd,J=2.2,7.3Hz),7.25(1H,t,J=2.2Hz),7.36(1H, d,J=8.1Hz),7 .56 (1H,s), 10.68 (1H,br)ppm
FABMS:485 (M+1)

Reference Examples 226

1,1-Diethylpropyl 2-(benzyloxy)-3'-fluoro-4'-nitro[1,1'-biphenyl]-4-carboxylate was obtained by the same procedure in Reference Example 120 using the compounds in Reference Examples 60 and 80. (yield 64.0%)
$^1$H-NMR ($CDCl_3$)δ: 0.89(9H, t, J=7.3Hz), 1.99(6H, q, J=7.3Hz), 5.18 (2H, s), 7.34-7.40 (6H, m), 7.47-7.56 (2H, m), 7.69-7.73 (2H, m), 8.10 (1H, t, J=8.3Hz) ppm

FABMS:466 (M+1)

Reference Examples 227

2-(Benzyloxy)-3'-fluoro-4'-nitro[1,1'-biphenyl]-4-carboxylic acid was obtained by the same procedure in Reference Example 185 using the compound in Reference Example 226. (yield 95.4%)
$^1$H-MMR($CDCl_3$) δ:5.27(2H,m),7.32-7.45(5H,m),7.62 (1H,d,J=8.3Hz),7.66-7.69(2H,m),7.76(1H,d,J=1.0Hz),7.84 (1H, dd, J=12.9,1. 7Hz), 8.21(1H, t, J=8.3Hz)ppm

Reference Examples 228

(4S)-1-[[2-(Benzyloxy)-3'-fluoro-4'-nitro[1,1'-biphenyl]-4-yl]carbonyl]-4-methyl-2,3,4,5-tetrahydro-1H-1,5-benzodiazepine was obtained by the same procedure in Reference Example 117 using the compounds in Reference Examples 5 and 227. (yield 74.9%)
$^1$H-NMR(DMSO-$d_6$,120˚C) δ:1.25(3H,d,J=6.8Hz), 1.60-1.73 (1H,m) , 1.91-1.96(1H,m),3.41(2H,br),4.09(1H,br), 4.83-4.94(2H,m), 5.01(1H,br),6.46-6.51(m,1H),6.62(1H,d,J=7.8Hz),6.90-7.04 (4H,m),7.25-7.36(6H,m),7.45-7.49(1H, m),7.54 (1H,dd,J=12.7,2.0Hz),8.05(1H,t,J=8.3Hz)ppm
FABMS:512(M+1)

Reference Examples 229

2'-(Benzyloxy)-3-fluoro-4'-[[(4S)-4-methyl-2,3,4,5-tetrahydro-1H-1,5-benzodiazepin-1-yl] carbonyl] [1,1'-biphenyl]-4-ylamine was obtained by the same procedure in Reference Example 125 using the compound in Reference Example 228. (yield 79.5%)
$^1$H-NMR(DMSO-$d_6$,120˚C)δ:1.25(3H,d,J=6.8Hz),1.58-1.71(1H,m), 1.89-1.96(1H,m),3.41(2H,br),4.09(1H,br),4.76-4.84 (2H,m), 4.92(2H,s),6.44-6.50(1H,m),6.59(1H,d,*J*=4.8Hz),6.75 (1H,dd,*J*=9.8,7.8Hz),6.89-7.02(5H,m),7.07-7.13(2H,m), 7.24-7.
35 (5H,m)ppm
FABMS:482 (M+1)

Reference Examples 230

A tetrahydrofuran solution (5 mL) of trifluoroacetic anhydride (0.69 mL. 4.96 mmol) was added to a tetrahydrofuran solution (20 mL) of the compound (2.12g, 4.51 mmol) in Reference Example 229 and pyridine (0.43 mL, 5.41 mmol) by cooling with ice in a nitrogen stream. After stirring for 2 hours, the reaction mixture was concentrated in vacuum. The residue obtained was poured into saturated saline solution, and the reaction product was extracted with ethyl acetate. The organic layer was washed with saturated saline solution, dried over anhydrous magnesium sulfate and concentrated in vacuum. The residue obtained was roughly purified by silica gel column chromatography (hexane:ethyl acetate/3:2). The roughly purified product obtained was directly used for the next reaction.
Subsequently, an acetone solution (60 mL) of the mixture obtained, ethane iodide (991 mg, 6.35 mmol) and potassium hydrogen carbonate (890 mg, 6.44 mmol) was heated at 60˚C in a nitrogen stream. After stirring for 2 hours, the reaction mixture was concentrated in vacuum. The residue obtained was poured into saturated saline solution, and the reaction product was extracted with ethyl acetate. The organic layer was washed with saturated saline solution, dried over anhydrous magnesium sulfate and concentrated in vacuum. The residue obtained was roughly purified by silica gel column chromatography (hexane : ethyl acetate / 3:2). The roughly purified product obtained was directly used for the next reaction. Palladium-carbon (10%, 1.20 g) was added to a methanol solution (85 mL) of the mixture obtained, and the solution was stirred for 3 hours in hydrogen stream at room temperature. The catalyst was filtered off, and the filtrate was concentrated in vacuum to obtain N-[2'-(benzyloxy)-3-fluoro-4'-[[(4S)-4-methyl-2,3,4,5-tetrahydro-1H-1,5-benzodiazepin-1-yl]carbonyl][1,1'-biphenyl]-4-yl]-N-ethyl-2,2,2-trifluorcacetamide. This compound was used for the reaction in Reference Example 231 without further purification.

Reference Example 231

N-[2'-(3-Bromopropoxy)-3-fluoro-4'-[[(4S)-4-methyl-2,3,4,5-tetrahydro-1H-1,5-benzodiazepin-1-yl]carbonyl] [1,1'-biphenyl]-4-yl]-N-ethyl-2,2,2-trifluoroacetamide was obtained by the same procedure in Reference Example 45 using the compound in Reference Example 230 and 3-bromopropanol. The reaction product was used for the reaction in Example 155 without further purification.

Reference Example 232

4-[2-Bromo-5-[[(4S)-4-methyl-2,3,4,5-tetrahydro-1H-1,5-benzodiazepin-1-yl]carbonyl]phenoxy]-N-methyl-N-propyl-1-butanamine was obtained by the same procedure in Reference example 210 using a compound, which was obtained by the same procedure in Reference Example 45 using the compound in Reference example 165 and 4-bromobutanol, and N-methyl propylamine. The reaction product was used for the reaction in Example 156 without further purification.

Reference Example 233

5-(Benzyloxy)-1-[2-bromo-5-[[(4S)-4-methyl-2,3,4,5-1H-1,5-benzodiazepin-1-yl] carbonyl] phenyl]-1-pentanone was obtained by the same procedure in Reference Example 117 using the compounds in Reference Examples 5 and 69. (yield 65.7%)
$^1$H-NMR(DMSO-$d_6$,120˚C) δ:1.23 (3H,d,J=6.3Hz), 1.55-1.96(4H,m), 2.65-2.67(2H,m),3.22-4.25(5H,m),4.45(2H,s)5.91 (IH,brs), 6.45-6.71(3H,m),6.91-6.92(2H,m),7.20-7.32(6H,m),7.46-7.49 (1H,m)ppm
FABMS:536 (M+1)

Reference Example 234

5-(Benzyloxy)-1-[2-(1H-indol-5-yl)-5-[[(4S)-4-methyl-2,3,4,5-tetrahydro-1H-1,5-benzodiazepzn-1-yl]carbonyl] phenyl]-1-pentanone was obtained by the same procedure in Reference Example 120 using the compounds in Reference Examples 233 and 70. (yield 70.9%)
$^1$H-NMR(DMSO-$d_6$, 120˚C) δ: 1.15-1.29(7H,m),1.53-1.74(1H,m), 1.91-1.96(1H,m),2.08(2H,t,J=7.3Hz),3.17(2H,t, J=7.3Hz),3.17 (2H,t,J=6.6Hz),3.41-4.29(5H,m),4.89(1H,brs),6.41-6.48 (2H,m),6.60(1H,d,J=7.3Hz),6.88-6.91(3H,m), 7.17-7.40(1H,m) 10.8(1H,brs)
FABMS:519(M+1)

Reference Example 235

Palladium-carbon (10%, 63 mg) and acetic acid (1 mL) were added to a methanol solution (3 mL) of the compound (301 mg, 0.526 mmol) in Reference Example 234, and the reaction mixture was stirred for 54 hours after substituting the reaction atmosphere with hydrogen. The reaction solution was concentrated in vacuum, and the residue was added to saturated saline solution. The reaction product was extracted with ethyl acetate. The organic layer was washed with saturated saline solution, dried over anhydrous magnesium sulfate and concentrated in vacuum to obtain 5-hydroxy-1-[2-(1H-indol-5-yl)-5-[[(4S)-4-methyl-2,3,4,5-tetrahydro-1H-1,5-benzodiazepin-1-yl]carbonyl]phenyl]-1-pentanone as an yellow solid (259 mg, quantitative yield).
$^1$H-NMR (DMSO-$d_6$, 120˚C) δ:1.10-1.26 (7H,m), 1.66-1.69 (1H,m), 1.94-1.96(1H,m),2.08(2H,t,J=7.3Hz),3.14-3.21(2H, m), 3.42-4.08 (4H, m), 4.90 (1H, brs), 6.42-6.49 (2H, m), 6.61 (1H,d,J=8.1Hz),6.89-6.93(3H,m),7.18-7.40(6H,m),10.8 (1H,brs) ppm
FABMS:482(M+1)

Reference Example 236

Triethylamine (0.1 mL, 0.727 mmol) and methanesulfonyl chloride (86 mg, 0.727 mmol) were added to a methylene chloride solution (6 mL) of the compound (250 mg, 0.519 mmol) in Reference Example 235 in a nitrogen atmosphere by cooling with ice, and the reaction mixture was stirred for 3 hours. After stirring for 30 minutes by adding methanesulfonyl chloride (56 mg, 0.489 mmol), the reaction mixture was poured into an aqueous citric acid solution, and the reaction product was extracted with chloroform. The organic layer was washed with distilled water, dried over anhydrous magnesium sulfate and concentrated in vacuum to obtain
5-[2-(1H-indol-5-yl)-5-[[(4S)-4-methyl-2,3,4,5-tetrahydro-1H-1,5-benzodiazepin-1-yl] carbonyl] phenyl]-5-oxypentyl methane sulfonate. The compound was used for the reaction in Example 158 without further purification.

Reference Example 237

(4S)-1-[4-Bromo-3-[3-(4-propyl-1-piperazinyl)propoxy] benzoyl]-4-methyl-2,3,4,5-tetrahydro-1H-1,5-benzodiazepine was obtained by the same procedure in Reference Example 210 using the compound in Reference Example 208 and N-propyl piperazine. The compound was used for the reaction in Example 159 without further purification.

Reference Example 238

(4S)-1-[4-Bromo-3-[2-(4-propyl-1-pzperazinyl)ethoxy] benzoyl]-4-methyl-2,3,4,5-tetrahydro-2H-1,5-benzodiazepine was obtained as an oily substance by the same procedure in Reference Example 210 using the compound, which was obtained by the same procedure in Reference example 45 using the compound obtained in Reference Example 164 and 2-chloroethanol, and N-propyl piperazine. The compound was used for the reaction in Example 160 without further purification.

Reference Example 239

An acetonitrile solution (50 mL) of the compound (774 mg, 1.66 mmol) in Reference Example 226, dimethylamine hydrochloride (272 mg, 3.33 mmol) and potassium carbonate (460 mg, 3.33 mmol) was stirred for 2 hours at 60˚C in a nitrogen stream. After completing the reaction, the reaction mixture was concentrated in vacuum, the residue was added to saturated saline solution, and the reaction product was extracted with ethyl acetate. The organic layer was washed with saturated saline solution, dried over anhydrous magnesium sulfate and concentrated in vacuum. The residue obtained was purified by silica gel column chromatography (hexane : ethyl acetate / 9:1) to obtain 1,1'-diethylpropyl 2-(benzyloxy),-3'-(dimethylamino)-4'-nitro[1,1'-biphenyl]-4-carboxylate as a pale yellow solid (791 g, 97.1%). The compound was used for the reaction in Example 240 without further purification.
$^1$H-N-MP,($CDCl_3$)δ:0·90(9H,t,J=7.3Hz)2.0(6H,q,J=7.3Hz)2.82 (6H,s),5.14(2H,s),7.01(1H,dd,J=8.3,2.0Hz),7.24 (1H,d, J=1.5Hz),7.34-7.36(5H,m),7.40(1H,d,J=7.8Hz), 7. 68-7.73 (2H,m) 7.84 (1H, d, J=8.8Hz) ppm
FABMS: 491 (M+1)

Reference Example 240

1,1'-Diethylpropyl 4'-aminc-2-(benzyloxy)-3'-(dimmethylamino)[1,1'-bipheny]-4-carboxylate was obtained by the same procedure in Reference Example 125 using the compound in Reference Example 239. (yield 82.0%)
$^1$H-NMR($CDCl_3$) δ: 098 (9H, t, J=7.3Hz) 2. 07 (6H, q, J=7.3Hz), 2.70 (6H,s),5.20(2H,s),6.85(1H,d,J=8.3Hz),7.24-7.49 (8H,m), 7.73-7.76(2H,m)ppm
FABMS : 4 61 (M+1)

Reference Example 241

A tetrahydrofuran solution (5mL) of trifluoroacetic acid anhydride (305 mL, 1.45 mmol) was dropped into a tetrahydrofuran solution (50 mL) of the compound (608 mg, 1.32mmol) in Reference Example 240 and pyridine (0.13 mL, 1.58 mmol) in a nitrogen stream by cooling with ice. After stirring for 0.5 hours, the reaction mixture was concentrated in vacuum. The residue obtained was poured into saturated saline solution, and the reaction product was extracted with ethyl acetate. The organic layer was washed with saturated saline solution, dried over anhydrous magnesium sulfate and concentrated in vacuum. The crude product obtained was used for the next reaction without further purification.

Subsequently, an acetone solution (30 mL) of the crude product obtained, ethane iodide (0.6 mL, 4.62 mmol) and potassium hydrogen carbonate (730 mg, 5.28 mmol) was heated at 60˚C in a nitrogen stream. After stirring the solution for 16 hours, the reaction mixture was concentrated in vacuum. The residue obtained was poured into saturated saline solution, and the reaction product was extracted with ethyl acetate. The organic layer was washed with saturated saline solution, dried over anhydrous magnesium sulfate and concentrated in vacuum. The crude product obtained was used for the next reaction without further purification.

Trifluoroacetic acid (3 mL) was added to the methylene chloride (30 mL) solution of the crude product obtained in a nitrogen stream at room temperature, and the mixed solution was stirred for 1 hour. After completing the reaction, the reaction mixture was concentrated in vacuum, the residue was added to saturated saline solution, and the reaction product was extracted with ethyl acetate. The organic layer was washed with saturated saline solution, dried over anhydrous magnesium sulfate and concentrated in vacuum. The residue obtained was purified by silica gel column chromatography (hexane: ethyl acetate / 2:3) to obtain
2-(benzyloxy)-3'-(dimethylamino)-4'-[ethyl(trifluoroacetyl) amino][1,1'-biphenyl]-4-carboxylic acid as a pale yellow oil (345 mg, 53.7%, three steps).
$^1$H-NMR($CDCl_3$)δ:1.18(3H,t,J=6.8Hz),2.68(3H,s),3.50(1H,br), 4.18(1H,br),5.16(2H,s),7.08-7.95(12H,m)ppm
FABMS:487(M+1)

Reference Example 242

N-[2'-(Benzyloxy)-3-(diethylamino)-4'-[[(4S)-4-methyl-2,3,4,5-tetrahydro-1H-1,5-benzodiazepin-1-yl] carbonyl]

[1,1'-biphenyl]-4-yl]-N-ethyl-2,2,2-trifluoroaceta mide was obtained by the same procedure in Reference Example 117 using the compounds in Reference Examples 241 and 5. (yield 41.2%)
$^1$H-NMR(DMSO-$d_6$,120˚C)δ:1.18(3H,t,J=6.8Hz),1.26 (3H,d,J=6.8Hz),1.66-1.70(1H,m),1.96(1H,br),2.60(3H,s), 2.85(m, 2H),3.42(2H,br),4.03(1H,br),4.06-4.86(2H,m),4.97 (1H,br),6.47-6.52(1H,m),6.63(2H,d,J=7.8Hz),6.90-7.26 (13H,m)ppm
FABMS : 632 (M+1)

Reference Example 243

N-[3-(Diethylamino)-2'-hydroxy-4'-[[(4S)-4-methyl-2,3,4,5-tetrahydro-1H-1,5-benzodiazepin-1-yl]carbonyl [1,1'-biphenyl]-4-yl]-N-ethyl-2,2,2-trifluoroacetamide was obtained by the same procedure in Reference Example 143 using the compound in Reference Example 242. (yield 55.1%) 1H-NMR(DMSO-$d_6$,120˚C)δ:1.18-1.25(6H,m),1.62-1.70 (1H,m), 1.89-1.96(1H,m),2.58 (3H, s), 2.67 (3H, s), 3.13-3.21 (2H,m), 3.44-3.75(2H,m), 4.03(1H,m)4.81(1H,br)6.46-6.68 (2H,m), 6.83-7.17(5H,m),7.33(1H,s),7.61(1H,d,J=7.8Hz)ppm
FABMS: 541 (M+1)

Reference Example 244

N-[2'-(3-Bromopropoxy) 3-(diethylamino)-4'-[[(4S)-4-methyl-2,3,4,5-tetrahydro-1H-1,5-benzodiazepin-1-yl] carbonyl][1,1'-biphenyl]-4-yl]-N-ethyl-2,2,2-trifluoroacetamide was obtained by the same procedure in Reference Example 45 using the compound in Reference Example 243 and 4-bromopropanol. (yield 67.9%)

Reference Example 245

Tricyclohexyl phosphine (79 mg, 0.282 mmol), tris (dibenzylideneactone) dipalladium (0) (93 mg, 0.102 mmol), the compound (2.65 g, 7.15 mmol) in Reference Example 60 and cesium carbonate (2.66 g, 8.17 mmol) were added to a dioxane solution (25 ml) of 5-chloro-2-nitroanisol (1.28 g, 6.81 mmol) at room temperature in a nitrogen atmosphere, and the mixture was stirred at 80˚C for 13 hours. The reaction solution was warmed to room temperature, and was filtered through celite after diluting with diethyl ether. The filtrate was concentrated in vacuum, and the residue was purified by column chromatography (hexane : ethyl acetate / 10:1) to obtain 1,1'-diethylpropyl 2-(benzyloxy)-3'-(dimethylamino)-4'-nitro[1,1'-biphenyl]-4-carboxylate as a pale yellow solid (3.25 g, 98.2%).
$^1$H-NMR($CDCl_3$)δ:0.90(9H,t,J=7.3Hz),2.00(6H,q,7.3Hz),3.78 (3H,s),5.15(2H,s),7.18(1H,dd,J=1.5 and 8.4Hz), 7.34-7.36 (5H,m)7.40-7.43(2H,m)7.71(1H,dd,J=1.5 and 7.8Hz), 7.75(1H,d,J=1.5Hz),7.92(1H,d,J=8.3Hz)ppm
FABMS:478(M+1)

Reference Example 246

2- (Benzyloxy) -3'- (dimethylamino)-4'-nitro [1, 1'-biphenyl] -4-carboxylic acid was obtained by the same procedure in Reference Example 185 using the compound in Reference Example 245. (yield 80.3%)
$^1$H-NMR (DMSO-$d_6$) δ:3.83 (3H, s), 5.23 (3H, s) 7.28-7.69 (0H,m), 7.76 (1H, s), 7.91 (1H, d, J=8.1 Hz) ppm

Reference Example 247

(4S)-1-[[2-(Benzyloxy)-3'-methoxy-4'-nitro[1,1'-biphenyl]-4-yl]carbonyl]-4-methyl-2,3,4,5-tetrahydro-1H-1,5-benzodiazepine was obtained by the same procedure in Reference Example 117 using the compounds in Reference Examples 246 and 5. (yield 81.7%)
$^1$H-NMR(DMSO-$d_6$, 120˚C) δ:1.25 (3H,d,J=6.4Hz), 1.68-1.96(2H,m), 3.42-4.08(6H,m),4.85-4.87(2H,m),4.97(1H,brs), 6.47-6.53 (1H,m),6.64(1H,d,J=7.8Hz),6.97-7.33(12H,m),7.78 (1H,d,J=8.3Hz) ppm
FABMS:524 (M+1)

Reference Example 248

2'-(Benzyloxy)-3-methoxy-4'-[[(4S)-4-methyl-2,3,4,5-tetrahydro-1H-1,5-benzodiazepin-1-yl]carbonyl][1,1'-biphenyl]-4-yl amine was obtained by the same procedure in Reference Example 125 using the compound in Reference Examples 247. (yield 81.7%)
$^1$H-NMR(DMSO-$d_6$,120˚C) δ:1.24 (3H, d, J=6.3Hz), 1.65-1.90 (2H,m), 3.4-4.10(6H,m),4.46(1H,br-s),4.76-4.78(2H,m), 4.93(1H,br-s), 6.44-6.52(1H,m),6.59-6.63(2H,m),6.82(1H,dd,J=2.0 and 8.3Hz),6.92-6.96(5H,m),7.12(1H,d,J=8.3Hz),7.25-7.30(5H,m) ppm
FABMS:494(M+1)

Reference Example 249

A methylene chloride solution (5 mL) of trifluoroacetic acid anhydride (0.38 mL, 2.72 mmol) was added to a methylene chloride solution (50 mL) of the compound (1.22 g, 2.47 mmol) in Reference Example 248 and pyridine (0.24 mL, 2.97 mmol) by cooling with ice in a nitrogen stream. After stirring the mixed solution at room temperature for 2 hours, trifluoroacetic acid anhydride (0.30 mL, 2.14 mmol) and pyridine (0.2 mL, 2.47 mmol) was added by cooling with ice, and the mixed solution was stirred at room temperature for 1 hour. The reaction solution was added to water, and the reaction product was extracted with chloroform. The organic layer was washed with saturated saline solution, dried over anhydrous magnesium sulfate and concentrated in vacuum. The residue obtained was purified by column chromatography (hexane : ethyl acetate / 3:2) to obtain N-[2'-(benzyloxy)-3-methoxy-4'-[[(4S)-4-methyl-2,3,4,5-tetrahydro-1H-1,5-benzodiazepin-1-yl]carbonyl][1,1'-biphenyl]-4-yl]-2,2,2-trifluoroacetamide (1.26 g. 86.3%) as a yellow solid.
$^1$H-NMR(DMSO-$d_6$,120˚C)δ:1.25(3H,d,J=6.4Hz),1.62-1.96(2H,m), 3.42-4.08(7H,m),4.80(1H,d,J=11.7Hz),4.85(1H,d, J=11.7Hz), 4.96(1H,br-s),6.48-6.50(1H,m),6.62(1H,d,J=7.3Hz),6.88-7.09 (4H,m),7.10-7.39(8H,m),7.48(1H,d, J=7.8Hz),9.94(1H,s)ppm
FABMS:590(M+1)

Reference Example 250

Ethyl iodide (0.521 mL, 4.08 mmol) was added to an acetone solution (12mL) of the compound (1.2 g, 2.04 mmol) in Reference Example 249 and potassium carbonate (0.569 g, 4.12 mmol) at room temperature in a nitrogen stream. After stirring the mixed solution at 60˚C for 4 . 5 hours, potassium carbonate (0.309 g, 2.24 mmol), ethyl iodide (0.28 mL, 2.19 mmol) and acetone (9 mL) were added to the solution, which was stirred at 60˚C for 65 hours. The reaction solution was concentrated in vacuum, the residue obtained was added to water, and the reaction product was extracted with ethyl acetate. The organic layer was washed with saturated saline solution, dried over anhydrous magnesium sulfate and concentrated in vacuum. The residue was purified by column chromatography (hexane : ethyl acetate/ = 2:1) to obtain N-[2'-(benzyloxy)-3-methoxy-4'-[[(4S)-4-ethyl-2,3,4,5-tetrahydro-1H-2,5-benzodiazepin-1-yl]carbonyl] [1,1'-biphenyl]-4-yl]-N-ethyl-2,2,2-trifluoroacetamide (0.907 g, 72.0%) as an yellow solid.
$^1$H-NM-R(DMSO-$d_6$,120˚C)δ:1.07(3H,t,J=7.3Hz),1.26 (3H,d,J=6.8Hz),1.69-1.96(2H,m),3.42-4.46(8H,m),4.82-4.83 (2H,m),4.97(1H,br-s),6.46-6.52(1H,m),6.62-6.64(1H,m), 6.90-7.06(5H,m) ,7.19-7.26(8H,m)ppm
FABMS:618(M+1)

Reference Example 251

N-Ethyl-2,2,2-trifluoro-N-[2'-hydroxy-3-methoxy-4'-[[(4S)-4-methyl-2,3,4,5-tetrahydro-1H-1,5-benzoazepin-1-yl] carbonyl][1,1'-biphenyll-4-yl]acetamide was obtained by the same procedure in Reference Example 143 using the compound in Reference Example 250. (yield 99.6%) The compound was used for the reaction in Reference Example 251 without further purification.
$^1$H-NMR (DMSO-$d_6$, 120˚C) δ:1.09 (3H, t, J=7.3Hz), 1.25 (3H, d, J=6.4Hz), 1.58-1.73 (1H, m), 1.92-1.96 (1H, m), 3.44-4.05 (8H, m), 4.83 (1H, br-s), 6.44-6.50 (1H, m), 6.63 (1H, d, J=7.82Hz), 6.69 (1H, d, J=8.3Hz), 6.91 (1H, d, J=3.9Hz), 6.97 (1H, d, J=1.46Hz), 7.09-7.33(5H, m) ppm
FABMS:528(M+1)

Reference Example 252

N-[2'-(3-Bromopropoxy)-3-methoxy-4'-[[(4S)-4-methyl-2,3,4,5-tetrahydro-1H-1,5-benzodiazepin-1-yl]carbonyl] [1,1'-biphenyl]-4-yl]-N-ethyl-2,2,2-trifluoroacetamide was obtained by the same procedure in Reference Example 45 using the compound in Reference Example 251 and 3-bromopropanol. The compound was used for the reaction in Reference Example 253 without further purification.

Reference Example 253

N-Ethyl-2,2,2-trifluoro-N-[3-methoxy-2'-[3-[methyl (propyl)amino]propoxy]-4'-[[(4S)-4-methyl-2,3,4,5-tetrahydro-1H-1,5-benzodiazepin-1-yl]carbonyl][1,1'-biphenyl]-4-yl]-acetamide was obtained by the same procedure in Reference Example 210 using the compound in Reference Example 252 and N-methyl propylamine. (yield 38.2%, total yield from the compound in Reference Example 251). $^1$H-NMR(DMSO-$d_6$,120˚C)δ:0.78(3H,t,J=6.8Hz),1.08 (3H,t,J=6.4Hz), 1.15-1.34(7H,m),1.64-1.69(3H,m),1.90-1.96 (1H,m),2.07(3H,s),2.18(2H,t,J=6.4Hz),2.28(2H,t,J=6.4Hz), 3.44-4.12(9H, m),4.92(1H,br-s),6.45-6.50(1H,m),6.63 (1H,d,J=8.3Hz),6.90-6.96(4H,m),7.04(1H,d,J=7.8Hz) 67.19-7.20(3H,m)ppm
FABMS: 641 (M+1)

Reference Example 254

An acetonitrile solution (20mL) of the compound (482 mg, 1.0 mmol) in Reference Example 208, propylamine (5 mL) and triethylamine (5 mL) was stirred for 4 hours by refluxing with heating in a nitrogen stream. After completing the reaction, the reaction solution was concentrated in vacuum, and the residue obtained was purified by NH-silica gel column chromatography (hexane : ethyl acetate / 3:2) to obtain
3-[[[(4S)-4-methyl-2,3,4,5-tetrahydro-1H-1,5-benzodiazepin-1-yl]carbonyl]phenoxy]-N-propyl-1-propanamine (315 mg, 68.5%) as a pale yellow oil.
$^1$H-NMR(DMSO-$d_6$, 120˚C)δ:0.87(3H,t,J=6.8Hz),1.24 (3H,d,J=5.9Hz),1.35-1.49(2H,m),1.63-1.80(3H,m),1.89(1H,br), 2.48-2.52(2H,m),2.64(2H,t,J=6.8Hz),3.38(2H,br),3.85-3.90 (2H,m),4.07(1H,br),4.90(1H,brs),6.46-6.50(1H,m),6.58 (1H,d,J=6.8Hz),6.75(1H,dd,J=8.8,2.0Hz),6.84-6.93(3H,m), 7.33(1H,d,J=8.8Hz)ppm
FABMS:461(M+1)

Reference Example 255

A methylene chloride solution (10 mL) of 1-ethyl-3-(3-dimethylaminopropyl)carbodiimide hydrochloride (271 mg, 1.42 mmol) was dropped into a methylene chloride solution (10 mL) of the compound in Reference Example 24 (416 mg, 0.944 mmol), N-benzyloxycarbonyl glycine (217 mg, 1.04 mmol), 3,4-dihydro-3-hydroxy-4-oxo-1,2,3-benzotriazine (231 mg, 1.42 mmol) and triethylamine (0.27 mL, 1.89 mmol) by cooling with ice in a nitrogen stream. After completing dropping, the reaction mixture was stirred at room temperature for 5 hours. After completing the reaction, the reaction mixture was concentrated in vacuum, and ethyl acetate was added to the residue. Saturated saline solution was poured into the ethyl acetate solution, and the reaction product was extracted. The organic layer was washed with saturated saline solution, dried over anhydrous magnesium sulfate and concentrated in vacuum. The residue obtained was purified by silica gel column chromatography (hexane : ethyl acetate / 1:4) to obtain benzyl 2-[[2-[2-(1H-indol-5-yl)-5-[(4-methyl-2,3,4,5-tetrahydro-1H-1,5-benzodiazepin-1-yl)carbonyl]phenoxy]ethyl]amino]-2-oxyethyl carbamate (382 mg, 64.1%) as an oily substance.
$^1$H-NMR(DMSO-d6,120˚C)δ:1.25(3H,d,J=6.8Hz) 1.63-1.70 (1H,m), 1. 91(1H,br),3.24-3.30(2H,m),3.41(2H,br),3.58-3.60 (2H,m), 2 . 3.72-3.77(2H,m),4.12(1H,br),4.87(1H,s),5.03(2H,s),6.41 3. (1H,s),6.46-6.52(1H,m),6.64(1H,d,J=7.8Hz), 6.75(1H,br), 4. 6.91-6.93(4H,m),7.11-7.37(9H,m),7.57(1H,s),10.66(1H,br) 5. ppm
FABMS:634(M+1)

Reference Example 256

Benzyl 2-[[2-[2-[(1H-indol-5-yl)-5-[(4-methyl-2,3,4,5-tetrahydro-1H-1,5-benzodiazepin-1-yl)carbonyl]phenoxy] propyl]amino]-3-oxyethyl carbamate was obtained by the same procedure in Reference Example 255 using the compound in Example 30. (yield 62.8%)
$^1$H-NMR(DMSO-$d_6$,120˚C)δ:1.36(3H,d,J=6.8Hz),1.71-1.88(3H,m), 2.02-2.07(1H,m),3.22(2H,q,J=6.8Hz),3.53(2H,br), 3.69 (2H,d,J=5.9Hz),3.81-3.86(2H,m),4.24(1H,br),5.01(1H,s),5.14 (2H,s),6.51(1H,s),6.57-6.63(1H,m),6.74(1H,d, J=7.8Hz),6.84 (1H,br),6.99-7.05(4H,m),7.33-7.47(7H,m),7.67(1H,s),10.76 (1H,br)ppm
FABMS:646(M+1)

Reference Example 257

3-Aminobutyric acid (5.1 g, 50 mmol) was added to a suspension of lithium aluminum hydride (5.7 g, 0.15 mmol) in tetrahydrofuran (100 mL) by cooling with ice. The reaction mixture was stirred and refluxed with heating for 1 day. The reaction mixture was cooled with ice, and water (5 mL), 15% aqueous sodium hydroxide solution (5 mL) and water (10 mL) were added to the solution in this order. The reaction solution was dried with potassium carbonate, filtered and concentrated in vacuum to obtain 3-amino-1-butanol (4.23 g, 94.9%) as a colorless oil.
$^1$H-NMR($CDCl_3$)δ:1.15(3H,d,J=6.3Hz)1.43-1.68(2H,m), 3.10-3.17 (1H,m),3.68-3.86(2H,m)ppm
FABMS:161(M+1)

Reference Example 258

A toluene solution (20 mL) of the compound (4.23 g, 47.5 mmol) in Reference Example 257 and phthalic anhydride (8.44 g, 57.0 mmol) was stirred and refluxed with heating for 17 hours. The reaction mixture was concentrated in vacuum, and the residue obtained was purified by silica gel column chromatography (hexane : ethyl acetate / 3:2) to obtain 2-(3-hydroxy-1-methylpropyl)-isoindole-1,3-dione (2.29 g, 22.1%) as a colorless solid.
$^1$H-NMR($CDCl_3$)δ:1.55(3H,d,J=6.8Hz),1.80(1H,br),1.92-2.04 (1H,m),2.20-2.33(1H,m),3.56-3.68(1H,m),7.70-7.74(2H,m), 7.81-7.84 (2H,m)ppm
FABMS:220(M+1)

Reference Example 259

An aqueous solution (750 mL) of 2-fluoronitrobenzene (127 g, 0.90 mol), D-aspartic acid (100 g, 0.75 mol) and sodium hydrogen carbonate (252 g, 2.99 mol) was stirred for 3 days by heating at 90˚C. The reaction mixture was cooled to room temperature, and washed with ethyl acetate. The aqueous layer was acidified by adding conc. hydrochloric acid (ca. 300 mL), and the reaction product was extracted with ethyl acetate. The organic layer was washed with 0.5 N aqueous hydrochloric acid and saturated saline solution, dried over anhydrous magnesium sulfate and concentrated in vacuum. The crude product obtained was used for the next reaction without further purification.

The crude product and acetic anhydride (500 mL) were stirred at room temperature for 15 hours. Ether was added to the reaction mixture, the mixture was stirred, and precipitated solids were filtered. The solid was thoroughly washed with ether to obtain
(3S)-3-(2-nitroanilino)dihydro-2,5-furandione (129.1 g, 72.9%) as an yellow solid. The filtrate was concentrated in vacuum with a bath temperature of 30 ˚C or less. Ether was poured into the residue obtained, the mixture was stirred, and the solid was thoroughly washed with ether to obtain (3S)-3-(2-nitroanilino)-dihydro-2,5-fulandione (28.5 g, 16.1%).
$^1$H-NMR ($CDCl_3$) δ:3.07 (1H, dd, J=7.3, 18.1Hz), 3.47 (1H,dd,J=9.8,18.1Hz),5.43-5.53(1H,m),6.80-6.86(1H,m),7.13 (1H, d,J=8.3Hz),7.59-7.65(1H,m),8.11(1H,dd,J=8.3,2.0Hz), 8.47(1H,d,J=9.3Hz)ppm
FABMS:236(M+1)

Reference Example 260

(3R)-3-(2-Nitroanilzno)dihydro-2,5-furnedione was obtained by the same procedure in Reference Example 259 using 2-fluoronitrobenzene and L-aspartic acid. (yield 78.1%)
$^1$H-NMR($CDCl_3$) δ:3.07(1H,dd,J=7.3,18.1Hz),3.47 (1H,dd,J=9.8,18.1Hz),5.43-5.53(1H,m),6.80-6.86(1H,m),7.13 (1H,d, J=8.3Hz),7.59-7.65(1H,m),8.11(1H,dd,J=8.3,2.0Hz), 8.47(1H,d,J=9.3Hz)ppm
FABMS:236(M+1)

Reference Example 261

Sodium borohydride (27.3 g, 0.722 mol) was added to a tetrahydrofuran solution (2.5 L) of the compound (160.5 g, 0.68 mol) in Reference Example 259 while the reaction temperature was kept below -5˚C. After stirring the reaction mixture at that temperature for 2 hours, the temperature was raised to room temperature followed by stirring for additional 2 hours . After completing the reaction, 6N aqueous hydrochloric acid was added until the reaction solution became acidic, and was concentrated in vacuum. After removing tetrahydrofuran by evaporation, the residue was poured into saturated saline solution, and the solution was washed with ethyl acetate. The organic layer was washed with saturated saline solution, dried over anhydrous magnesium sulfate and concentrated in vacuum. The crude product obtained was used for the next reaction without further purification.

A benzene solution (500 mL) of the crude product was stirred and refluxed with heating for 5 hours in a nitrogen stream. The reaction solution was filtered through a cotton plug while the solution was still hot, and the filtrate was concentrated in vacuum. The residue obtained was recrystallized from ethyl acetate to obtain (45)-4-(2-nitroanilino) dihydro-2(3H)-furanone. The filtrate was concentrated in vacuum. A tar-like substance remained on the cotton plug after filtration was dissolved in chloroform together with the residue of the filtrate after recrystallization. The chloroform solution was washed with saturated aqueous sodium hydrogen carbonate solution and saturated saline solution, dried over anhydrous magnesium sulfate and concentrated in vacuum. The residue obtained was recrystallized from ethyl acetate to obtain (4S)-4-(2-nitroanilino)dihydro-2(3H)-furanone as yellow crystalline granules. (93.98 g, 62.2%)
$^1$H-NMR($CDCl_3$)δ:2.64(1H,dd,J=4.2,27.6Hz),3.06 (1H,dd,J=7.3,17.6Hz),4.35(1H,dd,J=3.9,9.52Hz),4.70 (1H,dd, J=6.6,9.52Hz),6.73-6.88(2H,m),7.29-7.55(1H,m),8.14 (1H,br),8.24(1H,dd,J=1.7,8.6Hz)ppm
FABMS:223(M+1)

Reference Example 262

(4S)-4-(2-Nitroanilino)dihydro-2(3H)-furanone was obtained by the same procedure in Reference Example 261 using the compound in Reference Example 260. (yield 88.8%)
$^1$H-NMR($CDCl_3$)δ:2.64(1H,dd,J=4.2,17.6Hz),3.06 (1H, dd, J=7.3, 17.6 Hz), 4.35 (1H, dd, J=3.9, 9.52 Hz), 4.70 (1H,dd, J=6.6,9.52Hz),6.73-6.88(2H,m),7.29-7.55(1H,m),8.14 (1H, br), 8.24 (1H, dd, J=1.7, 8.6 Hz) ppm
FABMS:223(M+1)

Reference Example 263

Palladium-carbon (10%, 30 g) was added to a methanol solution (1.4 L) of the compound (93.98 g, 0.423 mol) in Reference Example 261 for catalytic reduction of the compound at room temperature in a hydrogen stream. After stirring for 19 hours, the catalyst was filtered off, and the filtrate was concentrated in vacuum. The crude product obtained was used for the next reaction without further purification.

A toluene solution (500 mL) of the crude product was stirred and refluxed with heating for 21 hours in a nitrogen stream. After completing the reaction, the reaction solution was stirred by cooling with ice. Crystals were filtered after confirming that the crystals were thoroughly precipitated. The crystals were washed with cooled ethyl acetate to obtain (4R)-4-(hydroxymethyl)-1,3,4,5-tetrahydro-2H-1,5-benzodiazepin-2-one (69.39 g, 85.3%) as a pale yellow solid.
$^1$H-NMR($CDCl_3$) $\delta$:2.25(2H, m), 3.34-3.40(3H, m), 3.44-3.66(1H, m), 4.91 (1H, br), 5.15 (1H, br), 6. 69-6.77 (2H, m), 6.83-6.89 (3H, m), 9.45 (1H, br) ppm
FABMS : 193 (M+1)

Reference Example 264

(4S)-4-(Hydroxymethyl)-1,3,4,5-tetrahydro-2H-1,5-benzodiazepin-2-one was obtained by the same procedure in Reference Example 263 using the compound in Reference Example 262. (yield 90.9%)
$^1$H-NMR($CDCl_3$)$\delta$:2.25(2H,m),3.34-3.40(3H,m),3.44-3.66(1H,m), 4.91(1H,br),5.15(1H,br),6.69-6.77(1H,m),6.83-6.89 (3H,m), 9.45(1H,br)ppm
FABMS:193 (M+1)

Reference Example 265-A

A tetrahydrofuran solution (150 mL) of mesyl chloride (34 mL, 0.22 mol) was dropped into a tetrahydrofuran solution (2.0 L) of the compound (76.88 g, 0.4 mol) in Reference Example 263, triethylamine (67.4 L, 0.48 mol) and 4-dimethylamino pyridine (24.4 g, 0.2 mol) for 3 hours at room temperature in a nitrogen stream. After stirring for 2 hours, the reaction solution was concentrated in vacuum, the residue was added to saturated saline solution, and the reaction product was extracted with ethyl acetate. The organic layer was washed with saturated saline solution, dried over anhydrous magnesium sulfate and concentrated in vacuum. The crude product obtained was used for the next reaction without further purification.

After adding a tetrahydrofuran solution (500 mL) of the crude product to 1M Super Hydride (R) (1.6 L, 1.6 mol) by cooling with ice in a nitrogen stream, the solution was warmed to room temperature and was stirred for 13 hours. Lithium aluminum hydride (45.6 g, 1.2 mol) was added to the reaction solution by cooling with ice, and the solution was warmed to room temperature with stirring for 24 hours. After completing the reaction, water (45 ml), 15% aqueous sodium hydroxide solution (45 ml) and water (90 ml) were added in this order to the solution followed by adding potassium carbonate with stirring. The residue of lithium aluminum hydride was filtered off, and the filtrate was concentrated in vacuum. The residue obtained was purified by silica gel column chromatography (hexane : methyl acetate / 9:1) to obtain (2S)-2-methyl-2,3,4,5-tetrahydro-1H-1,5-benzodiazepine (51.72 g, 79.7%) as a pale brown solid.
$^1$H-NMR($CDCl_3$) $\delta$ : 1.30(3H,d, J=6.35Hz),1.52-1.66(1H,m), 1.77-1.86(1H,m),2.70-2.80(1H,m),2.91-3.02(1H,m), 3.32-3.41 (1H,m),3.63(1H,br),6.69-6.78(4H,m)ppm
FABMS:163(M+1)

Reference Example 265-B

A tetrahydrofuran solution (3 mL) of mesyl chloride (257 mg, 2.2 mmol) was dropped into a tetrahydrofuran solution (10 mL) of the compound (384 mg, 2 mmol) in Reference Example 263, triethylamine (0.336mL, 2.4 mmol) and 4-dimethylaminopyridine (124 mg, 1 mmol) for 2 hours at room temperature in a nitrogen stream. After stirring for 2 hours, the reaction solution was filtered, and the filtrate was concentrated in vacuum. The crude product obtained was used for the next reaction without further purification.

A 0.5 M solution of sodium borohydride in 2-methoxy ethyl ether was added to the crude product and molecular sieve 3A (205 mg) by cooling with ice in a nitrogen stream, and the solution was warmed to room temperature with stirring for 30 minutes followed by warming to 50˚C with stirring for 30 minutes. Chlorotrimethylsilane (0.254 mL, 1.2 mmol) was added to the reaction solution at 50˚C, and the mixed solution was stirred for 1 hour. After completing the reaction, the reaction solution was acidified by adding 2N aqueous hydrochloric acid at room temperature. After stirring for 15 minutes, the reaction solution was made basic by adding 4N aqueous sodium hydroxide solution, and was filtered through celite. The filtrate was extracted with ethyl acetate, and the organic layer was washed with saturated saline solution, dried over anhydrous magnesium sulfate and concentrated in vacuum. The residue obtained was purified by

silica gel column chromatography (hexane : ethyl acetate / 2:1) to obtain (2S)-2-methyl-2,3,4,5-tetrahydro-1H-1,5-benzodiazepine (233 mg, 71.8%) as a pale brown solid.
$^1$H-NMR($CDCl_3$)δ:1.30(3H,d,J=6.35Hz) 1.52-1.66(1H,m), 1.77-1.86(1H,m),2.70-2.80(1H,m),2.91-3.02(1H,m), 3.32-3.41 (1H,m),3.63(1H,br),6.69-6.78(4H,m)ppm
FABMS:163(M+1)

Reference Example 266

(2R)-2-Methyl-2,3,4,5-tetrahydro-1H-1,5-benzodiazepine was obtained by the same procedure in Reference Example 265-A using the compound in Reference Example 264.
(yield 65%)
$^1$H-NMR($CDCl_3$)δ:1.30(3H,d,J=6.35),1.52-1.66(1H,m) 1.77-1.86 (1H,m), 2.70-2.80 (1H,m), 2.91-3.02 (1H,m), 3.32-3.41 (1H,m), 3.63 (1H,br), 6.69-6.78 (4H,m) ppm
FABMS:163(M+1)

Example 1

A mixed solution of the compound (0.3 g, 0.80 mmol) in Reference Example 117, the compound (0.13 g, 0.81mmol) in Reference Example 70, tetrakis(triphenylphosphine)palladium(0) (28 mg, 0.024 mmol) and sodium hydrogen carbonate (0.2 g, 2.38 mmol) in ethylene glycol dimethyl ether (6 mL) and water (6 mL) was heated with stirring in a nitrogen atmosphere at 100˚C for 3 hours. The solvent was concentrated in vacuum, acidified with an aqueous 0.5N citric acid solution, and then extracted with ethyl acetate. The organic layer was washed with an aqueous sodium hydrogen carbonate solution and successively a saturated saline solution, dried over anhydrous magnesium sulfate and concentrated in vacuum. The residue was purified by silica gel column chromatography (hexane:ethyl acetate = 20:5 to 18:7) to obtain 1-[4-(1H-indol-5-yl)-3-methoxybenzoyl]-4-methyl-2,3,4,5-tetrahydro-1H-1,5-benzodiazepine (0.255 g, 77.5%) as a colorless solid.

Example 2

1-[4-(1H-indol-5-yl)-3-methoxybenzoyl]-4,4-dimethyl-2,3,4,5-tetrahydro-1H-1,5-benzodiazepine was obtained by the same procedure as in Example 1 using the compounds in Reference Example 118 and Reference Example 70 (yield: 85.5%).

Example 3

To a solution of the compound (163 mg, 0.41 mmol) in Example 175 and pyridine (36 mg, 0.451 mmol) in tetrahydrofuran (4 mL), , a solution of acetyl chloride (35 mg, 0.451 mmol) in tetrahydrofuran (2 mL) was added dropwise in a nitrogen gas flow under ice cooling. After stirring for 0.5 hours, the reaction mixture was poured into a saturated sodium hydrogen carbonate solution and then extracted with ethyl acetate. The organic layer was washed with a saturated saline solution, dried over anhydrous magnesium sulfate and then concentrated in vacuum. The residue obtained was purified by silica gel chromatography (hexane:ethyl acetate = 1:4) to obtain
N-[2-(1H-indol-5-yl)-5-[(4-methyl-2,3,4,5-tetrahydro-1H-1,5-benzodiazepin-1-yl)carbonyl]phenyl]acetamide (88 mg, 49.0%) as a colorless solid.

Example 4

(4R)-1-[4-(2H-indol-5-yl)benzoyl]-4-methyl-2,3,4,5-tetrahydro-1H-1,5-benzodiazepine was obtained by the same procedure as in Example 1 using the compounds in Reference Example 121 and Reference Example 70 (yield: 49.2%).

Example 5

(4S)-1-[4-(1H-indol-5-yl)benzoyl]-4-methyl-2,3,4,5-tetrahydro-1H-1,5-benzodiazepine by the same procedure as in Example 1 using the compounds in Reference Example 122 and Reference Example 70 (yield: 65%).

Example 6

To a solution of the compound (100 mg, 0.252 mmol) in Example 175 and pyridine (30 mg, 0.378 mmol) in tetrahydrofuran (2 mL), a solution of methanesulfonyl chloride (32 mL, 0.277 mmol) in tetrahydrofuran (4 mL) was added

dropwise in a nitrogen gas flow under ice cooling. After stirring for 3 hours, the reaction mixture was concentrated in vacuum. The residue obtained was purified by silica gel column chromatography (chloroform:methanol = 95:5) to obtain N-[2-(1H-indol-5-yl)-5-[(4-methyl-2,3,4,5-tetrahydro-1H-1,5-benzodiazepin-1-yl)carbonyl]phenyl]methanesulfonamid e (80 mg, 66.9%) as a colorless solid.

Example 7

N-[2-(1H-indol-5-yl)-5-[(4-isopropyl-2,3,4,5-tetrahydro-1H-1,5-benzodiazepin-1-yl)carbonyl]phenyl]acetamide was obtained by the same procedure as in Example 3 using the compound in Example 176 (yield: 59.5%).

Example 8

N-[5-[(4-ethyl-2,3,4,5-tetrahydro-1H-1,5-benzodiazepin-1-yl)carbonyl]-2-(1H-indol-5-yl)-phenyl] acetamide was obtained by the same procedure as in Example 3 using the compound in Example 177 (yield: 37.7%).

Example 9

N-[2-(1H-indol-5-yl)-5-[(4-methyl-2,3,4,5-tetrahydro-1H-1,5-benzodiazepin-1-yl)carbonyl]phenyl]-N-[2-(4-methyl-1-piperazinyl)-2-oxoethyl]amine was obtained by the same procedure as in Example 1 using the compounds in Reference Example 128 and Reference Example 70 (yield: 49.5%).

Example 10

To a solution of the compound (198mg, 0.5 mmol) in Example 179 and pyridine (44 mg, 0.55 mmol) in tetrahydrofuran (4 mL), a solution of chloroacetyl chloride (57 mg, 0.5 mmol) in tetrahydrofuran (4 mL) was added dropwise in a nitrogen gas flow under ice cooling. After stirring for 4 hours, the reaction mixture was concentrated in vacuum. A solution of the crude product obtained, N-methylpiperazine (50 mg, 0.5 mmol) and potassium carbonate (69 mg, 0.5 mmol) in dimethylformamide (10 mL) was stirred in a nitrogen gas flow at 60˚C for 7.5 hours. After the completion of the reaction, the reaction mixture was concentrated in vacuum. The residue obtained was extracted with ethyl acetate and a saturated saline solution added. The organic layer was washed with a saturated saline solution, dried over anhydrous magnesium sulfate and then concentrated in vacuum. The residue obtained was purified by silica gel column chromatography (5-15% methanol in chloroform) to obtain N-[2-(1H-indol-5-yl)-5-[(4-methyl-2,3,4,5-tetrahydro-1H-1,5-benzodiazepin-1-yl)carbonyl]phenyl]-2-(4-methyl-1-piperazinyl)acetamide (163 mg, 60.8%) as a colorless solid.

Example 11

2-(1H-indol-5-yl)-N-methyl-5-[(4-methyl-2,3,4,5-tetrahydro-1H-1,5-benzodiazepin-1-yl)carbonyl]aniline was obtained by the same procedure as in Example 1 using the compounds in Reference Example 129 and Reference Example 70 (yield: 43.8%).

Example 12

The compound (203 mg, 0.5 mmol) in Reference Example 131 and nickel chloride hexahydrate (238 mg, 1.0 mmol) were added to methanol (20 mL), followed by stirring. Under ice cooling, sodium borohydride (189 mg, 5.0 mmol) was gradually added so as not to increase the reaction temperature. After stirring at room temperature for 30 minutes, to the solution, an aqueous 4N hydrochloric acid solution was added, followed by concentration in vacuum. The solution was washed with ether and then basified with an aqueous 4N sodium hydroxide solution. The aqueous solution was extracted with ethyl acetate. The organic layer was washed with a saturated saline solution, dried over anhydrous magnesium sulfate and then concentrated in vacuum. The residue obtained was purified by silica gel column chromatography (chloroform:methanol = 95:5 to 90:10) to obtain [2-(1H-indol-5-yl)-5-[(4-methyl-2,3,4,5-tetrahydro-1H-1,5-benzodiazepin-1-yl)carbonyl]phenyl]methanamine (123 mg, 59.9%) as a colorless solid.

Example 13

To a solution of the compound (66 mg, 0.17 mmol) in Example 179 and pyridine (17 mg, 0.21 mmol) in tetrahydrofuran (10 mL), a solution of methyl chloroformate (17 mg, 0.18 mmol) in tetrahydrofuran (10 mL) was gradually

added dropwise under ice cooling. After stirring at room temperature for 3 hours, the reaction solution was poured into water and then extracted with ethyl acetate. The organic layer was washed with water, dried over anhydrous sodium sulfate and then concentrated in vacuum. The residue obtained was purified by PTLC (chloroform:methanol = 98:2) to obtain methyl 2-(1H-indol-5-yl)-5-[(4-methyl-2,3,4,5-tetrahydro-1H-1,5-benzodiazepin-1-yl)carbonyl]phenylcarbamate (62 mg, 81.9%) as a pale yellow amorphous product.

Example 14

To a solution of the compound (0.215 g, 0.42 mmol) in Reference Example 135 in ethanol (13 mL), 10% palladium-carbon (0.22g) was added in a nitrogen gas flow under ice cooling. After refluxing in a hydrogen gas flow for 4.5 hours, the reaction solution was filtered through a filter (0.22 μm, Fuji Film FM-22) and palladium-carbon was washed with methanol, and then the filtrate and the wash were combined and concentrated in vacuum. The residue obtained was purified by column chromatography (chloroform:methanol = 248:2 to 247: 3) to obtain 2-(1H-indol-5-yl)-5-[(4-methyl-2,3,4,5-tetrahydro-1H-1,5-benzodiazepin-1-yl)carbonyl]phenylformamide (87 mg, 49.1%) as a colorless amorphous product.

Example 15

N-[2-(1H-indol-5-yl)-5-[(4-methyl-2,3,4,5-tetrahydro-1H-1,5-benzodiazepin-1-yl)carbonyl]phenyl]urea was obtained by the same procedure as in Example 14 using the compound in Reference Example 136 (yield: 71.0%).

Example 16

N-[2-(1H-indol-5-yl)-5-[[(4R)-4-methyl-2,3,4,5-tetrahydro-1H-1,5-benzodiazepin-1-yl]carbonyl]phenyl] acetamide was obtained by the same procedure as in Example 3 using the compound in Example 178 (yield: 37.1%).

Example 17

N-[2-(1H-indol-5-yl)-5-[[(4S)-4-methyl-2,3,4,5-tetrahydro-1H-1,5-benzodiazepin-1-yl]carbonyl]phenyl] acetamide was obtained by the same procedure as in Example 3 using the compound in Example 179 (yield: 70.2%).

Example 18

To a solution of the compound (0.2 g, 0.29 mmol) in Reference Example 144 in tetrahydrofuran (15 mL), tetrabutylammonium fluoride hydrate (0.15 g) was added at room temperature. The reaction solution was poured into ice water and then extracted with ethyl acetate. The organic layer was washed with water, dried over anhydrous sodium sulfate and then concentrated in vacuum. The residue obtained was purified by silica gel column chromatography (chloroform:methanol = 248:2 to 244:6) to obtain N-[5-[[4-(hydroxymethyl)-2,3,4,5-tetrahydro-1H-1,5-benzodiazepin-l-yl]carbonyl]-2-(1H-indol-5-yl)phenyl] acetamide (0.12 g, 91.0%) as a colorless amorphous product.

Example 19

2-amino-N-[2-(1H-indol-5-yl)-5-[(4-methyl-2,3,4,5-tetrahydro-1H-1,5-benzodiazepin-1-yl)carbonyl] phenyl] acetamide was obtained by the same procedure as in Example 14 using the compound in Reference Example 145 (yield: 27.8%).

Example 20

2-(dimethylamino)-N-[2-(1H-indol-5-yl)-5-[(4-methyl-2,3,4,5-tetrahydro-1H-1,5-benzodiazepin-1-yl) carbonyl] phenyl]acetamide was obtained by the same procedure as in Example 14 using the compound in Reference Example 146 (yield: 11.2%).

Example 21

2-hydroxy-N-[2-(1H-indol-5-yl)-5-[(4-methyl-2,3,4,5-tetrahydro-1H-1,5-benzodiazepin-1-yl)carbonyl] phenyl] acetamide was obtained by the same procedure as in Example 14 using the compound in Reference Example 147 (yield: 53.5%) .

Example 22

N-[5-[[4-[(dimethylamino)methyl]-2,3,4,5-tetrahydro-1H-1,5-benzodiazepin-1-yl]carbonyl]-2-(1H-indol-5-yl) phenyl] acetamide was obtained by the same procedure as in Example 1 using the compounds in Reference Example 148 and Reference Example 70 (yield: 51.9%).

Example 23

[2-(1H-indol-5-yl)-5-[(4-methyl-2,3,4,5-tetrahydro-1H-1,5-benzodiazepin-1-yl)carbonyl]phenyl] methanol was obtained by the same procedure as in Example 1 using the compounds in Reference Example 150 and Reference Example 70 (yield: 64.6%) .

Example 24

A solution of the compound (0.14 g, 0.24 mmol) in Reference Example 154 and hydrazine monohydrate (24 mg, 0.48 mmol) in methanol (13 mL) was stirred at room temperature for 4 hours. After the reaction solution was concentrated in vacuum, chloroform and ethyl acetate were added and insolubles were removed by filtration. The filtrate was concentrated in vacuum and the residue obtained was purified by NH silica gel column chromatography (chloroform: methanol = 97:3) to obtain 2-[2-(1H-indol-5-yl)-5-[(4-methyl-2,3,4,5-tetrahydro-1H-1,5-benzodiazepin-1-yl)carbonyl] phenoxy]ethaneamine (81 mg, 76.0%) as a white amorphous product.

Example 25

N-[5-[(8-amino-4-methyl-2,3,4,5-tetrahydro-1H-1,5-benzodiazepin-1-yl)carbonyl]-2-(1H-indol-5-yl) phenyl] acetamide was obtained by the same procedure as in Example 14 using the compound in Reference Example 160 (yield: 57.6%).

Example 26

N-[5-[(4,7-dimethyl-2,3,4,5-tetrahydro-1H-1,5-benzodiazepin-1-yl)carbonyl]-2-(1H-indol-5-yl)phenyl] acetamide was obtained by the same procedure as in Example 1 using the compounds in Reference Example 161 and Reference Example 70 (yield: 70.8%).

Example 27

N-[5-[(7-hydroxy-4-methyl-2,3,4,5-tetrahydro-1H-1,5-benzodiazepin-l-yl)carbonyl]-2-(1H-indol-5-yl)phenyl] acetamide was obtained by the same procedure as in Example 14 using the compound in Reference Example 163 (yield: 57.6%).

Example 28

To a solution of the compound (1.03 g, 2.60 mmol) in Example 175 and pyridine (0.27 g, 3.39 mmol) in tetrahydrofuran (200 mL), a solution of propionyl chloride (0.2 g, 2.86 mmol) in tetrahydrofuran (30 mL) was gradually added dropwise under ice cooling. After stirring at room temperature for 2 hours, the reaction solution was poured into water and then extracted with ethyl acetate . The organic layer was washed with water, dried over anhydrous sodium sulfate and then concentrated in vacuum. The residue obtained was purified by silica gel column chromatography (chloroform:methanol = 249:1) to obtain
N-[2-(1H-indol-5-yl)-5-[(4-methyl-2,3,4,5-tetrahydro-1H-1,5-benzodiazepin-1-yl)carbonyl]phenyl]propaneamide (0.83 g, 70.3%) as a colorless solid. Reference Example 29

A solution of benzyloxyacetic acid (0.14 g, 0.82 mmol) in thionyl chloride (0.5 mL) was refluxed for 3 hours. The reaction solution was concentrated in vacuum and then azeotroped with toluene. The resulting concentrated residue was dissolved in tetrahydrofuran (10 mL). The solution was gradually added dropwise to a solution of the compound (0.34 g, 0.68 mmol) in Example 177 and pyridine (70 mg, 0.89 mmol) in tetrahydrofuran (30 mL) under ice cooling. After stirring at room temperature for 3 hours, the reaction solution was poured into water and then extracted with ethyl acetate. The organic layer was washed with water, dried over anhydrous sodium sulfate and then concentrated in vacuum to obtain a crude product.

To a solution of crude crystals of the compound synthesized previously in ethanol (20 mL), 10% palladium-carbon (0.9 g) was added, followed by refluxing in a hydrogen gas flow for 16 hours. The catalyst was removed by

filtration and washed with methanol, and then the filtrate and the wash were combined and concentrated in vacuum. The residue obtained was purified by silica gel column chromatography (chloroform:methanol =245: 5) to obtain N-[5-[(4-ethyl-2,3,4,5-tetrahydro-1H-1,5-benzodiazepin-1-yl)carbonyl]-2-(1H-indol-5-yl)phenyl-2-hydroxyacetamide (0.14 g, 44.8%) as a colorless solid.

Example 30

3-[2-(1H-indol-5-yl)-5-[(4-methyl-2,3,4,5-tetrahydro-1H-1,5-benzodiazepin-1-yl)carbonyl]phenoxy]-1-propaneamine was obtained by the same procedure as in Example 24 using the compound in Reference Example 174 (yield: 65.3%).

Example 31

To a solution of the compound (0.15 g, 0.38 mmol) in Example 175 and pyridine (45 mg, 0.57 mmol) in tetrahydrofuran (20 mL), a solution of methoxyacetylchloride (45 mg, 0.57 mmol) in tetrahydrofuran (5 mL) was gradually added dropwise under ice cooling. After stirring at room temperature for 2 hours, the reaction solution was poured into water and then extracted with ethyl acetate. The organic layer was washed with water, dried over anhydrous sodium sulfate and then concentrated in vacuum. The residue obtained was purified by silica gel column chromatography (chloroform:methanol = 249:1) to obtain N-[2-(1H-indol-5-yl)-5-[(4-methyl-2,3,4,5-tetrahydro-1H-1,5-benzodiazepin-1-yl)carbonyl]phenyl]-2-methoxyacetamide (0.135 g, 75.8%) as a pale yellow oil.

Example 32

To a solution of 3-methoxypropionic acid (41 mg, 0.38 mmol) in tetrahydrofuran (3mL), thionyl chloride (0.11 g, 0.95 mmol) and one drop of N,N-dimethylformamide were added, followed by stirring at room temperature for 3 hours. The reaction solution was concentrated in vacuum and then azeotroped with toluene. The resulting concentrated residue was dissolved in tetrahydrofuran (10 mL). The solution was gradually added dropwise to a solution of the compound (0.15 g, 0.38 mmol) in Reference Example 126 and pyridine (45 mg, 0.57 mmol) in tetrahydrofuran (25 mL) under ice cooling. After stirring at room temperature for 3 hours, the reaction solution was poured into water and then extracted with ethyl acetate. The organic layer was washed with water, dried over anhydrous sodium sulfate and then concentrated in vacuum. The residue obtained was purified by silica gel column chromatography (chloroform:methanol = 248:2) to obtain N-[2-(1H-indol-5-yl)-5-[(4-methyl-2,3,4,5-tetrahydro-1H-1,5-benzodiazepin-1-yl)carbonyl]phenyl]-3-methoxypropaneamide (0.11 g, 60.0%) as a colorless solid.

Example 33

To a solution of the compound (199 mg, 0.5 mmol) in Example 182, ethylene glycol monomethyl ether (76 mg, 1.0 mmol) and triphenylphosphine (262 mg, 1.0 mmol) in tetrahydrofuran (4 mL), azodicarboxylic acid diethyl ester (40% toluene solution) (0.43 mL, 1.0 mmol) was added in a nitrogen gas flow at room temperature, followed by stirring for 96 hours. After the completion of the reaction, the reaction solution was concentrated in vacuum and the residue was dissolved in ethyl acetate. The solution was poured into a saturated saline solution and then extracted. The organic layer was washed with a saturated saline solution, dried over anhydrous magnesium sulfate and then concentrated in vacuum. The residue obtained was purified by silica gel column chromatography (hexane:ethyl acetate = 1:4) to obtain 1-[4-(1H-indol-5-yl)-3-(2-methoxyethoxy)benzoyl]-4-methyl-2,3,4,5-tetrahydro-1H-1,5-benzodiazepine (100 mg, 43.9%) as a colorless solid.

Example 34

To a solution of the compound (199 mg, 0.5 mmol) in Example 182, 2-benzyloxyethanol (76 mg, 0.5 mmol) and triphenylphosphine (131 mg, 0.5 mmol) in tetrahydrofuran (2 mL), azodicarboxylic acid diethyl ester (40% toluene solution) (0.218 mL, 1.0 mmol) was added in a nitrogen gas flow at room temperature, followed by stirring for 16 hours. After the completion of the reaction, the reaction solution was concentrated in vacuum and the residue was dissolved in ethyl acetate. The solution was poured into a saturated saline solution and then extracted. The organic layer was washed with a saturated saline solution, dried over anhydrous magnesium sulfate and then concentrated in vacuum.

The crude product obtained was used for the following reaction without being purified. A solution of the crude product in methanol (10 mL) was stirred under heating at reflux in the presence of 10% palladium-carbon (320 mg) in a hydrogen gas flow for 2 hours. After the catalyst was removed by filtration, the solvent was concentrated in vacuum.

The residue obtained was purified by silica gel column chromatography (hexane:ethyl acetate = 1:4 to 1:9) to obtain 2-[2-(1H-indol-5-yl)-5-[(4-methyl-2,3,4,5-tetrahydro-1H-1,5-benzodiazepin-1-yl)carbonyl]phenoxy]ethanol (156 mg, 70.7%) as a colorless solid.

Example 35

N-[2-[2-(1H-indol-5-yl)-5-[(4-methyl-2,3,4,5-tetrahydro-1H-1,5-benzodiazepin-1-yl)carbonyl]phenoxy] ethyl ] acetamide was obtained by the same procedure as in Example 33 using the compound in Example 182 and N-(2-hydroxyethyl)acetamide (yield: 37.8%).

Example 36

4-[2-(1H-indol-5-yl)-5-[(4-methyl-2,3,4,5-tetrahydro-1H-1,5-benzodiazepin-1-yl)carbonyl]phenoxy]-1-butaneamine was obtained by the same procedure as in Example 24 using the compound in Reference Example 175 (yield: 78.0%).

Example 37

5-[2-(1H-indol-5-yl)-5-[(4-methyl-2,3,4,5-tetrahydro-1H-2,5-benzodiazepin-1-yl)carbonyl]phenoxy]-1-pentaneamine was obtained using the compound in Reference Example 176 (yield: 82.7%).

Example 38

A solution of the compound (289 mg, 0.727 mmol) in Example 180, N-3-bromopropylphthalimide (205 mg, 0.763 mmol) and potassium carbonate (151 mg, 1.09 mmol) in N,N-dimethylformamide (10 mL) was stirred day-and-night under heating at 50˚C in a nitrogen gas flow. The reaction mixture was poured into an aqueous sodium hydrogen carbonate solution and then extracted with ethyl acetate. The organic layer was washed with a saturated saline solution, dried over anhydrous magnesium sulfate and then concentrated in vacuum. The crude product obtained was used for the following reaction without being purified.

To a solution of the crude product in methanol (10 mL), hydrazine hydrate (1.0 mL) was added, followed by stirring at room temperature for 2 hours. The reaction mixture was concentrated in vacuum and the residue obtained was dissolved in ethyl acetate, and then the solution was poured into a saturated saline solution. After extracting with ethyl acetate, the organic layer was washed with a saturated saline solution, dried over anhydrous magnesium sulfate and then concentrated in vacuum. The residue obtained was purified by NH-silica gel column chromatography (0-3% methanol-containing chloroform) to obtain
3-[2-(1H-indol-5-yl)-5-[[(4S)-4-methyl-2,3,4,5-tetrahydro-1H-1,5-benzodiazepin-1-yl]carbonyl]phenoxy] propylamine (274 mg, 82.4%) as a colorless solid.

Example 39

6-[2-(1H-indol-5-yl)-5-[(4-methyl-2,3,4,5-tetrahydro-1H-1,5-benzodiazepin-1-yl)carbonyl]phenoxy]hexylamine was obtained by the same procedure as in Example 24 using the compound in Reference Example 177 (yield: 77.3%).

Example 40

3-[5-[(4-ethyl-2,3,4,5-tetrahydro-1H-1,5-benzodiazepin-1-yl)carbonyl]-2-(1H-indol-5-yl)phenoxy]-1-propaneamine was obtained by the same procedure as in Example 38 using the compound in Example 183 (yield: 95.4%).

Example 41

Using the compounds of Example 182 and Reference Example 88, a compound was obtained by the same procedure as in Example 33. Using the resulting compound, 3-[2-(1H-indol-5-yl)-5-[(4-methyl-213,4,5-tetrahydro-1H-1,5-benzodiazepin-1-yl)carbonyl]phenoxyl-N-methyl-1-propaneamine was obtained by the same procedure as in Example 14 (yield: 44.4%).

Example 42

3-[2-(1H-indol-5-yl)-5-[(4-methy-2,3,4,5-tetrahydro-1H-1,5-benzodiazepin-1-yl)carbonyl]phenoxy]-N,N-dimethyl-1-propaneamine was obtained by the same procedure as in Example 33 using the compound in Example 182 and 3-dimethylamino-1-propanol (yield: 51.2%).

Example 43

To a solution of the compound (0.36 g, 0.91 mmol) in Example 182, compound (0.41 g, 1.81 mmol) in Reference Example 92 and triphenylphosphine (0.47 g, 1.81 mmol) in tetrahydrofuran (10 mL), azodicarboxylic acid diethyl ester (40% toluene solution) (0.8 mL, 1.8 mmol) was gradually added dropwise. After the completion of dropwise addition, stirring was conducted at room temperature for 15 hours. The reaction solution was concentrated in vacuum and the residue obtained was purified by silica gel column chromatography (benzene:ethyl acetate= 4: 1 to 3:2), and the resulting compound was heated by a hot jetter while evacuating for 10 minutes. The resulting reaction product was purified by NH-silica gel column chromatography (chloroform:methanol=248:2 to 246:4) to obtain 3-(1H-imidazol-5-yl)propyl 2-(1H-indol-5-yl)-5-[(4-methyl-2,3,4,5-tetrahydro-1H-2,5-benzodiazepin-1-yl)carbonyl]phenyl ether (0.19 g, 96.4%) as a pale yellow oil.

Example 44

N1-[2-(1H-indol-5-yl)-5-[(4-methyl-2,3,4,5-tetrahydro-1H-1,5-benzodiazepin-1-yl)carbonyl]phenyl]-1,3-propanediamine was obtained by the same procedure as in Example 24 using the compound in Reference Example 178 (yield: 49.6%).

Example 45

1-[4-(1H-indol-5-yl)-3-propoxybenzoyl]-4-methyl-2,3,4,5-tetrahydro-1H-1,5-benzodiazepine was obtained by the same procedure as in Example 33 using the compound in Example 182 and n-propanol (yield: 36.4%).

Example 46

2-(1H-indol-5-yl)-5-[(4-methyl-2,3,4,5-tetrahydro-1H-1,5-benzodiazepin-1-yl)carbonyl]phenyl 2- (4-morpholinyl) ethyl ether was obtained by the same procedure as in Example 33 using the compound in Example 182 and 2-(4-morpholinyl)ethanol (yield: 90.2%).

Example 47

(4S)-1-[4-(1H-indol-5-yl)-3-methoxybenzoyl]-4-methyl-2, 3, 4, 5-tetrahydro-1H-1, 5-benzodiazepine was obtained by the same procedure as in Example 1 using the compounds in Reference Example 179 and Reference Example 70 (yield: 61%).

Example 48

N-[2-(1H-indol-5-yl)-5-[[(4S)-4-methyl-2,3,4,5-tetrahydro-1H-1,5-benzodiazepi-n-1-yl]carbonyl]phenyl] methanesulfonamide was obtained by the same procedure as in Example 6 using the compound in Example 179 (yield: 47.1%).

Example 49

N-[2-(1H-indol-5-yl)-5-[[(4S)-4-methyl-2,3,4,5-tetrahydro-1H-1,5-benzodiazepin-1-yl] carbonyl] phenyl]-N-methylacetamide was obtained by the same procedure as in Example 1 using the compounds in Reference Example 180 and Reference Example 70 (yield: 87.5%).

Example 50

2'-methoxy-4'-[[(4S)-4-methyl-2,3,4,5-tetrahydro-1H-1,5-benzodiazepin-1-yl]carbonyl][1,1'-biphenyl]4-amine was obtained by the same procedure as in Example 14 using the compound in Reference Example 182 (yield: 51.3%).

Example 51

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N-methylamine was obtained by the same procedure as in Example 14 using the compound in Example 180

(yield: 90.8%).

Example 52

To a solution of the compound (397 mg, 1.0 mmol) in Example 180, the compound (500 mg, 2.0 mmol) in Reference Example 93 and triphenylphosphine (525 mg, 2.0 mmol) in tetrahydrofuran (10 mL), azodicarboxylic acid diethyl ester (40% toluene solution) (0.91 mL, 2.0 mmol) was added in a nitrogen gas flow at room temperature, followed by stirring for 17 hours. After the completion of the reaction, the reaction solution was concentrated in vacuum and the residue was dissolved in ethyl acetate. The solution was poured into a saturated saline solution and then extracted. The organic layer was washed with a saturated saline solution, dried over anhydrous magnesium sulfate and then concentrated in vacuum. The residue obtained was used for the following reaction without being purified.

A solution of the crude product in methanol (10 mL) was catalytically reduced in the presence of a 10% palladium-carbon (800 mg) in a hydrogen gas flow at room temperature. After stirring for 16 hours, the catalyst was removed by filtration and the reaction solution was concentrated in vacuum. The residue obtained was purified by NH-silica gel column chromatography (chloroform:methanol = 100:0 to 98:2) to obtain (4S)-1-[4-(1H-indol-5-yl)-3-(3-piperidinylmethoxy)benzoyl]-4-methyl-2,3,4,5-tetrahydro-1H-1,5-benzodiazepine (366 mg, 74%) as a colorless amorphous product.

Example 53

1-[2-[2-(1H-indol-5-yl)-5-[(4-methyl-2,3,4,5-tetrahydro-1H-1,5-benzodiazepin-1-yl)carbonyl]phenoxy ethyl]-2-pyrrolidinone was obtained by the same procedure as in Example 33 using the compound in Example 182 and 1-(2-hydroxyethyl)-2-pyrrolidinone (yield: 80.6%).

Example 54

2'-(3-aminopropoxy)-4'-[[(4S)-4-methyl-2,3,4,5-tetrahydro-1H-1,5-benzodiazepin-l-yl]carbonyl][1,1'-biphenyl]-4-amine was obtained by the same procedure as in Example 14 using the compound in Reference Example 189 (yield: 93.0%).

Example 55

3-[2-(1H-indol-5-yl)-5-[[(4R)-4-methyl-2,3,4,5-tetrahydro-1H-1,5-benzodiazepin-1-yl]carbonyl]phenoxy] propylamine was obtained by the same procedure as in Example 38 using the compound in Example 181 (yield: 48.8%) .

Example 56

1-[3-[2-(1H-indol-5-yl)-5-[(4-methyl-2,3,4,5-tetrahydro-1H-1,5-benzodiazepin-1-yl)carbonyl]phenoxy] propyl] -2-pyrrolidinone was obtained by the same procedure as in Example 33 using the compound in Example 182 and 1-(3-hydroxypropyl)-2-pyrrolidinone (yield: 59.3%).

Example 57

To a solution of the compound (0.172 g, 0.332 mmol) in Reference Example 190 and ethanolamine (0.1 mL, 1.66 mmol) in acetonitrile (15 mL), triethylamine (0.06 mL, 0.431 mmol) was added in a nitrogen atmosphere, followed by heating at reflux for 21 hours. The reaction mixed solution was concentrated in vacuum and the residue obtained was purified by NH-silica gel chromatography (chloroform:methanol = 80:1) to obtain 2-[[3-[2-(1H-indol-5-yl)-5-[(4-methyl-2,3,4,5-tetrahydro-1H-1,5-benzodsazepin-1-yl)carbonyl]phenoxy] propyl]amino] ethanol (161 mg, 97%) as a pale yellow oil.

Example 58

2-[[3-[2-(1H-indol-5-yl)-5-[(4-methyl-2,3,4,5-tetrahydro-1H-1,5-benzodiazepzn-1-yl)carbonyl]phenoxy] propyljamino]diethanol was obtained by the same procedure as in Example 57 using the compound in Reference Example 190 and diethanolamine (yield: 92%).

Example 59

(4S)-1-[4-(1H-indol-5-yl)-3-[2-(4-morpholinyl)ethoxy] benzoyl]-4-methyl-2,3,4,5-tetrahydro-1H-1,5-benzodiazepine was obtained by the same procedure as in Example 33 using the compound in Example 180 and N-(2-hydroxyethyl)morpholine
(yield: 92%).

Example 60

4'-[[(4S)-4-methyl-2,3,4,5-tetrahydro-1H-1,5-benzodiazepin-1-yl]carbonyl]-2'-[2-(4-morpholinyl) ethoxy][1,1'-biphenyl]-4-amine was obtained by the same procedure as in Example 14 using the compound in Reference Example 195 (yield: 88.5%).

Example 61

(4S)-1-[4-(1H-indol-5-yl)-3-[3-(4-morpholinyl) propoxy]benzoyl]-4-methyl-2,3,4,5-tetrahydro-1H-1,5-benzodiazepine was obtained by the same procedure as in Example 57 using the compound in Reference Example 191 and morpholine
(yield: 22%).

Example 62

2-(1H-indol-5-yl)-5-[(4-methyl-2,3,4,5-tetrahydro-1H-1,5-benzodiazepin-1-yl)carbonyl]phenyl 2- (1-piperidinyl) ethyl ether was obtained by the same procedure as in Example 33 using the compound in Example 182 and N-(2-hydroxyethyl)piperidine (yield: 90.2%).

Example 63

2-(1H-indol-5-yl)-5-[(4-methyl-2,3,4,5-tetrahydro-1H-1,5-benzodiazepin-1-yl)carbonyl]phenyl 3-(4-thiomorpholinyl)propyl ether was obtained by the same procedure as in Example 57 using the compound in Reference Example 190 and thiomorpholine (yield: 40%).

Example 64

Using the compound of Reference Example 190 and 1-piperazine carboxylate, a compound was obtained by the same procedure as in Reference Example 57. Using the resulting compound, 2-(1H-indal-5-yl)-5-[(4-methyl-2,3,4,5-tetrahydro-1H-1,5-benzodiazepin-1-yl)carbonyl]phenyl 3-(1-piperazinyl)propyl ether was obtained by the same procedure as in Example 14 (yield: 35%).

Example 65

2-(1H-indol-5-yl)-5-[(4-methyl-2,3,4,5-tetrahydro-1H-1,5-benzodiazepin-1-yl)carbonyl]phenyl 3-(4-methyl-1-piperazinyl)prapyl ether was obtained by the same procedure as in Example 57 using the compound in Reference Example 190 and N-methylpiperazine (yield: 42%).

Example 66

(4R)-1-[4-(1H-indol-5-yl)-3-[2-(4-morpholinyl)ethoxy] benzoyl]-4-methyl-2,3,4,5-tetrahydro-1H-1,5-benzodiazepine was obtained by the same procedure as in Example 33 using the compound in Example 181 and N-(2-hydroxyethyl)morpholine
(yield: 93%).

Example 67

(4S)-1-[4-(1H-indol-5-yl)-3-[4-(4-morpholinyl)butoxy] benzoyl]-4-methyl-2,3,4,5-tetrahydro-1H-1,5-benzodiazepine was obtained by the same procedure as in Example 57 using the compound in Reference Example 192 and morpholine (yield: 67%) .

Example 68

2-(1H-indal-5-yl)-5-[(4-methyl-2,3,4,5-tetrahydro-1H-1,5-benzodiazepin-1-yl)carbonyl]phenyl 3-(4-morpholinyl)propyl ether was obtained by the same procedure as in Example 57 using the compound in Reference Example 190 and morpholine (yield: 31%).

Example 69

(4S)-1-[4-(1H-indol-5-yl)-3-[[5-(4-morpholinyl) pentyl]oxy]benzoyl]-4-methyl-2,3, 4,5-tetrahydro-1H-1,5-benzodiazepine was obtained by the same procedure as in Example 57 using the compound in Reference Example 193 and morpholine (yield: 86%).

Example 70

(4S)-1-[4-(1H-indol-5-yl)-3-[3-(4-methyl-1-piperazinyl)propoxy]benzoyl]-4-methyl-2,3,4,5-tetrahydro-1H-1,5-benzodiazepine was obtained by the same procedure as in Example 57 using the compound in Reference Example 191 and 1-methylpiperazine (yield: 78%).

Example 71

(4S)-1-[4-(1H-indol-5-yl)-3-[3-(1-piperidinyl) propoxy]benzoyl]-4-methyl-2,3,4,5-tetrahydro-1H-1,5-benzodiazepine was obtained by the same procedure as in Example 57 using the compound in Reference Example 191 and piperidine (yield: 90.4%).

Example 72

(4S)-1-[4-(1H-indol-5-yl)-3-[3-(4-thiomorpholinyl) propoxy]benzoyl]-4-methyl-2,3,4,5-tetrahydro-1H-1,5-benzodiazepine was obtained by the same procedure as in Example 57 using the compound in Reference Example 191 and thiomorpholine (yield: 89.3%).

Example 73

(4S)-1-[3-[3-(4-acetyl-1-piperazinyl)propoxy]-4-(1H-indol-5-yl)]-4-methyl-2,3,4,5-tetrahydro-1H-1,5-benzodiazepine was obtained by the same procedure as in Example 57 using the compound in Reference Example 191 and N-acetylpiperazine (yield: 80%).

Example 74

N-methyl- 4' -[[(4S)-4-methyl-2,3,4,5-tetrahydro-1H-1,5-benzodiazepin-1-yl]carbonyl]-2'-[2-(4-morpholinyl) ethoxy][1,1'-biphenyl]-4-amine was obtained by the same procedure as in Example 1 using the compounds in Reference Example 196 and Reference Example 75 (yield: 58.1%).

Example 75

N,N-dimethyl-4'-[[(4S)-4-methyl-2,3,4,5-tetrahydro-1H-1, 5-benzodiazepin-1-yl] carbonyl]-2'-[2-(4-morpholinyl)ethoxy][1,1'-biphenyl]-4-amine was obtained by the same procedure as in Example 1 using the compound in Reference Example 196 and 4-(N,N-dimethylamino)phenylboronic acid (yield: 46.6%).

Example 76

N-[4'-[[(4S)-4-methyl-2,3,4,5-tetrahydro-1H-1,5-benzodiazepin-1-yl] carbonyl]-2'-[2-(4-morpholinyl) ethoxyl [1,1'-biphenyl]-4-yl]acetamide was obtained by the same procedure as in Example 1 using the compounds in Reference Example 196 and Reference Example 73 (yield: 75.7%).

Example 77

(4S)-1-[[4'-methoxy-2-[2-(4-morpholinyl)ethoxy] [1,1'-biphenyl]-4-yl]carbonyl]-4-methyl-2,3,4,5-tetrahydro-1H-1,5-benzodiazepine was obtained by the same procedure as in Example 1 using the compound in Reference Example 196 and 4-methoxyphenylboronic acid (yield: 98.9%).

Example 78

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N,N-dimethylamine was obtained by the same procedure as in Example 33 using the compound in Example 180 and 3-(dimethylamino)-1-propanol (yield: 73%).

Example 79

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N,N-dipropylamine was obtained by the same procedure as in Example 57 using the compound in Example 180 and dipropylamine (yield: 80.0%). Example 80

(45)-1-[3-[3-(2,6-dimethyl-4-morpholinyl) propoxy]4-(1H-indol-5-yl)benzoyl]-4-methyl-2,3,4,5-tetrahydro-1H-1,5-benzodiazepine was obtained by the same procedure as in Example 57 using the compound in Reference Example 191 and 2,6-dimethyl-4-morpholine (yield: 71%).

Example 81

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N-methyl-N-propylamine was obtained by the same procedure as in Example 57 using the compound in Reference Example 191 and N-methylpropylamine (yield: 81%).

In a nitrogen atmosphere, to a solution of the compound (204 mg, 0.4mmol) in Example 81 in ethyl acetate (11 mL), a 0.4N fumaric acid ethanol solution (1.33 mL) was added under heating at reflux, followed by stirring at room temperature for 8 hours. The precipitated solid was collected by filtration and vacuum dried at 60˚C to obtain Example 81 1/2 fumarate (183 mg, 80.6%) as colorless crystals.

In a nitrogen atmosphere, a solution of the compound (200 mg, 0.392mmol) in Example 81 in ethyl acetate (15 mL) was stirred under reflux conditions. Then, a solution of 0.5 M citric acid in ethyl acetate (25% ethanol, 0.256 mL, O.131 mmol) was added, followed by standing for 24 hours. The resulting colorless precipitate was collected by filtration to obtain crude crystals (0.160 g, 67.5%).

The resulting crude crystals (81 mg, 0.134 mmol) were added to ethanol (3 mL) , followed by heating at reflux. Then, water (12 mL) was added to the solution, followed by standing for 6 days to obtain Example 81 1/3 citrate (56 mg, 69.1%) as colorless crystals.

Example 82

N-(tert-butyl)-N-[3-[2-(1H-indol-5-yl)-5-[[(4S)-4-methyl-2,3,4,5-tetrahydro-1H-1,5-benzodiazepin-1-yl] carbonyl]phenoxy]propyl]-N-methylamine was obtained by the same procedure as in Example 57 using the compound in Reference Example 191 and N-tert-butylmethylamine (yield: 90%).

Example 83

1- [3-[2-(1H-indol-5-yl) -5- [[(4S)-4-methyl-2,3,4,5-tetrahydro-1H-1,5-benzodiazepin-1-yl]carbonyl] phenoxylpropyl]4-piperidine carboxamide was obtained by the same procedure as in Example 57 using the compound in Reference Example 191 and 4-piperidine carboxamide (yield: 78.4%).

Example 84

N-[3-[2-(1H-indol-5-yl)-5-[[(4S)-4-methyl-2,3,4,5-tetrahydro-1H-1,5-benzodiazepin-2-yl]carbonyl] phenoxy] propyl]-N,N-bis(2-methoxyethoxy)amine was obtained by the same procedure as in Example 57 using the compound in Reference Example 191 and N,N-bis(2-methoxyethyl)amine
(yield: 73.8%).

Example 85

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N-isopropyl-N-methylamine was obtained by the same procedure as in Example 57 using the compound in Reference Example 191 and isopropylmethylamine (yield: 91%) .

Example 86

4-[3-[2-(1H-indol-5-yl)-5-[[(4S)-4-methyl-2,3,4,5-tetrahydro-1H-1,5-benzodiazepin-1-yl]carbonyl] phenoxy] propyl]-2-piperazinone was obtained by the same procedure as in Example 57 using the compounds in Reference Example 191 and Reference Example 94 (yield: 78.9%).

Example 87

1- [3-[2- (1H-indol-5-yl) -5- [[(45)-4-methyl-2,3,4,5-tetrahydro-1H-1,5-benzodiazepin-1-yl]carbonyl] phenoxy] propyl]-4-piperidinylformamide was obtained by the same procedure as in Example 57 using the compounds in Reference Example 191 and Reference Example 96 (yield: 58.0%).

Example 88

(2S)-1-[3[2-(1H-indol-5-yl)-5-[[(4S)-4-methyl-2,3,4,5-tetrahydro-1H-1,5-benzodiazepin-1-yl] carbonyl] phenoxy]propyl]-2-pyrrolidine carboxamide was obtained by the same procedure as in Example 57 using the compound in Reference Example 191 and L-prolineamide (yield: 62.5%).

Example 89

To a solution of the compound (0.262 g, 0.661 mmol) in Example 179 and 4-morpholinyl carboxychloride (0.23 mL, 1.98mmol) in tetrahydrofuran (20 mL), triethylamine (0.28 mL, 1.98 mmol) was added in a nitrogen atmosphere, followed by heating at reflux for 6 hours. The reaction mixed solution was concentrated in vacuum and the residue obtained was purified by NH-silica gel chromatography (chloroform:methanol = 50:1) to obtain N-[2-(1H-indol-5-yl)-5-[[(4S)-4-methyl-2,3,4,5-tetrahydro-1H-1,5-benzodiazepin-1-yl]carbonyl[phenyl]-4-morpholine carboxamide (0.247 g, 73.3%) as a pale yellow oil.

Example 90

(4S)-1-[3-[3-(1H-imidazol-1-yl]propoxy]-4-(1H-indol-5-yl)benzoyl]-4-methyl-2,3,4,5-tetrahydro-1H-1,5-benzodiazepine was obtained by the same procedure as in Example 57 using the compound in Reference Example 191 and imidazole (yield: 76%).

Example 91

To a solution of 60% sodium hydride (14 mg, 0.34 mmol) in N,N-dimethylformamide (1 mL), 1-methylhydantoin (40 mg, 0.35 mmol) was added, followed by stirring at 50˚C for one hour. The reaction solution was cooled to room temperature and the compound (0.15g, 0.29 mmol) in Reference Example 191 was added, followed by stirring at 50˚C for one hour. The reaction solution was poured into ice water and then extracted with ethyl acetate. The organic layer was washed with a saline solution and then concentrated in vacuum. The residue obtained was purified by silica gel column chromatography (chloroform:ethyl acetate = 248:2) to obtain
1-[3-[2-(1H-indol-5-yl)-5-[[(4S)-4-methyl-2,3,4,5-tetrahydro-1H-1,5-benzodiazepin-1-yl]carbonyl]phenoxy] propyl]-3-methyl-2,4-imidazolidinedione (0.11 g, 57.0%) as a colorless oil.

Example 92

3-[3-[2-(1H-indol-5-yl)-5-[[(4S)-4-methyl-2,3,4,5-tetrahydro-1H-1,5-benzodiazepin-1-yl]carbonyl] phenoxy] propyl]-1,3-oxazolidin-2-one was obtained by the same procedure as in Example 91 using the compound in Reference Example 191 and 1,3-oxazolidin-2-one (yield: 87%).

Example 93

1-[3-[2-(1H-indol-5-yl)-5-[[(4S)-4-methyl-2,3,4,5-tetrahydro-1H-1,5-benzodzazepin-1-yl]carbonyl] phenoxy] propyl-4-piperidinol was obtained by the same procedure as in Example 57 using the compound in Reference Example 191 and 4-piperidinol (yield: 95%).

Example 94

(4S)-1-[4-(1H-indol-5-yl)-3-(4-morpholinylmethyl) benzoyl]-4-methyl-2,3,4,5-tetrahydro-1H-1,5-benzodiazepine was obtained by the same procedure as in Example 1 using the compounds in Reference Example 197 and

Reference Example 70
(yield: 92%).

Example 95

(4S)-1-[3-[3-[1,4-dioxa-8-azaspiro[4.5]dec-8-yl]propoxy]-4-(1H-indol-5-yl)benzoyl)-4-methyl-2,3,4,5-tetrahydro-1H-1,5-benzodiazepine was obtained by the same procedure as in Example 57 using the compound in Reference Example 191 and 1,4-dioxa-8-azaspiro[4.5]decane (yield: 99.9%).

Example 96

1-[3-[2-(1H-indol-5-yl)-5-[[(4S)-4-methyl-2,3,4,5-tetrahydro-1H-1,5-benzodiazepin-1-yl]carbonyl] phenoxy] propyl]-N,N-dimethyl-4-piperidine carboxamide was obtained by the same procedure as in Example 57 using the compounds in Reference Example 191 and Reference Example 99
(yield: 78.4%).

Example 97

3-[3-[2-(1H-indol-5-yl)-5-[[(4S)-4-methyl-2,3,4,5-tetrahydro-1H-1,5-benzodiazepin-2-yl]carbonyl] phenoxy] propyl]-2,4-imidazolidinedion was obtained by the same procedure as in Example 91 using the compound in Reference Example 191 and hydantoin (yield: 82%).

Example 98

1-[3-[2-(1H-indol-5-yl)-5-[[(4S)-4-methyl-2,3,4,5-tetrahydro-1H-1,5-benzodiazepin-1-yl]carbonyl] phenoxy] propyl]-4-piperidinone was obtained by the same procedure as in Example 57 using the compound in Reference Example 191 and 4-piperidinone (yield: 93%).

Example 99

(4S)-1-[4- (1H-indol-5-yl)-3-(4-isopropyl-1-piperazinyl)propoxy]benzoyl]-4-methyl-2,3,4,5-tetrahydro-2H-1,5-benzodiazepine was obtained by the same procedure as in Example 57 using the compounds in Reference Example 191 and Reference Example 101 (yield: 58.9%).
By the same procedure as in Example 81 1/2 fumarate, Example 99 1/2 fumarate was obtained as colorless crystals (yield: 84.2%).
In a nitrogen atmosphere, to a solution of the compound (170 mg, 0.366 mmol) in Example 99 in ethyl acetate (7 mL), a solution of 0.5N maleic acid in ethyl acetate (0.6 mL) was added under refluxing, followed by stirring at room temperature for 24 hours. The precipitated solid was collected by filtration and then vacuum dried at 60˚C to obtain Example 99 maleate (130 mg, 62.3%) as colorless crystals.

Example 100

3-[3-[2-(1H-indol-5-yl)-5-[[(4S)-4-methyl-2,3,4,5-tetrahydro-1H-1,5-benzodiazepin-1-yl]carbonyl] phenoxy] propyl]-1,3-thiazolidine-2,4-dione was obtained by the same procedure as in Example 91 using the compound in Reference Example 191 and 2,4-thiazolidinedione (yield: 50.1%).

Example 101

(4S)-1-[4-(1H-indol-5-yl)-3-(4-morpholinylcarbonyl)benzoyl]-4-methyl-2,3,4,5-tetrahydro-1H-1,5-benzodiazepine was obtained by the same procedure as in Example 1 using the compounds in Reference Example 204 and Reference Example 70 (yield: 28.4%). Example 102
(4S)-1-[4-(1H-indol-5-yl)-3-[(4-methyl-1-piperazinyl)carbonyl]benzoyl]-4-methyl-2,3,4,5-tetrahydro-1H-1,5-benzodiazepine was obtained by the same procedure as in Example 1 using the compounds in Reference Example 205 and Reference Example 70 (yield: 73.9%). Example 103
(4S)-1-[4-(1H-indol-5-yl)-3-[3-(4-methyl-1-piperazinyl)butoxy]benzoyl]-4-methyl-2,3,4,5-tetrahydro-1H-1,5-benzodiazepine was obtained by the same procedure as in Example 57 using the compound in Reference Example 192 and N-methylpiperazine (yield: 67.9%).

Example 104

5-[(4,7-dimethyl-2,3,4,5-tetrahydro-1H-1,5-benzodiazepin-1-yl]carbonyl]-2-(1H-indol-5-yl)phenyl 2- (4-morpholinyl) ethyl ether was obtained by the same procedure as in Example 1 using the compounds in Reference Example 207 and Reference Example 70 (yield: 43.1%).

Example 105

N-methyl-2'-[3-[methyl(propyl)amino]propoxy]-4'-[[(4S)-4-methyl-2,3,4,5-tetrahydro-1H-1,5-benzodiazepin-1-yl]carbonyl][1,1'-biphenyl]-4-amine was obtained by the same procedure as in Example 1 using the compounds in Reference Example 210 and Reference Example 75 (yield: 85.3%).

Example 106

3-[3-[[4'-(methylamino)-4-[[(4S)-4-methyl-2,3,4,5-tetrahydro-1H-1,5-benzodiazepin-1-yl]carbonyl] [1,1'-biphenyl]-2-yl]oxy]propyl]-2,4-imidazolidinedione was obtained by the same procedure as in Example 1 using the compounds in Reference Example 212 and Reference Example 75
(yield: 69.1%).

Example 107

(4S)-1-[4-(1H-indol-5-yl)-3-[3-[4-(4-morpholinyl)-1-piperidinyl]propoxy]benzoyl]-4-methyl-2,3,4,5-tetrahydro-1H-1,5-benzodiazepine was obtained by the same procedure as in Example 57 using the compound in Reference Example 191 and 4-morpholyl-1-piperidine (yield: 64.2%).

Example 108

N-[3-[2-(1H-indal-5-yl)-5-[[(4S)-4-methyl-2,3,4,5-tetrahydro-1H-1,5-benzodiazepin-1-y1]carbonyl] phenoxy] propyl]-N-methyl-1-pentaneamine was obtained by the same procedure as in Example 57 using the compound in Reference Example 191 and N-methylpentylamine (yield: 55.5%).

Example 109

(4S)-1-[4-(1H-indol-5-yl)-3-[3-(4-methyl-1,4-diazepane-1-yl)propoxy]benzoyl]-4-methyl-2,3,4,5-tetrahydro-1H-1,5-benzodiazepine was obtained by the same procedure as in Example 57 using the compound in Reference Example 191 and 1-methyl-1,4-benzodiazepine (yield: 53.4%).

Example 110

(4S)-1-[4-(1H-indol-5-yl)-3-[2-(4-methyl-1-piperazinyl)ethoxy]benzoyl]-4-methyl-2,3,4,5-tetrahydro-1H-1,5-benzodiazepine was obtained by the same procedure as in Example 57 using the compound in Reference Example 189 and N-methylpiperazine (yield: 38.2%).

By the same procedure as in Example 81 1/2 fumarate, Example 110 1/2 fumarate was obtained as colorless crystals (yield: 94.6%).

In a nitrogen atmosphere, a solution of the compound (8.58 g, 16.4mmol) in Example 110 in ethyl acetate (800 mL) was stirred under reflux conditions. Then, a solution of a 0.5M maleic acid in ethyl acetate (65.5 mL, 32.8 mmol) was added, followed by standing for 24 hours. The resulting colorless precipitate was collected by filtration to obtain Example 110 2 maleate (11.5 g, 89.8%).

Example 111

(4S)-4-methyl-1-[[2-[2-(4-morpholinyl)ethoxy]-4'-(trifluoromethoxy)[1,1'-biphenyl]-4-yl]carbonyl-2,3,4,5-tetrahydro-1H-1,5-benzodiazepine was obtained by the same procedure as in Example 1 using the compound in Reference Example 196 and 4-trifluoromethoxyphenylboronic acid (yield: 89.3%).

Example 112

N1-[3-[2-(1H-indol-5-yl)-5-[[(4S)-4-methyl-2,3,4,5-tetrahydro-1H-1,5-benzodiazepin-1-yl)carbonyl] phenoxy]propyl]-N1,N2,N2-trimethyl-1,2-ethaneamine was obtained by the same procedure as in Example 33 using the compound in Example 180 and 2-([2-(dimethylamino)ethyl]methylamino)ethanol (yield: 27.3%).

Example 113

(4S)-1-[4-(1H-indol-5-yl)-3-[3-(4-propyl-1-piperazinyl)propoxy]benzoyl]-4-methyl-2,3,4,5-tetrahydro-1H-1,5-benzodiazepine was obtained by the same procedure as in Example 57 using the compound in Reference Example 191 and N-propylpiperazine (yield: 67.4%).

Example 114

N-[3-[2-(1H-indol-5-yl)-5-[[(4S)-4-methyl-2,3,4,5-tetrahydro-1H-1,5-benzodiazepin-1-yl]carbonyl] phenoxy] propyl]-N-methyl-1-butaneamine was obtained by the same procedure as in Example 57 using the compound in Reference Example 191 and butylmethylamine (yield: 87.9%).

Example 115

(4S)-1-[3-[3-[(2R,6S)-2,6-dimethylmorpholinyl] propoxy]-4-(1H-indol-5-yl)benzoyl]-4-methyl-2,3,4,5-tetrahydro-1H-1,5-benzodiazepine was obtained by the same procedure as in Example 57 using the compound in Reference Example 191 and (2R,6S)-2,6-dimethylmorpholine (yield: 76.9%).

Example 116

N,N-diethyl-3-[2-(1H-indol-5-yl)-5-[[(4S)-4-methyl-2,3,4,5-tetrahydro-1H-1,5-benzodiazepin-1-yl] carbonyl] phenoxy]-1-propaneamine was obtained by the same procedure as in Example 57 using the compound in Reference Example 191 and diethylamine (yield: 52.9%).

Example 117

N-[4-[2-(1H-indol-5-yl)-5-[[(4S)-4-methyl-2,3,4,5-tetrahydro-1H-1,5-benzodiazepin-1-yl]carbonyl] phenoxy] butyl]-N-methyl-N-propylamine was obtained by the same procedure as in Example 57 using the compound in Reference Example 192 and N-methylpropylamine (yield: 67.9%).

In a nitrogen atmosphere, to a solution of the compound (12.3 g, 23.4 mmol) in Example 117 in acetone (500 mL), a solution of 0.5 M fumaric acid in isopropyl alcohol (23.4 mL) was added under heating at reflux, followed by stirring at room temperature for 24 hours. The precipitated solid was collected by filtration and then vacuum dried to obtain crude crystals (12.2 g, 89.2%). The resulting crude crystals were dissolved in an aqueous 50% ethanol solution by heating at reflux to obtain Example 117 1/2 fumarate (5.4 g, 39.6%) as colorless crystals.

In a nitrogen atmosphere, to a solution of the compound (1.41 g, 2.68 mmol) in Example 117 in acetone (60 mL), a solution of 0.5 M fumaric acid in isopropyl alcohol (5.36 mL) was added under heating at reflux, followed by stirring at room temperature for 17 hours. The precipitated solid was collected by filtration and then vacuum dried to obtain crude crystals (1.59 g, 92.5%). The resulting crude crystals were dissolved in an aqueous 50% ethanol solution by heating at reflux and then allowed to stand for one day to obtain Example 117 fumarate (1.23 g, 71.2%) as colorless crystals.

In a nitrogen atmosphere, a mixed solution of the compound (410 mg, 0.781 mmol) in Example 117 in acetonitrile (15 mL) and toluene (20 mL) was stirred under heat reflux conditions. Then, a solution of 0.5 M maleic acid in ethyl acetate (0.841 mL, 0.391 mmol) was added and concentrated in vacuum until crystals are precipitated, followed by standing for 24 hours. The resulting colorless precipitate was collected by filtration to obtain Example 117 maleate (0.285 g, 62.6%) as colorless crystals.

In a nitrogen atmosphere, a solution of the compound (200 mg, 0.381 mmol) in Example 117 in ethyl acetate (10 mL) was stirred under heat reflux conditions. Then, a solution of 0.5 M oxalic acid in ethyl acetate (0.381 mL, 0.191 mmol) was added, followed by standing for 24 hours. The resulting colorless precipitate was collected by filtration to obtain Example 117 oxalate (89 mg, 40.3%) as colorless crystals.

By the same procedure as in Example 81 citrate, crude crystals (0.395 mg, 86.9%) were obtained. Furthermore, the crude crystals were recrystallized from a mixed solvent of water and methanol (7/5) to obtain Example 117 citrate as colorless crystals (yield: 71.2%).

In a nitrogen atmosphere, a mixed solution of the compound (0.174 g, 0.332 mmol) in Example 117 in ethyl acetate (14 mL) and ethanol (4 mL) was stirred under heat reflux conditions. Then, a solution of 0.5 M tartaric acid and acetic acid in ethanol (0.332 mL, 0.166 mmol was added, followed by standing for 24 hours. The resulting colorless precipitate was collected by filtration to obtain Example 117 tartrate (78 mg, 39.2%) as colorless crystals.

Example 118

N-ethyl-3-[2-(1H-indol-5-yl)-5-[[(4S)-4-methyl-2,3,4,5-tetrahydro-1H-1,5-benzodzazepin-1-yl]carbonyl] phenoxy]-N-methyl-1-propaneamine was obtained by the same procedure as in Example 57 using the compound in Reference Example 191 and N-ethylmethylamine (yield: 65.9%).

Example 119

N- methyl- N-[2'-[3-[methyl (propyl) amino] propoxy]- 4'-[[(4S)- 4- methyl- 2,3,4,5- tetrahydro- 1H- 1,5- benzodiazepin-1-yl]carbonyl][1,1'-biphenyl]-4-yl]acetamide was obtained by the same procedure as in Example 3 using the compound in Example 105 (yield: 19.4%).

Example 120

Using the compound in Reference Example 202 and N,N-dimethylamine, a compound was obtained by the same procedure as in Example 57. Using the resulting compound and the compound in Reference Example 75, 3-(dimethylamino) propyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 1 (yield: 47%).

Example 121

To a solution of the compound (0.314 g, 0.790 mmol) in Example 180 and triphenylphosphine (0.414 g, 1.58 mmol) and benzyl 2-hydroxyethylmethylcarbamate (0.248 g, 1.18 mmol) in tetrahydrofuran (25 mL), azodicarboxylic acid diisopropyl ester (40% toluene solution) (0.921 mL, 1.82 mmol) was added in a nitrogen atmosphere, followed by stirring at room temperature for 7.5 hours. The reaction mixture was concentrated in vacuum and the residue obtained was purified by NH-silica gel chromatography (chloroform). The resulting compound was dissolved in ethanol in a nitrogen atmosphere and palladium-carbon (0.340 g) was added at 0˚C. Then, nitrogen was replaced by hydrogen, followed by stirring at room temperature for 6 hours . The catalyst was removed by filtration and the solution was concentrated in vacuum. The residue obtained was dissolved in an aqueous citric acid solution and then washed with ethyl acetate. Then, the aqueous layer was basified with sodium hydrogen carbonate and extracted with ethyl acetate. The organic layer was washed with a saturated saline solution, dried over anhydrous magnesium sulfate and then concentrated in vacuum. The residue obtained and potassium carbonate (0.063 g, 0.457 mmol) were further dissolved in dimethylformamide (5 mL) in a nitrogen atmosphere. To the solution, propyl iodide (0.071 g, 0.419 mmol) was added dropwise at room temperature, followed by stirring for 16.5 hours. The reaction mixture was poured into water and then extracted with ethyl acetate . The organic layer was washed with a saturated saline solution and then concentrated in vacuum. The residue obtained was purified by NH-silica gel chromatography (ethyl acetate:chloroform=0: 1 to 1 : 3 to obtain N-[2-[2-(1H-indol-5-yl)-5-[[(4S)-4-methyl-2,3,4,5-tetrahydro-1H-1,5-benzodiazepin-1-yl]carbonyl]phenoxy] ethyl]-N-methyl-1-propaneamine (42 mg, 19.0%, 3 steps) as a pale yellow oil.

Example 122

(4S)-1-[3-[3-(4-butyl-1-piperazinyl)propoxyl-4-(1H-indol-5-yl)benzoyl]-4-methyl-2,3,4,5-tetrahydro-1H-1, 5-benzodiazepine was obtained by the same procedure as in Example 57 using the compounds in Reference Example 191 and Reference Example 103 (yield: 61.9%).

By the same procedure as in Example 81 1/2 fumarate, Example 122 1/2 fumarate was obtained as colorless crystals (yield: 76.1%).

In a nitrogen atmosphere, to a solution of the compound (150 mg, 0.259 mmol) in Example 122 in ethyl acetate (7 mL), a solution of 0.5N maleic acid in ethyl acetate (0.51 mL) was added under heating at reflux, followed by stirring at room temperature for 24 hours. The precipitated solid was collected by filtration and then vacuum dried at 60˚C to obtain Example 122 maleate (137 mg, 62.3%) as colorless crystals.

In a nitrogen atmosphere, a solution of the compound (9.16 g, 15.799 mmol) in Example 122 in ethyl acetate (400 mL) was stirred under reflux conditions. Then, 63.2 mL of a solution of 0.5 M maleic acid in ethyl acetate was added, followed by stirring for 3 hours. The precipitated solid was collected by filtration to obtain crude crystals (12.38 g, 96.1%) . To the crude crystals (11.60 g, 14.287 mmol), a mixed solution (220 mL, 600 mL) of tetrahydrofuran and acetone was added, followed by heating at reflux for one hour. After stirring at room temperature for 12 hours, the precipitate was collected by filtration. The precipitate was vacuum dried at 60˚C to obtain Example 122 2 maleate (9.96 g, 85.8%) as colorless crystals.

In a nitrogen atmosphere, to a solution of the compound (159 mg, 0.274 mmol) in Example 122 in ethyl acetate

(7 mL), 0.5N citric acid isopropyl alcohol (0.37 mL) was added under heating at reflux, followed by stirring at room temperature for 168 hours. The precipitated solid was collected by filtration and then vacuum dried at 60˚C to obtain Example 122 citrate (113 mg, 53.3%) as a colorless solid.

Example 123

N-[1-[3-[2-(1H-indol-5-yl)-5-[[(4S)-4-methyl-2,3,4,5-tetrahydro-1H-1,5-benzodiazepin-1-yl]carbonyl] phenoxy] propyl-4-piperidinyl]-N, N-dimethylamine was obtained by the same procedure as in Example 57 using the compounds in Reference Example 191 and Reference Example 107 (yield: 32.3%).

Example 124

N-ethyl-N-[3-[2-(1H-indol-5-yl)-5-[[(4S)-4-methyl-2,3,4,5-tetrahydro-1H-1,5-benzodiazepin-1-yl] carbonyl]phenoxy]propyl]-1-butaneamine was obtained by the same procedure as in Example 57 using the compound in Reference Example 191 and butylethylamine (yield: 64.5%).

Example 125

N-ethyl-3-[2-(1H-indol-5-yl)-5-[[(4S)-4-methyl-2,3,4,5-tetrahydro-1H-1,5-benzodiazepin-1-yl]carbonyl] phenoxy]-N-propyl-1-propaneamine was obtained by the same procedure as in Example 57 using the compound in Reference Example 191 and N-ethylpropylamine (yield: 73.0%).

Example 126

N-[5-[2-(1H-indol-5-yl)-5-[[(4S)-4-methyl-2,3,4,5-tetrahydro-1H-1,5-benzodiazepin-1-yl] carbonyl] phenoxy] pentyl]-N-methyl-N-propylamine was obtained by the same procedure as in Example 57 using the compound in Reference Example 193 and N-methylpropylamine (yield: 51.2%).

Example 127

N-[3-[2-(1H-indol-5-yl)-5-[[(4R)-4-methyl-2,3,4,5-tetrahydro-1H-1,5-benzodiazepin-1-yl)carbonyl] phenoxy] propyl]-N-methyl-N-propylamine was obtained by the same procedure as in Example 57 using the compound in Reference Example 194 and N-methylpropylamine (yield: 59.9%).

Example 128

(4R)-1-[4-(1H-indol-5-yl)-3-[3-(4-propyl-1-piperazinyl)propoxy]benzoyl]-4-methyl-2,3,4,5-tetrahydro-1H-1,5-benzodiazepine was obtained by the same procedure as in Example 57 using the compound in Reference Example 194 and N-propylpiperazine (yield: 73.4%).

Example 129

N-ethyl-2'-[3-[methy2(propyl)amzno]propoxy]-4'-[[(4S)-4-methyl-2,3,4,5-tetrahydro-1H-l,5-benzodiazepin-1-yl]carbonyl][2,2'-biphenyl]-4-amine was obtained by the same procedure as in Example 1 using the compounds in Reference Example 210 and Reference Example 76 (yield: 46.1%).

Example 130

N-methyl-2'-[3-[methyl(propyl)amino]propoxy]-4'-[[(4R)-4-methyl-2,3,4,5-tetrahydro-1H-1,5-benzodiazepin-1-yl]carbonyl][1,1'-biphenyl]-4-amine was obtained by the same procedure as in Example 1 using the compounds in Reference Example 211 and Reference Example 75 (yield: 85.9%).

Example 131

N-[3-[[4'-methoxy-4-[[(4S)-4-methyl-2,3,4,5-tetrahydro-1H-1,5-benzodiazepin-1-yl]carbonyl][1,1'-biphenyl]-2-yl]oxy]propyl]-N-methyl-N-propylamine was obtained by the same procedure as in Example 1 using the compound in Reference Example 210 and 4-methoxyphenylboronic acid (yield: 94.5%).

Example 132

3-[methyl(propyl)amino]propyl 2-(1H-indol-5-yl)-5-[[(4S)-4-methyl-2,3,4,5-tetrahydro-1H-1,5-benzadiazepin-1-yl]carbonyl]benzoate was obtained by the same procedure as in Example 57 using the compound in Reference Example 202 and N-methylpropylamine (yield: 32.7%).

Example 133

Using the compound of Reference Example 200 and N,N-dimethylamine, a compound was obtained by the same procedure as in Example 57. Using the resulting compound and 4-methoxyphenylboronic acid, 3-(dimethylamino) propyl 4'-methoxy-4-[[(4S)-4-methyl-2,3,4,5-tetrahydro-1H-1,5-benzodiazepin-1-yl]carbonyl][1,1'-biphenyl]-2-carboxylate was obtained by the same procedure as in Example 1 (yield: 58%).

Example 134

(4S)-1-[4-(1H-indol-5-yl)-3-(4-morpholinyl) benzoyl]-4-methyl-2,3,4,5-tetrahydro-1H-1,5-benzodiazepine was obtained by the same procedure as in Example 1 using the compounds in Reference Example 216 and Reference Example 70 (yield: 70.5%).

Example 135

3-(4-morpholinyl)propyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 57 using the compound in Reference Example 202 and morpholine (yield: 88%).

Example 136

3-(4-methyl-2-piperazinyl)propyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 57 using the compound in Reference Example 202 and 1-methylpiperazine (yield: 84%).

Example 137

3-(4-butyl-1-piperazinyl)propyl 2-(1H-indol-5-yl)-5-[[(4S)-4-methyl-2,3,4,5-tetrahydro-1H-1,S-benzodiazepin-1-yl]carbonyl]benzoate was obtained by the same procedure as in Example 57 using the compound in Reference Example 202 and 1-butylpiperazine (yield: 60%).

Example 138

1-propyl-4-piperazinyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 1 using the compounds in Reference Example 217 and Reference Example 70 (yield: 84%).

Example 139

4-pyridinylmethyl 2-(1H-indol-5-yl)-5-[[(4S)-4-methyl-2,3,4,5-tetrahydro-1H-1,5-benzodiazepin-1-yl]carbonyl] benzoate was obtained by the same procedure as in Example 1 using the compounds in Reference Example 218 and Reference Example 70 (yield: 59%).

Example 140

(4S)-2-[3-[3-[(3R,5S)-4-butyl-3,5-dimethylpiperazinyl]propoxy]-4-(1H-indol-5-yl)benzoyl]-4-methyl-2,3,4,5-tetrahydro-1H-1,5-benzodiazepine was obtained by the same procedure as in Example 57 using the compounds in Reference Example 191 and Reference Example 110 (yield: 83.4%).

Example 141

A solution of the compound (304 mg, 0.587 mmol) in Reference Example 191 and 4-(N-Boc-amino)piperidine

and triethylamine (123 μL, 0.881 mmol) in acetonitrile (10 mL) was stirred under heating at reflux for 7 hours. After concentrating in vacuum, the following reaction was conducted without being purified.

In a nitrogen atmosphere, the concentrated residue was dissolved in dichloromethane/trifluoroacetic acid (1/1) (6mL), followed by stirring at room temperature for 30 minutes. After the solvent was distilled off by concentrating in vacuum, an aqueous saturated sodium hydrogen carbonate solution was added. After extracting with dichloromethane, the organic layer was washed with a saturated saline solution. The organic layer was concentrated in vacuum and then purified by NH-silica gel chromatography (methanol: ethyl acetate = 0 : 1 to 1: 6) to obtain 1-[3-[2-(1H-indol-5-yl)-5-[[(4S)-4-methyl-2,3,4,5-tetrahydro-1H-1,5-benzodiazepin-1-yl]carbonyl]phenoxy] propyl]-4-piperidinylamine (108 mg, 34.2%, 2 steps).

Example 142

(4S)-1-[4-(1H-indol-5-yl)-3-[3-(4-isobutyl-l-piperazinyl)propoxy]benzoyl]-4-methyl-2,3,4,5-tetrahydro-1H-1,5-benzodiazepine was obtained by the same procedure as in Example 57 using the compounds in Reference Example 191 and Reference Example 105 (yield: 47.6%).

Example 143

A solution of sodium (45 mg, 1.95 mmol) and naphthalene (256 mg, 2.0 mmol) in ethylene glycol dimethyl ether (20 mL) was stirred in a nitrogen gas flow at room temperature for one hour. To the reaction solution, a solution of the compound (683 mg, 1.0 mmol) in Reference Example 219 in ethylene glycol dimethyl ether (20 mL) was added at -60˚C, followed by stirring for 2 hours . After the completion of the reaction, the reaction solution was quenched with water at -78˚C and then solvent was concentrated in vacuum. The residue obtained was poured into a saturated saline solution and then extracted with ethyl acetate. The organic layer was washed with a saturated saline solution, dried over anhydrous magnesium sulfate and then concentrated in vacuum. The residue obtained was purified by NH-silica gel column chromatography (hexane:ethyl acetate = 2:3) to obtain
N-isopropyl-2'-[3-[methyl(propyl)amino]propoxy]-4'-[[(4S)-4-methyl-2,3,4,5-tetrahydro-1H-1,5-benzodiazepin-1-yl]carbonyl] [1,1'-biphenyl]-4-amine (346 mg, 65.4%) as a pale yellow oil.

Example 144

Using the compound in Reference Example 199 and N, N-dimethylaminoethanol, a compound was obtained by the same procedure as in Example 33. Using the resulting compound and the compound in Reference Example 70, 2-(dimethylamino)ethyl 2-(1H-indol-5-yl)-5-[[(4S)-4-methyl-2,3,4,5-tetrahydro-1H-1,5-benzodiazepin-1-yl]carbonyl] benzoate was obtained by the same procedure as in Example 1 (yield: 62%).

Example 145

1-ethyl-3-pyrrolidinyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 1 using the compounds in Reference Example 220 and Reference Example 70 (yield: 72%).

Example 146

A mixed solution of the compound (0.4 g, 0.82 mmol) in Reference Example 201, morpholine (0.214 g, 2.4 mmol) and potassium iodide (0.014 g, 0.08 mmol) in dimethylformamide (2 mL) and acetonitrile (2 mL) was stirred at 110˚C for 20 hours. After cooling, an aqueous sodium carbonate solution and a saturated saline solution were added, followed by extraction with ethyl acetate. The organic layer was extracted with an aqueous citric acid solution, basified with an aqueous sodium carbonate solution and then extracted with chloroform. The extract was dried over anhydrous magnesium sulfate and then concentrated in vacuum. The residue obtained was purified by NH-silica gel column chromatography (chloroform:methanol = 100:1) to obtain 2-(4-morpholinyl)ethyl 2-(1H-indol-5-yl)-5-[[(4S)-4-methyl-2,3,4,5-tetrahydro-1H-1,5-benzodiazepin-2-yl]carbonyl]benzoate (0.36 g, 81%) as a colorless amorphous product.

Example 147

2-(4-methyl-1-piperazinyl)ethyl 2-(1H-indol-5-yl)-5-[[(4S)-4-methyl-2,3,4,5-tetrahydro-1H-1,5-benzodiazepin-1-yl-carbonyl]benzoate was obtained by the same procedure as in Example 146 using the compound in Reference Example 201 and 1-methylpiperazine (yield: 68%).

Example 148

(4S)-1-[3-[2-(4-butyl-1-piperazinyl)ethoxy]-4-(1H-indol-5-yl)benzoyl]-4-methyl-2,3,4,5-tetrahydro-1H-2,5-benzodiazepine was obtained by the same procedure as in Example 146 using the compounds in Reference Example 189 and Reference Example 103 (yield: 79.8%).

By the same procedure as in Example 81 1/2 fumarate, Example 148 1/2 fumarate (yield: 79.9%) was obtained as colorless crystals.

By the same procedure as in Example 99 maleate, Example 148 maleate (yield: 80.2%) was obtained as colorless crystals.

Example 149

(4S)-1-[4-(1H-indol-5-yl)-3-(4-methyl-1-piperazinyl)benzoyl]-4-methyl-2,3,4,5-tetrahydro-1H-1, 5-benzodiazepine was obtained by the same procedure as in Example 1 using the compounds in Reference Example 223 and Reference Example 70 (yield: 10.5%).

Example 150

4-ethyl-1-[4-(1H-indol-5-yl)-3-[2-(4-morpholinyl)ethoxy]benzoyl]-2,3,4,5-tetrahydro-1H-1,5-benzodiazepine was obtained by the same procedure as in Example 33 using the compound in Example 183 and 4-(2-hydroxyethyl) morpholine (yield: 55.0%).

Example 151

N-[l-[2-[2-(1H-indol-5-yl)-5-[[(4S)-4-methyl-2,3,4,5-tetrahydro-1H-1,5-benzodiazepin-1-yl]carbonyl] phenoxy] ethyl]-4-piperidinyl]-N-methyl-N-propylamine was obtained by the same procedure as in Example 146 using the compounds in Reference Example 189 and Reference Example 112 (yield: 61.2%) .

Example 152

N-[4-[2-(1H-indol-5-yl)-5-[[(4S)-4-methyl-2,3,4,5-tetrahydro-1H-1,5-benzodiazepin-1-yl]carbonyl] phenoxy]-2-butynyl]-N-methyl-N-propylamine was obtained by the same procedure as in Example 146 using the compound in Reference Example 224 and N-methylpropylamine (yield: 84.5%).

Example 153

(2E)-4-[2-(1H-indol-5-yl)-5-[[(4S)-4-methyl-2,3,4,5-tetrahydro-1H-1,5-benzodiazepin-1-yl]carbonyl) phenoxy]-N-methyl-N-propyl-2-butene-1-amine was obtained by the same procedure as in Example 33 using the compounds in Reference Example 180 and Reference Example 115 (yield: 37.7%).

Example 154

Using the compound in Reference Example 231 and N-methylpropylamine, a compound was obtained by the same procedure as in Example 57. To the resulting compound, an aqueous 4N sodium hydroxide solution (10 mL) and methanol (40 mL) were added, followed by stirring under heating at reflux for 5 hours. After the completion of the reaction, the reaction mixture was concentrated in Vacuum and the residue obtained was added to a saturated saline solution, followed by extraction with ethyl acetate. The organic layer was washed with a saturated saline solution, dried over anhydrous magnesium sulfate and then concentrated in vacuum. The residue obtained was purified by NH-silica gel column chromatography (hexane:ethyl acetate = 3:2) to obtain
N-ethyl-3-fluoro-2'-[3-[methyl(propyl)amino]propoxy]-4'-[[(4S)-4-methyl-2,3,4,5-tetrahydro-1H-1,5-benzodiazepin-1-yl] carbonyl][1,1'-biphenyl]-4-amine(365 mg, 15,2%) as a pale yellow solid.

Example 155

N-ethyl-3-methyl-2'-[3-[methyl(propyl)amino]propoxy]-4'-[[(4S)-4-methyl-2,3,4,5-tetrahydro-1H-1,5-benzodiazepin-1-yl]carbonyl][1,1'-biphenyl]-4-amine was obtained by the same procedure as in Example 1 using the compounds in Reference Example 210 and Reference Example 83 (yield: 59.1%).

Example 156

N-ethyl-2'-[4-[methyl(propyl)amino]butoxy]-4'-[[(4S)-4-methyl-2,3-4,5-tetrahydro-1H-1,5-benzodiazepin-1-yl] carbonyl][1,1'-biphenyl]-4-amine was obtained by the same procedure as in Example 1 using the compounds in Reference Example 232 and Reference Example 76 (yield: 71.2%).

Example 157

N-[3-[[4'-(difluoromethoxy)-4-[[(4S)-4-methyl-2,3,4,5-tetrahydro-1H-1,5-benzodiazepin-1-yl]carbonyl] [1,1'-biphenyl]-2-yl]oxy]propyl]-N-methyl-N-propylamine was obtained by the same procedure as in Example 1 using the compounds in Reference Example 210 and Reference Example 79 (yield: 70%).

Example 158

5-(dimethylamino)-1-[2-(1H-indol-5-yl)-5-[[(4S)-4-methyl-2,3,4,5-tetrahydro-1H-1,5-benzodiazepin-1-yl]carbonyl]phenyl]-1-pentanone was obtained by the same procedure as in Example 57 using the compound in Reference Example 236 and N-methylpropylamine (yield: 36.0%).

Example 159

N-ethyl-4'-[[(4S)-4-methyl-2,3,4,5-tetrahydro-1H-1,5-berzodiazepin-1-yl] carbonyl]-2'- [3- (4-propyl-1-piperazinyl) propoxy] [1,1'-biphenyl]-4-amine was obtained by the same procedure as in Example 1 using the compounds in Reference Example 237 and Reference Example 76 (yield: 73.3%).

Example 160

N-ethyl-4'-[[(4S)-4-methyl-2,3,4,5-tetrahydro-1H-1,5-benzodiazepin-1-yl]carbonyl]-2'-[2-(4-propyl-1-piperazinyl) ethoxy] [1, 1' -biphenyl] -4-amine was obtained by the same procedure as in Example 1 using the compounds in Reference Example 238 and Reference Example 76 (yield: 53.6%).

Example 161

$N^4$-ethyl-$N^3$,$N^3$-dimethylamino-2'- [3-[methyl (propyl)amino]propoxy]-4'-[[(4S)-4-methyl-2,3,4,5-tetrahydro-1H-1,5-benzodiazepin-1-yl] carbonyl][1,1'-biphenyl] -3, 4-diamine was obtained by the same procedure as in Example 154 using the compound in Reference Example 244 and N-methylpropylamine (yield: 34.2%).

Example 162

4-(dimethylamino)butyl 2-(1H-indol-5-yl)-5-[[(4S)-4-methyl-2,3,4,5-tetrahydro-1H-1,5-benzodiazepin-1-yl]carbonyl]benzoate was obtained by the same procedure as in Example 146 using the compound in Reference Example 203 and dimethylamine (yield: 24%).

Example 163

To a solution of the compound (0.268 g, 0.418 mmol) in Reference Example 253 in a solution of methanol: water= 4:1 (5 mL), an aqueous 4N sodium hydroxide solution (0.52 mL, 349 mmol) was added. Then, the reaction solution was heated at reflux and stirred for 13 hours. After the completion of the reaction, the reaction solution was concentrated and the residue was poured into water, followed by extraction with ether and further washing with a saturated saline solution. The extract was dried over anhydrous magnesium sulfate and then concentrated in vacuum. The residue obtained was purified by NH-silica gel column chromatography (hexane:ethyl acetate = 2:1) to obtain N-ethyl-3-methoxy-2'-[3-[methyl(propyl)amino]propoxy]-4'-[[(4S)-4-methyl-2,3,4,5-tetrahydro-1H-1,5-benzodiazepin-1-yl] carbonyl][1,1'-biphenyl]-4-amine (0.142 g, 62.3%) as a yellow amorphous product.

Example 164

A mixed solution of the compound (849 mg, 1.79 mmol) in Reference Example 210, the compound (535 mg, 2.15 mmol) in Reference Example 76, tetrakis (triphenylphosphine)palladium(0) (62 mg, 0.0537 mmol) and sodium hydrogen carbonate (452 mg, 5.37 mmol) in ethylene glycol dimethyl ether (17 mL) and water (17mL) was stirred under

heating at reflux in a nitrogen gas flow for 6 hours. After the completion of the reaction, the reaction solution was concentrated in vacuum. The residue obtained was poured into a saturated saline solution and then extracted with ethyl acetate. The organic layer was washed with a saturated saline solution, dried over anhydrous magnesium sulfate and then concentrated in vacuum. The residue obtained was used for the following reaction without being purified.

To a solution of the crude product in methanol (20 mL), 10% palladium-carbon (300 mg) was added, followed by stirring in a hydrogen gas flow at room temperature for 4 hours. The catalyst was removed by filtration and the filtrate was concentrated in vacuum. The residue obtained was purified by NH-silica gel column chromatography (hexane:ethyl acetate = 1:1) to obtain 2'-[3-[methyl(propyl)amino]propoxy]-4'-[[(4S)-4-methyl-2,3,4,5-tetrahydro-1H-l,5-benzodiazepin-1-yl]carbonyl][1,1'-biphenyl]-4-amine (521 mg, 59.8%) as a colorless oil.

Example 165

N-ethyl-4'-[[(4S)-4-methyl-2,3,4,5-tetrahydro-1H-1,5-benzodiazepin-1-yl]carbonyl]-2'-[3-(propylamino) propoxy] [1,1' -biphenyl]-4-amine was obtained by the same procedure as in Example 1 using the compounds in Reference Example 254 and Reference Example 76 (yield: 53.9%).

Example 166

N-[2'-[3-[methyl(propyl)amino]propoxy]-4'-[[(4S)-4-methyl-2,3,4,5-tetrahydro-1H-1,5-benzodiazepin-1-yl]carbonyl][1,1'-biphenyl]-4-yl]acetamide was obtained by the same procedure as in Example 1 using the compounds in Reference Example 210 and Reference Example 73 (yield: 67.2%).

Example 167

4-[2-[(1H-indol-5-yl)-5-[(4-methyl-2,3,4,5-tetrahydro-1H-1,5-benzodiazepin-1-yl]carbonyl]phenoxy]-2-butaneamine was obtained by the same procedure as in Example 38 using the compounds in Reference Example 182 and Reference Example 258 (yield: 37.6%).

Example 168

2-amino-N-[2-[2-(1H-indol-5-yl)-5-[(4-methyl-2,3,4,5-tetrahydro-1H-1,5-benzodiazepzn-1-yl]carbonyl] phenoxy] ethyl] acetamide was obtained by the same procedure as in Example 14 using the compound in Reference Example 255 (yield: 96.30%.

Example 169

2-amino-N-[3-[2-(1H-indol-5-yl)-5-[(4-methyl-2,3,4,5-tetrahydro-1H-1,5-benzodzazepin-1-yl]carbonyl] phenoxy]propyl] acetamide was obtained by the same procedure as in Example 14 using the compound in Reference Example 256 (yield: 80.1%).

Example 170

To a solution of the compound (500 mg, 1.10 mmol) in Example 38 and pyridine (0.9 mL, 1.87 mmol) in tetrahydrofuran (80 mL), a solution of ethylmalonyl chloride (175 mg, 1.16 mmol) in tetrahydrofuran (20 mL) was added dropwise over 10 minutes under ice cooling. After stirring at room temperature for 5 hours, the reaction solution was concentrated in vacuum. The residue obtained was poured into water, followed by extracting with ethyl acetate and further washing with a saturated saline solution. The solution was dried over anhydrous magnesium sulfate and then concentrated in vacuum. The residue obtained was purified by NH-silica gel column chromatography (chloroform) to obtain ethyl 3-[[3-[2-(1H-indol-5-yl)-5-[[(4S)-4-methyl-2,3,4,5-tetrahydro-1H-1,5-benzodiazepin-1-yl]carbonyl]phenoxy] propyl]amino]-3-oxopropanoate (176 mg, 28.1%) as a colorless oil.

Example 171

Ethyl 5-[[3-[2-(1H-indol-5-yl)-5-[[(4S)-4-methyl-2,3,4,5-tetrahydro-1H-1,5-benzodiazepin-1-yl]carbonyl] phenoxy]propyl]amino]-5-oxopentanoate was obtained by the same procedure as in Example 170 using the compound in Example 38 and ethyl 5-chloro-5-oxopentanoate (yield: 49.6%).

Example 172

N-ethyl-4-[2-(1H-indol-5-yl)-5-[[(4S)-4-methyl-2,3,4,5-tetrahydra-1H-1,5-benzodiazepin-1-yl]carbonyl] phenoxy]-N-methyl-1-butaneamine was obtained by the same procedure as in Example 57 using the compound in Reference Example 192 and N-ethylmethylamine (yield: 56.5%).

Example 173

N-butyl-4-[2-(1H-indol-5-yl)-5-[[(4S)-4-methyl-2,3,4,5-tetrahydro-1H-1,5-benzodiazepin-1-yl]carbonyl] phenoxy]-N-methyl-1-butaneamine was obtained by the same procedure as in Example 57 using the compound in Reference Example 192 and butylmethylamine (yield: 31.Q°).

Example 174

N-[4-[2-(1H-indol-5-yl)-5-[[(4S)-4-methyl-2,3,4,5-tetrahydro-1H-1,5-benzodiazepin-1-yl]carbonyl] phenoxy] butyl]-N-methyl-1-pentaneamine was obtained by the same procedure as in Example 57 using the compound in Reference Example 192 and N-methylpentylamine (yield: 39.5%).

Example 175

2-(1H-indol-5-yl)-5-[(4-methyl-2,3,4,5-tetrahydro-1H-1,5-benzodiazepin-1-yl)carbonyl]anilinewas obtained by the same procedure as in Example 14 using the compound in Reference Example 120 (yield: 49.7%).

Example 176

Using the compound in Reference Example 123, the same procedure as in Example 1 was conducted to obtain a compound. Using the resulting compound, 2-(1H-indol-5-yl)-5-[(4-isopropyl-2,3,4,5-tetrahydro-1H-1,5-benzodiazepin-1-yl)carbonyl]aniline was obtained by the same procedure as in Example 12 (yield: 84.3%).

Example 177

Using the compound in Reference Example 124, the same procedure as in Example 1 was conducted to obtain a compound. Using the resulting compound, 5-[(4-ethyl-2,3,4,5-tetrahydro-1H-1,5-benzodiazepin-1-yl)carbonyl]-2-(1H-indol-5-yl)aniline (quantitative yield) was obtained by the same procedure as in Example 14.

Example 178

2-(1H-indol-5-yl)-5-[[(4S)-4-methyl-2,3,4,5-tetrahydro-1H-1,5-benzodiazepin-1-yl]carbonyl]aniline was obtained by the same procedure as in Example 14 using the compound in Reference Example 138 (yield: 67.2%).

Example 179

2-(1H-indol-5-yl)-5-[[(4R)-4-methyl-2,3,4,5-tetrahydro-1H-1,5-benzodiazepin-1-yl]carbonyl]aniline was obtained by the same procedure as in Example 14 using the compound in Reference Example 140 (yield: 63.1%).

Example 180

2-(1H-indol-5-yl)-5-[[(4S)-4-methyl-2,3,4,5-tetrahydro-H-1,5-benzodiazepin-1-yl]carbonyl]phenol was obtained by the same procedure as in Example 1 using the compound in Reference Example 165 (yield: 78.7%).

Example 181

2-(1H-indol-5-yl)-5-[[(4R)-4-methyl-2,3,4,5-tetrahydro-H-1,5-benzodiazepin-1-yl]carbonyl]phenol was obtained by the same procedure as in Example 14 using the compound in Reference Example 168 (yield: 90.8%).

Example 182

2-(1H-indol-5-yl)-5-[[4-methyl-2,3,4,5-tetrahydro-1H-1,5-benzodiazepin-1-yl]carbonyl]phenol was obtained by

the same procedure as in Example 14 using the compound in Reference Example 171 (yield: 83.6%).

Example 183

5-[(4-ethyl-2,3,4,5-tetrahydro-1H-1,5-benzodiazepin-1-yl)carbonyl]2-(1H-indol-5-yl)-phenol was obtained by the same procedure as in Example 14 using the compound in Reference Example 173 (yield: 80.1%).

Structures, MS data, NMR data and IR data of the compounds of the above Examples are shown in the following tables. [NMR] in the table is determined by using $(CH_3)_4Si$ as an internal standard.

Table 1-1

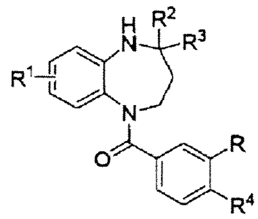

(1)

| 実施例 | $R^1$ | $R^2$ | $R^3$ | $R^4$ | R |
|---|---|---|---|---|---|
| 1 | H | $-CH_3$ | H | 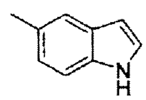 | $OCH_3$ |
| 2 | " | " | $-CH_3$ | " | " |
| 3 | " | " | H | " | $NHCOCH_3$ |
| 4 | " | $CH_3$ | " | " | H |
| 5 | " | $-CH_3$ | " | " | " |
| 6 | " | $-CH_3$ | " | " | $NHSO_2CH_3$ |
| 7 | " | 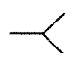 | " | " | $NHCOCH_3$ |
| 8 | " | $-CH_2CH_3$ | " | " | " |
| 9 | " | $-CH_3$ | " | " | 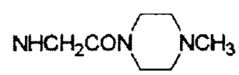 |
| 10 | " | " | " | " | 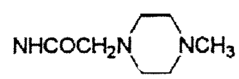 |
| 11 | " | " | " | " | $NHCH_3$ |
| 12 | " | " | " | " | $CH_2NH_2$ |
| 13 | " | " | " | " | $NHCOOCH_3$ |
| 14 | " | " | " | " | NHCOH |
| 15 | " | " | " | " | $NHCONH_2$ |
| 16 | " | 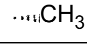 | " | " | $NHCOCH_3$ |
| 17 | " | 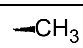 | " | " | " |
| 18 | " | $-CH_2OH$ | " | " | " |
| 19 | " | $-CH_3$ | " | " | $NHCOCH_2NH_2$ |
| 20 | " | " | " | " | $NHCOCH_2N(CH_3)_2$ |
| 21 | " | " | " | " | $NHCOCH_2OH$ |

(continued)

| 実施例 | $R^1$ | $R^2$ | $R^3$ | $R^4$ | R |
|---|---|---|---|---|---|
| 22 | " | - $CH_2N(CH_3)2$ | " | " | $NHCOCH_3$ |
| 23 | " | $-CH_3$ | " | " | $CH_2OH$ |
| 24 | " | " | " | " | $O(CH_2)_2NH_2$ |
| 25 | $8{\cdot}NH_2$ | " | " | " | $NHCOCH_3$ |
| 26 | $7{\cdot}CH_3$ | " | " | " | " |
| 27 | 7·OH | " | " | " | " |
| 28 | H | " | " | " | $NHCOCH_2CH_3$ |
| 29 | " | $-CH_2CH_3$ | " | " | $NHCOCH_2OH$ |
| 30 | " | $-CH_3$ | " | " | $O(CH_2)_3NH_2$ |

Table 1-2

| | | | | | |
|---|---|---|---|---|---|
| 31 | 〃 | " | " | " | $NHCOCH_2OCH_3$ |
| 32 | " | " | " | " | $NHCO(CH_2)_2OCH_3$ |
| 33 | " | " | " | " | $O(CH_2)_2OCH_3$ |
| 34 | " | " | " | " | $O(CH_2)_2OH$ |
| 35 | " | " | " | " | $O(CH_2)_2NHCOCH_3$ |
| 36 | " | " | " | " | $O(CH_2)_4NH_2$ |
| 37 | " | " | " | " | $O(CH_2)_2NH_2$ |
| 38 | " | $\blacktriangleleft CH_3$ | " | " | $O(CH_2)aNH_2$ |
| 39 | " | $-CH_3$ | " | " | $O(CH_2)_6NH_2$ |
| 40 | " | $-CH_2CH_3$ | " | " | $O(CH_2)_3NH_2$ |
| 41 | " | $-CH_3$ | " | " | $O(CH_2)_3NHCH_3$ |
| 42 | " | " | " | " | $O(CH_2)_3N(CH_3)_2$ |
| 43 | " | " | " | " | 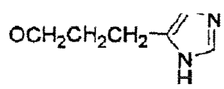 |
| 44 | " | " | " | " | $NH(CH_2)_3NH_2$ |
| 45 | " | " | " | " | $O(CH_2)_2CH_3$ |
| 46 | " | 〃 | " | " | 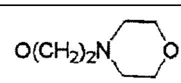 |
| 47 | " | $\blacktriangleleft CH_3$ | " | " | $OCH_3$ |
| 48 | " | " | " | " | $NHSO_2CH_3$ |
| 49 | " | " | " | " | $N(CH_2)COCH_3$ |
| 50 | " | " | " | 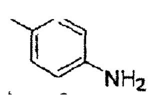 | $OCH_3$ |
| 51 | 〃 | 〃 | 〃 | 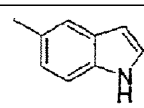 | $O(CH_2)_3NHCH_3$ |

(continued)

| | | | | | |
|---|---|---|---|---|---|
| 52 | " | " | " | " | 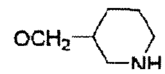 |
| 53 | " | $-CH_3$ | " | " | 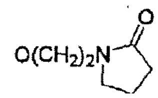 |
| 54 | " | $CH_3$ | " | 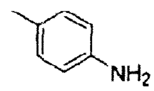 | $O(CH_2)_3NH_2$ |
| 55 | " | $CH_3$ | " | 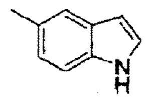 | $O(CH_2)_3NH_2$ |
| 56 | " | $-CH_3$ | " | " | 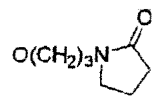 |
| 57 | " | " | " | " | $O(CH_2)_3NH(CH_2)_2OH$ |
| 58 | " | " | " | " | $O(CH_2)_3N(CH_2CH_2OH)_2$ |

Table 1-3

| | | | | | |
|---|---|---|---|---|---|
| 59 | " | $CH_3$ | " | " | 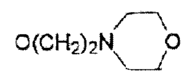 |
| 60 | " | " | " | 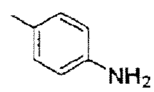 | " |
| 61 | " | " | " | 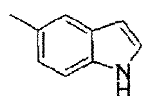 | 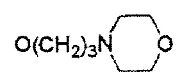 |
| 62 | " | $-CH_3$ | " | " | 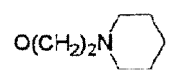 |
| 63 | " | " | " | " | 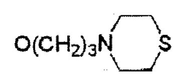 |
| 64 | " | " | " | " | 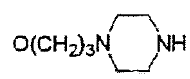 |
| 65 | " | " | " | " | 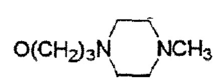 |
| 66 | " | $CH_3$ | " | " | 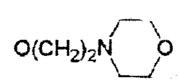 |
| 67 | " | $CH_3$ | " | " | 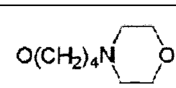 |

(continued)

| | | | | | |
|---|---|---|---|---|---|
| 68 | " | $-CH_3$ | " | " | 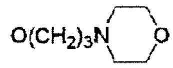 |
| 69 | " | $CH_3$ | " | " | 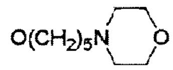 |
| 70 | " | " | " | " | 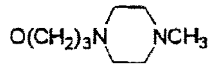 |
| 71 | " | " | " | " | 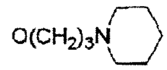 |
| 72 | " | " | " | " | 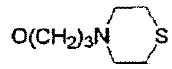 |
| 73 | " | " | " | " | 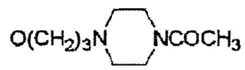 |
| 74 | " | " | " | 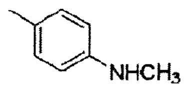 | 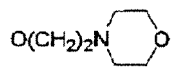 |
| 75 | " | " | " | 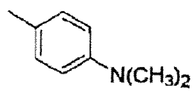 | " |
| 76 | " | " | " | 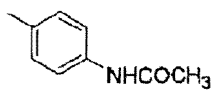 | " |
| 77 | " | " | " | 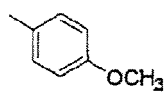 | " |
| 78 | " | " | " | 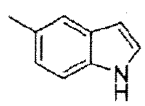 | $O(CH_2)_3N(CH_3)_2$ |

Table 1-4

| | | | | | |
|---|---|---|---|---|---|
| 79 | " | " | " | " | $O(CH_2)_3N(CH_2CH_2CH_3)_2$ |
| 80 | " | " | " | " | 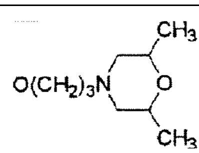 |
| 81 | " | " | " | " | 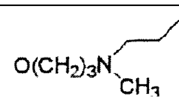 |
| 82 | " | " | " | " | 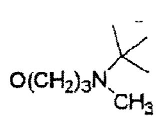 |

(continued)

| | | | | | |
|---|---|---|---|---|---|
| 83 | " | " | " | " | 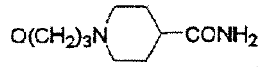 |
| 84 | " | " | " | " | $O(CH_2)_3N(CH_2CH_2OCH_3)_2$ |
| 85 | " | " | " | " | 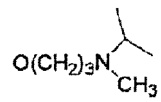 |
| 86 | " | " | " | " | 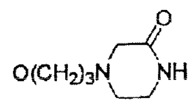 |
| 87 | " | " | " | " | 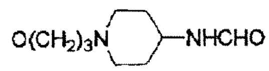 |
| 88 | " | " | " | " | 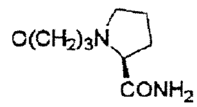 |
| 89 | " | " | " | " | 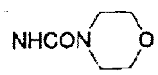 |
| 90 | " | " | " | " | 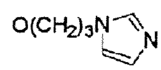 |
| 91 | " | " | " | " | 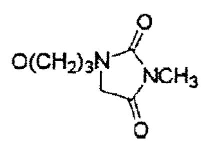 |
| 92 | " | " | " | " | 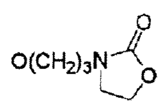 |
| 93 | " | " | " | " | 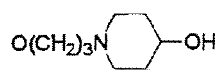 |
| 94 | " | " | " | " | 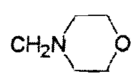 |
| 95 | " | " | " | " | 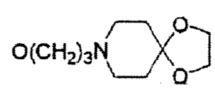 |
| 96 | " | " | " | " | 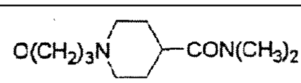 |

Table 1-5

| | | | | | |
|---|---|---|---|---|---|
| 97 | " | " | " | " | 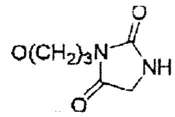 |
| 98 | " | " | " | " | 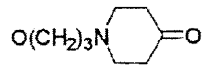 |
| 99 | " | " | " | " | 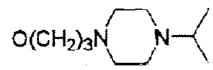 |
| 100 | " | " | " | " | 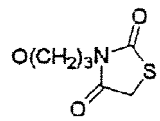 |
| 101 | " | " | " | " | 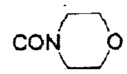 |
| 102 | " | " | " | " | 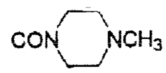 |
| 103 | " | " | " | " | 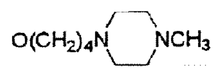 |
| 104 | 7-$CH_3$ | -$CH_3$ | " | " | 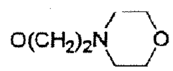 |
| 105 | H | -$CH_3$ | " | 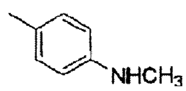 | 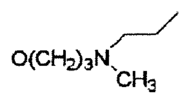 |
| 106 | " | " | " | " | 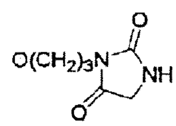 |
| 107 | " | " | " | 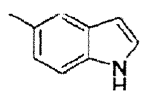 | 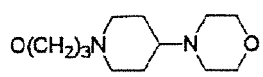 |
| 108 | " | " | " | " | 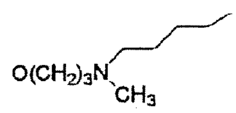 |
| 109 | " | " | " | " | 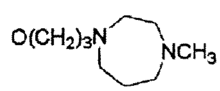 |
| 110 | " | " | " | " | 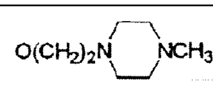 |
| 111 | " | " | " | 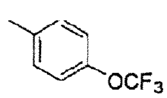 | 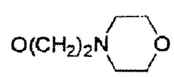 |

(continued)

| | | | | | |
|---|---|---|---|---|---|
| 112 | " | " | " | 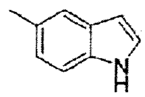 | 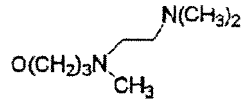 |
| 113 | " | " | " | | 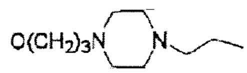 |

Table 1-6

| | | | | | |
|---|---|---|---|---|---|
| 114 | " | " | " | " | 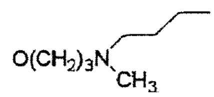 |
| 115 | " | " | " | " | 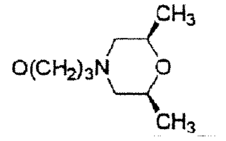 |
| 116 | " | " | " | " | $O(CH_2)_3N(CH_2CH_3)_2$ |
| 117 | " | " | " | " | 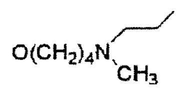 |
| 118 | " | " | " | " | 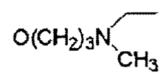 |
| 119 | " | " | " | 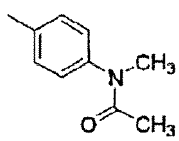 | 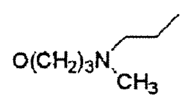 |
| 120 | " | " | " | N | $COO(CH_2)_3N(CH_3)_2$ |
| 121 | " | " | " | H " | 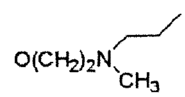 |
| 122 | " | " | " | " | 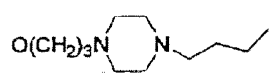 |
| 123 | " | " | " | " | 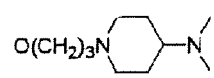 |
| 124 | " | " | " | " | 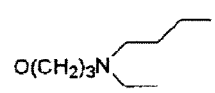 |
| 125 | " | " | " | " | 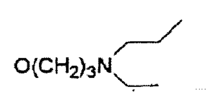 |
| 126 | " | " | " | " | 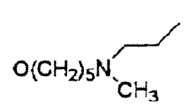 |

(continued)

| | | | | | |
|---|---|---|---|---|---|
| 127 | " | $\cdots CH_3$ | " | " | 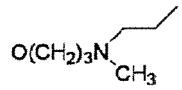 |
| 128 | " | " | " | " | 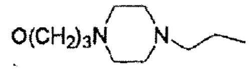 |
| 129 | " | $-CH_3$ | " | 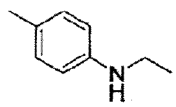 | 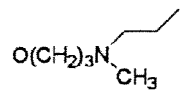 |
| 130 | " | $\cdots CH_3$ | " | 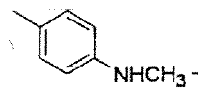 | " |

Table 1-7

| | | | | | |
|---|---|---|---|---|---|
| 131 | " | $-CH_3$ | " | 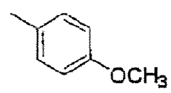 | " |
| 132 | " | " | " | 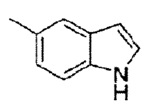 | 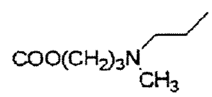 |
| 133 | " | " | " | 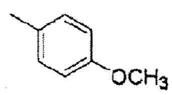 | $COO(CH_2)_3N(CH_3)_2$ |
| 134 | " | " | " | 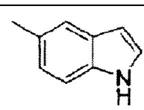 | 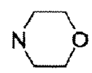 |
| 135 | " | " | " | " | 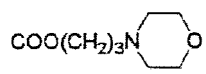 |
| 136 | " | " | " | " | 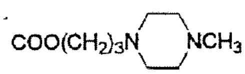 |
| 137 | " | " | " | " | 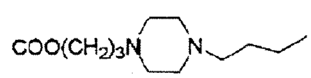 |
| 138 | " | " | " | " | 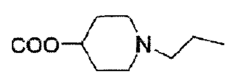 |
| 139 | " | " | " | " | 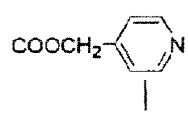 |

(continued)

| | | | | | |
|---|---|---|---|---|---|
| 140 | " | " | " | " | 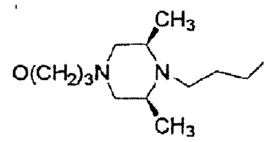 |
| 141 | " | " | " | " | 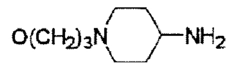 |
| 142 | " | " | " | " | 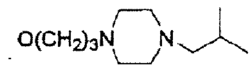 |
| 143 | " | " | " | 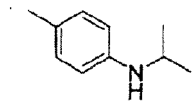 | 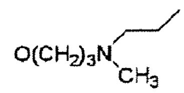 |
| 144 | " | " | " | 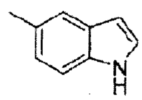 | $COO(CH_2)_2N(CH_3)_2$ |
| 145 | " | " | " | " | 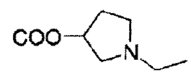 |
| 146 | " | " | " | " | 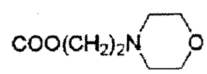 |
| 147 | " | " | " | " | 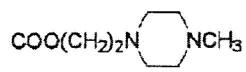 |
| 148 | " | " | " | " | 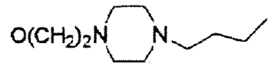 |

Table 1-8

| | | | | | |
|---|---|---|---|---|---|
| 149 | " | " | " | " | 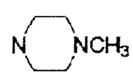 |
| 150 | " | $-CH_2CH_3$ | " | " | 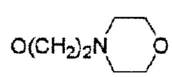 |
| 151 | " | $-CH_3$ | " | " | 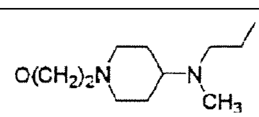 |
| 152 | " | " | " | " | 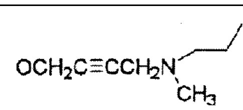 |

(continued)

| | | | | | |
|---|---|---|---|---|---|
| 153 | " | " | " | " | 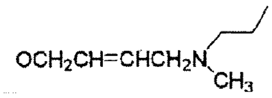 |
| 154 | " | " | " | 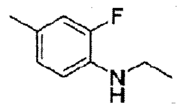 | 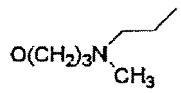 |
| 155 | " | " | " | 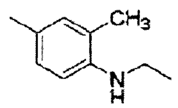 | " |
| 156 | " | " | " | 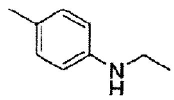 | 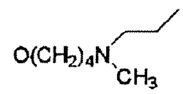 |
| 157 | " | " | " | 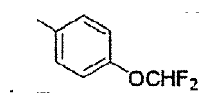 | 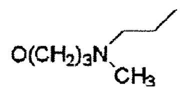 |
| 158 | " | " | " | 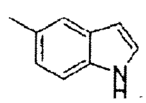 | CO $(CF_2)_4N(CH_3)_2$ |
| 159 | " | " | " | 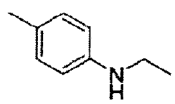 | 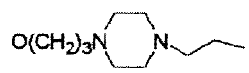 |
| 160 | " | " | " | " | 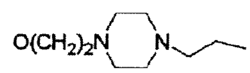 |
| 161 | " | " | " | 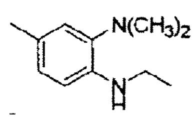 | 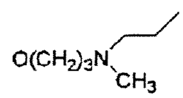 |
| 162 | " | " | " | 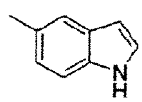 | COO $(CH_2)_4N(CH_3)_2$ |
| 163 | " | " | " | 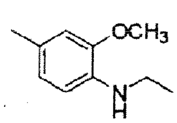 | 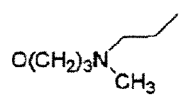 |

Table 1-9

| | | | | | |
|---|---|---|---|---|---|
| 164 | " | " | " | 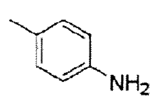 | " |
| 165 | " | " | " | 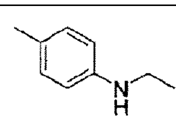 | 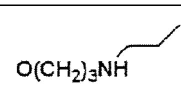 |

(continued)

| | | | | | |
|---|---|---|---|---|---|
| 166 | " | " | " | 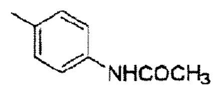 | 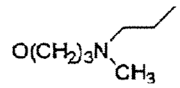 |
| 167 | " | $-CH_3$ | " | 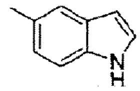 | 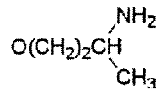 |
| 168 | " | " | " | " | $O(CH_2NHCOCH_2NH_2$ |
| 169 | " | " | " | " | $O(CH_2)_3NHCOCH_2NH_2$ |
| 170 | " | ◄$CH_3$ | " | " | 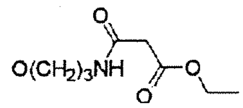 |
| 171 | " | " | " | " | 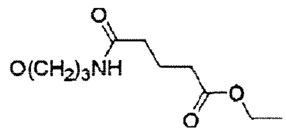 |
| 172 | " | " | " | " | 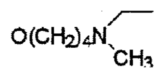 |
| 173 | " | " | " | " | 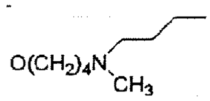 |
| 174 | " | " | " | " | 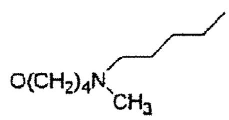 |
| 175 | " | $-CH_3$ | " | " | $NH_2$ |
| 176 | " | 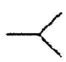 | " | " | " |
| 177 | " | $-CH_2CH_3$ | " | " | " |
| 178 | " | ····$CH_3$ | " | " | " |
| 179 | " | ◄$CH_3$ | " | " | " |
| 180 | " | " | " | " | OH |
| 181 | " | ····$CH_3$ | " | " | " |
| 182 | " | $-CH_3$ | " | " | " |
| 183 | " | $-CH_2CH_3$ | " | " | " |

Table 2-1

| Exam. | Spectrum data | |
|---|---|---|
| 1 | $^1$H-NMR($CDCl_3$) δ : 2.38(3H,s), 6.59-6.64(1H, m),7.14-7.20(1H,m), 7.26-7.29(1H,m), 7.36-7.47 (2H,m), 7.60(1H,s), 7.95-8.02(1H,m), 8.05(1H, s), 8.24(1H,brs) ppm FABMS:251(M+1) IR(KBr): 3328, 2958, 1615, 1595, 1415, 1323, 1135 $cm^{-1}$ | 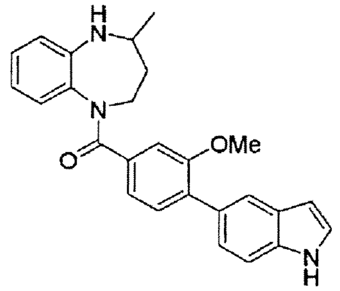 |
| 2 | $^1$H-NMR(DMSO-$d_6$, 120˚C)δ:1.24(6H,s), 1.76 (2H,t,J=5.9Hz), 3.49 (3H,s,), 3.67-3.99(2H,m), 4.59(1H,s), 6.36-6.43(1H,m), 6.52-6.62 (1H,m), 6.65(1H,d,*J*=7.8Hz), 6.83(1H,d,*J*=2.0Hz), 6.87-6.99(3H,m), 7.09(2H,d,*J*=7.8Hz). 7.24(1H,t,J= 2.9Hz), 7.34(1H,t,J=7.8Hz), 7.52 (1H,s). 10.66(1 H,brs), 7.26-7.29(1H,m), 7.36-7.47(2H,m), 7.60(1H, s)ppm FABMS:425(M+1) IR(KBr):3350, 2962, 1613, 1593, 1417, 1319, 1265$cm^{-1}$ | 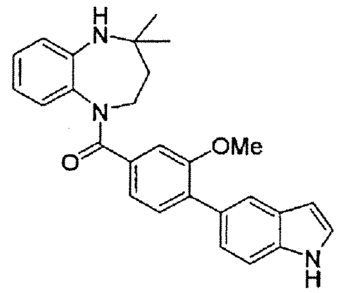 |
| 3 | $^1$H-NMR(DMSO-$d_6$,120˚C) δ: 1.24(3H,d,*J*=6.4Hz), 1.59-1.72(1H,m), 1.77(3H,s), 1.89-198(1H,m), 3.39-3.63(2H,m), 4.03-4.13(1H,m), 4.84(1H,s), 6.41-6.43(1H,m), 6.45-6.51(1H,m), 6.65(1H,d,*J*=8.3Hz), 6.90-7.07(5H,m), 7.27-7.29(1H,m), 7.40(1H,d,*J*=8.3Hz), 7.45(1H,s), 7.77(1H,s), 8.36(1H,s)ppm FABM:439(M+1) IR(KBr):3402, 1620, 1562, 1423, 1323$cm^{-1}$ | 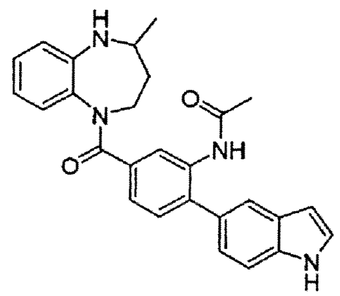 |

Table 2-2

| | | |
|---|---|---|
| 4 | $^1$H-NMR(DMSO-$d_6$,120˚C) δ : 1.26(3H,d,*J*=6.4Hz), 1.60-1.73(1H, m), 1.92-2.06(1H,m), 3.45(2H,br), 4.09(1H,br), 4,09(1H,br), 4.87(1H, br), 6.41-6.47(2H,m), 6.60(1H,d,*J*=7.3Hz), 6.87-6.94(2H,m), 7.24-7.28(4H,m), 7.30-7.46(3H,m), 7.73(1H,s)ppm FABMS:383 (M+1) IR(KBr):3322, 2930, 1622, 1408, 1323 $cm^{-1}$ | 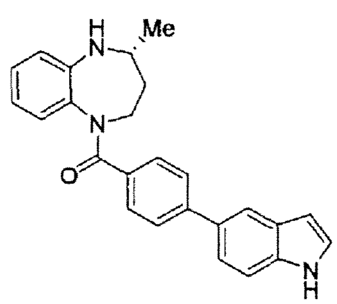 |

(continued)

| | | |
|---|---|---|
| 5 | $^1$H-NMR(DMSO-ds,120°C) δ : 1.36(d,3H,*J*=6.3Hz), 1.7-1,84(m,1H), 2.0-2.06(m,1H), 3.5-3.8(br,2H), 4.1-4.3(br,1H), 4.97(s,1H), 6.52-6,58(m,2H), 6.71(d,1H,*J*=7.3Hz), 6.97-7.05(m,2H), 7.36-7.42 (m,4H), 7.51-7.56(m,3H), 7.83(s,1H)ppm FABMS:383(M+1) IR (KBr):3326, 2934, 1622, 1560, 1408, 1323, 1255, 1112cm$^{-1}$ | 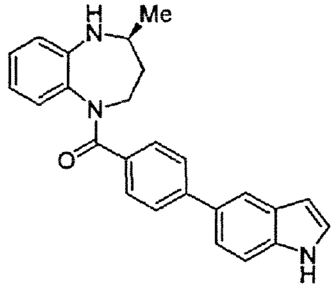 |
| 6 | $^1$H-NMR(DMSO-$d_6$, 120°C) δ: 1.25(3H,d,*J*=6.4Hz), 1.60-1.73(1H,m), 1.96(1H,br), 2.63(3H,s), 3.47(2H,br), 4.03-4.06(1H,m), 4.86(1H,s), 6.43-6.52(2H,m), 6.65(1H, d, *J* =7.3Hz), 6.91-6.92(2H,m), 7.02-7.12 (3H,m), 7.29-7.31(1H,m), 7.41-7.54(3H,m), 7.86(1H,br)ppm FABMS:475(M+1) IR(KBr):3356, 2364, 1628, 1562, 1419, 1323, 1156cm$^{-1}$ | 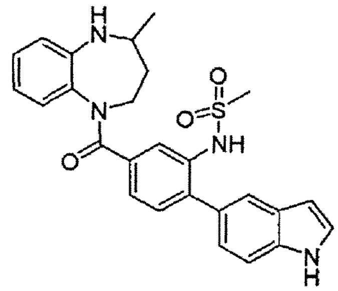 |

Table 2-3

| | | |
|---|---|---|
| 7 | $^1$H-NMR($CDCl_3$) δ :0.92-1.10(6H,m), 1.69-2.00(4H,m), 3.14(1H,brs) 3.55-4.20(2H,m), 4.71(1H,s), 6.40-6.52(2H,m) 6.64(1H,d,*J*=7.8Hz), 6.86-7.12(5H,m), 7.26-7.32(1H,m) 7.38-7.48(2H,m), 7.67-7.73(1H, m), 8.35(1H,brs), 10.75(1H,brs)ppm FABMS:466(M+1) IR(KBr): 3406, 2962, 1626, 1564, 1423, 1319cm$^{-1}$ | 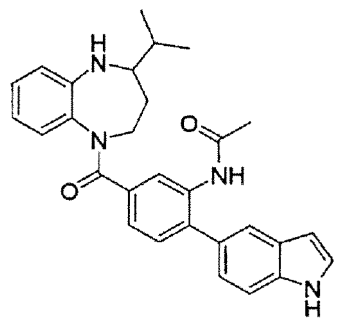 |
| 8 | $^1$H-NMR(DMSO-$d_6$,120°C) δ :0.99(3H,t,J=7.8Hz), 1.50-1.75(3H,m), 1.77(3H,s), 1.92-2,08(1H,m), 3.15-3.32(1H,m), 3.55-4.25(2H,m), 4.81(1H,s), 6.41-6.53(2H,m), 6.65(1H,d,*J*=7.8Hz), 6.88-7.09(5H,m), 7.26-7.32(1H,m), 7.38-7.48(2H,m), 7.66-7.71(1H,m), 8.36(1H,brs), 10.75(1H,brs)ppm FABMS:452(M+1) IR(KBr):3406, 2964, 1624, 1562, 1423, 1319, 1251 cm$^{-1}$ | 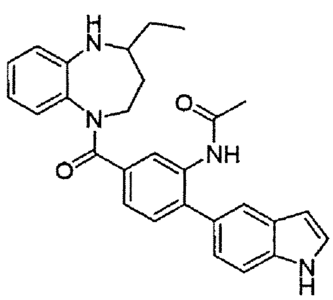 |

(continued)

| | | |
|---|---|---|
| 9 | $^1$H-NMR(DMSO-$d_6$, 120˚C) δ : 1.25(3H,d,*J*=6.4Hz), 1.59-1.73(1H, m), 1.92-1.96(1H,m), 2.21(3H,s), 2.31(4H,t,*J*=5.1Hz), 3.40(4H,t, *J*=5.1Hz), 3.62(2H,d,*J*=4.9Hz), 3.38-3.72(2H,m), 4.06(1H,br), 4.85 (1H,s), 5.04(1H,t,*J*=4.4Hz), 6,40-6.42(1H,br), 6.47-6.53(2H,m), 6.61-6.67(2H,m), 6.85(1H,d,*J*=7.8Hz), 6.92(2H,d,*J*=3.4Hz), 7.00 (1H,dd,*J*=8.3,2.0Hz), 7.28-7.29(1H,m), 7.40-7.44(2H,m)ppm FABMS:537(M+1) IR(KBr):3404, 2936, 1638, 1431, 1321, 1294 1257cm$^{-1}$ | 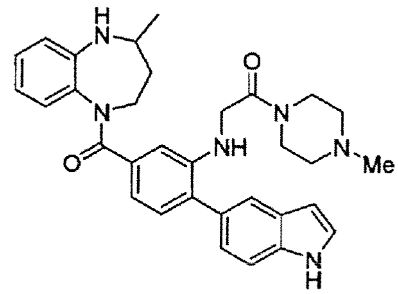 |

Table 2-4

| | | |
|---|---|---|
| 10 | $^1$H-NMR(DMSO-$d_6$,120˚C) δ : 1.36(3H,d,*J*=6.4Hz), 1.78-1.84 (5H,m), 1.94(3H,s), 2.03-2.18(2H,m), 2.30-2.33(4H,m), 3,57-3.74(2H,m), 4.12-4.29(1H,m) 4.92(1H,br), 6.55-6.64(2H, m), 6.76(1H,d,*J*=7.8Hz), 6.95-7.09(5H,m), 7.43-7.61(3H,m), 8.55(1H,d,*J*=1.5Hz), 9.38(1H,br), 10.97(1H,br)ppm FABMS: 436 (M+1) IR(KBr):3422, 2942, 1628, 1562, 1432, 1321cm$^{-1}$ | 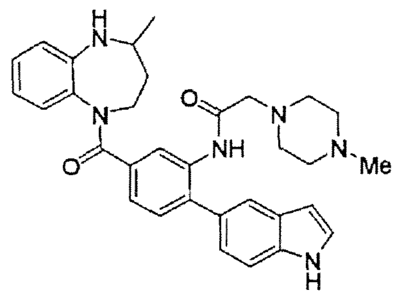 |
| 11 | $^1$H-NMR(DMSO-$d_6$,120˚C) δ : 1.25(3H.d,*J*=6.3Hz), 1.64-1.72 (1H,m), 1.91(1H,br), 2.52(3H,d,*J*=5.4Hz),3.44(2H,br), 4.11-4.24 (2H,m), 4.85(1H,br), 6.40-6.66(5H,m), 6.81(1H,d,*J*=7.3Hz), 6.91-6.97(3H,m), 7.26-7.28(1H,m), 7.40-7.42(2H,m) FABMS: 411(M+1) IR(KBr):3322, 2928, 1613, 1564, 1417, 1321, 1257cm$^{-1}$ | 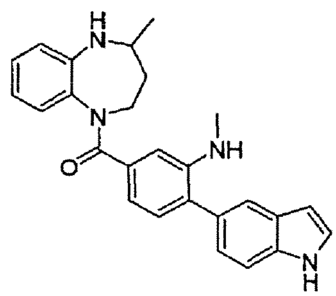 |
| 12 | $^1$H-NMR(DMSO-$d_6$,120˚C) δ: 1.26(3H,d,*J*=6.4Hz), 1.65-1.74 (1H,m), 1.92(1H,br), 3.35-3.54(2H,m), 3.54(2H,s), 4.13(1H,br), 4.87(1H,s), 6.41-6.63(3H,m), 6.80-7.00(4H,m), 7.09-7.12(1H, m), 7.27-7.40(4H,m)ppm FABMS:475(M+1) IR(KBr):3348, 2972, 1624, 1419, 1321, 1257cm$^{-1}$ | 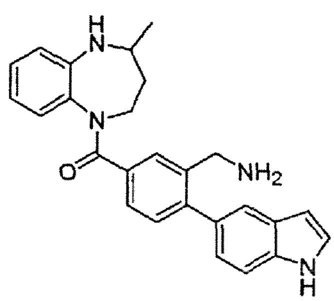 |

Table 2-5

| | | |
|---|---|---|
| 13 | $^1$H-NMR(DMSO,120°C) δ: 1.25(3H,d,*J*=5.9Hz), 1.58-1.74(1H,m), 1.86-2.01(1H,m), 3.32-3.72(5H,m), 3,95-4.18(1H,m), 4.84(1H, brs), 6.39-6.52(2H,m), 6.65(1H,d,J=7.8Hz), 6.68-7.04(5H,m), 7.30 (1H,t,J=2.0Hz), 7.38-7.44(2H,m) 7.49-7.55(1H,br), 7.68(1H,brs), 10.78(1H,brs)ppm FABMS:455(M+1) IR(KBr):3408, 2962,1725, 1626, 1568, 1425, 1321, 1216, 1067cm$^{-1}$ | 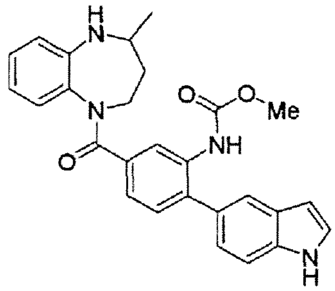 |
| 14 | $^1$H-NMR(DMSO,120°C) δ : 1.25(3H,d,*J*=6.8Hz), 1.58-1.74(1H,m), 1.86-2.04(1H,m) 3.22-3.85(2H,m), 3.92-4.24(1H,m), 4.87(1H, brs), 6.42-6.53(2H,m), 6.65(1H,d,*J*=7.8Hz), 6.87-7,08(5H,m), 7.26-7.32(1H,m), 7.39-7.46(2H,m), 7.62-7.79(1H,br), 8.02-8.07 (1H,m),8.54-8.65(1H,br), 10.78(1H,brs)ppm FABMS:425(M+1) IR (KBr):3352, 2932, 1686, 1626, 1425, 1323cm$^{-1}$ | 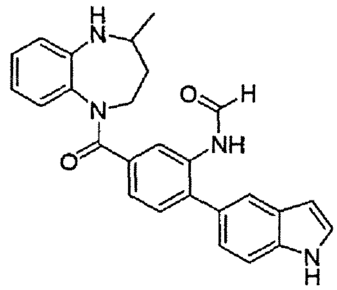 |
| 15 | $^1$H-NMR(DMSO, 120°C) δ: 1.25(3H,d,*J*=6.8Hz), 1,58-1.71(1H,m), 1.77(3H,s), 1.36-2.02(1H,m), 3.32-3.73(2H,m), 3.92-4.17(1H,m), 4.84(1H,brs), 6.41-6.52(2H,m), 6.62-6.68(1H,m), 6.87-7.08(5H, m), 7.28(1H,t,*J*=2.9Hz), 7.37-7.45(2H,m), 7.66(1H,s), 8.36(1H, brs), 10,75(1H,brs)ppm FABMS:439(M+1) IR(KBr):3406, 2924, 1626, 1562, 1423, 1321, 1112cm$^{-1}$ | 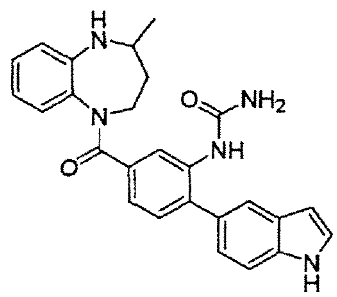 |

Table 2-6

| | | |
|---|---|---|
| 16 | $^1$H-NMR(DMSO-d6,120°C) δ: 1.24(3H,d,*J*=6.4Hz), 1.59-1.72(1H, m), 1.77(3H,s), 1.89-1.98(1H,m), 3.39-3.63(2H,m), 4.03-4.13(1H, m), 4.84(1H,s),6.41-6.43(1H,m), 6.45-6.51(1H,m), 6.65(1H,d, *J*=8.3Hz), 6.90-7.07(5H,m), 7.27-7.29(1H,m), 7.40(1H,d, *J*=8.3Hz), 7.45(1H,s), 7.77(1H,s), 8.36(1H,s)ppm FABMS: 439 (M+1) IR(KBr):3334, 1684, 1628, 1421, 1323, 1255cm$^{-1}$ | 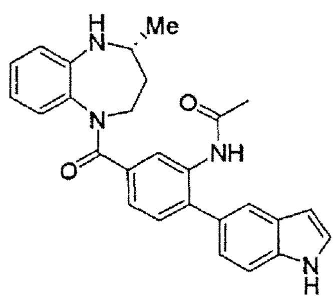 |

(continued)

| | | |
|---|---|---|
| 17 | $^1$H-NMR(DMSO-$d_6$,120˚C) δ :1.25(3H,d,*J*=6.8Hz), 1.60-1.77(1H, m), 1.77(3H,s), 1.87-2.05(1H,m), 7.27-7.31(1H,m), 7.40(1H,d, J=7.8Hz), 7.45(1H,brs), 7.67(1H,brs), 8.36(1H,brs), 10.75(1H,brs) ppm FABMS:439(M+1) IR(KBr):3334, 1684,1628, 1421, 1323, 1255cm$^{-1}$ | 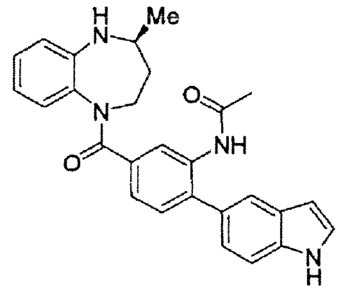 |
| 18 | $^1$H-NMR(DMSO-d6,120˚C) δ : 1.52-1.83(4H,m), 1.85-2.02(1H,m), 2.49-4.38(6H,m), 6,38-6.55(2H,m), 6.64-6.71(1H,m), 6.84-7.07 (5H,m), 7.24-7.31(1H,m), 7.35-7.47(2H,m), 7.62-7.68(1H,m), 8.36 (1H,brs), 10.75(1H,brs)ppm FABMS:455(M+1) IR(KBr):3404, 2934, 1622, 1564, 1423, 1319cm$^{-1}$ | 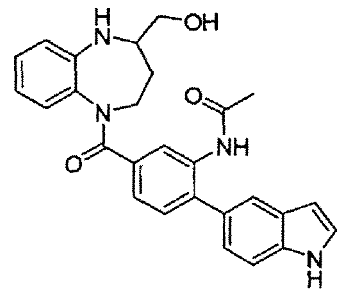 |

Table 2-7

| | | |
|---|---|---|
| 19 | $^1$H-NMR(DMSO-d6,120˚C) δ: 1.25(3H.d,*J*=5.9Hz), 1.56-1.76 (1H,m), 1.86-2.04(1H,m), 3.12(2H,s), 3.28-8.83(2H,m), 3.88-4.25(1H,m), 4.82(2H,brs), 6,39-6.54(2H,m), 6.61-6.69 (1H,m), 6.83-7.04(5H,m), 7,25-7.33(1H,m), 7.38-7.49(2H,m), 8.31(1H,brs), 10.77(1H,brs)ppm FABMS: 454(M+1) IR(KBr): 3430, 1673, 1622,1564,1423, 1323, 1257cm$^{-1}$ | 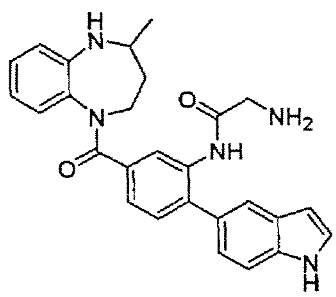 |
| 20 | $^1$H-NMR(DMSO-$d_6$,120˚C) δ: 1.25(,3H,d,*J*=6.8Hz), 1.58-1.74 (1H,m), 1.84-2.04(6H,m), 3.32-3.80(2H,m), 3.91-4.22(1H,m), 4.82(1H,brs), 6.41-6.54(2H,m), 6.61-6.69(1H,m), 6.84-6.92 (3H,m), 6.93-6.99(1H,m), 7.00-7.05(1H,m), 7.29-7.34(1H,m), 7.38-7.52(3H,m), 8.28(1H,d,*J*=2.0Hz), 9.27(1H,brs), 10.80(1H, brs)ppm FABMS:482(M+1) IR(KBr):3320, 2948, 1626, 1564,1421, 1257cm$^{-1}$ | 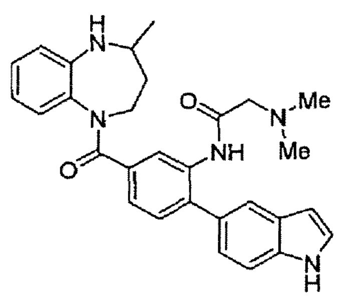 |

(continued)

| | | |
|---|---|---|
| 21 | $^1$H-NMR(DMSO-$d_6$,120˚C) δ: 1.62(3H,d,*J*=5.9Hz), 1.56-1.74 (1H,m), 1.88-2.04(1H,m), 3.34-3.88(4H,m), 3.98-4.18(1H,m), 4.75-4.88(1H,br), 5.08-5.36(1H,br), 6,41-6.63(2H,m), 6.60-6.70(1H,m), 6.84-6.94(3H,m), 6.95-7.05(2H,m), 7.27-7.34(1H,m), 7.40-7.50(1H,m), 8.34(1H,brs), 8.83(1H, brs), 10.81(1H,brs)ppm FABMS:455(M+1) IR(KBr): $cm^{-1}$ | 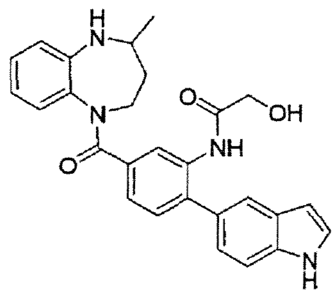 |

Table 2-8

| | | |
|---|---|---|
| 22 | $^1$H-NMR(DMSO-d6,120˚C) δ: 1.45-1.65(m,1H), 1.85(3H,s), 2.05-2.20(2H,m), 2.19(6H,s), 2.30-2.40(1H,m), 3.10-3.25(1H,m), 3.50-3.65(1H,m.), 4.90-5.10(1H,m), 6.88(1H,s), 6.35(1H, t*J*=7.3Hz), 6.52(1H,d,J=7.3Hz), 6.67(1H,d,*J*=7.3Hz), 6.87-7.00 (4H,m), 7.30-7.45(3H,m), 7.62(1H,s), 8.96(1H,s)ppm FABMS:454 (M+1) IR(KBr):3402, 1630, 1506, 1451, 1348, 1317$cm^{-1}$ | 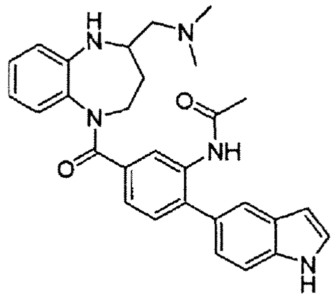 |
| 23 | $^1$H-NMR(DMSO-$d_6$, 120˚C) δ: 1.25(H,d,*J*=5.9Hz), 1.56-1.77(1H, m), 1.88-2.03(1H,m), 3.34-3.78(2H,m), 3.94-4.24(1H,m), 4.33(2H, d,*J*=5.9Hz), 4.84(1H,brs), 6.38-6.53(2H,m), 6.62(1H,d,*J*=7.8Hz), 6,85-7.08(5H,m), 7.28(1H,t,*J*=2.9Hz), 7.38(2H,d,*J*=8.8Hz), 7.53-7.60(1H,m)ppm FABMS:412(M+1) IR(KBr):3410, 2932, 1620, 1417, 1323, 1257$cm^{-1}$ | 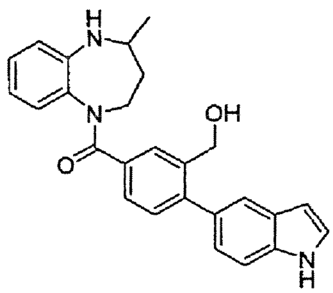 |
| 24 | $^1$H-NMR(DMSO-$d_6$, 120˚C) δ: 1.26(3H,d,*J*=5.9Hz), 1.58-1.76(1H, m), 1.85-2.01(1H,m), 2.62-2.72(2H,m), 3.26-3.73(4H,m), 3.91-4.26(1H,m), 4.80-5.02(1H,m), 6.36-6.42(1H,m), 6.44-6.54 (1H,m), 6.63(1H,d,*J*=6.8Hz), 6.84-6.99(4H,m), 7,08-7.19(2H,m), 7.24(1H,t,*J*=1.9Hz), 7.35(1H,d,*J*=8.8Hz), 7.53-7.61(1H,m), 10.66 (1H,brs)ppm FABMS:441(M+1) IR(KBr):3306, 2934, 1622, 1560, 1419, 1321, 1253$cm^{-1}$ | 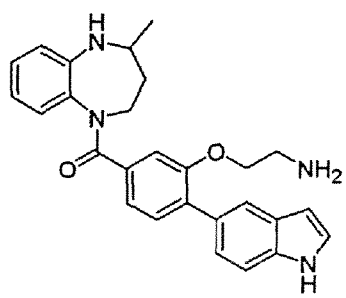 |

Table 2-9

| | | |
|---|---|---|
| 25 | $^1$H-NMR(DMSO-d6,120˚C) δ: 1.19(3H,d,*J*=6.4Hz), 1.50-1.64 (1H,m), 1.78(3H,s), 1.78·1.85(1H,m), 3.09(2H,br), 4.03-4.25(4H, m), 6.08(1H,d, *J*=2.4Hz), 6.29(1H,dd,*J*= 8.3,2.4Hz), 6.42-6.43 (1H,m), 6.71(1H,d,*J*=8.3Hz), 6,98-7.08(3H,m), 7.28-7.29(1H, m), 7.40(1H,d,*J*=8.3Hz), 7.47(1H,d,*J*=1.0Hz), 7.68(1H,d, *J*=1.0Hz), 8.37(1H,br), 10.8(1H,br)ppm FABMS:454(M+1) IR (KBr):3332, 2934, 1626, 1516, 1423, 1330, 1241cm$^{-1}$ | 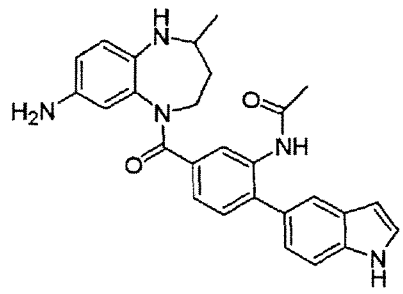 |
| 26 | $^1$H-NMR(DMSO-$d_6$, 120˚C) δ: 1.23(3H,d,*J*=6.4Hz), 1.53·1.70 (1H, m), 1.77(3H,s), 1.82·2.00(1H, m), 2.13(3H,s), 3.41-3.58(2H, m), 4.03-4.06(2H,m), 6.32(1H,d,*J*=7.8Hz), 6,42-6.43(1H,m), 6.53(1H,d,J=7.8Hz), 6.74(1H,s), 6.97-7.09(3H, m), 7.28-7.30 (1H,m), 7.40(1H,d,*J*=8.3Hz), 7.46(1H, d,*J*=1.0Hz), 7.63(1H,d, *J*=1.0Hz),8.37(1H,br), 10.8(1H,br)ppm FABMS:453(M+1) IR (KBr):3326, 2970, 1618, 1562, 1489, 1423 cm$^{-1}$ | 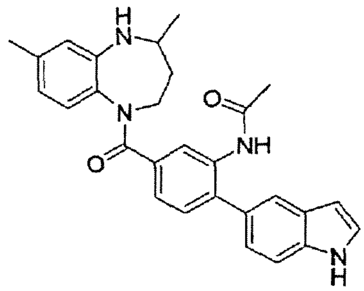 |
| 27 | $^1$H-NMR(DMSO-$d_6$,120˚C) δ: 1.33(3H,d,*J*=6.4Hz), 1.73-1.88 (1H,m), 1.89(3H,s), 1.98(1H, br), 3.49(2H,br), 4.15(1H,br), 4.79 (1H, br), 6.04(1H,d,*J*=8.3Hz) 6.44(3H,m), 7.06(3H,m), 7.39(3H, m), 7,75(1H,s), 8.48(1H,br), 8.71(1H,br), 10.9(1H,br)ppm FABMS:455(M+1) IR(KBr):3324, 1611, 1562, 1427, 1321, 1181 cm$^{-1}$ | 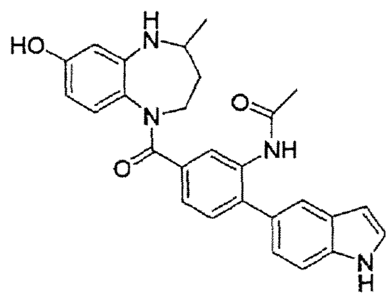 |

Table 2-10

| | | |
|---|---|---|
| 28 | $^1$H-NMR(DMSO-$d_6$,120˚C) δ: 0.93(3H,d,*J*=6.8Hz), 1.24(3H,d, *J*=6.8Hz), 1.54-1.73(1H,m), 1.87-1.99(1H,br), 2.05(2H,q, *J*=6.8Hz), 3.33-3.80(2H,m), 3.97-4.21(1H,br), 4.84(1H,brs), 6.40-6.54(2H,m), 6.66(1H,d,*J*=6.8Hz), 6.87-7.08(5H,m), 7.28 (1H,t,*J*=2.9Hz), 7.40(1H,d,*J*=8.8Hz), 7.45(1H,s), 7.72(1H,s), 8.26(1H,s), 10.75(1H,brs)ppm FABMS:453(M+1) IR(KBr): 3404, 2974, 1626, 1564, 1421, 1321cm$^{-1}$ | 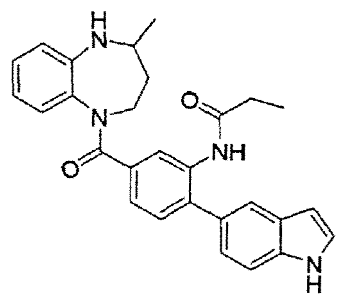 |

(continued)

| | | |
|---|---|---|
| 29 | $^1$H-NMR(DMSO-$d_6$,120°C) δ : 0.99(3H,t,*J*=7.8Hz), 1.45-1.80 (2H,m), 1.90-2.07(1H,m), 3.11-3.32(1H,m), 3.70-4.12(3H,m), 4.79(1H,brs) 5.20(1H,brs), 6.41-6.52(2H,m), 6.61-6.70(1H,m), 6.85-7.07(5H,m), 7.28-7.34(1H,m), 7.41-7.48(2H,m), 8.34(1H, s), 8.83(1H,brs), 10.80(1H,brs)ppm FABMS:469(M+1) IR(KBr): 3346, 2932, 1620, 1564, 1425, 1319cm$^{-1}$ | 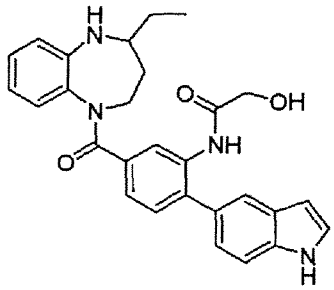 |
| 30 | $^1$H-NMR(DMSO-$d_6$,120°C) δ: 1.09(2H,brs), 1.25(3H,d, *J*=6.8Hz), 1.53-1.77(3H,m), 1.86-2.02(1H,m), 2.57(2H,t, *J*=7.8Hz), 3.30-3.87(4H,m), 3.96-4.34(1H,m), 4.91(1H,s),6.40 (1H,s), 6.43-6.56(1H,m), 6.63(1H,d,*J*=6.8Hz), 6.84-6.98(4H,m), 7.07-7.18(2H,m) 7.20-7.27(1H,m), 7.34(1H,d,*J*=8.8Hz), 10.66 (1H,brs)ppm FABMS:456(M+ 1) IR(KBr): 3310, 2932, 1624, 1560, 1419, 1321, 1253cm$^{-1}$ | 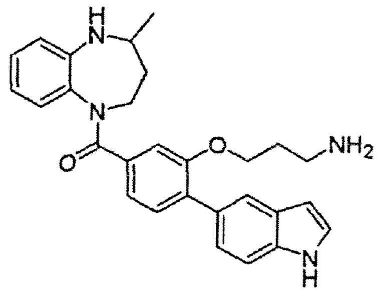 |

Table 2-11

| | | |
|---|---|---|
| 31 | $^1$H-NMR(DMSO-$d_6$,120°C) δ: 1.25(3H,d,*J*=6.8Hz), 1.57-1.74(1H, m), 1.87-2.03(1H,m), 3.09(3H,s), 3.35-3.75(3H,m), 3.77(1H,s), 3.92-4.28(1H,br), 4.83(1H,brs), 6.42-6.54(2H,m), 6.64(1H,d, *J*=7.8Hz), 6.85-7.08(5H,m), 7.23(1H,t,*J*=2.9Hz), 7.41-7.50(2H, m), 8.19(1H,d,*J*=2.0Hz), 8.58(1H,s), 10.82(1H,brs)ppm FABMS: 469(M+1) IR(KBr): 3352, 2936, 1626, 1566, 1423, 1321, 1255, 1114cm$^{-1}$ | 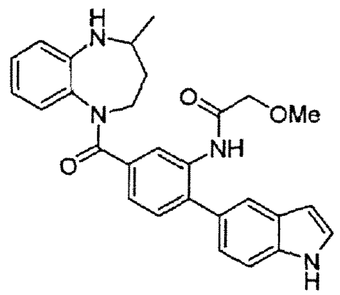 |
| 32 | $^1$H-NMR(DMSO-$d_6$,120°C) δ: 1.26(3H,d,*J*=5.9Hz),1.57-1.74(1H, m), 1.87-2.02(1H,m),2.32(2H,t,*J*=5.9Hz), 3.01(3H,s), 3.36-3.80 (4H,m), 3.95-4.26(1H,br), 4.83(1H,brs), 6.40-6.54(2H,m), 6.60 (1H,d,*J*=7.8Hz), 6.87-7.07(5H,m), 7.29(1H,t,*J*=2.9Hz), 7.38-7.48 (2H,m), 7.86(1H,brs), 8.43(1H,brs), 10.75(1H,brs)ppm FABMS: 483(M+1) IR(KBr): 3322, 2932, 1620, 1562, 1423, 1323, 1114 cm$^{-1}$ | 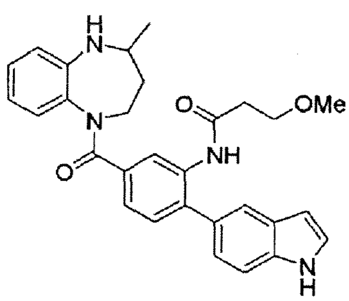 |

(continued)

| | | |
|---|---|---|
| 33 | $^1$H-NMR(DMSO-$d_6$, 120˚C) δ: 1.26(3H,d,*J*=6.8Hz), 1.60-1.74(1H, m), 1.91-196(1H,m), 3.20(3H,m), 3.46-3.49(4H,m), 3.80-3.84(2H, m), 4.09(1H,br), 4.91(1H,s), 6.39(1H,s), 6.46-6.53(1H,m), 6.63 (1H,d,*J*=7.8Hz), 6.87-6.96(4H,m), 7.13-7.18(m,2H), 7.23-7.25 (1H,m), 7.34(1H,d,*J*=8.8Hz), 7.60(1H,s), 10.65(1H,br)ppm FABMS:456(M+1) IR(KBr): 3290, 2932, 1628,1562,1417,1321, 1122cm$^{-1}$ | 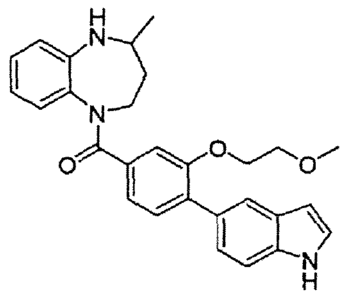 |

Table 2-12

| | | |
|---|---|---|
| 34 | $^1$H-NMR(DMSO-$d_6$,120˚C) δ : 1.26(3H,d,*J*=6.8Hz), 1.63-1.74 (1H,m), 1.91(1H,br), 3.43-3.78(6H,m), 4,06-4.10(2H,m), 4.89 (1H,s), 6.39(1H,s), 6.46-6.53(1H,m), 6.64(1H,d,*J*=7,8Hz), 6.90-6.94(4H,m), 7.11-7.25(3H,m), 7.34(1H,d,*J*=7.8Hz), 7.60 (1H,s), 10.65(1H,br)ppm FABMS:442(M+1) IR(KBr): 3338,2934,1620,1560,1419,1321, 1253cm$^{-1}$ | 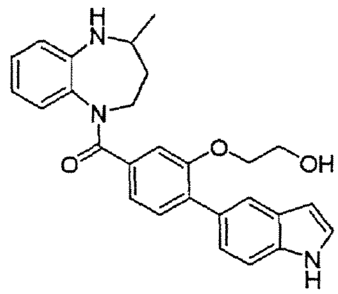 |
| 35 | $^1$H-NMR(DMSO-$d_6$,120˚C) δ: 1.25 (3H,d,J=6.8Hz), 1.58-1.76 (3H,m), 1.87-2.01 (1H,m), 3.17-3.28(2H,m), 3.32-3.82(4H,m), 3.94-4.32(1H,br), 4.89(1H,brs), 6.40(1H,brs), 6.45-6.55(1H, m), 6.64(1H,d,*J*=7.8Hz), 6.88-6.97(4H,m), 7.08-7.20(3H,m), 7.24(1H,t,*J*=2.9Hz), 7.35(1H,d,*J*=7.8Hz), 7.58(1H,s), 10.67 (1H,brs)ppm FABMS:483(M+1) IR(KBr): 3316, 2936, 1626, 1560, 1419, 1321, 1131cm$^{-1}$ | 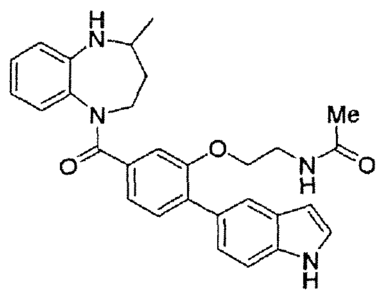 |
| 36 | $^1$H-NMR(DMSO-$d_6$,120˚C) δ: 1.12(2H,brs), 1.25(3H,d, J=6.7Hz), 1.38-1.41(2H,m), 1.50-1.75(3H,m), 1.88-2.01(1H, m), 2.46-2.55(2H,m), 3.30-3.75(4H,m), 3.90-4.40(1H,m), 4.91 (1H,s), 6.39(1H,d,*J*=2.9Hz), 6.45-6.53(1H,m), 6.63(1H,d, *J*=7.8Hz), 6.85(1H,s), 6.90-6.98(3H,m), 7.24(1H,brs), 7.34 (1H,d,*J*=8.8Hz), 7.56(1H,s), 10.65(1H,brs)ppm FABMS:469 (M+1) IR(KBr): 3322, 2938, 1624,1560, 1419, 1321, 1253 1125 cm$^{-1}$ | 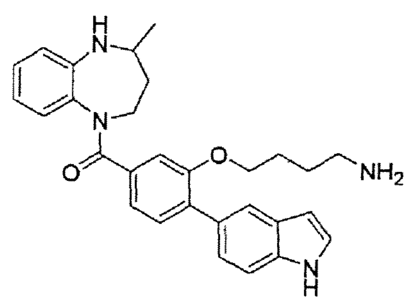 |

Table 2-13

| | | |
|---|---|---|
| 37 | $^1$H-NMR(DMSO-d6,120˚C) δ : 1.20-1.38 (5H,m), 1.49-1.75(5H, m), 1.88-2.01(1H,m), 3.33-3.73(6H,m), 3.95-4.35 (1H,m), 4.88 (1H,s) 6,36-6.40(1H,m), 6.44-6,52(1H,m), 6.62(1H,d,*J*=7.8Hz), 6.82-6.97(4H,m), 7.09-7.16(2H,m), 7,2I (1H,t, *J*=2.9Hz) 7.31 (1H,d, *J*=8.8Hz) 7.54(1H,m), 7.76-7.85(4H,m), 10.62(1H,brs) ppm FABMS : 613 (M+1) JR(KBr): 3326,2938,1622,1560,1321,1253, 1125cm$^{-1}$ | 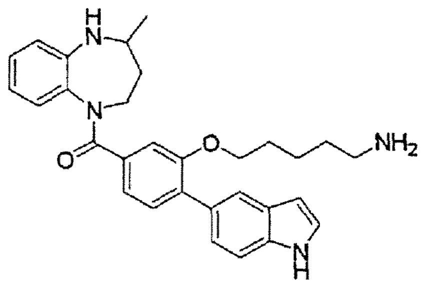 |
| 38 | $^1$H-NMR(DMSO-d6,120˚C) δ: 1.26(3H,d,*J*=5.9Hz), 1.61-1.74 (1H,m), 1.80-1.97(3H.m), 2.75-2.81(2H,m), 3.30-3.44(2H,m), 3.83(2H,t,*J*=5.4Hz), 4.10(1H,br), 5.95(1H,br), 6.42(1H,s), 6.47-6.53(1H,m), 6.64(1H,d,*J*=7.8Hz), 6.89-7.10(4H,m), 7.11-7.15(2H,m), 7.26(1H, t,*J*.=2.9Hz), 7.37(1H,d,*J*=8.8Hz), 7.55(1H,s), 10.73(1H,br)ppm FABMS:455(M+1) IR(KBr): 3322, 2634, 1622, 1560, 1419, 1621, 1253cm$^{-1}$ | 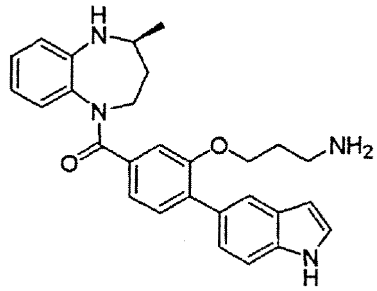 |

Table 2-14

| | | |
|---|---|---|
| 39 | $^1$H-NMR(DMSO-$d_6$, 120˚C) δ: 1.15-1.36(11H,m), 1.44-1.57(2H, m), 1.58-1.76(1H,m), 1.87-2.01(1H,m), 2.45-2.54(2H,m), 3.30-3.75(4H,m), 3.90-4.40(1H,br), 4.90(1H,s), 6.39(1H,d, *J*=2.9Hz), 6.45-6.53(1H,m), 6.63(1H,d,*J*=6.8Hz), 6.82-6.87(1H, m), 6.90-6.88(3H,m), 7.10-7.17(2H,m), 7.24(1H,d,*J*=2.9HZ), 7.34(1H,d,*J*=8.8Hz), 7.56(1H,s), 10.65(1H,brs)ppm FABMS: 498(M+1) IR(KBr): 3408, 2934, 1620, 1560, 1419, 1321cm$^{-1}$ | 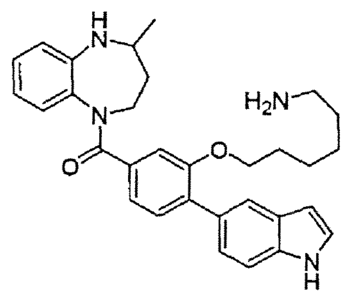 |
| 40 | $^1$H-NMR(DMSO-d6,120˚C) δ: 1.0(3H,t,*J*=7.3Hz), 1.50-1.77(5H, m), 1.95-2.00(1H,m), 2,57(2H,t,*J*=6.8Hz), 3.23(1H,br), 3.77(2H, t,*J*=6.8Hz), 3.75-4.03(2H,m), 4.89(1H,br), 6.40(1H,s), 6.45-6.51 (1H,m), 6.62(1H,d,*J*=7.8Hz), 6.89-6.95(4H,m), 7.11-7.16(2H, m), 7.23-7.25(1H,m), 7.34(1H,d,*J*=8.8Hz), 7.56(1H,s), 10.65 (1H,br)ppm FABMS:469(M+1) IR(KBr): 3360, 2934, 1624, 1560, 1419, 1319, 1249, 1129cm$^{-1}$ | 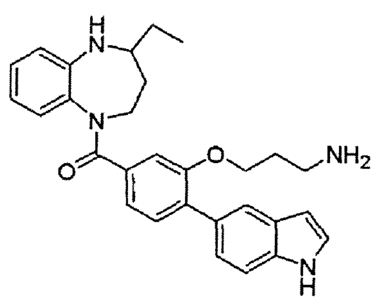 |

(continued)

| | | |
|---|---|---|
| 41 | $^1$H-NMR(DMSO·$d_6$,120°C) δ : 1.25 (3H,d,*J*=6.8Hz), 1.58-1.75 (3H,m), 1.88-2.02 (1H,m), 2.21(3H,s), 2.44-2.52(3H,s), 3.30·3.83 (3H,m), 3.90-4.35(1H,br), 4.89(1H,s), 6.39 (1H,s), 6.45-6.54(1H,m), 6.63(1H,d,*J*=6.8Hz), 6.85-6.98(4H,m), 7.09-7.17(2H,m), 7.24(1H,t,*J*=2.9Hz), 7.34(1H,d,*J*=8.8Hz), 7.56(1H,s), 10.67(1H,brs)ppm FABMS:469(M+1) IR(KBr): 3318, 2936, 1624, 1560, 1419, 1321, 1253, 1125cm$^{-1}$ | 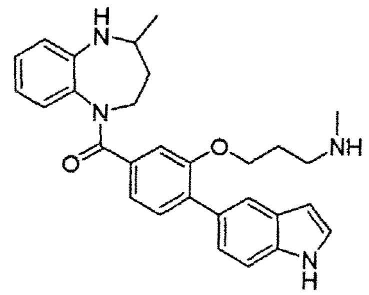 |

Table 2-15

| | | |
|---|---|---|
| 42 | $^1$H-NMR(DMSO-$d_6$, 120°C) δ: 1.13-1.30(6H, m), 1.52-1.76(3H, m), 1.87-2.03(1H, m), 2.05-2.26(5H, m), 3.42-3.81(4H,m), 3.95-4.30(1H,br), 4.88(1H,s), 6.39(1H,s), 6.45-6.54(1H,m), 6.63 (1H,d,*J*=6.8Hz) 6.85-6.98(4H, m), 7.09-7.17(2H, m), 7.24(1H, t, *J*=2.9Hz), 7.34(1H,d,*J*=8.8Hz), 7.56(1H,s), 10.65(1H,brs)ppm FABMS:484(M+1) IR(KBr): 3332, 2842, 1.626, 1560, 14I9, 121, 1255, 1110cm$^{-1}$ | 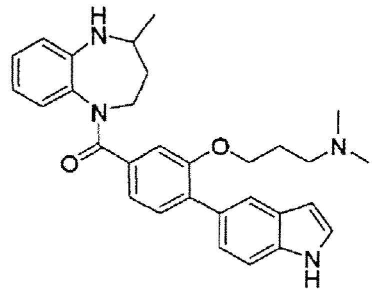 |
| 43 | $^1$H-NMR(DMSO-d6,120°C) δ : 1.25(3H,d,*J*=6.8Hz), 1.59-1.75 (1H,m), 1.76-2.00(3H,m), 2.46-2.55(2H,m), 3.32-3.80(4H,m), 3.90-4.35(1H,br), 4.88(1H,brs), 6.38-6.43(1H,m), 6.44-6.54(1H, m), 6.58-6.67(2H,m), 6.83-6.98(4H,m), 7.11-7.19(2H,m), 7.24 (1H,t,*J*=2.9Hz), 7.35(1H,d,*J*=8.8Hz), 7.40(1H,s), 7.59(1H,s), 10.65(1H,brs)ppm FABMS:506(M+1) IR(KBr): 3410, 1620, 1562, 1419, 1321, 1253, 1127cm$^{-1}$ | 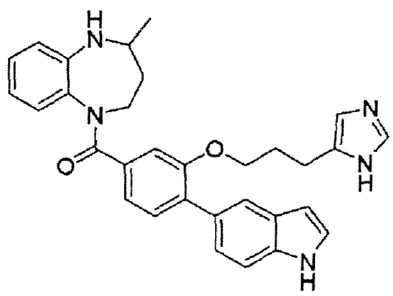 |
| 44 | $^1$H-NMR(DMSO-$d_6$,120°C) δ: 1.12-1.45(4H,m), 1.54-1.74(1H, m), 1.80-2.00(3H,m) 2.59(1H,t,*J*=6.8Hz), 3.36-3.75(2H,m), 4.20 (2H,t,*J*=6.8Hz) 4.36(2H,brs), 4.79(1H,brs), 6.40(1H,d,*J*=2.9Hz), 6.43·6.56(2H,m), 6.66(1H,d,*J*=7.8Hz), 6.74-6.82(2H,m) 6.86-6.94(2H,m), 7.03-7.09(1H,m), 7,29(1H,d,*J*=2.9Hz), 7.43-7.49(2H,m), 10.66(1H,brs)ppm FABMS:454(M+1) IR(KBr): 3446, 2930, 1620, 1427, 1325, 1255cm$^{-1}$ | 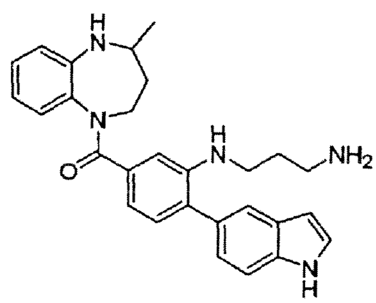 |

Table 2-16

| | | |
|---|---|---|
| 45 | $^1$H-NMR(DMSO-$d_6$,120˚C) δ: 0.82(3H,t,*J*=7.8Hz), 1.25(3H,d, *J*=6.8Hz), 1.45-1.75(3H,m), 1.88-2.02(1H,m), 3.32-3.74(4H,m), 3.94-4.40(1H,br), 4.91(1H,s), 6,37-6.41(1H,m), 6.45-6.54(1H,m) 6.68(1H,d,*J*=6.8Hz), 6.85(1H,s), 6.91-6.99(3H,m), 7.11-7.18(2H, m), 7.22-7.27(1H,m), 7.34(1H,d,*J*=7,8Hz), 7.55-7.59(1H,m), 10.65(1H,brs)ppm FABMS:440(M+1) IR(KBr): 3408, 2966, 1626, 1562, 1419, 1321, 1255,1129cm$^{-1}$ | 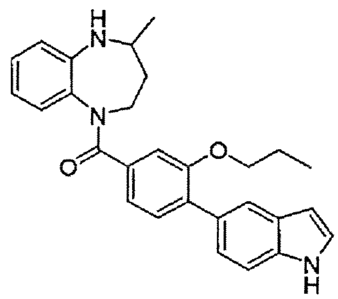 |
| 46 | $^1$H-NMR(DMSO-$d_6$,120˚C) δ : 1,25(3H,d, *J=6.9Hz),* 1.60-1.75 (1H,m), 1.88-2,01(1H,m), 2.27-2.35(4H,m), 2.45-2.55(4H,m), 3.35-3.52(4H,m) 3.75-3.86(2H,m), 3.95-4.35(1H,br), 4,90(1H, brs), 6.36-6.42(1H,m), 6.46-6.55(1H,m), 6.63(1H,d,*J*=7.3Hz), 6.84-6.99(4H,m), 7.11-7.19(2H,m), 7.24(1H,t,*J*=2.9Hz), 7.34 (1H,d,*J*=8.8Hz), 7.58(1H,s), 10.65(1H,brs)ppm FABMS:512(M+ 1) IR(KBr): 3414, 2962, 1626, 1560, 1419, 1321, 1116cm$^{-1}$ | 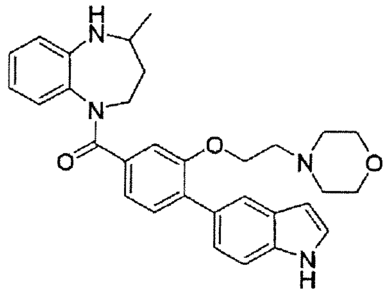 |
| 47 | $^1$H-NMR(DMSO-$d_6$, 120˚C) δ :1.26(3H,d,*J*=6.6Hz), 1.66(1H,m), 1.89(1H,m), 3.38(2H,br), 3.64(3H,s), 4.11(1H,br), 4.94(1H,br), 6.39(1H,d,*J*=2.2Hz), 6.49(1H,m), 6.63 (1H,d,*J*=8.0Hz), 6.87-6.95 (4H,m), 7.09-7.13(2H,m), 7.24(1H,t,*J*=2.9Hz),7.34(1H,d, *J*=8.0Hz), 7.52(1H,s), 10.66(1H,br)ppm FABMS:413(M+1) IR (KBr): 3328, 2958, 1618, 1560, 1415, 1321, 1253 cm$^{-1}$ | 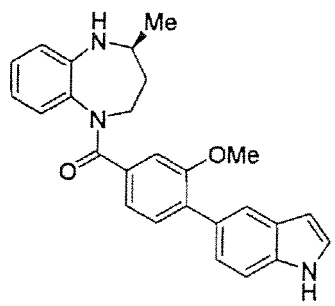 |

Table 2-17

| | | |
|---|---|---|
| 48 | $^1$H-NMR(DMSO-$d_6$,120˚C) δ: 1.25(3H,d,*J*=6.6Hz), 1.58-1.75(1H, m), 1.88-2.03(1H,m), 2.63(3H,s), 3.35-4.35(3H,m), 4.75-5.05(1H, br), 6.42-6.55(2H,m), 6.66(1H,d,*J*=8.1Hz), 6.91(2H,d,*J*=3.7Hz), 7.01-7.14(3H,m), 7.28-7.32(1H,m), 7.38-7.51(3H,m) 7.84(1H,s), 10.79(1H,brs)ppm FABMS:474(M+1) IR(KBr): 3346, 2932, 1626, 1562, 1419, 1323, 1156cm$^{-1}$ | 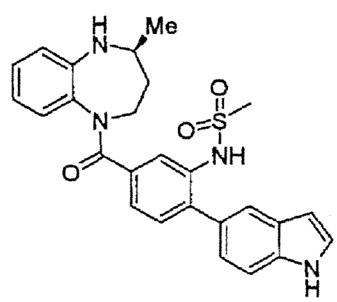 |

(continued)

| | | |
|---|---|---|
| 49 | $^1$H-NMR(DMSO-d6,120˚C) δ: 1.25(3H,d,*J*=6.6Hz), 1.41(3H,brs), 1.52-1.56(1H,m), 1.57-2.01(1H,m), 2.71(3H,s), 3.25-3.70(1H,br), 3.90-4.45(1H,br), 4.95(1H,brs), 6.39-6.43(1H,m), 6.44-6.54(1H, m), 6.64(1H,d,*J*=7.3Hz), 6.88-7.01(4H,m), 7.26-7.42(5H,m), 10.77 (1H,brs)ppm FABMS:453(M+1) IR(KBr): 3322, 1636, 1553, 1421, 1323, 1255cm$^{-1}$ | 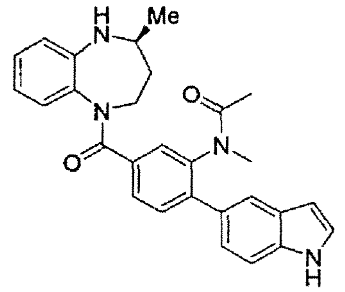 |
| 50 | $^1$H-NMR(DMSO-$d_6$,120˚C) δ: 1.36(3H,d,*J*=6.3Hz), 1.70-1.83(1H, m), 2.00-2.07(1H,m), 3.52(2H,br), 3.62(3H,s), 4.23(1H,br), 4.83 (2H,s), 5.01(1H,s), 6.56-6.61(1H,m), 6.66-6.72(3H,m), 6.94-7.05 (4H,m), 7.12-7.23(3H,m)ppm FABMS:388(M+1) IR(KBr): 3334, 2934, 1624, 1557, 1493, 1402, 1323, 1185, 1031cm$^{-1}$ | 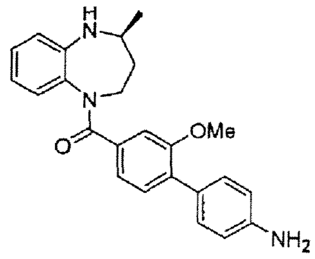 |

Table 2-18

| | | |
|---|---|---|
| 51 | $^1$H-NMR(DMSO-$d_6$,120˚C) δ: 1.25(3H,d,*J*=6.8Hz), 1.58-1.75(3H, m), 1.88-2.02(1H,m), 2.21(3H,s), 2.44-2,52(3H,s), 3.30.3,83(3H, m), 3.90-4.35(1H,br), 4.89(1H,s), 6.39(1H,s), 6.45-6.54(1H,m) 6.63(1H,d,*J*=6.8Hz), 6,85-6.98(4H,m), 7.09-7.17(2H,m), 7.24(1H, t,*J*=2.9Hz), 7.34(1H,d,*J*=8.8Hz), 7.56(1H,s), 10,67(1H,brs)ppm FABMS:469(M+1) IR(KBr): 3324, 2934, 1626, 1560, 1419, 1321, 1255cm$^{-1}$ | 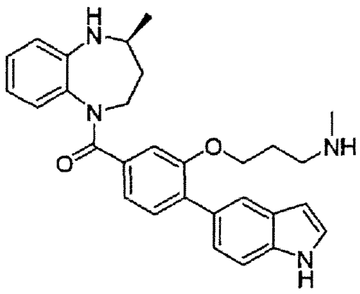 |
| 52 | $^1$H-NMR(DMSO-$d_6$,120˚C) δ: 1.19-1.45(3H,m), 1.36(3H,d, *J*=5.9Hz), 1.55-1.60(1H,m), 1.62-1.83(3H,m), 2.02(1H,br), 2.30-2.38(1H,m), 2.45-2.54(1H,m), 3.61-3.70(5H,m), 4.16(1H,br), 5.00(1H,s), 6.49(1H,s), 6.56-6.61(1H,m), 6.72(1H,d,*J*=7.8Hz), 6.94-7.05(4H,m), 7.21-7.25(2H,m), 7.33-7.35(1H,m), 7.44(1H,d, J=7.8Hz), 7.66(1H,s), 10.75(1H,br)ppm FABMS:4950(M+1) IR (KBr): 3332,2934,1719, 1566, 1419, 1321, 1253, 1125cm$^{-1}$ | 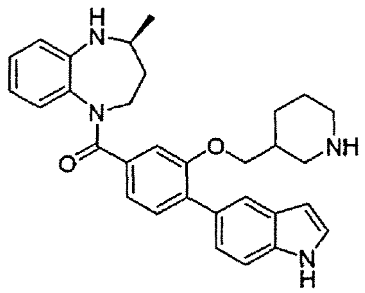 |

Table 2-19

| | | |
|---|---|---|
| 53 | $^1$H-NMR(DMSO-$d_6$, 120˚C) δ: 1.26(3H,d,*J*=5.9Hz), 1.60-1.77 (3H,m), 1.88-2.02(1H,m), 2.07(2H,t,*J*=7.3Hz), 3.10(2H,t, *J*=7.3Hz), 3.30-3.70(3H,m) 3.73-3.87(2H,m), 3.95-4.35(1H,br), 4.91(1H,brs), 6.40(1H,t,*J*=2.9Hz), 6.46-6.55(1H,m), 6.64(1H,d, *J*=6.6Hz), 6.85-6.98(4H,m), 7.08-7.16(2H,m), 7.25(1H,t, *J*=2.2Hz), 7.35(1H,d,*J*=8.1Hz), 7.54(1H,s), 10.66(1H,brs)ppm FABMS:510(M+1) IR(KBr): 3326, 2936c 1669, 1560, 1419, 1321, 1127m$^{-1}$ | 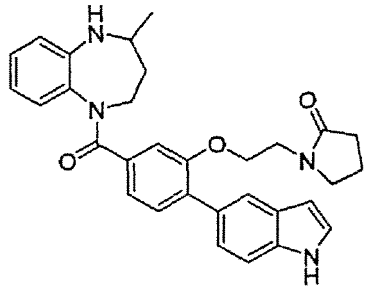 |
| 54 | $^1$H-NMR(DMSO-$d_6$,120˚C) δ: 1.25(3H,d,*J*=6.8Hz), 1.57-1.72 (3H,m), 1.88-1,95(1H,m), 2,57-2.62(2H,m), 3.42(2H,br), 3.65-3.80(2H,m), 4.11(1H,br), 4.70(2H,br), 4.89(1H,br), 6.44-6.68(4H,m), 6.61-6.95(4H,m), 7.03(1H,d,*J*=7.8Hz), 7.11-7.15(2H,m)ppm FABMS:431(M+1) IR(KBr): 3358, 2938, 1624, 1557, 1493, 1415, 1323, 1125 cm$^{-1}$ | 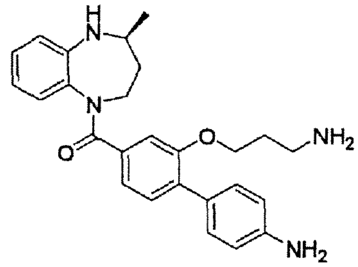 |
| 55 | $^1$H-NMR(DMSO-$d_6$,120˚C) δ: 1.09(2H,brs), 1.25(3H,d, *J*=6.8Hz), 1.53-1.77(3H,m) 1.86-2.02(1H,m), 2.57(2H,t, *J*=7.8Hz), 3.30-3.87(4H,m), 3.96-4.34(1H,m), 4.91(1H,s), 6.40 (1H,s), 6.43-6.56(1H,m), 6.63(1H,d,*J*=6.8Hz), 6,84-6.98(4H,m), 7.07-7.18(2H,m), 7.20-7.27(1H,m), 7.34(1H,d,*J*=8.8Hz), 10.65 (1H,brs)ppm FABMS:456(M+1) IR(KBr): 3328, 2932, 1624, 1560, 1419, 1321, 1255, 1125cm$^{-1}$ | 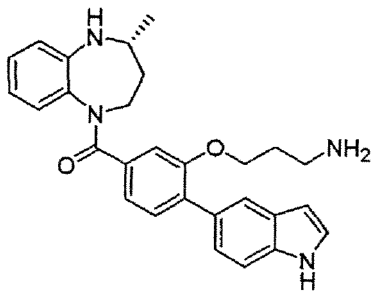 |

Table 2-20

| | | |
|---|---|---|
| 56 | $^1$H-NMR(DMSO-$d_6$,120˚C) δ: 1.26(3H,d,*J*=6,6Hz), 1.60-2.00 (6H,m), 2.13(2H,t,*J*=7.3Hz), 3.16(4H,t,*J*=7.3Hz), 3.30-3.75 (5H,m), 3.90-4.40(1H,br), 4.92(1H,brs), 6.39-6.43(1H,m), 6.46-6.54(1H,m), 6.63(1H,d,*J*=7.3Hz), 6.83-6.88(1H,m), 6.89·6.99(3H,m), 7.12-7.19(2H,m), 7.24(1H,t,*J*=2.9Hz), 7.36 (1H,d,*J*=8.1Hz), 7.58(1H,brs), 10.65(1H,brs)ppm FABMS:522 (M+1) IR(KBr): 3322, 2934, 1669, 1560, 1419, 1321, 1255, 112,5cm$^{-1}$ | 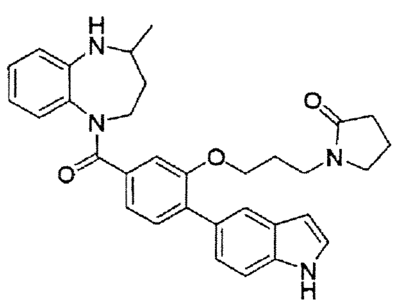 |

(continued)

| | | |
|---|---|---|
| 57 | $^1$H-NMR(DMSO-$d_6$,120˚C) δ: 0,87-0.89(1H,m), 1.26(4H,d, *J*=5.9Hz), 1.65-1.69(4H,m), 1.92(1H,br), 3.41(4H,m), 3.73-3.77(4H,complex), 4.15(1H,br), 4.89(1H,br), 6.39(1H,s), 6.50-6.62(1H,m), 6.64(1H,m), 6.87-6.94(4H,m), 7.12-7.15 (2H,m), 7.25(1H,t,*J*=2.9Hz), 7.34 (1H, d, *J*=8.8 Hz), 7.56(1H, s)ppm FABMS:500(M+1) IR(KBr): 3328, 2932, 1620, 1560, 1419, 1321, 1253, 1069cm$^{-1}$ | 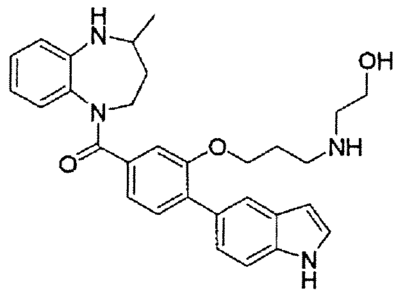 |
| 58 | $^1$H-NMR (DMSO-$d_6$, 120˚C) δ: 0.87-0.89(1H,m), 1.26 (4H, d, *J*=5.9 Hz), 1.61-1.73(4H,m), 1.94(1H,br), 3.41(6H,m), 3.74-3.77 (5H, complex), 4.14 (2H, br), 4.89(1H,br), 6.39 (1H, m), 6.47-6.53(1H,m), 6,63(1H,d,*J*=7,3Hz), 6.86(1H,m), 6.90-6.94(3H,m), 7.11-7.15(2H,m), 7.24(1H,t,*J*=2.9Hz),7.35 (1H,d,*J*=8.8Hz), 7.56(1H,s), 10.65(1H,br)ppm FABMS:544 (M+1) IR(KBr): 3324, 2936, 1620, 1560, 1419, 1321, 1253. 1042cm$^{-1}$ | 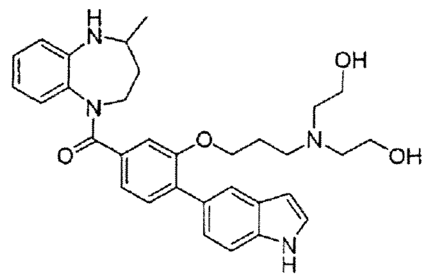 |

Table 2-21

| | | |
|---|---|---|
| 59 | $^1$H-NMR(DMSO-$d_6$,120˚C) δ: 1.17-1.27(5H,m), 1.65-1.70(1H, m), 1.92(1H,br), 2.31(4H,m), 3.47(2H,t,*J*=4.4Hz), 3,77-3.81(2H, m), 4.15(1H,br), 4.91(1H,s), 6.38(1H,s), 6.46-6.52(1H,m), 6.61 (1H,d,*J*=7.3Hz), 6.87-6.96(4H,m), 7.12-7.15(2H,m), 7.24(1H,t, *J*=2.9Hz), 7.33(1H,d,*J*=8.8Hz), 7.58(1H,s), 10.65(1H,br)ppm FABMS:512(M+1) IR(KBr): 3326, 2932, 1622, 1560, 1419, 1321, 1118cm$^{-1}$ | 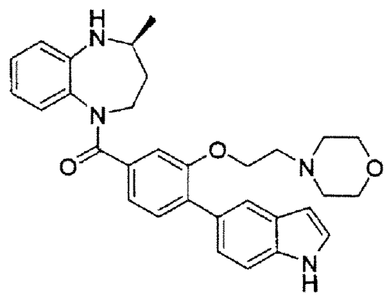 |
| 60 | $^1$H-NMR(DMSO-$d_6$,120˚C) δ: 1.25(3H,d,*J*=6.8Hz), 1.67(1H,br), 1.91(1H,br), 2.34-2.37(2H,m), 2.52-2.54(2H,m), 3.51-3.55(4H, m), 3.77(2H,br), 4.06(1H,br), 4.72(2H,br), 4.90(1H,br), 6.47(4H, m), 6.81-6.93(4H,m), 7.04(1H,d,*J*=7.8Hz), 7.15-7.18(2H,m) ppm FABMS:487(M+1) IR (K.Br): 3360, 2934, 1626, 1557, 1412, 1323, 1116cm$^{-1}$ | 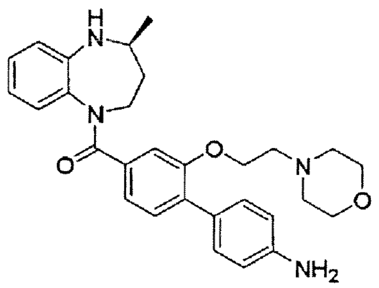 |

(continued)

| | | |
|---|---|---|
| 61 | $^1$H-NMR(DMSO-d6:120˚C) δ: 1.26(3H,d,*J*=6.6Hz), 1.60-1.70 (3H,m), 1.62(2H,m), 1.91(1H,m), 2.22(6H,m), 3.45-3.52(5H, complex), 3.73(2H,m), 4.15(1H,br), 4.89(1H,s), 6.38(1H,m), 6,47-6.62(1H,m), 6.63(1H,d,*J*=7.3Hz), 6.88-6.94(4H,m), 7.13 (2H,dd,*J*=2.2,8.1Hz), 7.24(1H,t, *J*=2.9Hz), 7.34(1H,d, *J*=8.8Hz), 7.56(1H,s), 10.65(1H,br)ppm FABMS:526(M+1) IR (KBr): 3332, 962, 1624, 1560, 1419, 1321, 1253, 1116 cm$^{-1}$ | 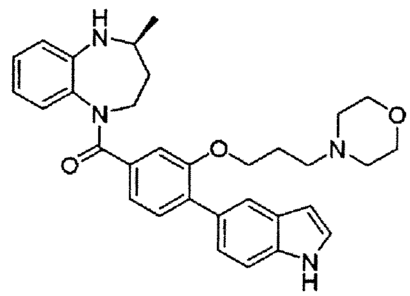 |

Table 2-22

| | | |
|---|---|---|
| 62 | $^1$H-NMR(DMSO-$d_6$,120˚C) δ: 1.20-1.52(9H,m), 1.55-1.80 (1H,m), 1.83-2.06(1H,m) 2.15-2.35(6H,m), 3.20-4.40(4H, m), 4.90(1H,brs) 6.38(1H,brs), 6.49(1H,brs), 6.61(1H,brs), 6.80-7.00(4H,m) 7.04-7.38(2H,m), 7.42-7.70(3H,m), 10.60 (1H,brs)ppm FABMS:510(M+1) IR(KBr): 3324, 2936, 1626, 1562, 1417, 1321, 1122cm$^{-1}$ | 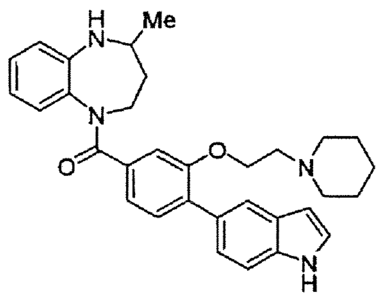 |
| 63 | $^1$H-NBR(DMSO-*$d_6$*, 120˚C) δ: 1.24(3H,d,*J*=6.6Hz), 1.60-1.65(3H, m), 1.92(1H,m), 2.12(3H,s), 2.29(2H, t, *J*=7.3Hz), 3.42(2H,br), 3.70-3.72(3H, m), 4.13(1H,br), 4.89 (1H,s), 6,39(1H,s), 6.46-6.52(1H, m), 6.63 (1H, d, *J*=7.3Hz), 6.87-6.94(4H, m), 7.10-7.13(2H,m), 7.25(1H,s), 7.34 (1H, d, *J*=8.8Hz), 7.49-7.65 (4H, m), 10.65(1H,br)ppm FABMS:542 (M+1) IR(KBr): 3322, 2934, 1719, 1626, 1660, 1419, 1321, 1251, 1112cm$^{-1}$ | 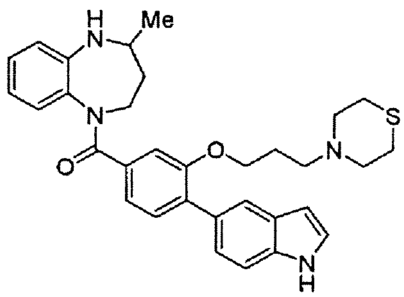 |
| 64 | $^1$H-NMR(DMSO-d6, 120˚C) δ : 1.25(3H,d, *J*=6.6Hz). 1.62-1.67(3H,m), 1.91(1H,m), 2.12-2.26 (8H,complex), 2.65 (6H,m), 3.35-3.48(2H,m), 3.65-3.75(3H,m), 4.11(1H,br), 4.88 (1H,s), 6.38 (1H,s), 6.46-6.52(1H,m), 6.61(1H,d, *J*=7.3Hz), 6.87-6.94(4H,m), 7.10-7.14(2H,m), 7.24 (1H, t, *J*=2.9Hz), 7.33(1H,d,*J*=8.8Hz), 7.56(1H,s), 10.65(1H,br) ppm FABMS:525(M+1) IR(KBr): 3320, 2944, 1624, 16241419, 1321, 1253, 1131cm$^{-1}$ | 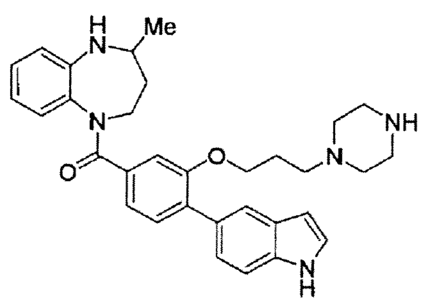 |

Table 2-23

| | | |
|---|---|---|
| 65 | $^1$H-NMR(DMSO-$d_6$,120˚C) δ: 1.26(3H,d,$J$=6.6Hz), 1.59-1.68 (3H,m), 1.91(1H,m), 2.12(3H,s), 2.27(10H,s)3.4(2H,br), 3.70-3.75(3H,m), 4,11(1H,br), 4.88(1H,bz), 6.38(1H,s), 6.46-6.52(1H,m), 6.63(1H,d,$J$=7.3Hz),6.87·6.94(4H,m), 7.10-7.15(2H,m), 7.24(1H,t,$J$=2.9Hz), 7.33(1H,d, $J$=8.8Hz), 7.56(1H,s), 10.65(1H,br)ppm FABMS:539(M+1) IR(KBr): 3342, 2944, 1626, 1560, 1419, 1321, 1151cm$^{-1}$ | 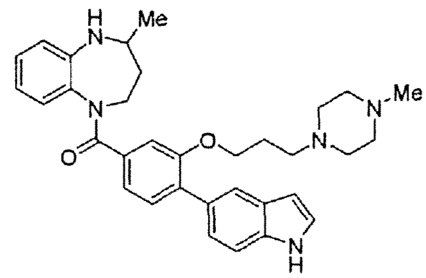 |
| 66 | $^1$H-NMR(DMSO-d$_6$, 120˚C) δ: 1.26 (3H, d, $J$=5.9Hz), 1.68(1H, m), 1.92(1H,m), 2.48(4H,m), 3.47 (4H, t, $J$=4.4), 3.79(2H,m), 4.11(1H,br), 4.91(1H,s), 6.38(1H,s), 6.46-6.52(1H,m), 6.61 (1H, d, $J$=7.3 Hz), 6.87-6.96(4H.m), 7.12-7.15(2H,m), 7.24 (1H, t, $J$=2.9 Hz), 7.33(1H,d,J=8.8Hz), 7.58(1H,s), 10.65(1H,br)ppm FABMS:512(M+1) IR(KBr): 3430, 2926, 1626, 1562, 1419, 1321, 1116cm$^{-1}$ | 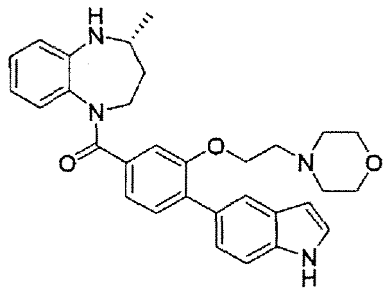 |
| 67 | $^1$H-NMR(DMSO-$d_6$, 120˚C) δ : 1,25(3H,d,$J$=7.3Hz), 1.39(2H, m), 1.62(2H,m), 1.68(1H,m), 1.96(1H,m), 2.15-2.22(6H,m), 3.46-3.50(5H,m), 3.69-3.84(3H,m), 4.89(1H,m), 6.38(1H,t, $J$=2.2Hz ), 6.46-6.52(1H,m), 6,62(1H,d,$J$=7.3Hz), 6.86-6.95 (4H,m), 7.11-7.16(2H,m), 7.24(1H,t,$J$=2.9Hz), 7.33(1H,d, $J$=8.8), 7.57(1H,s), 10.65(1H,br)ppm FABMS:540(M+1) IR (KBr): 3328, 2958, 1624, 1560, 1419, 1321, 1118cm$^{-1}$ | 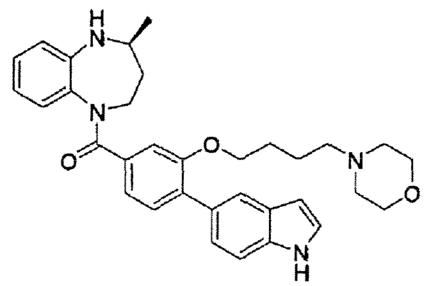 |

Table 2-24

| | | |
|---|---|---|
| 68 | $^1$H-NMR(DMSO-d$_6$,120˚C) δ : 1.26(3H,d,$J$=5.9Hz), 1.68 (3H,m), 1.96(1H,m), 2.22-2.23(6H,m), 3.48-3.52(5H,m), 3.73(2H,m), 4.14(1H,br), 4.89(1H,s), 6.33(1H,m), 6.46-6.52(1H,m), 6.63(1H,d,$J$=7.3Hz), 6.88-6.94(4H,m), 7.13(2H,dd,$J$=2.2, 8.1Hz), 7.24(1H,$J$=2.9Hz), 7.34(1H,d, $J$=8.8Hz), 7.56(1H,s), 10.66(lH,br)ppm FABMS:526(M+1) R(KBr): 3330, 2962, 1624, 1560, 1419, 1321, 1253, 1116cm$^{-1}$ | 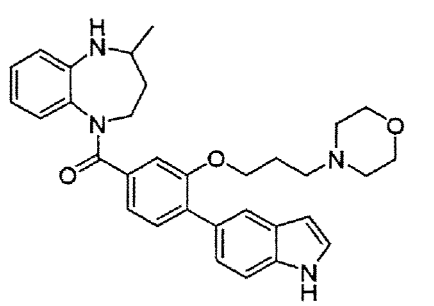 |
| 69 | $^1$H-NMR(DMSO-$d_6$,120˚C)δ:1.12-1.50(H,m), 1.55(2H,m), 1.68(1H,m), 1.91(1H,br), 2.17(2H,t,$J$=6.6Hz), 2.25-2.28 (4H,m), 3.49-3.53(6H,m), 3.67-3.71(2H,m), 4.16(1H,m), 4.89(1H,m), 6.38(1H,t,$J$=2.2Hz), 6.46-6.52(1H,m), 6.62 (1H,d,$J$=7.3Hz), 6.86-6.95(4H,m), 7.11-7.16(2H,m), 7,24 (1H,t,$J$=2.9Hz), 7.33(1H,d,$J$=8.8Hz), 7.57(1H,s), 10.65 (1H,br)ppm FABMS:554(M+1) IR(KBr): 3324, 2938, 1624, 1560, 1419, 1321, 1118 cm$^{-1}$ | 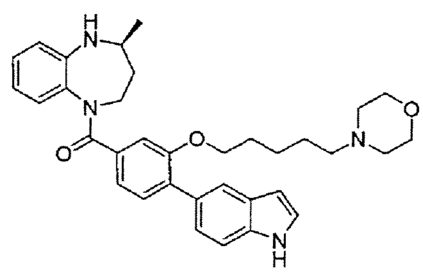 |

Table 2-25

| | | |
|---|---|---|
| 70 | $^1$H-NMR(DMSO-$d_6$, 120°C) δ: 1.27(3H,d,*J*=5.9Hz), 1.69(4H,m), 1.93(1H,m), 2.06(1H,s), 2.27(1H,m), 3.35(2H,m), 3.75(1H,m), 4,12(1H,br), 4.90(1H,br), 6.40(1H,d,*J*=2.9Hz), 6.50(1H,m), 6.64 (1H,d,*J*=7.3Hz), 6.89(1H,s), 6.94(3H,m), 7.24(2H,dd,*J*=8.8, 2.9Hz), 7.26(1H,d,*J*=7.3Hz), 7.36(1H,d,*J*=8.8Hz), 7.57(1H,s), 10.67(1H,s)ppm FABMS:539(M+1) IR(KBr): 3326, 2940, 1624, 1560, 1419, 1321, 1151cm$^{-1}$ | 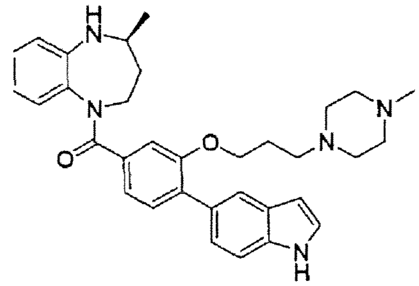 |
| 71 | $^1$H-NMR(DMSO-$d_6$=120°C) δ: 1.26(3H,d,*J*=6.6Hz), 1.80-1.52 (6H,m), 1.55-1.80(3H,m), 1.90-2.02(1H,m), 2.20-2.30(3H,m), 3.28-3.93(4H,m), 3.95-4.40(1H,br), 4.88(1H,s), 6.38(1H,t, *J*=2.9Hz), 6.46-6.54(1H,m), 6.63(1H,d,*J*=7.3Hz), 6.85-6.97(4H, m) 7.10-7.17(2H,m), 7.24(1H,t,*J*=2.9Hz), 7.35(1H,d,*J*=8.1Hz), 7.56(1H,s), 10.65(1H,brs)ppm FARMS:524(M+1) IR(KBr): 3322, 2936, 1626, 1560, 1419, 1321, 1253, 1127cm$^{-1}$ | 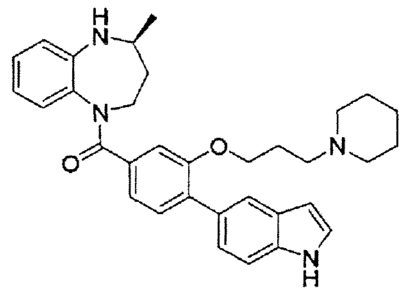 |

Table 2-26

| | | |
|---|---|---|
| 72 | $^1$H-NMR(DMSO-$d_6$,120°C) δ : 1.25(3H,d,*J*=6.6Hz), 1.57-1.75 (3H,m), 1.88-2.02(1H,m), 2.29(2H,t,*J*=6,6Hz), 2.48-2.60(7H, m),3.28-3.92(5H,m), 3,95-4.40(1H,br), 4.88(1H,s), 6.37-6.41 (1H,m), 6.46-6.54(1H,m), 6.63(1H,d,*J*=7.3Hz), 6.85-6.98(4H, m),7.10-7.17(2H,m) 7.23-7.27(1H,m), 7.35(1H,d,*J*=8.8Hz), 7.54-7.58(1H,m), 10.65(1H,brs)ppm FABMS:542(M+1) IR (KBr): 3320, 2926, 1628, 1557, 1417, 1321, 1253, 1125cm$^{-1}$ | 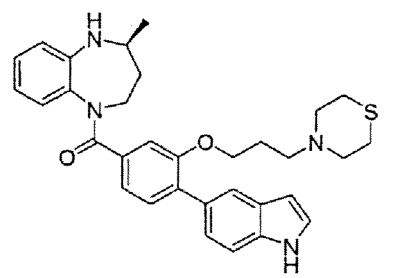 |
| 73 | $^1$H-NMR(DMSO-$d_6$, 120°C) δ: 1.27(3H,d,*J*=5.9Hz), 1.68(4H, m), 1.95(1H,s), 2.28(4H,m), 3.33-3.44(7H,m), 3.75(2H,m), 4.13 (1H,br), 4.90(1H,br), 6.40(1H,d,*J*=2.9Hz), 6.47-6.53(1H,m), 6.64(1H,d,*J*=7.3Hz), 6.89(1H,s), 6.94(3H,m), 7.14(2H,dd, *J*=8.8,2.9Hz), 7.26(1H,d,*J*=2.9Hz), 7.36 (1H, d, *J*=8.8 Hz), 7.57 (1H, s), 10.67(1H,s)ppm FABMS:567 (M+1) IR(KBr): 3322, 2936, 1626, 1560, 1421, 1321, 1257, 1127 cm$^{-1}$ | 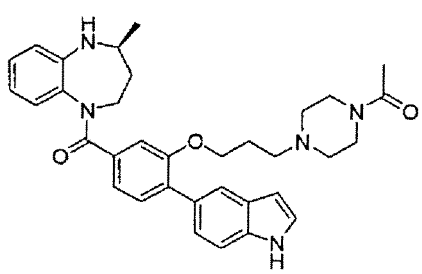 |

Table 2-27

| | | |
|---|---|---|
| 74 | $^1$H-NMR(DMSO-$d_6$,120°C) δ: 1.25(3H,d,*J*=5.9Hz), 1.59-1.72 (1H,m), 1.91-1.93(1H,m), 2.34-2.38(4H,m), 2.51-2.56(2H,m), 2.71(3H,d,J=2.9Hz), 3.42-3.55(2H,m), 3.51-3.55(4H,m), 3.75-3.80(2H,m), 4,10(1H,br), 4.89(1H,br), 5.21(1H,br), 6.44-6.68(4H,m), 6.71-6.98(4H,m), 7.05(1H,d,*J*=7.8Hz), 7.21-7.25(2H,m)ppm FABMS:501 (M+1) IR(KBr): 3376, 2934, 1615, 1533, 1497, 1400, 1323, 1116cm$^{-1}$ | 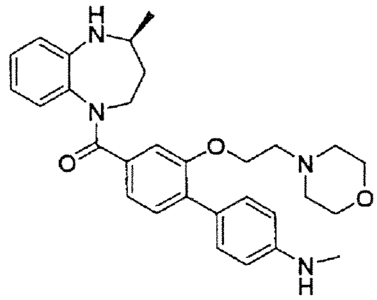 |
| 75 | $^1$H-NMR(DMSO-$d_6$,120°C) δ: 1.25(3H,d,*J*=6.8Hz), 1.64-1.69 (1H,m), 1.91(1H,br), 2.34-2.37(4H,m), 2.51-2.55(2H,m), 2.90 (3H,s), 3.43-3.54(2H,m), 3.51-3.54(4H,m), 3.77-3.81(2H,m), 4.11(1H,br), 4.91(1H,br), 6,45-6.50(1H,m), 6.61(1H,d, *J*=7.8Hz), 6.69(2H,d,*J*=8.8Hz). 6.83-6.93(4H,m), 7.07(2H,d, *J*=7.8Hz), 7.31(2H,d,*J*=8.8Hz)ppm FABMS:515 (M+1) IR (KBr): 3332, 2934, 1615, 1568, 1396, 1361, 1118cm$^{-1}$ | 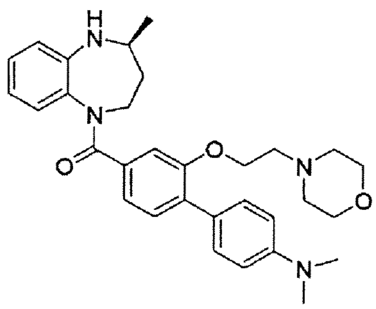 |
| 76 | $^1$H-NMR(DMSO-$d_6$,120°C)δ: 1.25(3H,d,*J*=6.8Hz), 1.59-1.73 (1H,m), 1.91-1.93(1H,m), 2.02(3H,s), 2.32-2.36(4H,m), 2.50-2.54(2H,m), 3.43-3.54(2H,m), 3.50-3.54(4H,m), 3.76-3.85(2H,m), 4.14(1H,br), 4.91(1H,br), 6.45-6.51(1H,m), 6,62(1H,d,*J*=6.8Hz), 6.86-6,95(4H,m), 7.10(1H,d,*J*=7.8Hz), 7.35-7.39(2H,m), 7.50-7.53(2H,m), 9.49(1H,br)ppm FABMS: 529 (M+1) IR(KBr):3316, 2964, 1601, 1562, 1402, 1323, 1116cm$^{-1}$ | 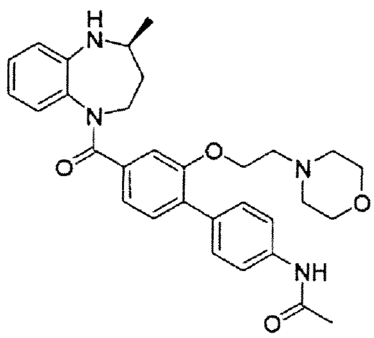 |

Table 2-28

| | | |
|---|---|---|
| 77 | $^1$H-NMR(DMSO-$d_6$,120°C) δ : 1.25(2H,d,*J*=6.8Hz), 1.63-1.77(1H,m), 1.91(1H,br), 2.33-2.36(4H,m), 2.50-2.55(2H,m), 3.42-3.54(2H,m), 3.50-3.54(4H,m), 3.77-3.83(2H,m), 4.11(1H,br), 6.45-6.51(1H,m), 6.61(1H,d,*J*=6.8Hz), 6.86-6.94(6H,m), 7.09(1H,d,*J*=7.8Hz), 7.36-7.39(2H,m)ppm FABMS:502 (M+1) IR(KBr): 3338, 2936, 1630, 1557, 1404, 1323, 1249, 1118cm$^{-1}$ | 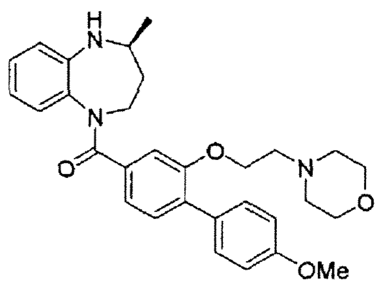 |

(continued)

| | | |
|---|---|---|
| 78 | $^1$H·NMR(DMSO-$d_6$,120°C) δ : 1.26(3H,d,$J$=6.3Hz), 1.63(3H,m), 1.93 (1H,m), 2.07(1H,s), 2.21(2H,t,$J$=6.8Hz) 3.42(1H,br), 3.73(2H,m), 4.12(1H,br), 4.88(1H,br), 6.38(1H,t,$J$=1.9Hz), 6.46(1H,s), 6.49(1H, m), 6,63(1H,d,$J$=7.3Hz), 6.88(1H,s), 6.94(3H,m), 7.12(2H,m), 7,24 (1H,t,$J$=8.1Hz), 7.34(1H,d,$J$=8.3Hz), 7.56(1H,s), 10.65(1H,s)ppm FABMS:484(M+1) IR(KBr): 3330, 2940, 1626, 1560, 1419, 1321, 1253, 1125cm$^{-1}$ | 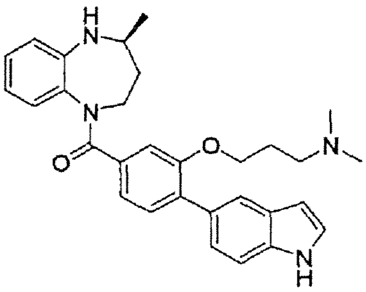 |

Table 2-29

| | | |
|---|---|---|
| 79 | $^1$H-NMR(DMSO-d$_6$,120°C) δ: 0.72-0.85(4H,m), 1.16-1.40(6H, m), 1.52-1.75(3H,m) 1.85-2.02(1H,m),2.10-2.50(4H,m), 2.77-2.95(2H,m) 3.32-3.90(4H,m), 3.95.4.35(1H,m),4.80.4.95 (1H,m), 6.34-6.41(1H,m), 6.45-6.53(1H,m), 6.60-6.67(1H,m), 6.84-6.98(4H,m), 7.08-7.12(2H,m),7.22-7.27(1H,m), 7.31-7.38(1H,m), 7.55(1H,s), 10.66(1H,s)ppm FABMS:540 (M+1) IR(KBr): 3318, 2962, 1626, 1560, 1419, 1321, 1253, 1125 cm$^{-1}$ | 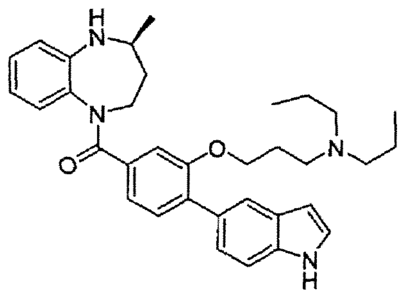 |
| 80 | $^1$H-NMR(DMSO-d$_6$, 120°C) δ: 0.99(6H,d,$J$=6.6Hz), 1.26(3H, d,$J$=5.9Hz), 1.51(2H,t,$J$=10.2Hz), 1.64(3H,m), 1.83(3H,m), 1.91(1H,m), 2.23(2H,t,$J$=7.3Hz), 2.55(2H,m), 3.48(3H,m), 3.74(2H,m), 4.11(1H,br), 4.89(1H,br), 6.38(1H,m), 6.50(1H, m), 6.63(1H,d,$J$=7.3Hz), 6.88-6.94(4H,m), 7.11-7.15(2H,m), 7.24(1H,t,$J$=2.9Hz), 7.34(1H,d,$J$=8.1Hz), 7.55(1H,s), 10.66 (1H,s)ppm FABMS:554(M+1) IR(KBr): 3326, 2972, 1626, 1560, 1419, 1223, 1253, 1145cm$^{-1}$ | 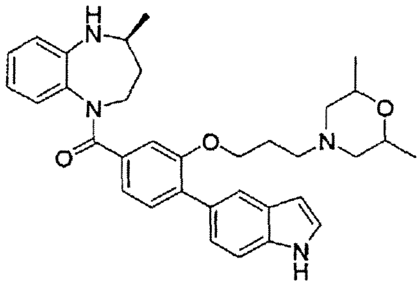 |

Table 2-30

| | | |
|---|---|---|
| 81 | $^1$H-NMRODMSO·$d_6$, 120°C) δ : 0.81(3H,t,$J$=7.3Hz), 1.26(3H,d,$J$=8.0Hz), 1.38(2H,m), 1.41-1.46(1H,m), 1.83(3H,m), 2.60(2H,m), 3.42(2H,br), 3.79(2H,m), 4.13(1H,br), 4.85(1H,br), 6.41(1H,m), 6.52(1H,m), 6.65(1H,d,$J$=6.6Hz), 6.93(4H,m), 7.12(2H,dd, $J$=8.0,1.5Hz), 7.27(1H,d,$J$=2.9Hz), 7.37(1H,d, $J$=8.0Hz), 7.55(1H,s), 10.72(1H,s)ppm FABMS:512 (M+1) IR(KBr): 3316, 2964, 1624, 1562, 1419, 1321, 1253cm$^{-1}$ | 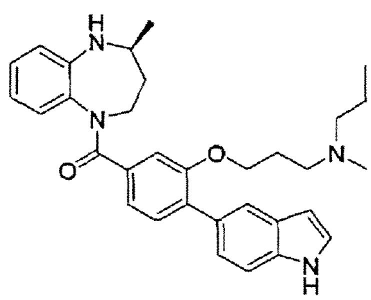 |

(continued)

| | | |
|---|---|---|
| 81 ·1/2 fumarate | $^1$H-NMR(DMSO-$d_6$, 120°C) δ:0.78(3H, t, J=7.3Hz), 1.25(3H, d, J=6.6Hz), 1.31(2H, q, J=7.3Hz), 1.64(4H, m), 1.94(1H, m), 2,10(3H, s), 2.21(2H, t, J=7.3Hz), 2.32(2H, t, J=7.3Hz), 3.74(2H, m), 6.38(1H, s), 6-48 (1H, m), 6.60(2H, m), 6.87(1H, s), 6.94(3H, m), 7.11 (2H, m), 7.24(1H, m), 7.33(1H, d, J=8.8Hz), 7.56(1H, s), 10.64(1H, br)ppm IR(KBr):3350, 2970, 1630,1593,1502, 1419, 1386, 1321, 1257,1125, 1054, 810, 754, 675cm$^{-1}$ Melting point : 165°C~166°C | |
| 81 ·1/3 citrate | $^1$H-NMR(DMSO-$d_6$,120°C) δ: 0.79(3H, t, J=7.3Hz), 1.26(3H, d, J=6.6Hz), 1.37(2H, q, J=7.3Hz), 1.72(4H, m), 1.96(1H, m), 2.26(3H, s), 2.40(2H, t, J=7.3Hz), 2.58(4H, m), 3.77(2H, m), 4.08(1H, br), 6.39(1H, s), 6.49(1H, m), 6.63(2H, m), 6.90(4H, m), 7.13(2H, m), 7.25(1H, t, J=2.2Hz), 7.35(1H, d, J=8.0Hz), 7.56(1H, s), 10.68(1H, br)ppm 1R(KBr) : 3330, 2964, 1696, 1630, 1597, 1502, 1419, 1388, 1323, 1257,1123, 754 cm$^{-1}$ Melting point: 134°C~135°C | |

Table 2-31

| | | |
|---|---|---|
| 82 | $^1$H-NMR(DMSO-$d_6$,120°C) δ : 0.91(9H,s), 1.25(3H,d,J=5.9Hz), 1.64(3H,m), 1.94(1H,m), 2.07(3H,s), 2.32(2H,t,J=7.3Hz), 3.35 (2H,m), 3.43(1H,br), 3.74(1H,m), 4.11(1H,br), 4.87(1H,br), 6.37 (1H,m), 6.48(1H,m), 6.63(1H,d,J=6.6Hz), 6.86-6.91(4H,m), 7.11-7.16(2H,m), 7.23(2H,t,J=2.2Hz), 7.33(1H,d,J=8.0Hz), 7.55(1H,s), 10.64(1H,s)ppm FABMS: 526(M+1) IR(KBr): 3428, 2972, 1626, 1562, 1419, 1321, 1255, 1125cm$^{-1}$ | 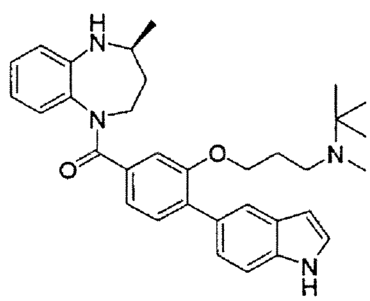 |
| 83 | $^1$H-NMR(DMSO-$d_6$,120°C) δ: 1.26(3H,d, J=6.6Hz), 1.50-1.75 (7H,m), 1.85-2.02(1H,m) 2,27(2H,d,J=7.3Hz), 2.69-2.75(2H, m), 3.20-3.85(4H,m) 3.90-4.40(1H,br), 4.88(1H,s), 6.37-6.41 (1H,m) 6.46-6.54(1H,m), 6.64(1H,d,J=7.3Hz), 6.84-6.89(1H,m) 6.90-6.98(4H,m), 7.10-7.17(2H,m), 7.24(1H,t,J=2.9Hz) 3.34 (1H,t,J=8.8Hz), 7.56(1H,s), 10.68(1H,br)ppm FABMS : 594 (M+1) IR(KBr): 3404, 2944, 1671, 1620, 1421, 4654, 1131cm$^{-1}$ | 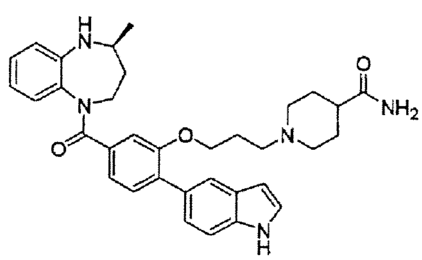 |

Table 2-32

| | | |
|---|---|---|
| 84 | $^1$H-NMR(DMSO-$d_6$,120˚C) δ: 1.26(3H,d,J=6.6Hz), 1.56-1.75 (3H,m), 1.88-2.02(1H,m), 2.44-2.52(2H,m), 2.55(4H,t,J=5.9Hz), 3.17(6H,s) 3.29(4H,t,J=5.9Hz), 3.35-3.85(4H,m), 3.90-4.4(1H, br) 4.87(1H,s), 6.37-6.41(1H,m), 6.45·6.6.54(1H,m), 6.63(1H,d, J=7.33Hz), 6.85-6.97(4H,m), 7.10-7.18(2H,m) 7.22-7.26(1H,m), 7.34(1H,d,J=7.1Hz), 7.56(1H,s), 10.65(1H,brs)ppm FABMS : 572(M+1) IR(KBR) : 3322, 2934, 1626, 1560, 1419, 1321, 1253, 1118cm$^{-1}$ | 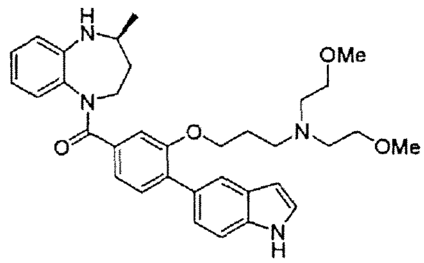 |
| 85 | $^1$H-NMR(DMSO-$d_6$,120˚C) δ: 0.85(6H,d,J=6,6Hz), 1.26(3H,d, J=6.6Hz), 1.55-1.70(3H,m), 1.89-1.94(3H,m), 2.31(2H,t, J=7.3Hz), 2.65(1H,m), 3.43(2H,br), 3.73(2H,m), 4.12(1H,m), 4.88(1H,br), 6.46-6.52(4H,m), 6.63(1H,d,J=7.3Hz), 6.87(1H,s), 6.90-6.94(3H,m), 7.11-7.16(2H,m), 7.24(1H,d,J=2.9), 7,24(1H,t, J=7.3Hz), 7.33(1H,d,J=8.8Hz), 7.56(1H,d,J=1.5Hz), 10.65(1H, s)ppm FABMS : 512(M+1) IR(KBr): 3414, 2986, 1626, 1562, 1419, 1321, 1143cm$^{-1}$ | 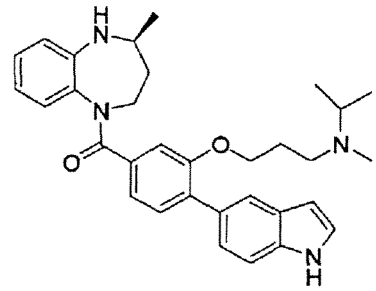 |

Table 2-33

| | | |
|---|---|---|
| 86 | $^1$H-NMR(DMSO-$d_6$,12O˚C)δ 1.26(311,d,J=6.8Hz), 1.60-1.75 (3H,m), 1.87-2.02(1H,m), 2.36(2H, t,J=6.8Hz), 2.43(2H, t, J=6.8Hz), 2.77-2.90(2H,m), 3.07-3.14(2H,m), 3.25-3.80(4H, m), 3.90-4.40(1H,m), 4.89(1H,s), 6.38-6.42(1H,m), 6.45-6.54 (1H,m), 6.63(1H,d,J=7.8Hz), 6.86(1H,brs), 6.91-6.97(3H,m), 7.09-7.21(3H,m), 7.24(1H,t,J=2.9Hz), 7.35(1H,d,J=8.8Hz), 7.55(1H,s), 10.66(1H,brs)ppm FABMS : 538(M+1) IR(KBr) : 3320, 2936, 1657, 1560, 1419, 1321cm$^{-1}$ | 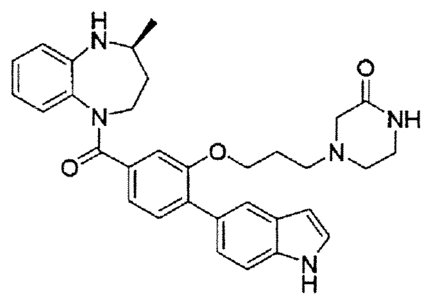 |
| 87 | $^1$H-NMR(DMSO-$d_6$, 120˚C) δ : 1.25 (3H, d, J=6.6 Hz), 1.30-1.50 (2H,m), 1.57-1.75(5H,m), 1.85-2.03(3H,m), 2.26 (2H, t, J=7.3 Hz), 2.56-2.68(2H,m), 3.30-3.80(5H,m), 3.90-4.40(1H,m), 4,88 (1H,s), 6.38 (1H, t, J=2.2 Hz), 6.45-6.54(1H,m), 6.63(1H,d, J=7,3Hz), 6.85-6.98(4H,m), 7.10-7.17(2H,m), 7.23-7.27(1H, m), 7.30-7.52(2H,m), 7.S5(1H,s), 7.95(1H,s), 10.66(1H,brs) ppm FABMS : 566(M+1) IR(KBr): 3308, 2934, 1671, 1622, 1557, 1419, 1321, 1253, 1127 cm$^{-1}$ | 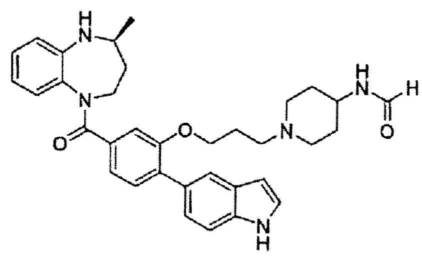 |

Table 2-34

| | | |
|---|---|---|
| 88 | $^1$H-NMR(DMSO-$d_6$, 120˚C) δ : 1.26(3H,d,*J*=6.6Hz), 1.55-1.76 (7H,m), 1.80-2.05(2H,m), 2.15-2.26(1H,m), 2.33-2.45(1H,m), 2.51.2.66(1H,m), 2.77-2.85(2H,m), 2.95-3.05(1H,m), 3.35-3.82(4H,m), 3.90-4.40(1H,br), 4.91(1H,s), 6,38-6.42(1H, m), 6.42-6.68(4H,m), 6.85-6.88(1H,m), 6.91-6.98(4H,m), 7.10-7.17(2H,m), 7.22-7.26(1H,m), 7.34(1H,d,*J*=8.1Hz), 7.56 (1H,s), 10.66(1H,brs)ppm FABMS : 553(M+1) IR(KBr): 3420, 2986, 1671, 1626, 1560, 1419, 1321, 1255cm$^{-1}$ | 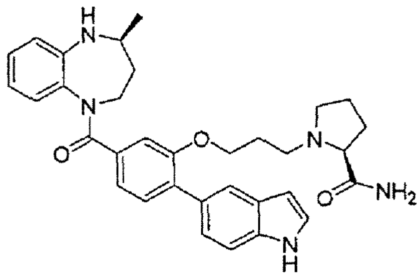 |
| 89 | $^1$H-NMR(DMSO-$d_6$,120˚C) δ : 1.25(3H,d,*J*=5.9). 1.66(1H,m), 1.91(1H,m), 3,15(2H,t,J=5.1Hz), 3.43(3H,t,*J*=4.4Hz), 3.65 (1H,br), 4.08(1H,br), 4.35(1H,s), 4,81(1H,m), 6.41-6,53(2H, m), 6.66(1H,d,j=7.3Hz), 6.78(1H,m), 6.90(3H,t,*J*=3.6Hz), 7.02 (1H,dd,*J*=8.8,2.2Hz), 7.29(1H,m), 7.43(2H,m), 7.72(2H,s), 10.70(1H,br)ppm FABMS : 511(M+1) IR(KBr) : 3420, 2968, 1630, 1564, 1423, 1321, 1245, 1116cm$^{-1}$ | 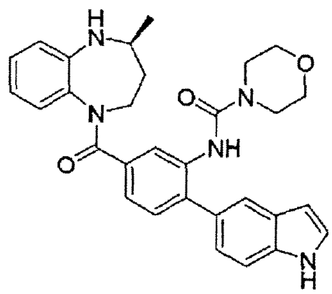 |
| 90 | $^1$H-NMR(DMSO-d6,120˚C) δ: 1.26(3H,d,*J*=6.6), 1.68(1H,m), 1.93(3H,m), 3.43(1H,br), 3.66(2H,m), 3.86(2H,t,*J*=6,6Hz), 4.06(1H,br), 4.87(1H,br), 6.41(2H,m), 6.61(1H,m), 6.82-6.98 (5H,m) 7.12-7.17(2H,m), 7.26(1H,m), 7.38(1H,d,*J*=8.1Hz), 7.58(1H,s), 10.70(1H,s)ppm FABMS: 507(M+1) IR(KBr) : 3328, 2932, 1626, 1560, 1419, 1321, 1253, 1110cm$^{-1}$ | 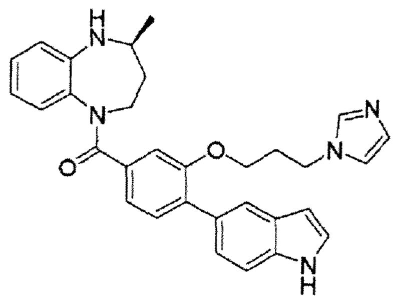 |

Table 2-35

| | | |
|---|---|---|
| 91 | $^1$H-NMR(DMSO-$d_6$,120˚C) δ : 1.26(3H,d,*J*=6.6Hz), 1.58-2.08 (4H,m), 2.80(2H,m), 3.40-3.56(8H,m), 3.85-4.40(1H,br), 4.70-5,10(1H,br), 6.41(1H,t,*J*=2.9Hz), 6.46-6.54(1H,m), 6.61-6.66(1H,m), 6.82-6.88(1H,m), 6.90-6.98(3H,m), 7.13 (1H,d,*J*=8.1Hz), 7.15-7.26(2H,m), 7.35(1H,d,*J*=8.1Hz), 7.59 (1H,s), 10.65(1H,bs)ppm FABMS: 416(M+1) IR(KBr): 3344, 2938, 1771, 1705, 1626, 1415, 1321, 1249, 1166cm$^{-1}$ | 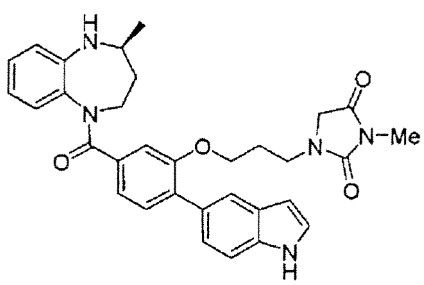 |

(continued)

| | | |
|---|---|---|
| 92 | $^1$H-NMR(DMSO-$d_6$, 120˚C) δ : 1.26(3H,d,*J*=6.6Hz), 1.64(3H, m), 1.91(1H,m), 2.52-2.57(3H,m), 3.22(1H,m), 3.41(3H,t, *J*=5.9Hz), 3.76(3H,m), 4.12(1H,br), 4.90(1H,br), 6.39(1H,s), 6.50(1H,m) 6.88-6.94(4H,m), 7.11-7.16(2H,m), 7.24(1H,t, *J*=2.9Hz), 7.34(1H,d,*J*=8.8Hz), 7.56(1H,s), 10.66(1H,s)ppm FABMS: 526(M+1) IR(KBr): 3406, 2934, 1622, 1562, 1421, 1321, 1253, 1125cm$^{-1}$ | 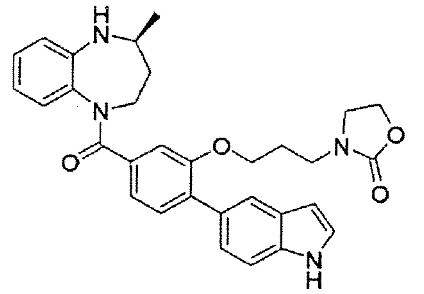 |
| 93 | $^1$H-NMR(DMSO-$d_6$, 120˚C) δ: 1.25(3H,d,*J*=6.6Hz), 1.37(2H, m), 1.63(7H,m), 1.95(3H,m), 2.24(1H,t,*J*=6.6Hz), 2.55(2H,m), 3.72(3H,m), 4.18(1H,br), 4.88(1H,br), 6.38(1H,s) 6.50(1H,m), 6.63(1H,d,*J*=7.3Hz), 6.87(1H,s), 6.94(3H,m), 7,13(2H,m), 7.25(1H,t,*J*=2.9Hz), 7.34(1H,d,*J*=8.8Hz), 7.56(1H,s), 10.63 (1H,br)ppm FABMS : 540(M+1) IR(KBr): 3316, 2936, 1622, 1560, 1419, 1321, 1133cm$^{-1}$ | 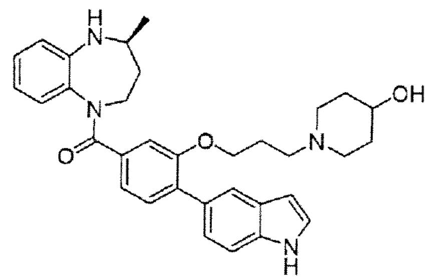 |

Table 2-36

| | | |
|---|---|---|
| 94 | $^1$H-NMR(DMSO-$d_6$, 120˚C) δ: 1.26(3H,d,*J*=5.9Hz), 1.68(1H,m), 1.96 (1H,m), 2.06(4H,m), 3.21(2H,d,*J*=1.5Hz), 3.47(5H,t,*J*=5,1Hz), 4.03 (1H,br), 4.89(1H,br), 6.39-6.46(2H,m), 6.69(1H,d,*J*=7.3Hz), 6.85-6.93(2H,m), 7.01(1H,dd,*J*=8.8,2.2Hz), 7.08(1H,d,*J*=7.3Hz), 7.22(1H,dd,*J*=8.1,2.2Hz), 7.26-7.30(3H,m), 7.37(1H,d,*J*=8.0Hz), 7.45(1H,s), 10.70(1H,br)ppm FABMS : 482(M+1) IR(KBr) : 3324, 2962, 1626, 1410, 1321, 1116cm$^{-1}$ | 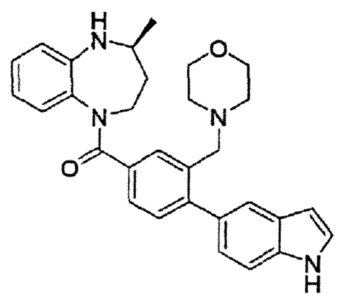 |
| 95 | $^1$H-NMR(DMSO-$d_6$,120˚C) δ: 1.25(3H,d,*J*=6.8Hz), 1.48-1.75(8H,m), 1.88-2.02(1H,m), 2.25-2.40(6H,m), 3.33-3.80(4H,m), 3.95-4.40(1H, m), 4.88(1H,s), 6.36-6.40(1H,m), 6,45-6.54(1H,m), 6.63(1H,d, J=6.8Hz), 6.85-6.97(4H,m), 7.10-7.17(2H,m), 7.24(1H,t,*J*=2.9Hz), 7.34(1H,d,*J*=8.8Hz), 7.55(1H,s), 10.65(1H,s)ppm FABMS : 581 (M+1) IR(KBr) : 3444, 2946, 1628, 1562, 1400, 1321, 1100cm$^{-1}$ | 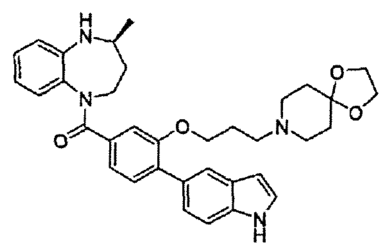 |

Table 2-37

| | | |
|---|---|---|
| 96 | $^1$H-NMR(DMSO-$d_6$,120˚C) δ : 1.26(3H,d,*J*=6.6Hz), 1.50-1.75 (7H,m), 1.85-2.02(1H,m), 2.27(2H,d,*J*=7.3Hz), 2.69-2.75(2H, m), 3.20-3.85(4H,m), 3.90-4.40(1H,br), 4.88(1H,s), 6.37-6.41 (1H,m), 6.46-6.54(1H,m), 6.64(1H,d,*J*=7.3Hz), 6.84-6.89(1H, m), 6.90-6.98(4H,m), 7.10-7.17(2H,m), 7.24 (1H, t, *J*=2.9 Hz), 3.34(1H,t,*J*=8.8Hz), 7.56(1H,s), 10.69(1H,br)ppm FABMS:594 (M+1) IR(KBr) : 3406, 2932, 1626, 1560, 1419, 1321, 1255, 1122cm$^{-1}$ | 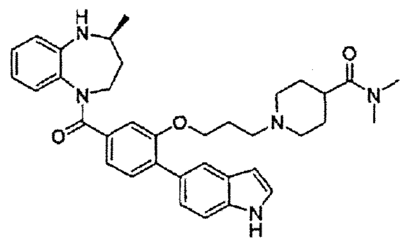 |
| 97 | $^1$H-NMR(DMSO-$d_6$, 120˚C) δ : 1.25(3H,d,*J*=6.6Hz), 1.37(2H, m), 1.63(7H,m), 1.95(3H,m), 2.25(2H,t,*J*=6.6Hz), 2.55(2H,m), 3.42(3H,br), 3.72(2H,m), 3.90(2H,br), 4.14(1H,br), 4.88(1H,br), 6.38(1H,m), 6.63(1H,d,*J*=7.3Hz), 6.86(1H,s), 6.91-6.94(3H,m), 7.11-7.15(2H,m), 7.25(1H,t,*J*=2.9Hz), 7.34(1H,d,*J*=8.8Hz), 7.56(1H,s), 10.63(1H,s)ppm FABMS : 539(M+1) IR(KBr): 3342, 2938, 1773, 1709, 1620, 1458, 1421, 1321, 1158cm$^{-1}$ | 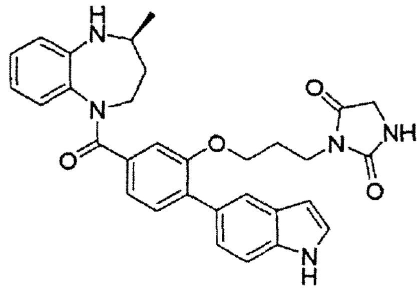 |

Table 2-38

| | | |
|---|---|---|
| 98 | $^1$H-NMR (DMSO-$d_6$, 120˚C) δ: 1.26(3H,d,*J*=6.6Hz), 1.71(3H, m), 1.96(1H,m), 2.24(3H,t,*J*=5.8Hz), 2.41(2H,t,*J*=7.3Hz), 2.58 (2H,t,*J*=5.8Hz), 3.43(2H,br), 3.78(2H,m), 4.12(1H,br), 4.90(1H, br), 6.38(1H,t,*J*=2.9Hz), 6.47-6.52(1H,m), 6.63(1H,d,*J*=7.3Hz), 6.90(1H,s), 6.93(3H,m), 7.11-7.15(2H,m), 7.24(1H,t,*J*=2.2Hz), 7.34(1H,d,*J*=8.8Hz), 7.57(1H,s), 10.66(1H,s)ppm FABMS: 538 (M+1) IR(KBr) : 3406, 2964, 1715, 1626, 1562, 1419, 1321, 1127cm$^{-1}$ | 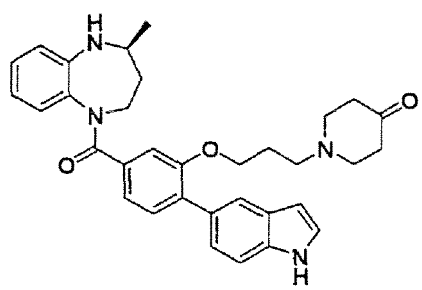 |
| 99 | $^1$H-NMR(DMSQ-$d_6$,120˚C) δ: 0.94(6H,d,*J*=6.6Hz), 1.25(3H,d, *J*=6.6Hz), 1.58.1.75(3H,m), 1.88-2.02(1H,m), 2.20-2.33(6H, m), 2.34-2.43(4Hm), 2.61-2.66(1H,m), 3.20-3.85(4H,m), 3.90-4.36(1H,br), 4.88(1H,s), 7.59(1H,d,*J*=8.1Hz), 6.37-6.41 (1H,m), 6.46-6.54(1H,m), 6.64(1H,d,*J*=7.3Hz), 6.85-6.98(4H, m), 7.10-7.17(2H,m), 7.24(1H,t,*J*=2.9Hz), 7.34(1H,d,*J*=8,8Hz), 7.56(1H,s), 10.65(1H,s)ppm FABMS:566(M+1) IR(KBr): 3334, 2968, 1626, 1560, 1419, 1321, 1149cm$^{-1}$ | 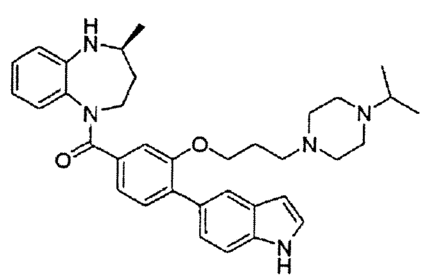 |

Table 2-39

| | | |
|---|---|---|
| 99 · 1/2 fumarate | $^1$H -NMR(DMSO-$d_6$,120˚C) δ:0.95(GH,d,*J*=6.6Hz), 1.26(3H,d,*J*=6.GHz),1.6 2-1.67(3H,m),1.91-1.98(1H, m),2.24-2.46(10H, m),2.68-2.62(1H,m),3.40-4.09(5H, m),6.38-6.64 (4H,m), 6.88-6.94(4H,m), 7.11-7.14(2H, m), 7.24-7.25(1H,m),7.34(1H,d,J=8.1Hz),7.56(1H,S), 10,7(1H,s)ppm IR(KBr):3330, 2964, 1628, 1595, 1497, 1419, 1390, 1321, 1253, 1I41, 1033, 806, 758, 667cm$^{-1}$ Melting point: 203˚C~205˚C | |
| 99· male -ate | $^1$H-NMR(DMSO-$d_6$,120˚C) δ : 1.14(6H,d,*J*=6.6Hz), 1.25(3H,d,*J*=6.6Hz),1.69 1.76(3H,m),1.91-1.98(1H, m),2.60-3,12(11H,m) ,3.43-4.03(6H,m),6.00(2H,s), 6.40(1H,s),6.49-6. 53(1H,m),6.64(1H,d,J=8.8Hz), 6.89-6.94(4H,m) ,7.10-7.15(2H,m),7.26-7.27(1H,m), 7.36(1H,d,J =8,1Hz),7.56(1H,s),10.7(1H,s)ppm IR (KBr) : 3330, 2948, 1632, 1589, 1499, 1419, 1386, 1321, 1255, 1007, 864, 756, 567 cm$^{-1}$ Melting point: 160˚C~161˚C | |
| 100 | $^1$H-NMR(DMSO-$d_6$, 120˚C) δ: 1.26(6H,d,*J*=6.6Hz), 1.60-2.00(4H,m), 2.65-3.20(1H,m), 3.30-3.60(4H,m), 3.65-3.77(2H,m), 3.90-4.35(1H,br), 6.39-6.43(1H,m), 6.46-6.54(1H,m), 6.63(1H,d,*J*=7.3Hz), 6.83-6.88(1H, m), 6.90-6.99(3H,m), 7.12(1H,s), 7.14-7.18(1H,m), 7.19(1H,d,*J*=1.5Hz), 7.23(1H,t,*J*=2.6Hz), 7.35(1H,d, *J*=8.8Hz), 7.68(1H,s), 10.66(1H,s)ppm FABMS:555 (M+1) IR(KBr): 3406, 2934, 1682, 1626, 1390, 1321, 1170cm$^{-1}$ | 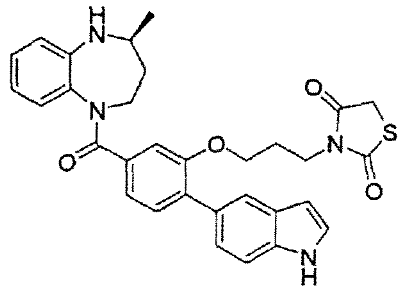 |

Table 2-40

| | | | |
|---|---|---|---|
| 101 | $^1$H-NMR (DMSO-$d_6$:120˚C) δ : 1.25(3H,d, *J*=6.6Hz), 1.58-1.78(1H,m), 1.84-2.02(1H, m), 2.20-4.50(11H,m), 4.92(1H,brs), 6.41-6,52(1H,m), 6.61(1H,d,*J*=8.1Hz), 6.89-6.98(2H,m), 7.00-7.09(2H,m), 7.26-7.33(2H,m), 7.34-7.45(2H,m), 7.48-7.53(1H,m), 8.14(1H,brs)ppm FABMS:495 IR(KBr): 3418, 1626,1408, 1321, 1114cm$^{-1}$ | 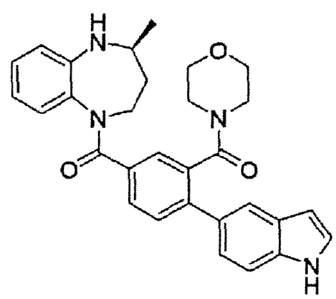 | |
| 102 | $^1$H-NMR (DMSO-$d_6$:120˚C) δ: 1.25(3H,d, *J*=5.9Hz), 1,45-2.10(5H,m), 2.30-4.50(11H, m), 4.91(1H,br), 6,38-6.50(2H,m), 6.61(1H, d,*J*=8.1Hz), 6.88-6.96(2H,m), 6.96-7.08 (2H,m), 7.25-7.31(2H,m),7.32-7.42(2H,m), 7.47-7.53(1H,m), 10.60-11.90(1H,br)ppm FABMS:508(M+1) IR(KBr): 3428, 2940, 1626, 1446, 1323cm-$^1$ | 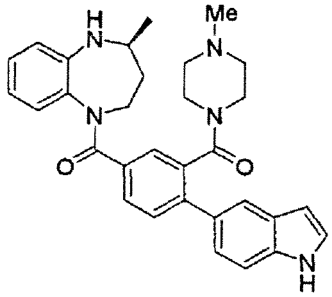 | |

(continued)

| | | |
|---|---|---|
| 103 | $^1$H-NMR (DMSO-$d_6$:120˚C) δ : 1.25(3H,d, *J*=6,6Hz), 1.31-1.44(2H,m), 1.45-1.58 (2H.m), 1.60-1.77(1H,m), 1.86-2.01(1H,m), 2.12-2.29(13H,m), 3.30-4.50(5H,m), 4.90 (1H,brs), 6.38(1H,s), 6.44-6.58(1H,m), 6.63 (1H,d,*J*=6.3Hz), 6.85(1H,s), 6.90-7.00(3H, m), 7.10-7.19(2H,m), 7.33(1H,d,*J*=8.8Hz), 7.56(1H,s), 10.65(1H,brs)ppm FABMS:552 (M+1) IR(KBr): 3328, 2940, 1624, 1560, 1419, 1321, 1266cm$^{-1}$ | 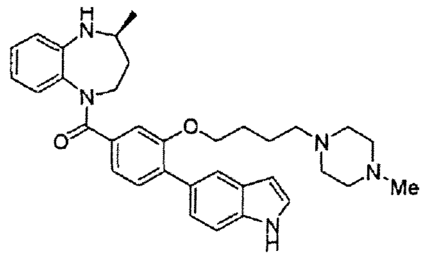 |

Table 2-41

| | | |
|---|---|---|
| 104 | $^1$H-NMR (DMSO-$d_6$: 120˚C) δ: 1.25 (3H, d, *J*=7.8 Hz), 1.55-1.79 (1H,m), 1.83-2.01(1H,m), 2.13(3H,s), 2.28-2.38(4H,m), 2,47-2.54(4H,m), 3.30-3.65(5H,m), 3.70-3.87(2H,m), 3.95-4.45(1H,br), 4.79(1H,br), 6.30-6.41 (2H, m), 6.60 (1H, d, *J*=7.8 Hz), 6.76(1H,s), 6.85(1H,s), 6.92-7.00(1H,m), 7.10-7.28 (3H,m), 7.34 (1H, d, *J*=8.8 Hz), 7.59(1H,s), 10.65(1H,brs)ppm FABMS:525(M+1) IR(KBr): 3332, 2928, 1626, 1560, 1417, 1323, 1118 cm$^{-1}$ | 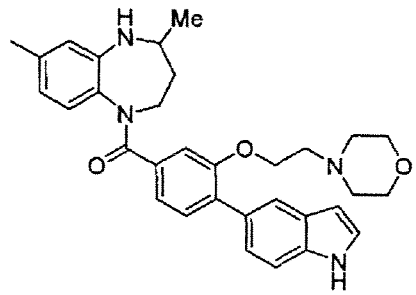 |
| 105 | $^1$H-NMR (DMSO-$d_6$:120˚C) δ : 0.82 (3H, t, *J*=7.8 Hz), 1.24 (3H, d, *J*=6.8 Hz), 1.30-1.44(2H,m), 1.59-1.75(3H,m), 1.87-2.00(1H, m), 2.11.(3H,s), 2.22 (2H, t, *J*=7.8 Hz), 2.30 (2H, t, *J*=7.8 Hz), 2.71 (3H, d, *J*=4.9 Hz), 3.30-3.83(4H,m), 3.90-4.30(1H,br), 4.86 (1H,brs), 5.15-5.29(1H,m), 6.41-6.62(4H,m), 6.79-6.97(4H,m), 7.04(2H,d,*J*=7.8Hz), 7.20(1H,d,*J*=7.8Hz)ppm FABMS=502 (M+1) IR(KBr): 3362, 2964, 1615, 1557, 1390, 1323, 1189cm$^{-1}$ | 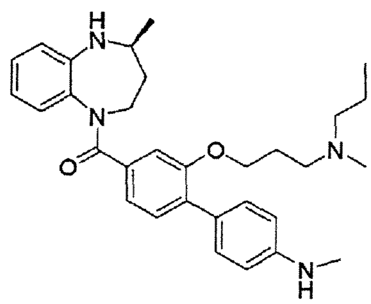 |
| 106 | $^1$H-NMR (DMSO-$d_6$:120˚C) δ: 1.25(3H,d,*J*=6.8Hz), 1.58.1.76 (1H,m), 1.80-2.01(4H,m), 2.71(3H,d,*J*=5.9Hz), 3.32-3.77(5H, m), 3.81(2H,s), 3.90-4.30(1H,br), 4.88(1H,brs), 5.14-5.29(1H, m), 6.42-6.65(4H, m), 6.80(1H,s), 6.89-6.99(3H, m), 7.05(1H, d, *J*=7.8Hz), 7.23(2H,d,*J*=8.8Hz), 7.50(1H,br)ppm FABMS:527 (M+1) IR(KBr): 3350, 2934, 1773, 1709, 1609, 1456, 1323, 1191, cm$^{-1}$ | 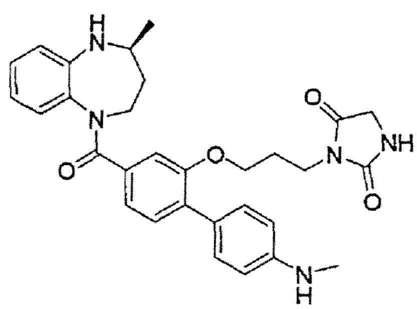 |

Table 2-42

| | | |
|---|---|---|
| 107 | $^1$H-NMR (DMSO-$d_6$:120˚C) δ : 1.23-1.47(4H,m), 1.58-2.15(6H, m), 2.26(2H,t,*J*=7.3Hz), 2.40-2.53(4H,m), 2.60-3.10(4H,m), 3.31-4.50(7H,m), 4.89(1H,brs), 6.38-6.42(1H,m), 6.47-6.58 (1H,m), 6.85-7.00(4H,m), 7.26(1H,t,*J*=2.9Hz), 7.35(1H,d, *J*=8.8Hz), 7.58(1H,s), 10.67(1H,brs)pprm. FABMS:608(M+1) IR(KBr): 3328, 2956, 1628, 1560, 1419, 1321, 1120cm$^{-1}$ | 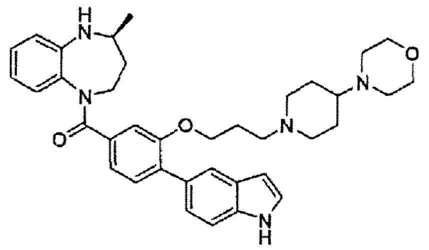 |
| 108 | $^1$H-NMR (DMSO-$d_6$:120˚C) δ: O.89(3H,d,*J*=6,6Hz), 1.31(9H, m), 1.63(6H,m), 1.92(1H,m), 2.09(3H,s), 2.22(2H,m), 2.31(2H, m), 3.45(1H,br), 3.73(2H,m), 4.88(1H,br), 6.38(1H,s), 6.49(1H, m), 6,63(1H,d,*J*=7.3Hz), 6.88(1H,s), 6.93(3H,m), 7.11(2H,m), 7.24(1H,t,*J*=2.9Hz), 7.33 (1H,d,*J*=8.1Hz), 7.56(1H,s), 10.65 (1H,br)ppm FABMS:539(M+1) IR(KBr): 3320, 2934, 1628, 1560, 1419, 1321, 1253, 1123cm$^{-1}$ | 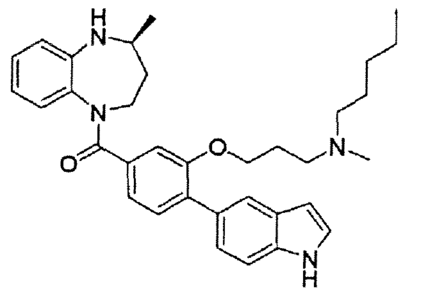 |
| 109 | $^1$H-NMR (DMSO-$d_6$:120˚C) δ : 1.25(3H,d,*J*=6.8Hz), 1.55-1.78 (5H,m), 1.90-2.03(1H,m), 2.23(3H,s), 2.35-2.65(10H,m), 2.80-3.05(3H,m), 2.23(3H,s), 2.35-2.65(10H,m), 2.80-3,05 (3H,m), 3.30-3.83(4H,m), 3.95-4.32(1H,m), 4.88(1H,s), 6.36-6.40(1H,m), 6.45-6.53(1H,m), 6.63(1H,d, *J*=6.8Hz), 6.85-6.89(4H,m), 7.09-7.17(2H,m), 7.24(1H,t,*J*=2.9Hz), 7.34 (1H.d,*J*=8.8Hz), 7.55(1H,s), 10.66(1H,brs)ppm FABMS:552 (M+1) IR (KBr): 3336, 2936, 1626, 1562, 1419, 1321, 1255, 1125cm$^{-1}$ | 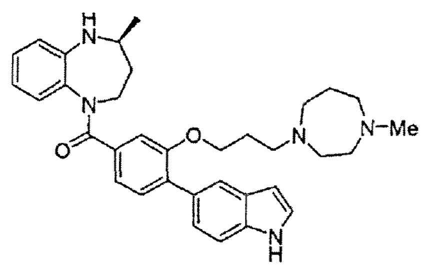 |

Table 2-43

| | | |
|---|---|---|
| 110 | $^1$H-NMR (DMSO-$d_6$:120˚C) δ: 1.26(3H,d, *J*=6.8Hz),1.65-1.68(1H,m), 1.92(1H,br), 2.13(3H, s), 2.23-2.27(4H,m), 2.46-2.51(2H,m), 3.42(2H, br), 3.76-3.80(2H,m), 4.12(1H,br), 4.90(1H,brs), 6.39(1H,s), 6.47-6.51(1H,m), 6.63(1H,d, *J*=7.8Hz), 6.86-6.96(4H,m), 7.12.7.19(2H,m), 7,24 (1H,t,*J*=2.9Hz), 7.33(1H,d,*J*=8.8Hz), 7.59(1H,s), 10.65(1H,br)ppm FABMS:524(M+1) IR(KBr): 3318, 2940, 1626, 1560, 1417, 1321, 1255, 1168, cm$^{-1}$ | 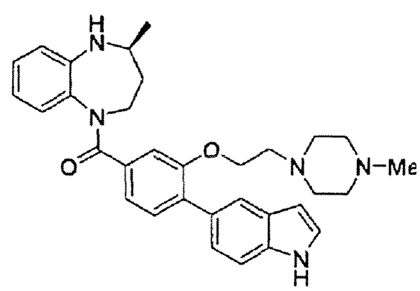 |

(continued)

| | | |
|---|---|---|
| 110 · 1/2 fuma -rate | $^1$H-NMR(DMSO-$d_6$, 120˚C) δ: 1.26(3H, d, *J*=6.6Hz), 1.69(1H, m), 1.96(1H, m), 2.14(3H, s), 2.34(8H, m), 3.43(1H, br), 3.78(2H, t, *J*=5.8Hz), 4.05(1H, br), 6.38(1H, s), 6.51(1H, m), 6.60(2H, m), 6.86(1H, s), 6.94(3H, m), 7.12-7.18(2H, m), 7.24(1H, t, *J*=2.9Hz), 7.33(1H, d, *J*=8.0Hz), 7.59 (1H, s), 10.67(1H, br) IR(KBr) : 3402, 2956, 1647, 1483, 1450, 1383, 1340, 1319, 1257, 1168, 1141, 1054, 986, 888, 804,756,671 $cm^{-1}$ Melting point: 209˚C~210˚C | |

Table 2-44

| | | |
|---|---|---|
| 110· 2 maleate | $^1$H-NMR(DMSO-$d_6$,120˚C) δ : 1.26(3H, d, *J*=6.8Hz), 1.69 (1H, m), 1.94(1H, m), 2.66(9H, m), 2.93(4H, m), 3.45(1H, m), 3.85(2H, m), 4.12(1H, m), 6.09(4H, m), 6.41(1H, s), 6.51(1H, m), 6.64(1H, d, *J*=7.8Hz). 6.94(4H, m), 7.12(2H, d, *J*=6.8Hz), 7.27(1H, m), 7.36(1H, d, *J*=8.3Hz), 7.56(1H, s), 10.67(1H, brs) ppm IR(KBr) 3340, 2938, 1618, 1578, 1475,1388, 1357, 1321, 1253, 1193, 1071, 866, 758, 648, 569 $cm^{-1}$ Melting point : 112˚C~113˚C | |
| 111 | $^1$H-NMR (DMSO-$d_6$:120˚C) δ : 1.25(3H,d,*J*=6.8Hz), 1.58-1.77(1H,m), 1.85-2.02(1H,m), 2.28-2.37(4H,m), 2.40-2.58(4H,m), 3.25-3.70(6H,m), 3.75-3.92(2H,m), 4.11(1H,brs), 6.42-6.57(1H,m), 6.62(2H,d,*J*=6.8Hz), 6.88-7.00(4H,m), 7.14(1H,d,*J*=7.8Hz), 7.26 (1H, d, *J*=7.8 Hz) ppm FABMS:556(M+1) IR(KBr): 3332, 2940, 1634, 1562, 1404, 1118 $cm^{-1}$ | 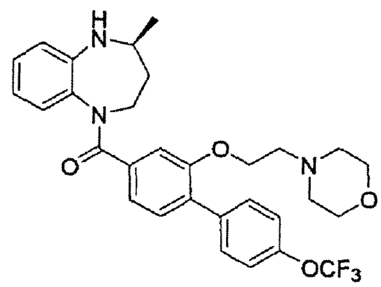 |
| 112 | $^1$H-NMR (DMSO-$d_6$:120˚C) δ: 0.83 (3H, t, *J*=7.3 Hz), 1.24 (9H,m), 1.65(3H,m), 1.91(1H,m), 2.07(3H,s), 2.20(2H,t, J=6,6Hz), 2.29(2H,t,J=6.6Hz), 2.83(6H,s), 3.43(2H,br), 3.73(3H,m), 4.11(1H,br), 4.88(1H,br), 6.38(1H,s), 6.49 (1H,m), 6.63(1H,d,*J*=7.3Hz), 6.87(1H,s), 6.93(3H,m), 7.11(2H,m), 7.24(1H,t,*J*=2.9Hz), 7,33(1H,d,*J*=8.8Hz), 7.56(1H,s), 1064(1H,br)ppm FABMS:526(M+1) IR(KBr): 3320, 2934, 1626, 1562, 1419, 1321, 1255, 1122$cm^{-1}$ | 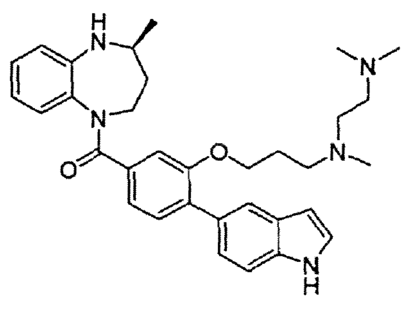 |

Table 2-45

| | | |
|---|---|---|
| 113 | $^1$H-NMR (DMSO-d6:120˚C) δ : 0.83(3H,t,*J*=7.3Hz), 1.14-1.38(8H, m), 1.58-1.77(3H,m), 1.88-2.10(4H,m), 2.18-2,32(4H,m), 3.20-4.50(1H,d,*J*=7.3Hz), 6.84-6.89(1H,m), 6.90-6.98(3H,m), 7.09-7.18(2H,m), 7,24(1H,t,*J*=2.9Hz), 7.33(1H,d,*J*=8.8Hz), 7.56 (1H,s), 10.50-10.78(1H,br)ppm FABMS:566(M+1) IR(KBr): 3326, 2962, 1626, 1560, 1419, 1321, 1160$cm^{-1}$ | 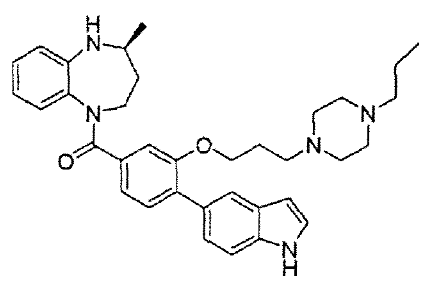 |

(continued)

| | | |
|---|---|---|
| 114 | $^{1}$H-NMR (DMSO-$d_6$:120˚C) δ: 0.84(3H,t,*J*=7.3Hz), 1.25(3H,d, *J*=6.6Hz), 1.31-1.49(2H,m), 1.55-1.78(3H,m), 1.90-2.04(1H,m), 2.16-2,40(10H,m), 3.30-4.35(5H,m), 4.88(1H,brs), 6.36-6.42(1H, m), 6.45-6.57(1H,m), 6.63(1H,d,*J*=7.3Hz), 6.82-7.00(4H,m), 7.08-7.19(2H,m), 7.24(1H,t,*J*=2.9Hz), 7.34(1H,d,*J*=8.8Hz), 7.56 (1H,s), 10.65(1H,brs)ppm FABMS:566(M+1) IR(KBr): 3324, 2960, 1626, 1562, 1419, 1321, 1253, 1125 $cm^{-1}$ | 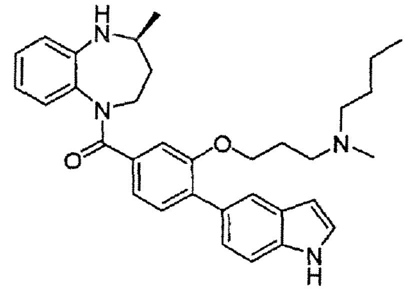 |

Table 2-46

| | | |
|---|---|---|
| 115 | $^{1}$H-NMR (DMSO-$d_6$:120˚C) δ : 0.99(6H,d,*J*=6.6Hz), 1.25(3H,d, J=6.6Hz), 1.47-1.78(5H,m), 1.86-2.03(1H,m), 2.23(2H,t, J=7.3Hz), 2.40-2.60(2H,m), 3.30-3.89(4H,m), 3.90-4.40(1H,br), 4.88(1H,brs), 6.38(1H,d,*J*=2.9Hz), 6.43-6.58(1H,m), 6.63(1H,d, *J*=7.3Hz), 6.87-6.98(4H,m), 7.10-7.17(2H,m), 7.24(1H,d, J=2.9Hz), 7.34(1H,d,*J*=8.1Hz), 7.56(1H,s), 10.66(1H,brs)ppm FABMS:553(M+1) IR(KBr): 3326, 2972, 1626, 1560, 1419, 1323, 1153, 1145$cm^{-1}$ | 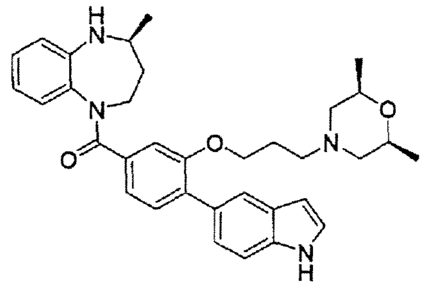 |
| 116 | $^{1}$H-NMR (DMSO-$d_6$:120˚C) δ: 0.86(3H,t,*J*=7.3Hz), 1.25(3H,d, *J*=6.6Hz), 1.54-1.78(3H,m), 1.85-2.03(1H,m), 2.20-2.45(6H,m), 3.20-3.85(4H,m), 3.90-4.40(1H,br), 4.87(1H,brs), 6.88(1H,brs), 6.42-6.55(1H,m), 6.80-7.00(4H,m), 7.08-7.20(2H,m), 7.24(1H, brs), 7.33(1H,d,*J*=8.1Hz), 7.56(1H,s), 10.65(1H,brs)ppm FABMS: 511(M+1) IR(KBr): 3322, 2972, 1626, 1562, 1419, 1321, 1253, 1125 $cm^{-1}$ | 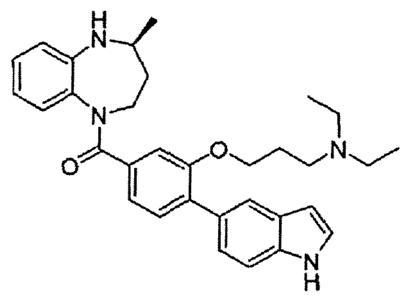 |

Table 2-47

| | | |
|---|---|---|
| 117 | $^{1}$H-NMR, (DMSO- $d_6$:120˚C) δ :0.80(3H,t, *J*=7.3Hz), 1.26(4H,*J*=6.6Hz), 1.36(4H,m), 1.52 (2H,m), 1.67(1H, m), 1.96(1H,m)2.5(3H,s), 2.19 (4H,m), 3.43(1H,br), 3.70(2H,dt,*J*=5.9,2.2Hz), 4.12(1H,br), 4.90(1H,br), 6.38(1H,s), 6.49(1H, m), 6.63(1H,d,*J*=7.3Hz), 6.86(1H,s), 6.94(3H, m), 7.14(2H,m), 7.24(1H,m), 7.33(1H,d, *J*=8.8Hz), 7.56(1H,s), 10.66(1H,br) FABMS:525 (M+1) IR(KBr): 3324, 2962, 1624, 1560, 1417, 1321, 1251, 1125 $cm^{-1}$ | 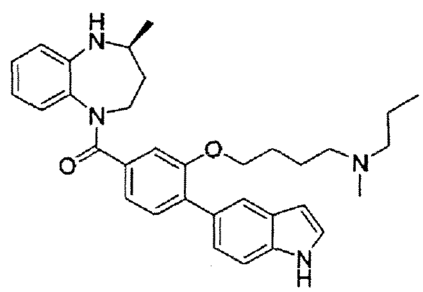 |

(continued)

| | | |
|---|---|---|
| 117 · 1/2 fuma -rate | $^1$H-NMR(DMSO-$d_6$, 120˚C) δ: 0.81(3H, t, *J*=7.3Hz), 1.26(3H, d, *J*=6.6Hz), 1.37(4H, m), 1.51(2H, q, *J*=6,6Hz), 1.65(1H, m), 1.96(1H, m), 2.08(3H,s), 2.21(4H, m), 3.43(1H, br), 3.71(2H, td, *J*=5.9, 2.2Hz), 4.11(1H, br), 6.38(1H, s), 6.49 (1H, m), 6.60(1H, s), 6.63(1H, d, *J*=7.3 Hz), 6.86 (1H, s), 6.94(3H, m), 7.14(2H, m), 7.24(1H, t, *J*=2.2 Hz), 7.33(1H, d, *J*=8.0 Hz), 7,56(1H, s), 10.65(1H, br)ppm IR(KBr) : 3330, 2968, 1630, 1597, 1419,1388, 1321, 1255, 1125, 756 $cm^{-1}$ Melting point : 164˚C~166˚C Anal. Calcd for $C_{35}H_{42}N_4O_4 \cdot H_2O$: C, 69.98; H, 7.38; N, 9.33. Found: C, 70.15; H, 7.31; N, 9.27 | |

Table 2-48

| | | |
|---|---|---|
| 117 · fuma -rate | $^1$H-NMR (DMSO-$d_6$, 120˚C) δ: 0.81 (3H, t, J=7.3Hz), 1.26(3H, d, *J*=5.9 Hz), 1.37(4H, m), 1.51(2H, q, *J=5.9Hz),* 1.65(1H, m), 1.96(1H, m), 2.20(3H, s), 2.26(4H, m), 3.45(1H, br), 3.71(2H, td, *J*=5.9, 2.2Hz), 4.11(1H, br), 6.38(1H, s), 6.49(1H, m), 6.60(2H, s), 6.63(1H, d, J=7.3Hz), 6.86(1H, s), 6.92(3H, m), 7.14(2H, m), 7.24(1H, t, *J*=2.9 Hz), 7.33(1H, d, J=8.8Hz), 7.56(1H, s), 10.65(1H, br)ppm IR(KBr) : 3327, 2968, 1631, 1593, 1419, 1384, 1320, 1262, 1180, 754 $cm^{-1}$ Melting point: 156˚C~157˚C Anal. Calcd far $C_{37}H_{44}N_4O_6$. $H_2O$ :C, 67.46; H, 7.04; N, 8.50. Found: C, 67.76; H, 7.31; N, 8.56. | |
| 117 · male -ate | $^1$H-NMR(DMSO-$d_6$,120˚C) δ : 0.84(3H, t, J=7.3Hz), 1.26(3H, d, J=6.6Hz), 1.60(7H, m), 1.94(1H, m), 2.71(3H, t, *J*=7,3Hz), 2.79(3H, s), 3.43(1H, br), 3.78(2H, s), 4.12(1H, br), 5.99 (2H, s), 6.40(1H, s), 6.49(1H, m), 6.63(1H, d, *J*=7.3Hz), 6.90(4H, m), 7.09(2H, m), 7.26(1H, t, *J*=2,9Hz), 7.36(1H, d, *J*=8.8Hz), 7.56(1H, s), 10.67(1H, br)ppm IR(KBr): 3330, 2930, 1702, 1632, 1504, 1419, 1386, 1365, 1253, 1216, 1046, 866, 754 $cm^{-1}$ Melting point: 164˚~166˚C | |
| 117 · oxalate | $^1$H-NMR(DMSO-$d_6$,120˚C)δ : 1 0.82(3H, t, *J*=7.3Hz), 1.26(3H, d, *J*=6.6Hz), 1.43(6H, m), 1.65 (1H, m), 1.96(1H, m), 2.26(3H, s), 3.73(2H, m), 4.09(1H, br), 6.40(1H, s), 6.49(1H. m), 6.63(1H, d, *J*=8.8Hz), 6.94(4H, m), 7.10(2H, m), 7.24(1H, s), 7.34(1H, d, *J*=8.0Hz), 7.66(1H, s), 10.67(1H, br)ppm IR(KBr) : 3326, 2962, 1628, 1597, 1499, 1450, 1417, 1386, 1319, 1255, 1125, 1104, 756 $cm^{-1}$ Melting point 170˚C~171˚C | |

Table 2-49

| | | |
|---|---|---|
| 117 · citrate | $^1$H-NMR(DMSO-$d_6$, 120˚C) δ: 0.82(3H, t, *J*=7.3Hz), 1.25(3H, d, *J*=6.6Hz), 1.43(6H, m), 1.69 (1H, m), 1.96(1H, m), 2.24(3H, s), 2.58(4H, m), 3.73(2H, m), 4.09(1H, br), 6.39(1H, s), 6.49 (1H, m), 6.63(1H, d, *J*=8.8Hz), 6.93(4H, m), 7.13(2H, m), 7.25(1H, t, *J*=2.9Hz), 7.34(1H, d, *J*=8.8Hz), 7.56(1H, s), 10.65(1H, br)ppm IR(KBr): 3330, 2964, 1717, 1632, 1599, 1502, 1419, 1386, 1321, 1257, 1135, 754 $cm^{-1}$ Melting point: 165˚C~166˚C | |
| 117 · tart -rate | $^1$H-NMR(DMSO-*d*6.,120˚C) δ : 0.82(3H, t, *J*=7.3Hz), 1.26(3H, d, *J*=6,6Hz), 1.36-1.58(6H, m), 1.68(1H, m), 1.96(1H, m), 2.19(3H, s), 2.38(4H, m), 3.73(2H, m), 4.02(1H, br), 6.38(1H, s), 6.49(1H, m), 6.63(1H, d, *J*=8,8Hz), 6.91(4H, m), 7.13(2H, m), 7.25(1H, t, *J*=2.9Hz), 7.34 (1H, d, *J*=8.1Hz), 7.56(1H, s), 10.66(1H,br)ppm IR(KBr) : 3350, 2964,1630, 1419, 1388,1321, 1257, 1123, 1069, 756 $cm^{-1}$ Melting point 159˚C~160˚C | |

Table 2-50

| | | |
|---|---|---|
| 118 | $^1$H-NMR (DMSO-$d_6$:120°C) δ: 0.89(3H,t,$J$=7.3Hz), 1.26(4H,d, $J$=6.6Hz), 1.62(3H,t,$J$=7.3Hz), 1.96(1H,m), 2.07(3H,s), 2.29(4H,m), 3.43(1H,br), 3.73(2H,m6), 4.13(1H,br), 4.88(1H,br), 6.38(1H,s), 6.50 (1H,m), 6.63(1H,d,$J$=7.3Hz), 6.87(1H,s), 6.93(3H,m), 7.14(2H,m), 7.24(1H,d,$J$=2.9Hz), 7.34(1H,d,$J$=8.8Hz), 7.56(1H,s), 10.65(1H,br) ppm FABMS:497(M+ 1) IR(KBr): 3322, 2970, 1624, 1560, 1419, 1321, 1147cm$^{-1}$ | 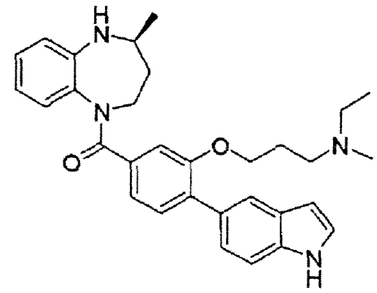 |
| 119 | $^1$H-NMR (DMSO-$d_6$:120°C) δ : 0.80(3H,t,$J$=7.3Hz), 1.25(3H,d, $J$=6.8Hz), 1.29-1.40(2H,m), 1.60-1.73(3H,m), 1.83(3H,s), 1.91-1.93 (1H,m), 2.08(3H,s), 2.19(2H,t,J=6.8Hz), 2.28(2H,t,$J$=7.3Hz), 3.17 (3H,s), 3.43(2H,brs), 3.71-3.83(2H,m), 4.12(1H,brs), 4.89(1H,s), 6.44-6.50(1H,m), 6.62(1H,t,$J$=7.8Hz), 6.89-6.95(4H,m), 7.14(1H,d, $J$=7.8Hz), 7.22-7.27(2H,m), 7.43-7.48(2H,m)ppm FABMS:54,3 (M+1) IR(KBr): 3326, 2962, 1640, 1383, 1323, 1143cm$^{-1}$ | 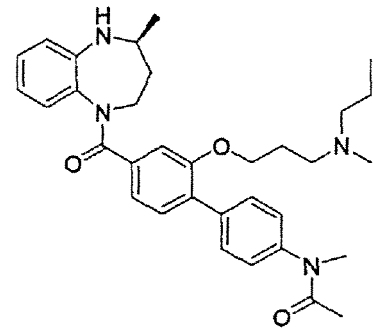 |

Table 2-51

| | | |
|---|---|---|
| 120 | $^1$H-NMR (DMSO-$d_6$:120°C)δ 1.25(3H,d, $J$=6.3Hz), 1.39(2H, quintet,$J$=6.8Hz), 1.58-1.80(1H,m), 1.90-2.05(aH,m), 1.92(2H,t, J=6.8Hz), 1.97(6H,s), 3.2-3.6(2H,m), 3.93(2H,t,J=6.8Hz), 4.05-4.30(1H,m), 4.87(1H,s), 6.30.6.42(1H,m), 6.45-6.52(1H,m), 6.63(1H,d,J=7.8Hz), 6.91-6.95(3H,m), 7.20(1H,d,$J$=7.8Hz), 7.25-7.37(4H,m), 7.53(1H,d,$J$=2.0Hz)ppm FABMS:511(M+1) IR (KBr): 3350, 2966 1717, 1630, 1394, 1290, 1174cm$^{-1}$ | 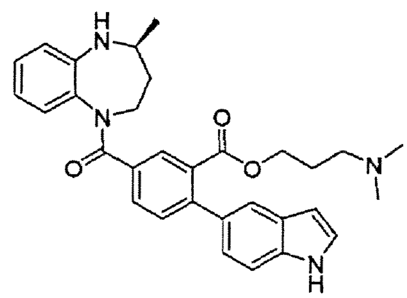 |
| 121 | $^1$H-NMR(DMSO-$d_6$, 120°C) δ: 0.87(7H,d,$J$=5.9Hz), 1.25(3H,d, $J$=6.6Hz), 1.65(1H,m), 1.96(1H,br), 2.10(3H,s), 2.54(2H,t, $J$=5.9Hz), 2.70(1H,m), 3.47(2H,br), 4.12(1H,br), 6.38(1H,d, $J$=2.9Hz), 6.48(1H,m), 6.63(1H,d,$J$=7.3Hz), 6.86(1H,d,$J$=1.5Hz), 6.93(1H,m), 7.11(6H,m), 7.27(1H,m), 7.38(1H,d,$J$=8.0Hz), 7.55 (1H,s), 10.77(1H,br)ppm FABMS:498(M+1) IR(KBr): 3326, 2970, 1626, 1562, 1417, 1321, 1127cm$^{-1}$ | 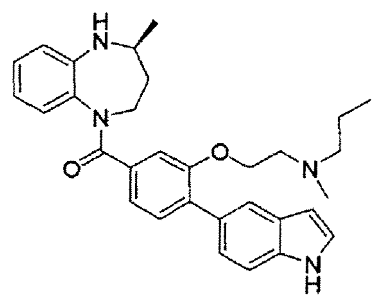 |
| 122 | $^1$H-NMR(DMSO-$d_6$,120°C) δ : 0.87(3H,t,$J$=7.3Hz), 1.20-1.45(7H, m), 1.59-1.75(3H,m), 1.87-2.01(1H,m), 2.18.2.38(12H,m), 2.77.2.97(1H,m), 3.30-4.45(5H,m), 4.87(1H,s), 6.36-6.41(1H,m), 6.46-6.54(1H,m), 6.63(1H,d,$J$=7.3Hz), 6.84-6.98(4H, m), 7.08-7.12(2H,m), 7.22-7.27(1H,m), 7.31-7.38(1H,m), 7.55(1H,s), 10.66(1H,s)ppm FABMS:580(M+1) IR(KBr): 3324, 2934, 1628, 1560, 1419, 1321, 1160cm$^{-1}$ | 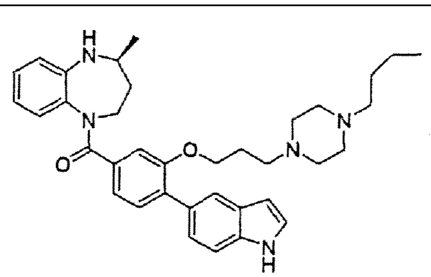 |

Table 2-52

| | | |
|---|---|---|
| 122 1/2 fuma -rate | $^1$H-NMR(DMSO-$d_6$, 120˚C) δ :0.87(3H, t, *J*=7.3Hz), 1.26(3H, d, *J*=6.6Hz), 1.30(4H, m), 1.64(3H, m), 1.92(1H, m), 3.72(2H, m), 4.11(1H, br), 6.38(1H, s), 6.49(1H, m), 6.61-6.64 (2H,m), 6.93(4H, m), 7.12(2H, d, *J*=7.3Hz), 7.23(1H, m), 7.34(1H, d, *J*=8.1Hz), 7.55(1H, s), 10.64(1H,br)ppm IR(KBr) : 3332, 2964, 1632, 1419, 1386, 1321, 1255, 1147, 984, 806, 756, 665 $cm^{-1}$ Melting point : 169˚C~170˚C | |
| 122 · male -ate | $^1$H-NMR(DMSO-$d_6$,120˚C)δ: 0.90(3H, t, *J*=7.3Hz), 1.24(3H, d, *J*=6.6Hz), 1.31-1.36(2H, m), 1.45-1.53(2H, m), 1.69-1.76(3H, m), 1.92-1.96(1H, m), 2.59-2.79(12H, m), 3.41-4.10 (5H, m), 6.01(2H, s), 6.40(1H, brs), 6.50-6.53(1H, m), 6.64(1H, d, *J*=7.3Hz), 6.89-6.94 (4H, m), 7.10-7.14(2H, m), 7.26(1H, dt, *J*=2.94Hz), 7.36(1H, d, *J*=8.8Hz), 7.56(1H, s), 10.7(1H, brs) ppm IR(KBr) : 3330, 2966, 1632, 1589, 1499, 1419, 1386, 1321, 1253, 864, 756 $cm^{-1}$ Melting point : 140˚C~ 141˚C | |

Table 2-53

| | | |
|---|---|---|
| 122 · 2 male ate | $^1$H-NMR(DMSO-d6, 120˚) δ: 0.91(3H, t, *J*=7.3Hz), 1.26(3H, d, *J*=5.9Hz), 1.29.1.37(2H, m), 1.47-1.56(2H, m), 1,69-1.78(3H, m),1.92-2.00(1H, m), 2.64(2H, t, J=7.3Hz),2.65(4H, t, *J*=5.1Hz), 2.77(2H, t, *J*=7.3Hz),2.88(4H, t, *J*=5,1Hz), 3.38-4.27(5H, m), 6.09(4H, s),6.41 (1H, brs), 6.46-6.55(1H, m), 6.64(1H, d, *J*=8.1Hz), 6.90-6.95(4H, m), 7.10-7.14(2H, m), 7.25-7.26(1H, m), 7.36(1H, d,*J*=8.1Hz), 7.56(1H, s), 10.7(1H, brs) ppm IR(KBr) : 3284, 2960, 2444, 1700, 1617, 1211, 1094, 864, 756, 647, 584 $cm^{-1}$ Melting point : 146~147˚C | |
| 122 · citrate | $^1$H-NMR(DMSO-$d_6$, 120˚C) δ: 0.88(3H, t, *J*=7.3Hz), 1.25(3H, d, *J*=6.6Hz), 1.29-1.46(4H, m), 1,65-1.70(3H, m), 1.96-2.00(1H, m), 2.32-2.69(16H, m), 3.43-4.09(5H, m), 6.39(1H, s), 6.49-6.52(1H, m), 6.63(1H, d, *J*=8.1Hz), 6.89-6.94(4H, m), 7,10-7.15(2H, m), 7.24-7.25 (1H, m), 7.34(1H, d, J=8.1Hz), 7.55(1H, s), 10.7(1H, s) ppm IR(KBr): 3332,2964,1719,1632,1599,1502, 1450, 1419, 1383, 1321, 1253, 1137, 971, 754$cm^{-1}$ Melting point: 148˚C~152˚C | |

Table 2-54

| | | |
|---|---|---|
| 123 | $^1$H-NMR(DMSO-$d_6$, 120˚C) δ : 1.25(3H,d,*J*=6.6Hz), 1.56(2H,m), 1.67(3H,t,*J*=7.3Hz), 1.94(1H,m), 2.35(5H,m), 2.64(5H,m), 3.41 (2H,br), 3.76(3H,dt,*J*=5.9,2.9Hz), 4.12(1H,br), 4.54(1H,s), 4.89 (1H,s), 6.38(1H,t,*J*=2.2Hz), 6.49(1H,dt,*J*=6.6,2.2Hz), 6.63(1H,d, *J*=6.6Hz), 6.77(1H,t,7.3Hz), 6.93(6H,m), 7,15(5H,m), 7,22(1H,t, *J*=2.9Hz), 7.33(1H,d,*J*=8.OHz), 7,56(1H,s), 8.02(1H,s), 10.65(1H, br)ppm FABMS:567(M+1) IR(KBr): 3332, 2950, 1626, 1560, 1417, 1321, 1255, 1123cm-1 | 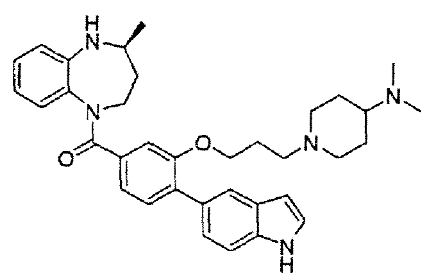 |
| 124 | $^1$H-NMR(DMSO-$d_6$, 120˚C) δ : 0.86(6H,m), 1.25(8H,m), 1.61(3H, m), 1.91(1H,m), 2.36(6H,m), 3.43(1H,br), 3.71(3H,m), 4.11(1H, br), 4.87(1H,br), 6.37(1H,d,*J*=3.6Hz), 6.48(1H,m), 6.62(1H,d, *J*=7.3Hz), 6.86(1H,s), 6.93 (3H,m), 7.14(2H,m), 7.23(1H,m), 7.33 (1H,d,*J*=8.8Hz), 7.56(1H,s), 10.65(1H,br)ppm FABMS:526(M+1) IR(KBr): 3320, 2964, 1624, 1560, 1419, 1321, 1253, 1125$cm^{-1}$ | 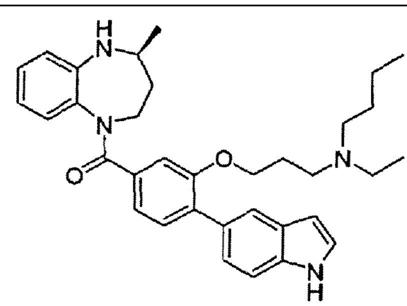 |

Table 2-55

| | | |
|---|---|---|
| 125 | $^1$H-NMR(DMSO-$d_6$ 120˚C) δ: 0.77(3H,t,*J*=7.3Hz), 0.86(3H,t, *J*=6.6Hz), 1.24(5H,m), 1.61(3Hm), 1.91(1H,m), 2.25(2H,t, *J*=7.3Hz), 2.36(4H,t,*J*=6.6Hz), 3.41(2H,br), 3,72(2H,m), 4.10 (1H,br), 4.87(1H,s), 6.38(1H,d,*J*=2.9Hz), 6.48(1H,m), 6.63(1H, d,*J*=8.0Hz), 6.86(1H,s), 6.92(4H,m), 7. 11(2H,m), 7.24(1H,d, *J*=2.9Hz), 7.33(1H,d,*J*=8.0Hz), 7.56(1H,s), 10.65(1H,br)ppm FABMS:626(M+1) IR(KBr): 3324, 2966, 1626, 1562, 1419, 1321, 1253,1125cm$^{-1}$ | 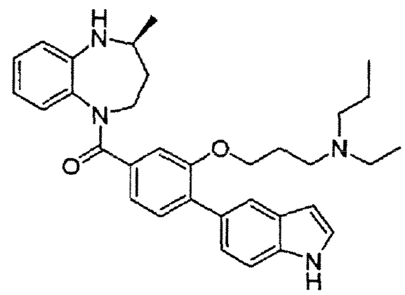 |
| 126 | 1H-NMR(DMSO- d6, 120˚C) δ : 0.82(3H,t,*J*=7.3Hz),1.27(10H, m),1.52(2H,m), 1.68(1H,m), 1.96(1H,m), 2.08(1H,s), 2,20(2H, t,*J*=7.3Hz),3.43(2H,br), 3.68(2H,dt,J=6.6,2.9Hz), 4.12(1H,br), 4.89(1H,s), 6.38(1H,s), 6.49(1H,m) 6.63(1H,d,*J*=7.3Hz), 6.85 (1H,s), 6.94(3H,m), 7.11(1H,m), 7.14(1H,d,*J*=1.5Hz), 7.24(1H, d,*J*=2.9Hz),7.33(1H,d,*J*=8.81Hz) 7.56(1H,s), 10,65(1H,br) ppm FABMS:540(M+1) IR(KBr): 3320,2938,1624,1560,1419,1321, 1253, 1125 cm$^{-1}$ | 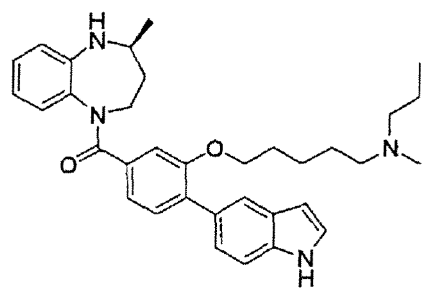 |

Table 2-56

| | | |
|---|---|---|
| 127 | $^1$H-NMR(DMSO-$d_6$,120˚C) δ: 0.78(3H,t,J=7.3Hz), 1.25(3H,d, *J*=6.6Hz), 1.24-1.36(2H,m), 1.58-1.78(3H,m), 1.95(1H,m), 2.07 (3H,s), 2.17(2H,t,*J*=7.33Hz), 2.28(2H,t,*J*=7.33Hz), 3.25(2H,br), 3.71-3.76(2H,m), 4.10(1H,br), 4.88(1H,brs), 6.37-6.38(1H,m), 6.47-6.52(1H,m), 6.63(1H,d,*J*=6.6Hz), 6.87-6.94(4H,m), 7.11-7.15(2H,m), 7.23-7.24(1H,n), 7.33(1H,d,*J*=8.1Hz), 7.56(1H, s), 10.7(1H,br)ppm FABMS:512(M+1) IR(KBr): 3324, 2962, 1624, 1560, 1419, 1321, 1125cm$^{-1}$ | 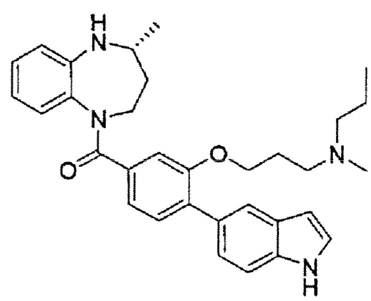 |
| 128 | $^1$H-NMR(DMSO-$d_6$,120˚C) δ : 0.84(3H,t,*J*=7.3Hz), 1.15-1.44(3H, m), 1.26(3H,d,*J*=6.6Hz), 1.61-1.69(3H,m), 1.85- 1.95(1H,m), 2.18-2.32(11H,m), 3.40(2H,br), 3.70-3.75(2H,m), 4.10(1H,br), 4.88(1H,br), 6.38(1H,brs), 6.47-6.52(1H,m), 6.63(1H,d,*J*=7.3Hz), 6.86-6.94(4H,m), 7.11-7.15(2H,m),7.23-7.24(1H,m), 7.33(1H,d, *J*=8.1Hz), 7.56(1H,s), 10.7(1H,br)ppm FABMS:566(M+1) IR (KBr): 3336,2934,1626,1560,1419,1321, 1253, 1160cm$^{-1}$ | 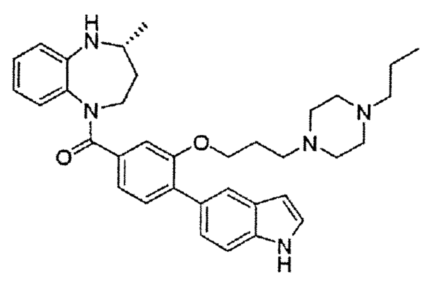 |

Table 2-57

| | | |
|---|---|---|
| 129 | $^1$H-NMR(DMSO-$d_6$,120˚C) δ: 0.82(3H,t,*J*=7.3Hz), 1.16(3H,t, *J*=6.8Hz), 1.25(3H,d,*J*=6.8Hz), 1.29-1.43(2H,m), 1.58-1.72(3H, m), 1.86-1.96(1H,m), 2.11(3H,s), 2.22(2H,t,*J*=6.8Hz), 3.03-3.13 (2H,m), 3.45(2H,br), 4.09(1H,br), 4.86(1H,br), 5.11(1H,br), 6.43-6.61(4H,m), 6.82-6.94(4H,m), 7.04(1H,d,*J*=7.8Hz), 7.16-7.22(2H,m)ppm FABMS:515(M+1) IR(KBr): 3348, 2964, 1615, 1557, 1390, 1323, 1189cm$^{-1}$ | 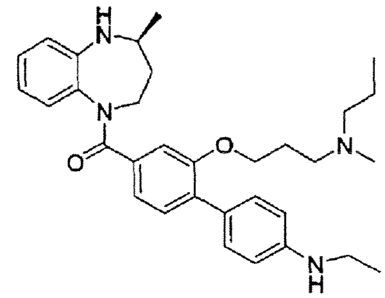 |
| 130 | $^1$H-NMR(DMSO-$d_6$, 120˚C) δ : 0.82 (3H, t, *J*=7.3 Hz), 1.25 (3H, d, *J*=6.8 Hz), 1.32-1.41(2H,m), 1.64-1.71(3H,m), 1.81-1.96(1H, m), 2.12(3H,s), 2.23 (2H, t, *J*=6.8 Hz), 2.33 (2H, t, *J*=6.8 Hz), 2.71 (3H, d, *J*=5.37 Hz), 3.31-4.20 (5H, m), 4.86(1H,br), 5.20(1H,br), 6.41-6.64(4H,m), 6.82-6.93(4H,m), 7.05 (1H, d, *J*=7.8 Hz), 7,19-7.22(2H,m)ppm FABMS:502(M+1) IR(KBr): 3372, 2962, 1615, 1557, 1392, 1323, 1189cm$^{-1}$ | 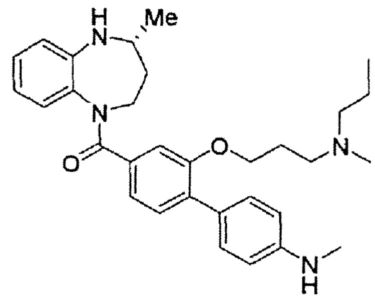 |
| 131 | $^1$H-NMR (DMSO-$d_6$, 120˚C) δ: 0.81 (3H, t, *J*=7.3 Hz), 1.25 (3H, d, *J*=5.9 Hz), 1.28-1.41(2H,m), 1.60-1.73(3H,m), 1,91-1.96(1H, m), 2.10(3H,s), 2.20(2H,t, *J*=6.8Hz), 2.29(2H,t, *J*=6.8Hz), 3.42 (2H,br), 3.70-3.80(2H,m), 3.77(3H,s), 4.03(1H,br), 4.88(1H,br), 6.44-6.49(1H,m), 6.61(1H,d, *J*=7.8Hz), 6.86-6.98(6H,m), 7.08 (1H,d, *J*=7.8Hz), 7.31-7.37(2H,m)ppm FABMS:502(M+1) IR (KBr): 3348, 2964, 1615, 1557, 1390, 1323, 1189cm$^{-1}$ | 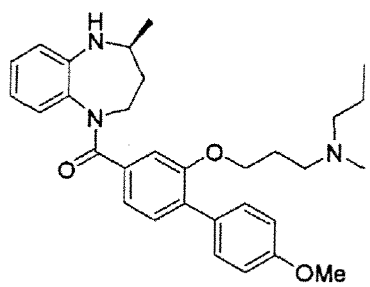 |

Table 2-58

| | | |
|---|---|---|
| 132 | $^1$H-NMR(DMSO-$d_6$,120˚C) δ: 0.79(3H,*t*,*J*=7.3Hz), 1.15-1.45 (7H,m), 1.69(1H,m), 1.97(9H,m), 2.09(4H,m), 2.84(3H,s), 3.43 (1H,br), 3.94(2H,t,*J*=6.6Hz), 4.14(1H,br), 4.88(1H,s), 6.40(1H, s), 6.50(1H,m), 6.63(1H,d,*J*=8.0Hz), 6.93(3H,m), 7.20(1H,d, *J*=8.0Hz), 7.27(1H,t,*J*=2.2Hz), 7.33(3H,m), 7.52(1H,d, *J*=1.5Hz), 10.73(1H,br)ppm FABMS:540(M+1) IR(KBr): 3342, 2964, 1717, 1630, 1450, 1323, 1255, 1174cm$^{-1}$ | 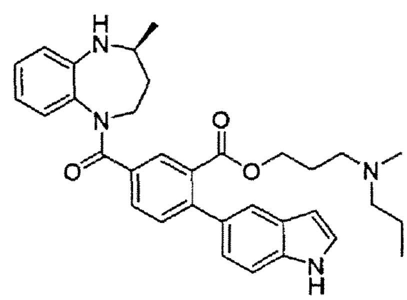 |

(continued)

| | | |
|---|---|---|
| 133 | $^1$H-NMR(DMSO-$d_6$,120˚C) δ: 1.24(3H,d,*J*=6.3Hz), 1.49(2H, quintet,*J*=6.8Hz), 1.58-1.75(1H,m), 1.90-2.05(1H,m),2.05(2H, t,*J*=6.8Hz), 2.06(6H,s), 3.4-3.6(2H,m), 3.77(3H,s), 3.99(2H,t, *J*=6.8Hz), 4.88(1H,s), 6.44-6.51(1H,m), 6.61(1H,d,*J*=7.3Hz), 6.88-6.95(4H,m), 7.08-7.16(3H,m), 7.33(1H,dd,*J*=7.8,1.5Hz), 7.69(1H,d,*J*=1.5Hz)ppm FABMS:502(M+1) | 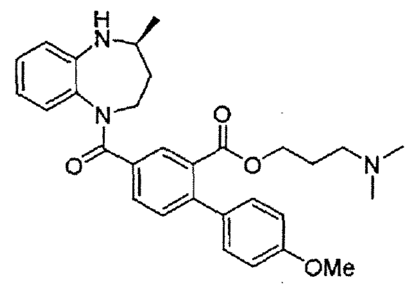 |
| 134 | $^1$H-NMR($CDCl_3$) δ: 1.3(3H,d,*J*=6.3Hz), 1.80-1.90(2H,m), 2.40-2.70(5H,m), 3.00-3.70(6H,m), 5.10-5.20(1H,m), 6.50-6.75(3H,m), 6.80-7.05(2H,m), 7.10-7.20(3H,m), 7.30-7.55(2H,m), 7.70-7.80(1H,m), 8.15-8.25(1H,m)ppm FABMS:467 (M+1) IR(KBr):3420,2964,1626,1556,1417,1317, 1116cm$^{-1}$ | 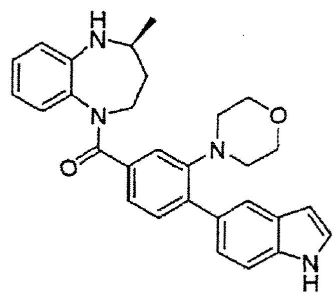 |

Table 2-59

| | | |
|---|---|---|
| 135 | $^1$H-NMR(DMSO-$d_6$,120˚C) δ: 1.36(3H,d,*J*=6,8Hz), 1.50(2H, quintet,*J*=6.8Hz), 1.72-1.90(1H,m), 2.00-2.15(1H,m),2.04(2H, t,*J*=6.8Hz), 2.20-2.25(4H,m), 3.45-3.65(5H,m), 4.05(2H,t, *J*=6.8Hz), 4.99(1H,s), 6.48-6.53(1H,m), 6,57.6.64(1H,m), 6.74 (1H,d,*J*=7.8Hz),6.98·7.07(3H,m), 7.31(1H,d,*J*=7.8Hz), 7.37-7.48(4H,m), 7.63(1H,d,*J*=1.5Hz)ppm FABMS:553(M+1) IR(KBr): 3346,2964,1717,1630,1396,1290, 1116cm$^{-1}$ | 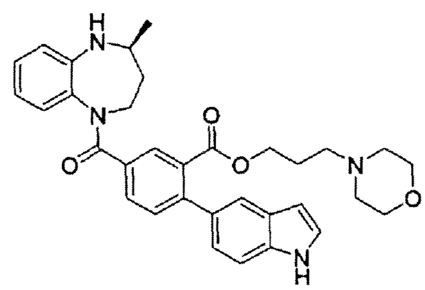 |
| 136 | $^1$H-NMR(DMSO-$d_6$, 120˚C) δ : 1.36(3H, d, *J*=6.8Hz), 1.50(2H, quintet, *J*=6,8Hz), 1.72-1.90(1H,m), 2.06(2H, t, *J*=6.8Hz), 2.00-2,15(1H,m), 2.23(3H,s), 2.25-2.40(8H,m), 3.45-3.65(2H, m), 4.04(2H,t,*J*=6.8Hz), 4.10-4.40(1H,m), 4.99(1H,s), 6.48-6.53(1H,m), 6.57-6.64(1H,m), 6.74(1H,d,*J*=7.8Hz), 6.98-7.07(3H,m), 7.31(1H,d,*J*=7.8Hz), 7.37-7.48(4H,m), 7.63 (1H, d, *J*=1.5Hz)ppm FABMS:566(M+1) IR(KBr): 3352, 2944, 1717, 1630, 1396, 1288, 1164cm$^{-1}$ | 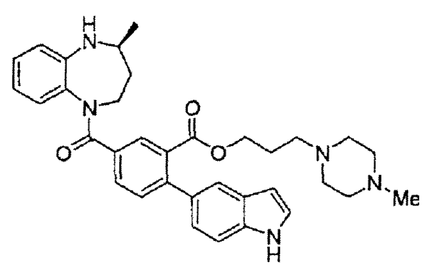 |

Table 2-60

| | | |
|---|---|---|
| 137 | $^{1}$H-NMR(DMSO·$d_6$,120°C) δ: 0.87(3H,t, *J*=6.8Hz), 1.36(3H, d,*J*=6.8Hz 1.40-1.60(6H,m), 1,72-1.90(1H,m), 2.06(2H,t, *J*=6.8Hz), 2.00-2.15(1H,m), 2.23(3H,s), 2.25-2.40(10H,m), 3.45-3.65(2H,m), 4,04(2H,t,*J*=6.8Hz), 4.10-4.40(1H,m), 4.99(1H,s), 6.48-6.53(1H,m), 6.57-6.64(1H,m), 6.74(1H,d, *J*=7.8Hz), 6.98-7.07(3H,m), 7.31(1H,d,*J*=7.8Hz), 7.37-7.48 (4H,m), 7.63(1H,d,*J*=1.5Hz)ppm FABMS:608(M+1) IR (KBr): 3350, 2958, 1717, 1630, 1394, 1290, 1158$cm^{-1}$ | 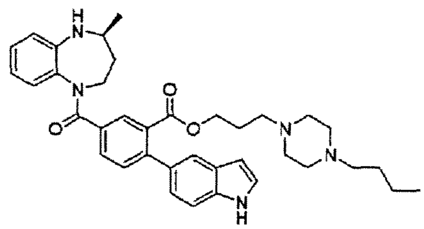 |
| 138 | $^{1}$H-NMR(DMSO-$d_6$,120°C) δ: 0,80(3H,t,*J*=7.3Hz), 1.25(3H, d,*J*=6.3Hz), 1.28-1.40(4H,m), 1.50-1.80(3H,m), 1.90-2.40 (7H,m), 3.30-3.50(2H,m), 4.00.4.30(1H,m), 4.58-4.70(1H, m), 4.86(1H,s), 6.37-6.42(1H,m), 6.45-6.53(1H,m), 6.62 (1H,d,*J*=7,8Hz), 6.88-6.95(3H,m), 7.20(1H,d,*J*=7.8Hz), 7.25-7.35(4H,m), 7.48(1H,d,*J*=2.0Hz)ppm FABMS:551 (M+1) IR(KBr): 3356, 2962, 1711, 1630, 1392, 11290, 1093$cm^{-1}$ | 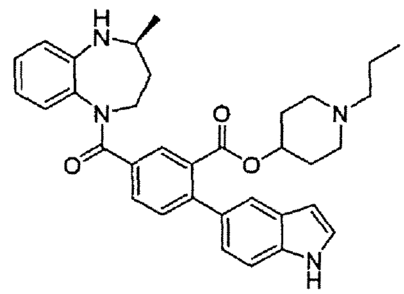 |
| 139 | $^{1}$H-NMR(DMSO-$d_6$,120°C) δ: 1.25(3H,d,*J*=6.3Hz), 1.6-1.8 (1H,m), 1.9·2.0(1H,m), 3.3·3.5(2H,m), 4.0-4.3(1H,m), 4.86 (1H,s), 4.99(2H,s), 6.37·6.42(1H,m), 6.45-6.53(1H,m), 6.63 (1H,d,*J*=7.8Hz), 6.8-6.95(5H,m), 7.23(1H,d,*J*=7.8Hz), 7.27-7.40(4H,m), 7.59(1H,d,*J*=2.0Hz), 8.28(1H,dd, *J*=4.5,2.0Hz)ppm FABMS:517(M+1) IR(KBr): 3346, 1721, 1630, 1419, 1288, | 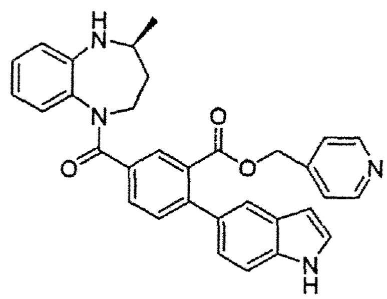 |

Table 2-61

| | | |
|---|---|---|
| 140 | $^{1}$H-NMR(DMSO-$d_6$, 120°C) δ: 0.90(9H, m), 1.24(8H,m), 1.62 (5H,m), 1.95(1H,m), 2.18(2H,t,*J*=6.6Hz), 3.43(1H,br), 3.72(2H, dt,*J*=6.6,2.9Hz), 4.12(1H, r), 4.87(1H,s), 6.38(1H,s), 6.50(1H, m), 6.63(1H,d,*J*=7.3Hz), 6.88(1H,s), 6.94(3H,m), 7.13(2H,m), 7.24(1H,t,*J*=2.9Hz), 7.33(1H,d,*J*=8.1Hz), 7.55(1H,s), 10.66 (1H,br)ppm FABMS:609(M+1) IR(KBr): 3316, 2962, 1626, 1562, 1419, 1158$cm^{-1}$ | 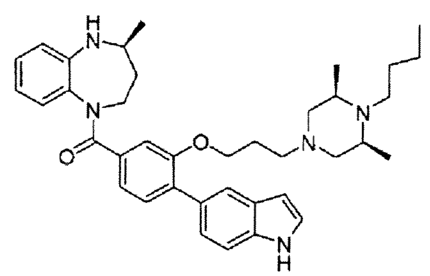 |
| 141 | $^{1}$H-NMR(DMSO-$d_6$, 120°C) δ:1.25(8H,m), 1,65(7H,m), 1.91 (4H,m), 2.27(4H,m), 2.60(4H,m),3.43(2H,br), 3.73(3H,m), 4.10 (1H,br), 4.88(1H,br), 6.39(1H,d,*J*=3.6Hz), 6.51(1H,m), 6.64 (1H,d,*J*=7.3Hz), 6.86(1H,s), 6.93 (3H,m), 7,13(2H,m), 7.24 (1H,m), 7.33(1H,d,*J*=8.8Hz), 7.55(1H,s)ppm FABMS:539 (M+1) IR(KBr): 3354, 2934, 1624, 1557, 1419, 1321, 1523, 1137$cm^{-1}$ | 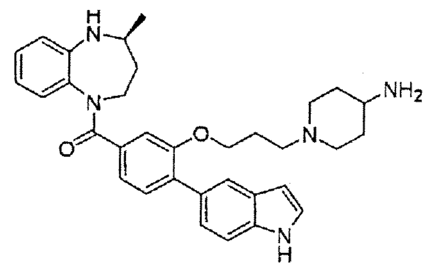 |

(continued)

| | | |
|---|---|---|
| 142 | $^1$H-NMR(DMSO-$d_6$, 120˚C) δ : 0.87(6H,d,*J*=6.6Hz), 1.25(4H, d,*J*=6.6Hz), 1.59-1.80(4H,m), 1.83-2.06(3H,m), 2.20-2.35(9H, m), 3.30-4.85(5H,m), 4.87(1H,s), 6.36-6.40(1H,m), 6.46-6.54 (1H,m), 6.63(1H,d,*J*=7.3Hz), 6.86-6.97(4H,m), 7.10-7.17(2H, m), 7.24(1H,t,*J*=2.9Hz), 7.34(1H,d,*J*=8.1Hz), 7.56(1H,s), 10.65(1H,s)ppm FABMS:580(M+1) IR(KBr): 3416, 2956, 1626, 1562, 1419, 1321, 1162cm$^{-1}$ | 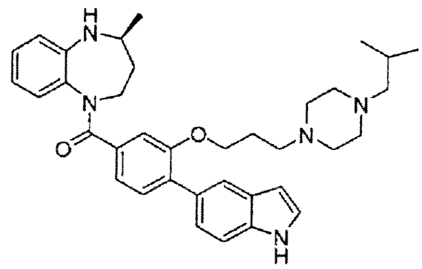 |

Table 2-62

| | | |
|---|---|---|
| 143 | $^1$H-NMR(DMSO-$d_6$,120˚C) δ: 0.82(3H,*t*,*J*=7.3Hz), 1.15(1H,d, J=5.9Hz), 1.25(3H,d,*J*=6.8Hz), 1.29-1.40(2H,m), 1.58-1.70(3H, m), 1.90-1.92(1H.m), 2.11(3H,s), 2.22(2H,t,*J*=7.3Hz), 2.32(2H,t, *J*=6.8Hz), 3.40(2H,br), 3.49-3.61(1H,m), 3.70-3.75(2H,m), 4.10 (1H,br), 4.86-4.92(2H,m), 6.43-6.61(4H,m), 6.81-6.92(4H,m), 7.04(1H,d,*J*=7.8Hz), 7.16-7.20(2H,m)ppm FABMS:529(M+1) IR (KBr): 3342, 2966, 1615, 1557, 1388, 1323, 1189cm$^{-1}$ | 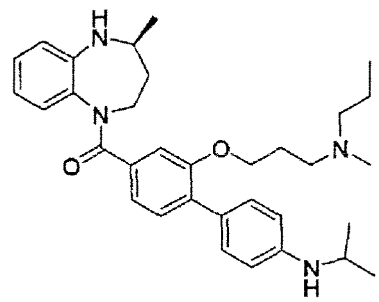 |
| 144 | $^1$H-NMR(DMSO-$d_6$, 120˚C) δ: 1.25(3H, d, *J*=6.3Hz), 1.58·1.80(1H, m), 1.90-2.05(1H, m), 2.01(6H,s), 2.19(2H,t,*J*=6.0Hz), 3.2-3.6(2H, m), 3.98(2H, t, *J*=6.0Hz), 4.05-4.30(1H, m), 4.87(1H,s), 6.30-6.42 (1H, m), 6.45-6.52(1H,m), 6.63(1H, d, *J*=7.8Hz), 6.91-6.95(3H, m), 7.20(1H,d,*J*=7.8Hz), 7.25-7.37(4H,m), 7.53(1H,d,*J*=2.0Hz) ppm FABMS:497(M+1) IR(KBr): 3348, 2970, 1719, 1630, 1398, 1290, 1158cm$^{-1}$ | 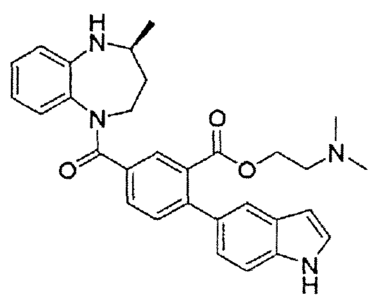 |

Table 2-63

| | | |
|---|---|---|
| 145 | $^1$H-NMR(DMSO-$d_6$,120˚C) δ: 0.89(3H,t,*J*=6.8Hz), 1.25(3H,d, *J*=6.3Hz), 1.3-1.45(1H,m), 1.6-1.8(1H,m), 1.85-2.0(2H,m), 2.05-2.15(1H,m), 2.25-2.35(4H,m), 2.55-2.65(1H,m), 3.3-3.5 (2H,m), 4.0-4.3(1H,m), 4.89(1H,s), 4.95-5.0(1H,m), 6.37-6.42 (1H,m), 6.45-6.55(1H,m), 6.63(1H,d,*J*=7.8Hz), 6.87-6.95(3H, m), 7.24(1H,d,*J*=7.8Hz), 7.2-7.35(4H,m), 7.5(1H, s)ppm FABMS:523(M+1) IR(KBr): 3346, 2972, 1715, 1630, 1392, 1290, 1174cm$^{-1}$ | 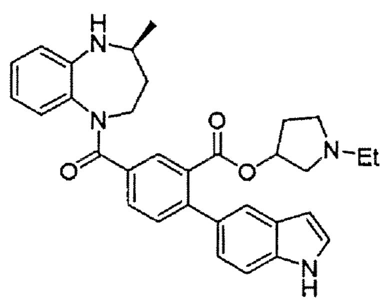 |

(continued)

| | | |
|---|---|---|
| 146 | $^1$H-NMR(DMSO-$d_6$,120˚C) δ: 1.25(3H,d,*J*=6.3Hz), 1.6-1.75 (1H,m), 1.9-2.0(1H,m), 2.14-2.17(4H,m), 2.23(2H,t,*J*=6.3Hz), 3.3-3.5(1H,m), 3.39-3.42(4H,m), 4.01(2H,t,*J*=6.3Hz), 4.0-4.2 (1H,m), 4.88(1H,s), 6.40(1H,brs), 6.45-6.55(1H,m), 6.63(1H,d, *J*=7.8Hz), 6.87-6.95(3H,m), 7.20(1H,d,*J*=8.3Hz), 7.25-7.35 (4H,m), 7,53(1H,d,*J*=1.5Hz)ppm FABMS:539(M+l) IR(KBr): 3410, 2962, 1719, 1628, 1390, 1290, 1116cm$^{-1}$ | 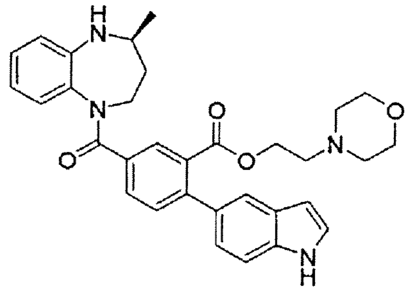 |

Table 2-64

| | | |
|---|---|---|
| 147 | $^1$H-MMR(DMSO-$d_6$,120˚C) δ: 1.25(3H,d, *J*=6.3Hz), 1.6-1.75(1H,m), 1.9-2.0(1H,m), 2.10(3H,s), 2.17.2.26(10H,m), 3.35-3.5(1H, m), 4.00(2H,t,*J*=5.9Hz), 4.88(1H,brs), 6.40 (1H,brs), 6.45-6.55(1H,m), 6.63(1H,d, *J*=7.8Hz), 6.87-6.95(3H,m), 7.20(1H,d, *J*=8.3Hz), 7.25-7.35(4H,m), 7.53(1H,d, *J*=1.5Hz)ppm FABMS:552(M+1) IR(KBr): 3336, 2942, 1719, 1628, 1450, 1290, 1170cm$^{-1}$ | 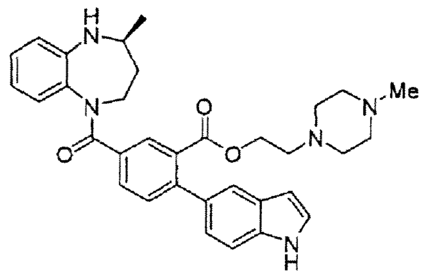 |
| 148 | $^1$H·NMR(DMSO·$d_6$,120˚C) δ : 1.15-1.26(7H, m), 1.65-1.69(1H,m), 1.88-1.97(9H,m), 2.06 (2H,t,*J*=7,3Hz), 3.41-4.15(3H,m), 4.91(1H, brs), 6.41-6.47(2H,m), 6.61(1H,d,*J*=7.3Hz), 6.89-6.93(3H,m), 7.18-7.41(6H,m), 10.8(1H, brs)ppm FABMS:509(M+1) IR(KBr): 3326, 2934, 1626, 1562, 1417, 1321, 1164cm$^{-1}$ | 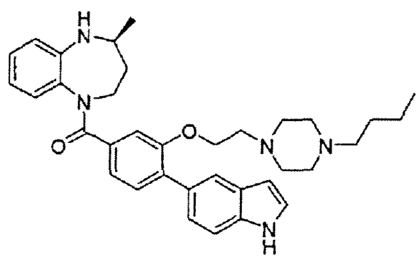 |
| 148 · 1/2 fuma -rate | $^1$H -NMR(DMSO-d6,120˚C) δ: 0.87(3H, t, J=6.6Hz), 1.25(3H, d, *J*=6.6Hz), 1.30-1.38 (4H, m), 1.65-1.68(1H, m), 1.91-1.96(1H, m), 2.23-2.51(12H, m), 3.42-4.08(5H, m), 6.38 (1H, s), 6.47-6.52(1H, m), 6.61-6.64(2H,m), 6.86-6.95(4H, m), 7.17-7.18(2H, m), 7.24(1H, t, *J*=2.2Hz), 7.33(1H, d, *J*=8.8Hz), 7.59(1H, s), 10.6(1H, brs) ppm IR(KBr): 3430, 2964, 1626, 1584, 1493, 1450, 1419, 1321, 1255,1127, 1042, 984, 806, 756, 648cm$^{-1}$ Melting point : 158˚C~159˚C | |

Table 2-65

| | | |
|---|---|---|
| 148 · male -ate | $^1$H-NMR(DMSO-$d_6$,120°C) δ : 0.90(3H, t, *J*=7.3Hz), 1.25 (3H, d, *J*=5.9Hz), 1.30-1.69(5H, m), 1.92-1.96(1H, m), 2.58-2.79(12H, m), 3.43-4.10(5H, m), 6.01(2H, s), 6.40-6.41(1H, m), 6.50-6.53(1H, m), 6.64(1H, d, *J*=8.1Hz), 6.92-6.94(4H, m), 7.11-7.14(2H, m), 7.25-7.27(1H, m), 7.35(1H, d, *J*=8.8Hz), 7.56(1H, s), 10.7(1H, brs) ppm IR (KBr) : 3332, 2944, 1632, 1584, 1499, 1419, 1388, 1321, 1257,876, 864, 756 $cm^{-1}$ Melting point: 112°C~113°C | |
| 149 | $^1$H-NMR($CDCl_3$) δ: 1.37(3H,d,*J*=6,3Hz), 1.80-1.90(2H,m), 2.23(3H,s), 2.50-2.8O(5H,m), 3.00-3.70(6H,m), 5.10-5.20 (1H,m), 6.50-6.75(3H,m), 6.80-7.05(2H,m), 7.10-7.20(3H, m), 7.30-7.55(2H,m), 7.70-7.80(1H,m), 8.15-8.26(1H,m) ppm FABMS:480 (M+1) IR(KBr): 3348, 2938, 1624, 1599, 1557, 1400, 1317, 1151 $cm^{-1}$ | 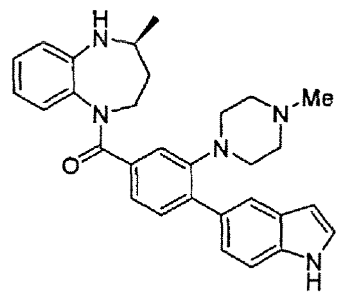 |
| 150 | $^1$H-NMR(DMSO-$d_6$, 120°C) δ: 1.00(3H,d,*J*=7.3Hz), 1.55-1.65(3H,m), 1.96·1.99(1H,m),2.31(4H,t,*J*=4.4Hz), 2.48-2.52(2H,m), 3.19-4.01(7H,m), 4.91(1H,brs), 6.39 (1H,s), 6.51(1H,m), 6.63(1H,d,*J*=7.3Hz), 6.88-6.97(4H, m), 7.13-7.18(2H,m), 7.25-7.26(1H,m), 7.34(1H,d, *J*=8.8Hz), 7.59(1H,s), 10.7(1H,brs)ppm FABMS:525 (M+1) IR(KBr): 3330, 2962, 1624, 1560, 1419, 1319, 1118$cm^{-1}$ | 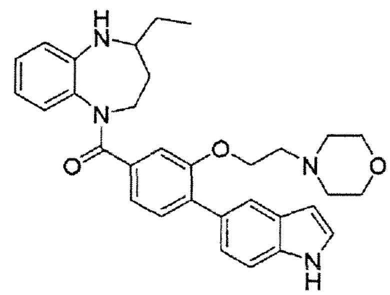 |

Table 2-66

| | | |
|---|---|---|
| 151 | $^1$H-NMR(DMSO-$d_6$,120°C) δ : 0.83(3H,t,*J*=7.3Hz), 1.26(3H, t, *J*=6.6Hz), 1.31-1.44(4H,m), 1.52(1H,s), 1.57(1H,s), 1.68(1H, m), 1.94(3H,dt,*J*=8.8,2.9Hz), 2.14(3H,s), 2.17(1H,m), 2.33(3H, t,*J*=6.6Hz), 2.76(2H,m), 3.43(1H, br), 3.76(2H,dt,*J*=5.9,L5Hz), 4.12(1H,br), 4.90(1H,s) 6.38(1H,t,*J*=2.2Hz), 6.50(1H,dt, *J*=5.9,2.2Hz), 6.63(1H,d,*J*=6.6Hz), 6.86(1H,s), 6.91-6.95(3H, m), 7.11-7.19(2H,m), 7.23(1H,t,J=2.9Hz), 7.24(1H,d,J=8.8Hz), 7.59(1H,s), 10.65(1H,br)ppm FABMS:581(M+1) IR(KBr): 3320, 2958, 1626, 1562, 1417, 1321, 1255, 1125$cm^{-1}$ | 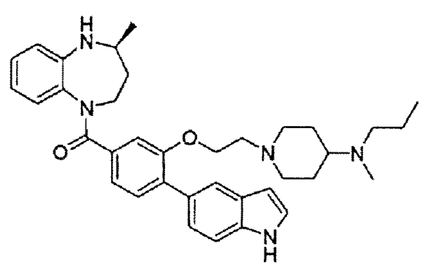 |
| 152 | $^1$H-NMR(DMSO-$d_6$,120°C) δ : 0.81(3H,t,*J*=7.3Hz), 1.26(3H,d,J. =6.6Hz), 1.37(2H,m), 1.67(1H,m), 1.96(1H,m), 2.14(3H,s), 2.25 (2H,t,J=6.6Hz), 3.25(2H,t,*J*=1.5Hz), 3.44(2H,br), 4.48(2H,t, *J*=1.5Hz), 4.91 (1H,s), 6.39(1H,t,*J*=2.2Hz), 6.51(1H,m), 6,63 (1H,d,*J*=7.3Hz), 6.91-6.95(4H,m), 7.03(1H,d,*J*=1.5Hz), 7.11 (1H,s), 7.14(1H,dd,*J*=6.6,1.5Hz), 7.25(1H,t,*J*=2.9Hz), 7.34(1H, d,*J*=8.1Hz), 7.57(1H,s), 10.67(1H,br)ppm FABMS:522(M+1) IR (KBr): 3326, 2966, 1626, 1560, 1419, 1321, 1257, 1112 $cm^{-1}$ | 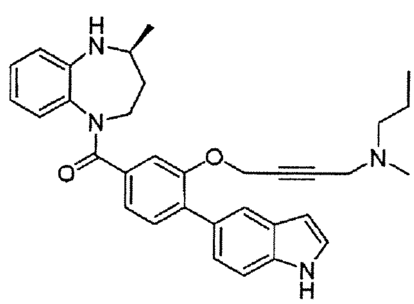 |

Table 2-67

| | | |
|---|---|---|
| 153 | $^1$H-NMR(DMSO-$d_6$,120˚C) δ: 0.81(3H,t,*J*=7.3Hz), 1.25(3H,d, *J*=5.9Hz), 1.37(3H,q,*J*=7.3Hz), 1.68(1H,m), 1.91(1H,m), 2.08 (3H,s), 2.22(2H,t,*J*=6.6Hz), 2.90(2H,d,*J*=5.1Hz), 3.43(2H,br), 4.24(3H,d,*J*=4.4Hz), 4.91 (1H,s), 5.64(2H,m), 6.38(1H,s), 6.52 (1H,m), 6,62(1H,d,*J*=7,3Hz), 6.88(1H,s), 6.95(3H,m), 7.14(2H, m), 7.24(1H,t,*J*=2.9Hz), 7.33(1H,d,*J*=8.8Hz), 7.56(1H,s), 10.66 (1H,br)ppm FABMS:524(M+1) IR(KBr): 3320, 2964, 1626, 1560, 1417, 1321, 1255, 1125cm$^{-1}$ | 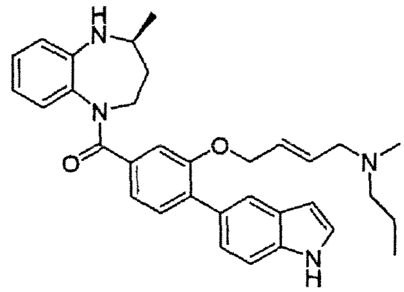 |
| 154 | $^1$H-NMR(DMSO·$d_6$, 120˚C) δ : 0.81(3H,t,*J*=6.8Hz), 1.18(3H,t, *J*=6.8Hz), 1.25(3H,d,*J*=6.8Hz), 1.29-1.42(2H,m), 1.62-1.72(3H, m), 1.90-1.96(1H,m), 2.11(3H,s), 2.21(2H,t,*J*=7.3Hz),2.32 (21-1,t,*J*=7.3Hz), 3,11-3.21(2H,m), 3.41(2H,br), 3.71-3.77(1H, m),4.09(1H,br), 4.87(2H,br), 6.43-6.50(1H,m), 6.59-6.71(2H, m), 6.84-6.93(4H,m), 7.07- 7.14(3H,m)ppm FABMS:533 (M+1) IR(KBr): 3332, 2966, 1630, 1562, 1410, 1323, 1156cm$^{-1}$ | 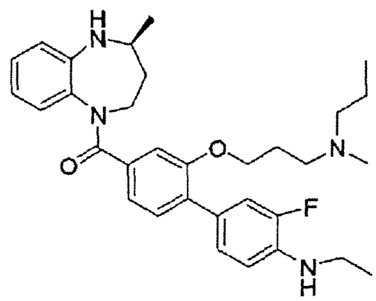 |
| 155 | $^1$H-NMR(DMSO-$d_6$, 120˚C) δ: 0.82 (3H, t, *J*=7.3 Hz), 1.20(6H, m), 1.36 (2H, q, *J*=7.3 Hz), 1.68(3H,m), 1.96(1H,m), 2.08 (3H, s), 2.11(3H,s), 2.22(2H,t,J=7.3Hz), 2.33 (2H, t, *J*=7.3 Hz), 3.15 (3H,m), 3.41 (3H,br), 3.72 (3H,m), 4.07 (3H,br), 4.37(2H,br), 4,86(1H,br), 6.53(4H,m), 6.82-6.93(4H,m), 7.04(2H,d, *J*=8,1Hz), 7.11 (2H,m)ppm FABMS:530(M+1) IR(KBr): 3398, 2964, 1630, 1560, 1390, 1323, 1292, 1164cm$^{-1}$ | 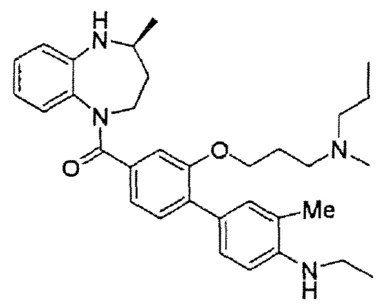 |

Table 2-68

| | | |
|---|---|---|
| 156 | $^1$H-NMR(DMSO-*d*$_6$, 120˚C) δ: 0.83(3H,t,*J*=7.3Hz), 1.13-1.26(8H, m), 1.37-1.72(8H,m), 2.10(3H,s), 2.24(4H,q,*J*=7.3Hz),3.03-3.12 (2H,m), 3.66-3.72(2H,m), 4.01-4.09(1H,m), 4.88(1H,brs), 5.10(1H, brs), 6.43-6.61(4H,m), 6.81-6.93(4H,m), 7.04(1H,d,*J*=8.1Hz), 7.19 (2H,d,*J*=8.8Hz)ppm FABMS:530 (M+2) IR(KBr): 3336, 2966, 1615, 1557, 1390, 1323, 1189, 1122cm$^{-1}$ | 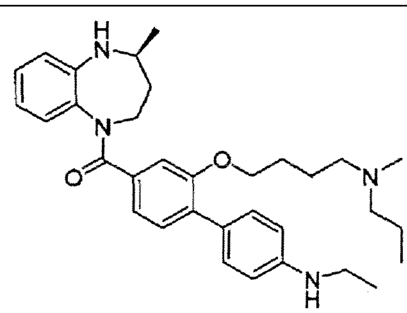 |

(continued)

| | | |
|---|---|---|
| 157 | $^{1}$H-NMR(DMSO-$d_6$, 120˚C) δ: 0.80(3H,t,*J*=7.3Hz), 1.25(3H,d, *J*=6.3Hz), 1.34(2H,sextet,*J*=7.3Hz), 1.64(2H,quintet,*J*=7.3Hz), 1.6-1.75(1H,m), 1.85-2.05(1H,m), 2.08(3H,s), 2.19(2H,t,*J*=7.0Hz), 2.27(2H,t,*J*=7.3Hz), 3.3-3.5(1H,m), 3.7-3.85(1H,m), 3.95-4.30(1H, m), 4.90(1H,s), 6.40-6.50(1H,m), 6.63(1H,d,*J*=7.8Hz), 6.85-7.00 (5H,m), 7.05(IH,t,*J*=74.2Hz), 7.06-7.15(3H,m), 7.43(2H,d,J =8.8Hz)ppm FABMS:538 (M+2) IR(KBr): 3364, 2966, 1628, 1599, 1402, 1323 12245, 1120, 1044cm$^{-1}$ | 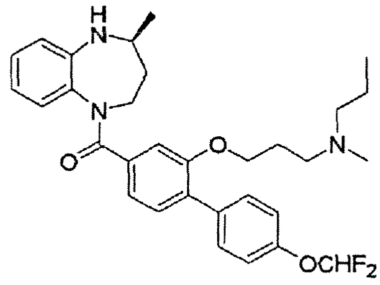 |
| 158 | $^{1}$H-NMR(DMSO-$d_6$,120˚C) δ: 1. 15-1.26(7H,m), 1.65-1.69(1H,m), 188-1.97(9H,m), 2.06(2H,t,*J*=7.3Hz), 3.41-4.15(3H,m), 4.91(1H, brs), 6.41-6.47(2H,m), 6.61(1H,d,*J*=7.3Hz), 6.89-6.93(3H,m), 7.18-7.41(6H,m), 10.8(1H,brs)ppm FABMS:509(M+1) IR(KBr): 3346, 2942, 1684, 1630, 1388, 1321, 1257cm$^{-1}$ | 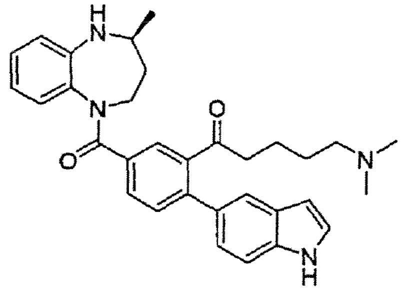 |

Table 2-69

| | | |
|---|---|---|
| 159 | $^{1}$H-NMR($CDCl_3$) δ : 0.84(3H,d,*J*=6.3Hz), 1.14(3H,d,*J*=6, 3Hz), 1.20-1.80(5H,m), 2,00-2.80(11H,m), 2.90-3.15(2H,m), 3.50-3.96(1H,m), 4.60-5.70(1H,m), 6.82-7.02(4H,m), 6.40-6.70(4H,m), 6.80-7.15(4H,m), 7.20-7.35(2H,m), 7,50-7.70(1H,m)ppm FABMS:570 (M+1) IR(KBr): 3344, 2964, 1615, 1557, 1390, 1323, 1162cm$^{-1}$ | 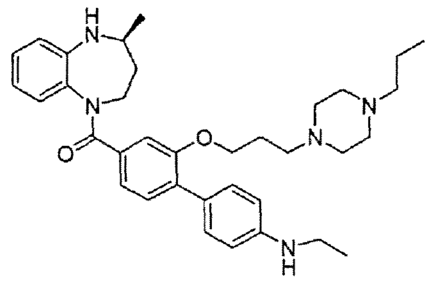 |
| 160 | $^{1}$H-NMR($CDCl_3$) δ:0.84(3H,d,*J*=6.3Hz), 1.14(3H,d,*J*=6.3Hz), 1.20-1.80(5H,m), 2.00-2.80(9H,m), 2.90-3.10(2H,m), 3.50-3.95(1H,m), 4.60-5.70(1H,m), 6,82-7.02(4H,m), 6.40-6.70(4H,m), 6.80-7.15(4H,m), 7.20-7.35(2H,m), 7.50-7.70(1H,m)ppm, FABMS:566 (M+1) IR(KBr): 3336, 2936, 1615, 1557, 1404, 1323, 1189, 1164 cm$^{-1}$ | 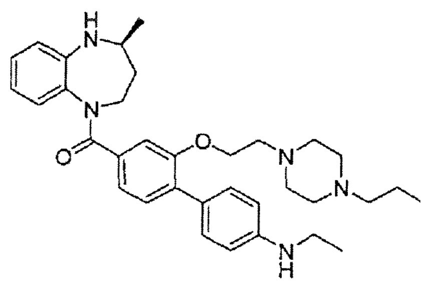 |

(continued)

| | | |
|---|---|---|
| 161 | $^1$H-NMR(DMSO-d6,120˚C) δ: 0.80(3H,t,*J*=6.8Hz), 1.20(3H,t, *J*=6,8Hz), 1.25(3H,t,*J*=6.8Hz), 1.31-1.42(2H,m), 1.63-1.73 (3H,m), 1.91(1H,br), 2.21(2H, t, *J*=6.8Hz), 2.32(2H,t, *J*=6.8Hz), 2.58(6H,s), 3.11-3.18(2H,m), 3.43(2H,br), 3.70-3.76(2H,m), 4.10(1H,br), 4.75(1H,br), 4.87(1H,br), 6.45-6.62(3H,m), 6.84-7.15(1H,m)ppm FABMS:558(M+1) IR (KBr): 3336, 2936, 1630, 1557, 1390, 1321, 1168cm$^{-1}$ | 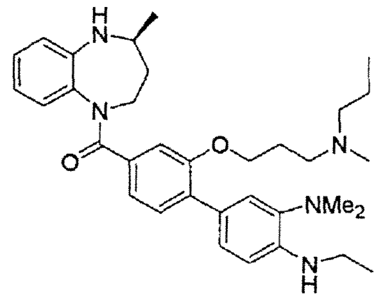 |

Table 2-70

| | | |
|---|---|---|
| 162 | 1H-NMR(DMSO-d6,120˚C) δ: 1.24(3H,d, *J*=6.3Hz), 1.55-1.70(1H, m), 1.85-2.00(1H,m), 2.0-2.15(3H,m), 3.08(6H,s), 3.35-3.55(5H, m), 3.3-3.7(1H,m), 3.9-4.2(1H,m), 4.7-4.9(1H,m), 6.33(1H,brs), 6.45-6.55(1H,m), 6.63(1H,d,*J*=7.8Hz), 6.80-6.95(4H,m), 7.15-7.25(3H,m), 7.33(1H,s), 7.61(1H,s)ppm FABMS:525(M+1) IR(KBr): 3428, 1622, 1394, 1321, 1255cm$^{-1}$ | 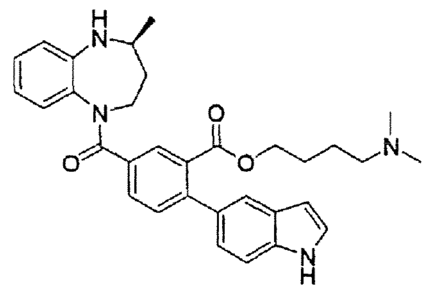 |
| 163 | $^1$H-NMR(DMSO-$d_6$,120˚C) δ: 0.81(3H,t,*J*=7.3Hz), 1.18(3H,t, *J*=7.3Hz), 1.25(2H,d,*J*=6.9Hz), 1.31-1.39(2H,m), 1.65-1.70(3H, m), 1.89-1.96(1H,m), 2.10(3H,s), 2.21(2H,t,*J*=7.8Hz), 2.32(2H,t, *J*=7.3Hz), 3.11-3.16(2H,m),3.42-4.10(8H,m), 4,48-4.52(1H,m), 4.87(1H,brs), 6.45-6.48(1H,m), 6.52(1H,d,*J*=7.8Hz), 6.61(1H,d, *J*=7,3Hz), 6,84-6.96(6H,m), 7.10(1H,d,*J*=7.8Hz)ppm FABMS:545 (M+1) IR(KBr): 3426, 2964, 1630, 1557, 1408, 1323, 1210, 1160cm$^{-1}$ | 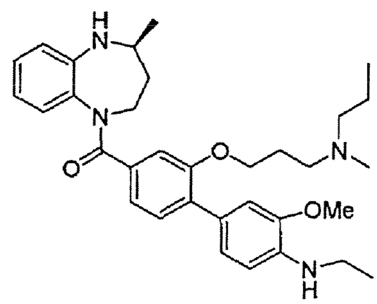 |

Table 2-71

| | | |
|---|---|---|
| 164 | $^1$H-NMR(DMSO-$d_6$,120˚C) δ: 0.82(3H,t,*J*=8,4Hz), 1.25(3H,d, *J*=6.8Hz), 1.30-1.4(2H,m), 1.58-1.71(3H,m), 1.90-1,95(1H,m), 2.11 (3H,s), 2.22(2H,t,*J*=6.88Hz), 2.32(2H,t,*J*=6.8Hz), 3.42(2H,br), 3.65-3.78(2H,m), 4.12(1H,br), 4.71(2H,brs), 4.87(1H,brs), 6.43-6.51 (1H,m), 6.55-6.61(3H,m), 6.82(1H,s), 6.85-6.94(3H,m), 7.03(1H,d, *J*=7.8Hz), 7.11-7.19(2H,m)ppm FABMS:487(M+1) IR(KBr): 3346, 2962, 1628, 1557, 1388, 1323, 1183, 1145cm$^{-1}$ | 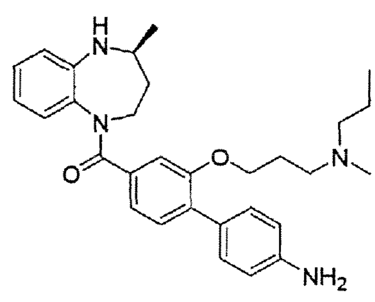 |

(continued)

| | | |
|---|---|---|
| 165 | $^1$H-NMR(DMSO-$d_6$,120°C) δ : 0.84(3H,t,*J*=6.8Hz),1.16(3H,t, *J*=6.8Hz), 1.25(3H,d,*J*=6.8Hz), 1.30-1.44(2H,m), 1.58-1.71(3H,m), 1.90-1.96(1H,m), 2.44(2H,t,*J*=6,8Hz), 2.54(2H,t,*J*=6.8Hz), 3.03-3.13(2H,m), 3.42(2H,br), 3,72-3,80(2H,m), 4.10(1H,br), 4.88 (1H,brs), 6.11(1H,br), 6.44-6.61(4H,m), 6.79-6.94(4H,m), 7.03(1H, d,*J*=7,8Hz), 7.17-7.20(2H,m)ppm FABMS:502(M+2) IR(KBr): 3342, 2966, 1615, 1557, 1392, 1323, 1255 1212, 1151cm$^{-1}$ | 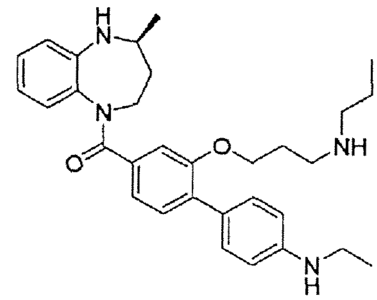 |

Table 2-72

| | | |
|---|---|---|
| 166 | $^1$H-NMR(DMSO-$d_6$,120°C) δ: 0.81(3H,t,*J*=6.8Hz), 1.25(3H,d, *J*=6.8Hz), 1.28-1.41(2H,m), 1.60-1.72(3H,m), 1.91-1.96(1H,m), 2.02(3H,s), 2.10(3H,s), 2.20(2H,t,*J*=6.8Hz), 2.29(2H,t,*J*=6.8Hz), 3.42(2H,br), 3.68-3.81(2H,m), 4.06(1H,br), 4.88(1H,brs), 6.44-6.50(1H,m), 6.61(1H,d,*J*=7.8Hz),6.86-6.95(4H,m), 7.09(1H, d,*J*=7.8Hz), 7.33(2H,d,*J*=8.8Hz), 7.52(2H,d,*J*=8.8Hz), 9.49(1H, brs)ppm FABMS:530(M+2) IR(KBr): 3318, 2964, 1673, 1601, 1562, 1402, 1323, 1257, 1149cm$^{-1}$ | 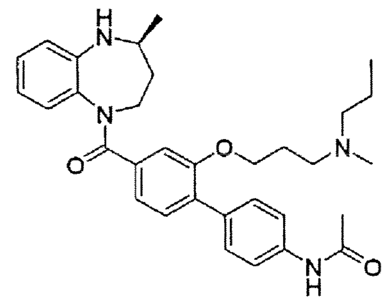 |
| 167 | $^1$H-NMR(DMSO-$d_6$,120°C) δ : 0.90-1.58(BH,m), 1.59-1.75(1H,m), 1.88-2.02(1H,m) 1.87-2.01(1H,m), 2.70-2.90(2H,m), 3.30-4.40 (5H,m), 4.90(1H,s), 6.39(1H,s), 6.45-6.54(1H,m), 6.64(1H,d, *J*=6.8Hz), 6.85-6.98(4H,m), 7.10-7.17(4H,m) 7.22-7.27(1H,m), 7.34(1H,d,*J*=7.8Hz), 7.55(1H,s), 10.66(1H,brs)ppm FABMS:469 (M+1) IR(KBr): 3320, 2972, 1624, 1560, 1419, 1323, 1255, 1125cm$^{-1}$ | 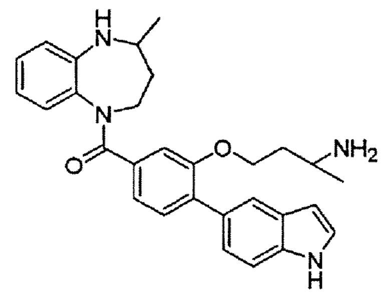 |
| 168 | $^1$H-NMR(DMSO-$d_6$,120°C) δ : 1.37(3H,d,*J*=6.8Hz), 1.71-1.85(1H, m), 2.02-2.05(1H,m), 3.16(2H,s), 3.38-3.42(2H,m), 3.54(2H,m), 3.86-3.92(2H,m), 4.25(1H,br), 5.01(1H,br), 6.52(1H,s), 6.57-6.64 (1H,m), 6.75(1H,d,*J*=7.8Hz), 7.02-7.05(4H,m), 7.23-7.29(2H,m), 7.34-7.37(1H,m), 7.47(1H,d,*J*=8.8Hz), 7.68(1H,s), 10.77(1H,br) ppm FABMS:497(M+1) IR(KBr): 3390,2934,1624,1560,1419,1321, 1129cm$^{-1}$ | 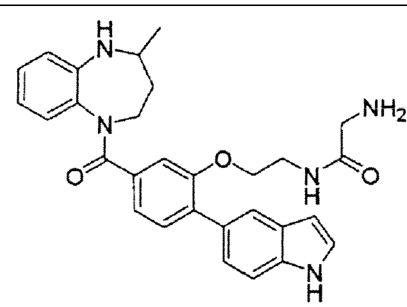 |

Table 2-73

| | | |
|---|---|---|
| 169 | $^{1}$H-NMR(DMSO-$d_6$,120˚C) δ :1,37(3H,d,*J*=6.3Hz), 1.59-1.92 (3H,m), 2.02-2.10(1H,m), 3.16(2H,s), 3.21-3.27(2H,m), 3.48-3.57(2H,m), 3,77-3.88(2H,m), 4.20(1H,br), 5.03(1H,s), 6.51-6.52(1H,m), 6.58-6.64(1H,m),6.74(1H,d,*J*=7.3Hz), 6.98-7.08(4H,m), 7.22-7.29(2H,m), 7.34-7.36(1H,m), 7.46 (1H,d,*J*=8.3Hz), 7.68(1H,d,*J*=1.5Hz), 10.76(1H,br)ppm FABMS:512(M+1) IR(KBr): 3320, 2934, 1624, 1562, 1421, 1321, 1125cm$^{-1}$ | 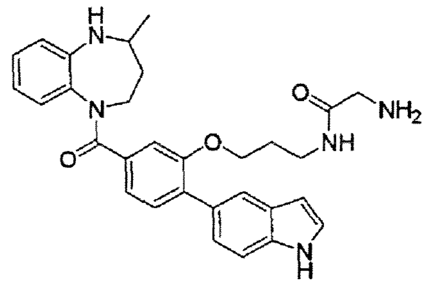 |
| 170 | $^{1}$H-NMR(DMSO-$d_6$, 120˚C) δ : 1.17(3H,t,*J*=6,6Hz), 1.26(3H,d, *J*=6.6Hz), 1.65-1.75(3H,m), 1.87-1.98(1H,m), 3.08-3.15(4H, m), 3,39-4.11(7H,m), 6.40(1H,s), 6.48-6.54(1H,m), 6.64(1H,d, *J*=7.3Hz), 6.89-6.95(4 H,m), 7.11-7.17(2H,m), 7.23-7.25(1H, m), 7.33-7.36(1H,m), 7.50(1H,br),7.57(1H,s), 10.7(1H,br)ppm FABMS:569(M+1) IR(KBr): 3320, 2940, 1738, 1622, 1560, 1419, 1321, 1253, 1031cm$^{-1}$ | 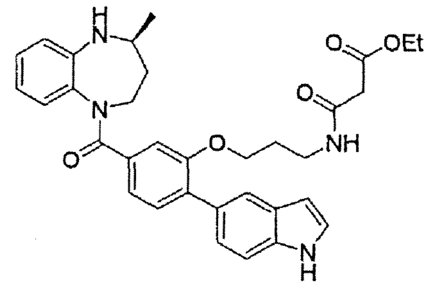 |
| 171 | $^{1}$H-NMR(DNSO-$d_6$,120˚C) δ : 1.17(3H,t,*J*=7.3Hz), 1.25(3H,d, *J*=5.9Hz), 1.60-1.80(5H,m), 1.91-1.96(1H,m), 2.03(2H,t, *J*=8.06Hz), 2.24(2H,t,*J*=7.3Hz), 3.05-3.12(2H,m), 3.42-4.22 (7H,m), 4.91(1H,s), 6.40(1H,s), 6.40-6.53(1H,m), 6.64 (1H, d, *J*=7.3 Hz), 6,88-6.94(4H,m), 7.11-7.25 (4H, m), 7.35 (1H, d, *J*=8.1 Hz), 7.56(1H,s), 10.7(1H,br)ppm FABMS:597(M+1) IR (KBr): 3314, 2934, 1931, 1626, 1560, 1419, 1321, 1253, 1154 cm$^{-1}$ | 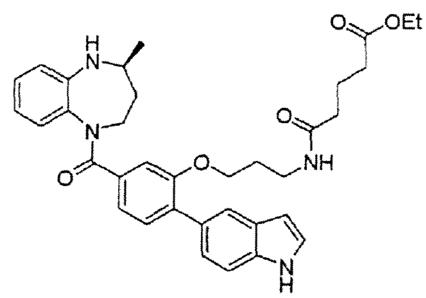 |

Table 2-74

| | | |
|---|---|---|
| 172 | $^{1}$H-NMR(DMSO-$d_6$, 120˚C) δ : 0.91(3H, t, *J*=6.6Hz), 1.26(3H, d, *J*=6.6Hz), 1.53(2H, q, *J*=6,6Hz), 1.50(2H, q, *J*=6.6Hz), 1.68 (1H, m), 1.92(1H, m), 2.05(3H, s), 2.25(4H, m), 3.44(1H, br), 3,71(2H, m), 4.90(1H, br), 6.48(1H, m), 6.68(1H, d, *J=7.8Hz),* 6.94(4H, m), 7.11(2H, m), 7.24(1H, m), 7.33(1H, d, *J*=8.0Hz), 7.56(1H, s), 10.64(1H, br)ppm FABMS : 512 (M+1) IR(KBr): 3330, 2938, 1626, 1560, 1491, 1448, 1419,1392,1321,1251,1123,1044,1006, 893, 806, 756 cm$^{-1}$ | 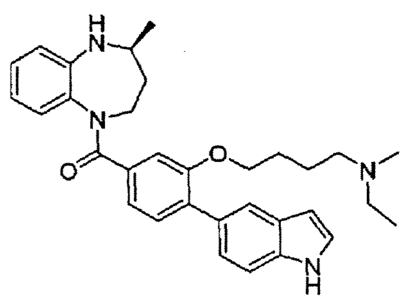 |
| 173 | $^{1}$H-NMR(DMSO-$d_6$,120˚C) δ: 0.84-0.95(3H,m),1.25-1.79 (12H,m),1.92-1.97(1 H,m),2.05(3H,s),2.21-2.26(4H,m), 3.44-4.16(5H ,m),4.88(1H,s),6.37(1H,s),6.49-6.69(2H,m), 6.8 5-6.93(4H,m),7.11-7.35(4H,m)7.56(1H,s), 10.7(1H,s) ppm FABMS : 639(M+1) IR(KBr):3326, 2934, 1626, 1560, 1491, 1448, 1419, 1321, 1253, 1123, 1042, 1006, 806, 756 cm$^{-1}$ | 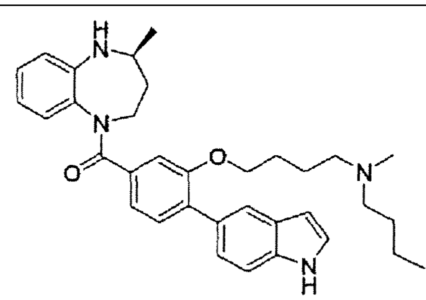 |

(continued)

| | | |
|---|---|---|
| 174 | $^1$H-NMR(DMSO-$d_6$,120˚C) δ : 0.84-0.95(3H,m),1.25-1.79 (12H,m),1.92.1.97(1 H,m),2.06(3H,s),2.21-2.26(4H,m), 3.44-4.16(5Hm),4.88(1H,s),6.37(1H,s),6.49.6.69(2H,m),6.8 5-6.93(4H,m),7.11-7.35(4H,m)7.56(1H,s),10.7( 1H,s)ppm FABMS: 539(M+1) IR(KBr)=3316, 2932, 1626, 1560, 1491, 1448, 1419, 1392, 1321, 1253, 1123, 1044, 1006, 806, 756 $cm^{-1}$ | 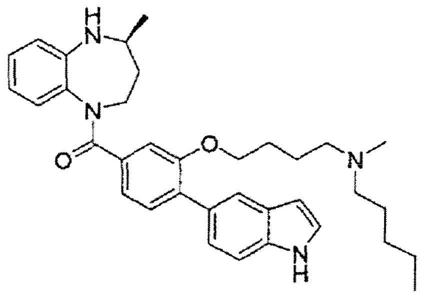 |

Table 2-75

| | | |
|---|---|---|
| 175 | $^1$H-NMR(DMSO-$d_6$,120˚C) δ : 1.35(3H,d,J=6.4Hz), 1.68-1.81(1H, m), 1.99-2.07(1H,m), 3.56(1H,br), 3.73(1H,br), 4.16(1H,br), 4.46(2H, br), 4.90(1H,br), 6.52-6.53(1H,m), 6.57(1H,dd,*J*=78,2.OHz), 6.60-6.65(1H,m), 6.78(1H,d,*J*=7.3Hz), 6.78(1H,d,*J*=5.9Hz), 6.91 (1H,s), 6.99-7.06(2H,m), 7.13(1H,ddJ=8.3,1.5Hz), 7.39(1H,t,2.9Hz), 7.53(1H,d,*J*=8.8Hz), 7.57(1H,s)ppm FABMS : 397(M+1) | 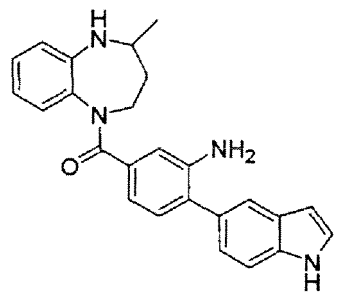 |
| 176 | $^1$H-NMR(DMSO-$d_6$,120˚C) δ: O,94-1.10(6H,m), 1.67-1.98(4H,m), 3.08-3.21(1H,m), 3.61-4.10(1H,m), 4.35(2H,brs), 4.64(1H,brs), 6.39-6.53(2H,m), 6.62-6.69(1H,m), 6.75-6.94(5H,m), 6.98-7.06(1H, m), 7.26-7.30(1H,m), 7.39-7.47(2H,m), 10.63-10.80(1H,br)ppm FABMS:425(M+1) | 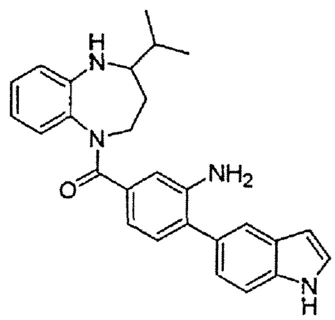 |
| 177 | $^1$H-NMR($CDCl_3$) δ: 0.98-1.17(3H,m), 1.50-2.00(3H,m), 2.17-2.93 (2H,m), 3.30-3.89(2H,m), 4.77-5.19(1H,m), 6.52-6.59(2H,m), 6.67-6.76(2H,m), 6.87-7.11(3H,m), 7.21-7.59(4H,m), 7.62-7.84(1H, m), 8.20-8.40(1H,m)ppm FABMS: 441(M+1) | 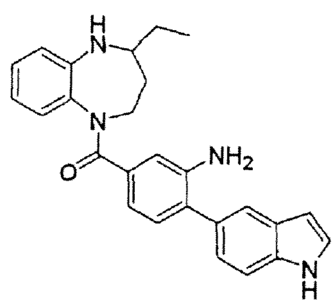 |

Table 2-76

| | | |
|---|---|---|
| 178 | $^1$H-NMR(DMSO-$d_6$,120˚C) δ : 1.35(3H,d,J=6.4Hz), 1.68-1.81(1H,m), 1.99-2.07(1H,m), 3.56(1H,br), 3.73(1H,br), 4.15(1H,br), 4.46(2H,br), 4.90(1H,br), 6.52-6.53(1H,m), 6.57(1H,dd,J=7.8,2.0Hz), 6.60-6.65 (1H,m), 6.78(1H,d,J=7.3Hz), 6.78(1H,d,J=5.9Hz), 6.91(1H,s), 6.99-7.06(2H,m), 7.13(1.H,dd,J=8.3,1.5Hz), 7.39(1H,t,2.9Hz), 7.53 (1H,d,J=8.8Hz), 7.57(1H,s)ppm FABMS: 397(M+1) | 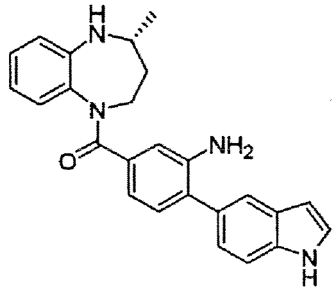 |
| 179 | $^1$H-NMR(DMSO-$d_6$,120˚C) δ: 1.24(3H,d,=6.8Hz), 1.55-1.73(1H,m), 1.85-2.04(1H,m), 3.30-3.79(1H,m), 3.93-4.18(1H,m), 4.35(2H,brs), 4.79(1H,brs), 6.39-6.56(3H,m), 6.66(1H,d,J=7.8Hz), 6.75-6.84(2H, m), 6.86-6.96(2H,m), 6.98-7.06(1H,m), 7.26-7.31(1H,m), 7.41(1H,d, J=8.8Hz), 7.45(1H,s), 10.71(1H,brs)ppm FABMS: 397(M+1) | 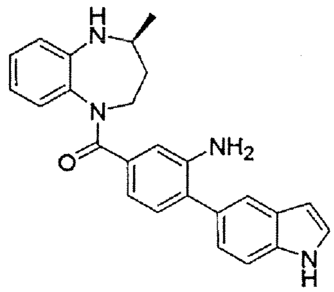 |
| 180 | $^1$H-NMR(DMSO·$d_6$,120˚C) δ: 1.25(3H,d,J=6.8Hz), 1.57-1.70(1H,m), 1.92·1.96(1H,m), 3.45-3.63(2H,m), 4.01-4.09(1H,m), 4.82(1H,s), 6.39 (1H,d,J=2.9Hz), 6.45-6.51(1H), 6.61-6.66(2H,m), 6.90-6.94(3H,m), 7.00(1H,d,J=7.8Hz), 7.18(1H,d,J=7.8Hz), 7.24(1H,d,J=2.9Hz), 7.34 (1H,d,J=7.8Hz), 7.60(1H,s), 8.72(1H,s), 10.64(1H,br)ppm FABMS: 398(M+1) | 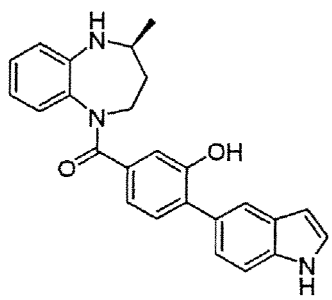 |

Table 2-77

| | | |
|---|---|---|
| 181 | $^1$H-NMR(DMSO-$d_6$,120˚C) δ: 1.2.5(3H,d,J=6.8Hz), 1.57-1.70(1H,m), 1.92-1.96(1H,m), 3.45-3.63(2H,m), 4.01-4.09(1H,m), 4.82(1H,s), 6.39(1H,d,J=2.9Hz), 6.45-6.51(1H), 6,61-6.66(2H,m), 6,90-6,94(3H, m), 7.00(1H,d,J=7.8Hz), 7.18(1H,d,J=7.8Hz), 7.24(1H,d,J=2.9Hz), 7.34(1H,d,J=7.8Hz), 7,60(1H,s), 8.72(1H,s), 10.64(1H,br) ppm FABMS : 398(M+1) | 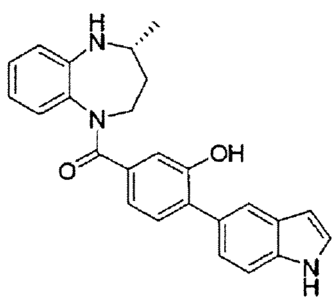 |

(continued)

| | | |
|---|---|---|
| 182 | $^1$H-NMR(DMSO-$d_6$,120°C) δ: 1.25(3H,d,J=6.8Hz), 1.57-1.70(1H,m), 1.92-1.96(1H,m), 3.45-3.63(2H,m), 4.01-4.09(1H,m), 4.82(1H,s), 6.39(1H,d,J=2.9Hz), 6.45-6.51(1H), 6.61-6.66(2H,m), 6.90-6.94(3H, m), 7.00(1H,d,J=7.8Hz), 7.18(1H,d,J=7.8Hz), 7.24(1H,d,J=2.9Hz), 7.34(1H,d,J=7.8Hz), 7.60(1H,s), 8.72(1H,s), 10.64(1H,br)ppm FABMS : 398(M+1) | 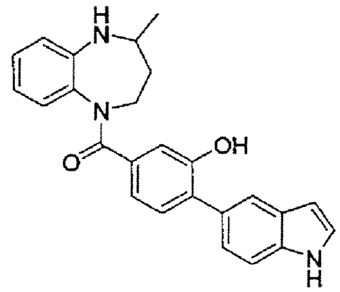 |
| 183 | $^1$H-NMR(DMSO-$d_6$, 120°C) δ: 1.11(3H,t,J=7.3Hz), 1.60-1.84(3H,m), 2.07-2.09(1H,m), 3.35(1H,br), 3.83(1H,br), 4.12(1H,br), 4.88(1H,s), 6.50(1H,s), 6.55-6.60(1H,m), 6.61-6.78(2H,m), 7.01.7.13(4H,m), 7.29(1H,dd,J=7.8,2.0Hz), 7.35(1H,t,J=2.9Hz), 7,46(1H,d,J=7.8Hz), 7.71(1H,s), 8.81(1H,s), 10.74(1H,br)ppm FABMS : 412(M+1) | 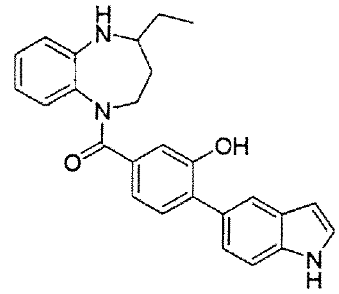 |

(1) Receptor binding study using cell membrane samples of a cell expressing human vasopressin V1a receptor and V2 receptor

(i) Production of membrane sample

Preparation of Chinese hamster ovary (CHO) cell which expresses human vasopressin receptor subtype and production of membrane sample of the cell was conducted according to the method of Tahara et al. (Br. J. Pharmacol., 125, 1463-1470, 1998).

(ii) Receptor binding study

In saturation binding experiment, the membrane sample and [$^3$H]AVP of several concentrations (0.125 nM to 4.0 nM) were mixed and incubated in microplate wells. In displacement experiment, [$^3$H]AVP of constant concentrations (0.5 nM to 1.0 nM) and the membrane sample were mixed with Compound A of several concentrations, and incubation was performed. Assay was performed using 50 mM of Tris-HCl (pH = 7.4) (total amount: 250 μL) (buffer composition: containing 10 mM of $MgCl_2$ and 0.1% BSA), and incubation was performed at room temperature (25°C) for one hour. After incubation, suck filtration was immediately performed and reaction was completed when the membrane sample was collected on a glass fiber filter fitted with the microplate. After the filter was washed with an ice-cold Tris-HCl buffer (50 mM Tris-HCl, 10 mM $MgCl_2$, pH 7.4), radioactivity measurement was performed. Nonspecific binding was determined using nonradioactive- labeled AVP (1 μM). Concentration of Compound A at which specific binding of [$^3$H]AVP in each membrane sample ($IC_{50}$ value) was inhibited by 50% was determined using a regression analysis of a displacement curve for Compound A. Inhibition constant (Ki value) of Compound A was determined using Ki = $IC_{50}$/ (1 + [L]/Kd) ([L] is [$^3$H]AVP concentration in well, and Kd (dissociation constant) is a value determined using a scatchard plot analysis about results of saturation binding experiment).

Table 3-1

| Test compound No. of Example | Human V1a Ki (nM) | Human V2 Ki (nM) |
|---|---|---|
| 1 | 0.39 | 1.7 |
| 2 | 0.31 | 4.4 |
| 3 | 0.18 | 22 |
| 7 | 0.38 | 15 |
| 8 | 0.16 | 15 |
| 16 | 0.33 | 63 |

(continued)

| Test compound No. of Example | Human V1a Ki (nM) | Human V2 Ki (nM) |
|---|---|---|
| 17 | 0.13 | 11 |
| 30 | 0.091 | 17 |
| 36 | 0.23 | 11 |
| 37 | 0.24 | 4.3 |
| 38 | 0.086 | 11 |
| 39 | 0.22 | 5.2 |
| 42 | 0.19 | 34 |
| 46 | 0.24 | 1.1 |
| 47 | 0.39 | 1.2 |
| 48 | 0.23 | 1.5 |
| 51 | 0.079 | 12 |
| 54 | 0.41 | 731 |
| 55 | 0,31 | 25 |
| 56 | 0.36 | 2.4 |
| 59 | 0.23 | 0.89 |
| 60 | 0.27 | 84 |
| 61 | 0.22 | 1.9 |
| 66 | 0.41 | 3.6 |
| 67 | 0.19 | 8.7 |
| 69 | 0.24 | 17 |
| 70 | 0.19 | 6.9 |
| 71 | 0.16 | 11 |
| 73 | 0.19 | 4.6 |
| 74 | 0.24 | 6.1 |
| 78 | 0.11 | 12 |
| 79 | 0.13 | 16 |
| 80 | 0.064 | 5.1 |
| 81 | 0.17 | 8.0 |
| 82 | 0.16 | 16 |
| 83 | 0.13 | 17 |
| 84 | 0.10 | 6.5 |
| 85 | 0.052 | 7.7 |
| 87 | 0.086 | 8.7 |
| 88 | 0.30 | 3.0 |
| 89 | 0.29 | 19 |

Table 3-2

| | | |
|---|---|---|
| 91 | 0.15 | 0.78 |

(continued)

| | | |
|---|---|---|
| 92 | 0.21 | 5.6 |
| 94 | 0.21 | 0.73 |
| 97 | 0.13 | 0.39 |
| 99 | 0.30 | 3.4 |
| 100 | 0.46 | 3.2 |
| 102 | 0.25 | 61 |
| 103 | 0.24 | 3.6 |
| 104 | 0.35 | 3.5 |
| 105 | 0.16 | 54 |
| 106 | 0.14 | 2.0 |
| 107 | 0.13 | 33 |
| 108 | 0.42 | 21 |
| 109 | 0.13 | 7.1 |
| 110 | 0.13 | 6.5 |
| 112 | 0.42 | 23 |
| 113 | 0.26 | 5.0 |
| 114 | 0.22 | 18 |
| 115 | 0.27 | 4.9 |
| 116 | 0.16 | 11 |
| 117 | 0.21 | 12 |
| 118 | 0.15 | 9.5 |
| 120 | 0.20 | 93 |
| 121 | 0.14 | 10 |
| 122 | 0.21 | 5.1 |
| 123 | 0.38 | 9.9 |
| 124 | 0.37 | 20 |
| 125 | 0.18 | 15 |
| 126 | 0.24 | 7.0 |
| 127 | 0.21 | 77 |
| 128 | 0.20 | 39 |
| 129 | 0.40 | 88 |
| 130 | 0.36 | 309 |
| 131 | 0.26 | 83 |
| 132 | 0.25 | 172 |
| 134 | 0.49 | 2.7 |
| 136 | 0.23 | 16 |
| 138 | 0.23 | 34 |
| 141 | 0.45 | 10 |
| 144 | 0.11 | 28 |

(continued)

| | | |
|---|---|---|
| 145 | 0.12 | 20 |
| 148 | 0.23 | 9.6 |
| 149 | 0.29 | 1.9 |

Table 3-3

| | | |
|---|---|---|
| 151 | 0.25 | 10 |
| 154 | 0.40 | 53 |
| 156 | 0.19 | 53 |
| 157 | 0.28 | 28 |
| 158 | 0.11 | 13 |
| 159 | 0.18 | 11 |
| 160 | 0.20 | 22 |
| 162 | 0.13 | 29 |
| 164 | 0.15 | 477 |
| 165 | 0.28 | 92 |
| 169 | 0.13 | 4.9 |
| 170 | 0.37 | 2.6 |
| 172 | 0.093 | 7.1 |
| 173 | 0.21 | 10 |
| 174 | 0.35 | 11 |
| SR-49059 | 0.51 | 143 |
| YM-087 | 4.5 | 1.5 |

(2) V1a receptor antagonism in awake rat (oral administration)

V1a receptor antagonism was examined using male Sprague-Dawley rats (body weight; 319 to 418 g) . One day before evaluation of test compounds, a cannula for blood pressure measurement was inserted into left carotid artery, and a cannula for arginine vasopressin (AVP) administration was inserted into left cervical vein. Blood pressure of artery cannula was measured via pressure transducer during rats were awake. During evaluation of test compounds, elevation breadth of mean blood pressure resulting from intravenous administration of 30 mU/kg of AVP was determined as a vasopressor control, in advance. After test compound was suspended in 0.5% methylcellulose solution and orally administered at 1 to 3 mg/kg, 30 mU/kg of AVP was intravenously administered at fixed time points from 0.5 to 8 hours, and vasopressure value was measured. The result is shown in Figure 1.

$$[\text{Vasopressure inhibition rate (\%) at each measurement point after administration}] = [(A - B)/A] \times 100$$

Wherein, control vasopressure = A, and vasopressure value at each measurement point = B

Example 110 maleate, 117 fumarate, and 122 maleate showed maximum inhibition rate of 65% or more after 2 to 6 hours since administration (68.5, 77.7, and 67.3%, respectively), and showed significant AVP vasopressure inhibition effect after 8 hours since administration, and 117 fumarate and 122 maleate maintained inhibition rate of 50% or more after 1 to 8 hours since administration and thus has a persistent effect. On the other hand, oral administration of 3 mg/kg of SR49059, which is 3 times more than the dose of the compound of this invention, showed maximum inhibition rate of 61.1% after 0.5 hours since administration, and showed significant AVP vasopressure inhibition effect until 4 hours after administration, and after that it was attenuated and the inhibition rate was 50% or less after 2 hours or longer since

administration.
From these results, it was demonstrated that V1a receptor antagonism of oral administration of the compound of this invention to awake rats was potent and persistent compared to that of SR49059.
(3) Acute toxicity study
Test compound suspended in 0.5% methylcellulose solution was orally administered to male ICR mice (body weight 25 to 35 g), which were separated into groups each having 5 mice, and body weight change and general symptom after 6 days since administration were observed. During the test period except for 17 to 20 hours before administration and after 2 hours since administration, mice were allowed to take feed and water ad libitum. Results of this test are showed in the Table. Test substance was showed to be highly safe because the toxicity expressing doses for all the substances were 300 mg/kg or more.

Table 4

| **Test compound** | **Toxicity expressing dose (mg/kg)** |
|---|---|
| **Example 110-Maleate** | **>300** |
| **Example 117-Fumarate** | **>300** |
| **Example 117 - Maleate** | **>300** |
| **Example 122-Maleate** | **>300** |

INDUSTRIAL APPLICABILITY

The compound and its salts in the present invention have a superior affinity to arginine vasopressin V1a receptor and V2 receptor, especially to an arginine vasopressin V1a receptor. That is, the compound and its salts in the present invention contain both antagonist for arginine vasopressin V1a receptor and V2 receptor, and arginine vasopressin V1a receptor-selective antagonist. The compound in the present invention for example has vasodilating action, hypotensive action, hepatic glucose release inhibiting action, mesangial cells proliferation inhibiting action, water diuretic action, platelet aggregation inhibiting action, vomit inhibiting action, urea excretion promoting reaction, the VIII factor secretion inhibiting reaction, cardiac function promoting reaction, mesangial cells contraction inhibiting reaction, hepatic gluconeogenesis inhibiting action, aldosterone secretion inhibiting action, endothelin production inhibiting action, renin secretion adjusting action, memory adjusting action, body temperature adjusting action, prostaglandin production adjusting action, etc., and therefore are useful as a vasodilating agent, a hypotensor, a water diuretic agent, a platelet aggregation inhibitor, a urea excretion promoter, an anti-cardiac failure agent, an anti-renal failure agent, etc., and is useful in prevention and treatment of hypertension, edema, brain edema, ascites, cardiac insufficiency, renal dysfunction, abnormal vasopressin secretion syndorome (SIADH), hepatocirrhosis, hyponatremia, hypokalemia, diabetes, circulatory failure, oscillation disease, water metabolism disorder, renal disease (nephritic syndrome, nephritis, diabetic nephropathy, chronic or acute renal failure), gastric ulcer, nausea, vomiting, various isochemic diseases such as faint, glomerulosclerosis, etc.

BRIEF DESCRIPTION OF THE DRAWINGS

Fig. 1 is a graph showing a V1a receptor antagonism in an awake rat.

**Claims**

**1.** A 2,3,4,5-tetrahydro-1H-1,5-benzodiazepine derivative represented with the following Formula (1)

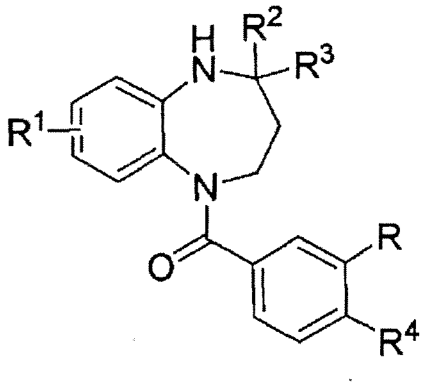 (1)

[In the Formula, $R^1$ represents hydrogen, lower alkyl, amino, or hydroxy. $R^2$ and $R^3$ are the same or different from each other representing hydrogen, lower alkyl, di-lower alkylamino lower alkyl, or hydroxy lower alkyl. However, the case that both of $R^2$ and $R^3$ are hydrogen is excluded. When $R^2$ and $R^3$are different, they represent an optically-active substance or a racemic body. $R^4$ is a group represented with

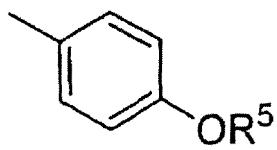

($R^5$ represents hydrogen, lower alkyl, or halogen-substituted lower alkyl.), a group represented with

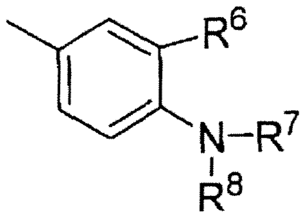

($R^6$ represents hydrogen, lower alkyl, lower alkoxy, halogen, or di-lower alkylamino. $R^7$ and $R^8$ are the same or different from each other representing hydrogen, lower alkyl, or lower alkanoyl.), or 1H-indol-5-yl. R represents hydrogen, hydroxy, morpholino, lower alkylpiperazinyl, lower alkoxy, amino lower alkyl, hydroxy lower alkyl, morpholino lower alkyl, di-lower alkylamino lower alkylcarbonyl, morpholinocarbonyl, lower alkylpiperazinylcarbonyl, piperazinyl lower alkoxy, a group represented with

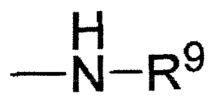

($R^9$ represents hydrogen, lower alkyl, amino lower alkyl, lower alkylpiperazinylcarbonyl lower alkyl, or lower alkylsulfonyl.), a group represented with

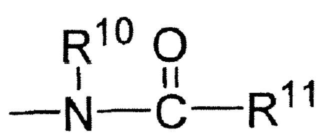

($R^{10}$ represents hydrogen, or lower alkyl. $R^{11}$ represents hydrogen, lower alkyl, lower alkylpiperazinyl lower alkyl, lower alkoxy, amino lower alkyl, amino, di-lower alkylamino, hydroxy lower alkyl, lower alkoxy lower alkyl, or morpholino.), a group represented with

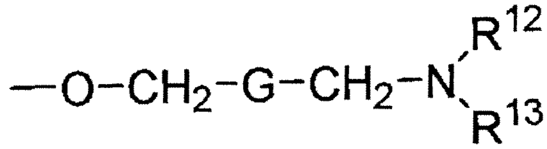

($R^{12}$ and $R^{13}$ are the same or different from each other representing hydrogen or lower alkyl. G represents -CH=CH- or -C≡C-.), a group represented with

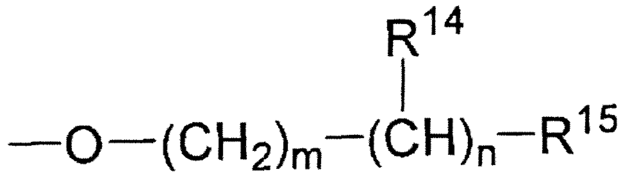

{$R^{14}$ represents lower alkyl . $R^{15}$ represents hydroxy, lower alkoxy, imidazolyl, 2,5-dioxoimidazolidin-l-yl , 3-methyl-2,4-dzoxoimidazolidin-1-yl, 2,4-dioxothiazolidin-3-yl, 4-oxopiperizin-1-yl,2-oxopyrrolidin-1-yl, 2-oxooxazolidzn-3-yl, 3-oxopiperazin-1-yl, or 1,4-dioxa-8-azaspiro[4,5]dec-8-yl, or a group represented with

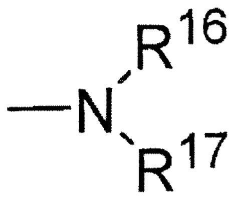

($R^{16}$ and $R^{17}$ are the same or different from each other representing hydrogen, lower alkyl, hydroxy lower alkyl, lower alkoxy lower alkyl, lower alkanoyl, amino lower alkanoyl, lower alkoxy lower alkylcarbonyl, or di-lower alkylamino lower alkyl. Further, $R^{16}$ and $R^{17}$ may form a 6- to 7-membered saturated heterocycle by bonding each other through a nitrogen atom or an oxygen atom and accompanying a nitrogen atom to which $R^{16}$ and $R^{17}$ are bonding. It may have lower alkyl or lower alkanoyl on the heterocycle as a substituent.), or a group represented with

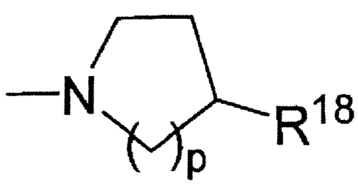

($R^{18}$ represents hydrogen, di-lower alkylamino, amino, aminocarbonyl, di-lower alkylaminocarbonyl, formamide, hydroxy, or morpholino. p represents 1 or 2.). m represents an integer of 2 to 6, and n represents an integer of 0 to 3. }, or a group represented with

$$-CO_2-(CH_2)_m-R^{19}$$

($R^{19}$ represents di-lower alkylamino, morpholino, lower alkylpiperazinyl, lower alkylpiperizino, pyridyl lower alkyl, or lower alkylpyrrolidinyl. m is the same as described above.)], and the pharmaceutically acceptable salt.

**2.** The compound or the pharmaceutically acceptable salt according to Claim 1 wherein $R^4$ is

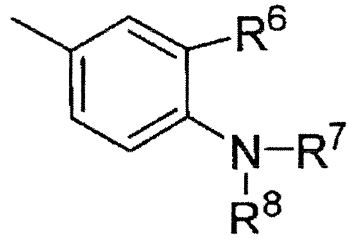

($R^6$, $R^7$, and $R^8$ are the same as described above.).

**3.** The compound or the pharmaceutically acceptable salt according to Claim 1 wherein $R^4$ is 1H-indol-5-yl.

**4.** The compound or the pharmaceutically acceptable salt according to Claim 2 wherein R is

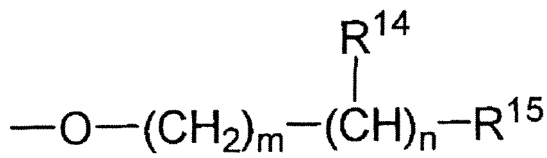

($R^{14}$ , $R^{15}$, m, and n are the same as described above.).

**5.** The compound or the pharmaceutically acceptable salt according to Claim 3 wherein R is

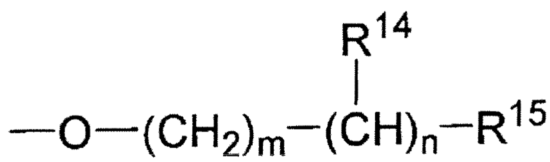

($R^{14}$, $R^{15}$, m, and n are the same as described above.).

**6.** The compound or the pharmaceutically acceptable salt according to Claim 1 which is selected from the group consisting of N-[3-[2-(1H-indol-5-yl)-5-[[(4S)-4-methyl-2,3,4,5-tetrahydro-1H-1,5-benzodiazepin-2-yl]carbonyl] phenoxy]propyl]-N-methyl-N-propylamine;
(4S)-1-[4-(1H-indol-5-yl)-3-[3-(4-isopropyl-1-piperazinyl)p ropoxy]benzoyl]-4-methyl-2,3,4,5-tetrahydro-1H-1,5-benzodiazepine;
(4S)-1-[4-(1H-indol-5-yl)-3-[3-(4-propyl-1-piperazinyl) propoxy]benzoyl]-4-methyl-2,3,4,5-tetrahydro-1H-1,5-benzodiazepine;
N-methyl-2'-[3-[methyl (propyl) amíno]propoxy]-4'-[[(4S)-4-methyl-2,3,4,5-tetrahydro-1H-1,5-benzodiazepin-1-yl] carbonyl][1,1'-biphenyl]-4-amine;
(4S)-1-[4-(1H-indol-5-yl)-3-[2-(4-methyl-1-piperazinyl) ethoxy]benzoyl]-4-methyl-2,3,4,5-tetrahydro-1H-2,5-benzodiazepine;

N-[4-[2-(1H- indol- 5- yl)- 5-[[(4S)- 4- methyl- 2,3,4,5- tetrahydro- 1H- 1,5- benzodiazepin- 1- yl] carbanyl] phenoxy] butyl]-N-methyl-N-propylamine;
(4S)-1-[3-[3-(4-butyl-1-piperazinyl)propoxy]-4-(1H-indol-5-yl)benzoyl]-4-methyl-2,3,4,5-tetrahydro-1H-1,5-benzodiazepine; and
N-ethyl-2'-[3-[methyl(propyl)amino]propoxy]-4'-[[(4S)-4-methyl-2,3,4,5-tetrahydro-1H-2,5-benzodiazepin-1-yl]carbonyl][1,1'-biphenyl]-4-amine.

**7.** The compound according to Claim 1 which is N-[4-[2-(1H- indol- 5- yl)- 5-[[(4S)- 4- methyl- 2,3,4,5- tetrahydro- 1H- 1,5- benzodiazepin- 1- yl] carbonyl] phernoxy] butyl]-N-methyl-N-propylamine·1/2 fumarate.

**8.** The compound according to Claim 1 which is (4S)-1-[4-(1H-indol-5-yl)-3-[2-(4-methyl-1-piperazinyl) ethoxy]benzoyl]-4-methyl-2,3,4,5-tetrahydro-1H-1,5-benzodiazepine·1/2 fumarate.

**9.** The compound according to Claim 1 which is (4S)-1-[3-[3-(4-butyl-1-piperazinyl)propoxy]-4-(1H-indol-5-yl)benzoyl]-4-methyl-2,3,4,5-tetrahydro-1H-1,5-benzodiazepine·1/2 fumarate.

**10.** A pharmaceutical composition containing the compound or the pharmaceutically acceptable salt according to any one of Claims 1 to 9.

**11.** A therapeutic and prophylactic agent for diabetes, diabetic nephropathy, and glomerulosclerosis having the compound or the pharmaceutically acceptable salt according to any one of Claims 1 to 9 as an effective component.

Fig. 1

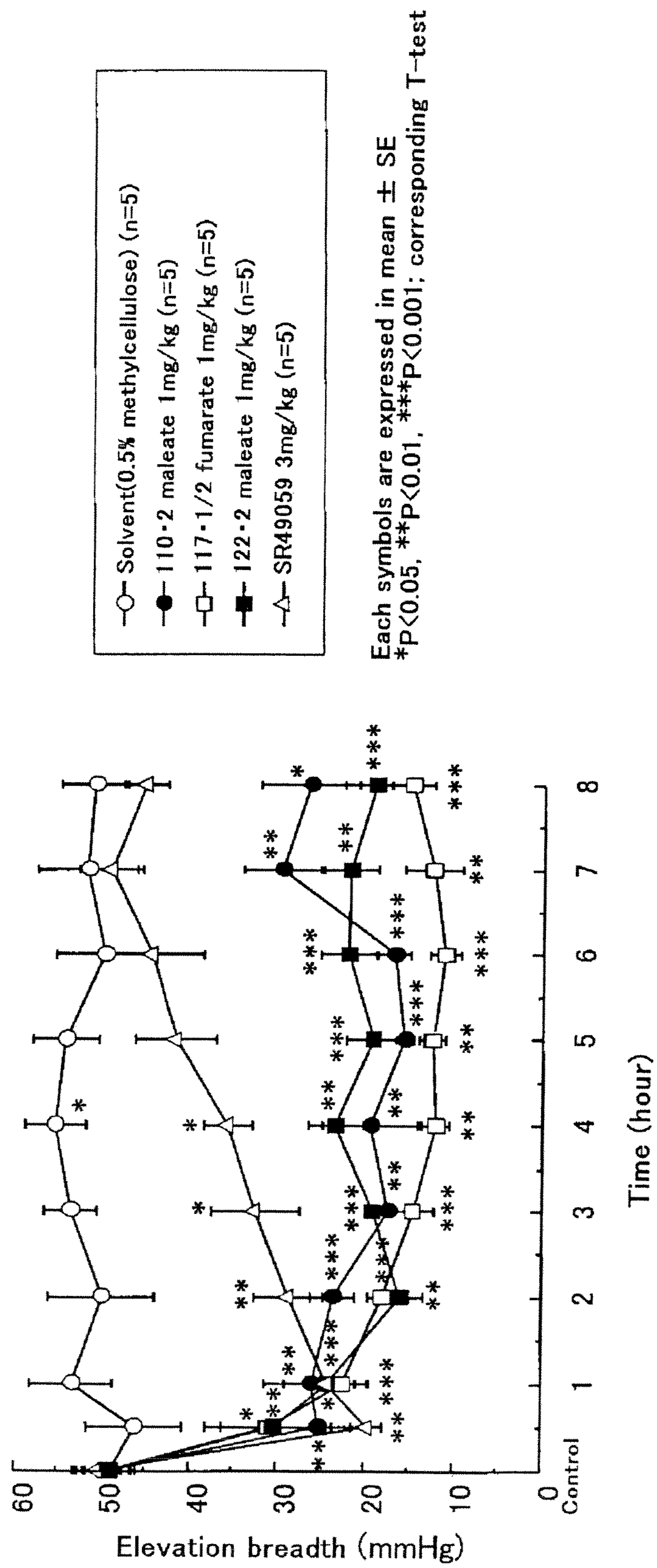
Solvent(0.5% methylcellulose) (n=5)
110·2 maleate 1mg/kg (n=5)
117·1/2 fumarate 1mg/kg (n=5)
122·2 maleate 1mg/kg (n=5)
SR49059 3mg/kg (n=5)
Each symbols are expressed in mean ± SE
*P<0.05, **P<0.01, ***P<0.001; corresponding T-test
Elevation breadth (mmHg)
60
50
40
30
20
10
0
Control
1
2
3
4
5
6
7
8
Time (hour)

**INTERNATIONAL SEARCH REPORT**

International application No.
PCT/JP2005/020599

A. CLASSIFICATION OF SUBJECT MATTER
***C07D243/12***(2006.01), ***A61K31/55***(2006.01), ***A61P3/10***(2006.01), ***A61P13/12***(2006.01), ***C07D401/14***(2006.01), ***C07D403/10***(2006.01), ***C07D403/14***(2006.01), ***C07D413/14***(2006.01), ***C07D491/113***(2006.01)

According to International Patent Classification (IPC) or to both national classification and IPC

B. FIELDS SEARCHED

Minimum documentation searched (classification system followed by classification symbols)
C07D243/12, A61K31/55, A61P3/10, A61P13/12, C07D401/14, C07D403/10, C07D403/14, C07D413/14, C07D491/113

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | JP 9-221476 A (OTSUKA PHARMACEUTICAL, CO., LTD.), 26 August, 1997 (26.08.97), Full text (Family: none) | 1-11 |
| A | WO 95/34540 A1 (OTSUKA PHARMACEUTICAL, CO., LTD.), 21 December, 1995 (21.12.95), Full text & JP 8-301848 A & JP 11-319570 A & JP 2000-351768 A & EP 765314 A1 & EP 1221440 A1 & US 6096735 A & US 6335327 B1 & US 2002/049194 A1 | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 January, 2006 (04.01.06) | 17 January, 2006 (17.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2005/020599

C (Continuation). DOCUMENTS CONSIDERED TO BE RELEVANT

| Category* | Citation of document, with indication, where appropriate, of the relevant passages | Relevant to claim No. |
|---|---|---|
| A | JP 6-16643 A (Yamanouchi Pharmaceutical Co., Ltd.), 25 January, 1994 (25.01.94), Full text (Family: none) | 1-11 |
| A | JP 7-258226 A (Yamanouchi Pharmaceutical Co., Ltd.), 09 October, 1995 (09.10.95), Full text (Family: none) | 1-11 |
| A | JP 8-81460 A (Yamanouchi Pharmaceutical Co., Ltd.), 26 March, 1996 (26.03.96), Full text (Family: none) | 1-11 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 218944
- WO 0202553 A
- WO 9503305 A
- JP 4234361 A
- JP 4154765 A
- JP 8301848 A
- JP 6016643 A


### Non-patent literature cited in the description


- *Kidney International,* 1993, vol. 44 (1), 19-23
- *Biological and Pharmaceutical Bulletin,* 2000, vol. 23 (11), 1323-1327
- *European Journal of Pharmacology,* 2002, vol. 450, 169-177
- *European Journal of Pharmacology,* 1999, vol. 376, 239-246
- *Drug Development Research,* 2003, vol. 60, 241-251
- *Proceeding of the National Academy of Sciences of the United State of America,* 1999, vol. 96, 10397-10402
- *Journal of Diabetes and Its Complications,* 1995, vol. 9, 326-329
- *Nephrology Dialysis Transplantation,* 2003, vol. 18, 497-506
- *Nephrology Dialysis Transplantation,* 2003, vol. 18, 1755-1763
- *Nephron,* 1996, vol. 73, 629-636
- **GREENE ; WUTS.** Protective Group in Organic Synthesis
- *Heterocycles,* 1991, vol. 32, 1131
- *Journal of Organic Chemistry,* 2000, vol. 65, 164
- Heterocycles. 1992, vol. 34, 1395
- Exact Organic Synthesis [Laboratory Manual. Seimitsu Yuki Gosei [Jikken Manual. 91
- **TAHARA et al.** *Br. J. Pharmacol,* 1998, vol. 125, 1463-1470